(19) Europäisches Patentamt

European Patent Office

Office européen des brevets

(11) **EP 2 441 838 A2**

(12) **EUROPEAN PATENT APPLICATION**

(43) Date of publication:
18.04.2012 Bulletin 2012/16

(51) Int Cl.:
*C12N 15/62* (2006.01)     *C07K 16/00* (2006.01)

(21) Application number: 11157601.3

(22) Date of filing: 24.01.2007

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK RS

(30) Priority: 24.01.2006  US 761708 P
05.12.2006  PCT/GB2006/004559

(62) Document number(s) of the earlier application(s) in
accordance with Art. 76 EPC:
07705002.9 / 1 976 991

(71) Applicant: Domantis Limited
Brentford, Middlesex TW8 9GS (GB)

(72) Inventor: Beckmann, Roland
Cambridge, CB4 0WG (GB)

(74) Representative: Easeman, Richard Lewis
GlaxoSmithKline
Corporate Intellectual Property (CN9.25.1)
980 Great West Road
Brentford, Middlesex TW8 9GS (GB)

Remarks:
This application was filed on 10-03-2011 as a
divisional application to the application mentioned
under INID code 62.

(54) **Fusion proteins that contain natural junctions**

(57)     A method for preparing recombinant fusion proteins that comprise at least one natural junction is described. Fusion proteins that contain at least one natural junction have reduced potential for immunogenicity, improved stability, reduced tendency to aggregate, improved expression and/or improved production yields relative to conventional fusion proteins. Novel fusion proteins that comprise at least one natural junction, compositions comprising the fusion proteins and methods of using the proteins are also disclosed.

FIG. 1A

EP 2 441 838 A2

**Description**

RELATED APPLICATIONS

This application is a continuation-in-part of International Application No. PCT/GB2006/004559, which designated the United States and was filed on December 5, 2006, and this application claims the benefit of U.S. Provisional Application No. 60/761,708, filed on January 24, 2006. The entire teachings of the above applications are incorporated herein by reference.

BACKGROUND OF THE INVENTION

Fusion proteins are a recognized class of potentially effective therapeutic and diagnostic agents. One benefit provided by fusion protein technology is the possibility of designing a fusion protein that has desired function, enhanced desirable properties and/or decreased undesirable properties.

Fusion proteins contain component polypeptides which are derived from different parental proteins, and bonded or fused to each other through a peptide bond. Each component polypeptide in a fusion protein contributes to the properties of the fusion protein, and it is desirable for the component polypeptide to be fused at positions that do not result in a reduction in the activity of the component polypeptides. Thus, conventional fusion proteins generally are fused at positions that correspond to domain boundaries, or the loops between domains, in the native parental proteins. For example, a conventional chimeric antibody light chain is a fusion protein that contains a non-human antibody light chain variable domain that is fused to a human light chain constant domain.

One aspect of conventional fusion proteins that can limit commercial applications is that the amino acid sequence and structure surrounding the fusion site does not match the corresponding amino acid sequence of either of the parental proteins. As a result, the fusion protein contains a "non-self' amino acid sequence that includes the amino acids adjacent to the fusion site. Even when a fusion protein contains polypeptides derived from proteins from the same species (*e.g.*, two human polypeptides are fused), the amino acid sequence at the fusion site will commonly comprise a non-self sequence generated by the juxtaposition of amino acid residues from different parental proteins. These non-self sequences can function as antibody and/or T-cell epitopes and render the fusion protein immunogenic, and can limit *in vivo* uses of the fusion protein, or render the fusion protein unsuitable for *in vivo* applications.

The juxtaposition of amino acid residues at the fusion site in conventional fusion proteins can also have other undesirable effects. For example, the juxtaposed amino acids can result in disruption of structural features important for expression, activity and/or stability. Consequently, conventional fusion proteins frequently form aggregates or oligomers, have low solubility and/or are more susceptible to proteolysis than are the parental proteins. In addition, conventional fusion proteins frequently can only be produced in lower yields than the parental proteins.

There is a need for improved fusion proteins and improved methods for designing and making fusion proteins.

SUMMARY OF THE INVENTION

The invention relates to recombinant fusion proteins that contain natural junctions. The fusion proteins of the invention comprise at least two portions derived from two different polypeptides, and at least one natural junction between the two portions.

The recombinant fusion proteins can comprise a hybrid domain, that contains a first portion derived from a first polypeptide and a second portion derived from a second polypeptide, wherein the first polypeptide comprises a domain that has the formula (X1-Y-X2), and the second polypeptide comprising a domain that has the formula (Z1-Y-Z2), wherein Y is a conserved amino acid motif, X1 and Z1 are the amino acid motifs that are located adjacent to the amino-terminus of Y in said first polypeptide and said second polypeptide, respectively, and X2 and Z2 are the amino acid motifs that are located adjacent to the carboxy-terminus of Y in said first polypeptide and said second polypeptide, respectively, provided that if the amino acid sequences of X1 and Z1 are the same, the amino acid sequences of X2 and Z2 are not the same; and when the amino acid sequences of X2 and Z2 are the same, the amino acid sequences of X1 and Z1 are not the same.

If desired, the hybrid domain can be bonded to an amino-terminal amino acid sequence D, and/or bonded to a carboxy-terminal amino acid sequence E, such that the recombinant fusion protein comprises a structure that has the formula D-(X1-Y-Z2)-E, wherein D is absent or is an amino acid sequence that is adjacent to the amino-terminus of (X1-Y-X2) in said first polypeptide; and E is absent or is an amino acid sequence that adjacent to the carboxy-terminus of (Z1-Y-Z2) in said second polypeptide. In particular embodiments, D is present, E is present, or D and E are present.

In some embodiments, the hybrid domain (X1-Y-Z2) is a hybrid immunoglobulin variable domain, such as hybrid antibody variable domain. Y can be in framework region (FR) 4, for example, Y can be GlyXaaGlyThr (SEQ ID NO:386) or GlyXaaGlyThrXaa(Val/Leu) (SEQ ID NO:387). In such embodiments, X1 can be a portion of an antibody variable domain comprising FR1, complementarity determining region (CDR) 1, FR2, CDR2, FR3, and CDR3.

In other embodiments Y is in FR3, for example Y can be GluAspThrAla (SEQ ID NO:388), ValTyrTyrCys (SEQ ID NO:389), or GluAspThrAlaValTyrTyrCys (SEQ ID NO:390). In such embodiments, X1 can be a portion of an antibody variable domain comprising FR1, CDR1, FR2, and CDR2.

In some embodiments, the hybrid domain (X1-Y-Z2) is a hybrid immunoglobulin constant domain, such as a hybrid antibody constant domain. Y can be (Ser/Ala/Gly)Pro(Lys/Asp/Ser)Val (SEQ ID NO:391), (Ser/Ala/Gly)Pro(Lys/Asp/Ser)ValPhe (SEQ ID NO:392), LysValAspLys(Ser/Arg/Thr) (SEQ ID NO:393) or ValThrVal (SEQ ID NO:394). For example, in particular embodiments, Y is selected from the group consisting of SerProLysVal (SEQ ID NO:398), SerProAspVal (SEQ ID NO:399), SerProSerVal (SEQ ID NO:400), AlaProLysVal (SEQ ID NO:401), AlaProAspVal (SEQ ID NO:402), AlaProSerVal (SEQ ID NO:403), GlyProLysVal (SEQ ID NO:404), GlyProAspVal (SEQ ID NO:405), Gly-ProSerVal (SEQ ID NO:406), SerProLysValPhe (SEQ ID NO:407), SerProAspValPhe (SEQ ID NO:408), SerProSer-ValPhe (SEQ ID NO:409), AlaProLysValPhe (SEQ ID NO:410), AlaProAspValPhe (SEQ ID NO:411), AlaProSerValPhe (SEQ ID NO:412), GlyProLysValPhe (SEQ ID NO:413), GlyProAspValPhe (SEQ ID NO:414), GlyProSerValPhe (SEQ ID NO:415), LysValAspLysSer (SEQ ID NO:416), LysValAspLysArg (SEQ ID NO:417), LysValAspLysThr (SEQ ID NO:418), and or ValThrVal (SEQ ID NO:394).

In some embodiments, D is absent, (X1-Y-Z2) is a hybrid immunoglobulin variable domain, and E is an immunoglobulin constant domain. The fusion protein can further comprise a second immunoglobulin variable domain that is amino terminal to or carboxyl terminal to (X1-Y-Z2).

In some embodiments, D is an immunoglobulin variable domain, and (X1-Y-Z2) is a hybrid immunoglobulin constant domain. In other embodiments, (X1-Y-Z2) is a hybrid immunoglobulin constant domain, and E is an immunoglobulin constant domain. In other embodiments, E is absent, (X1-Y-Z2) is a hybrid immunoglobulin constant domain, and the fusion protein comprises a further domain that is amino terminal to (X1-Y-Z2).

In other embodiments, D is an immunoglobulin constant domain, and (X1-Y-Z2) is a hybrid immunoglobulin constant domain.

The fusion protein of the invention, can comprise a first portion from a first polypeptide and a second portion from a second polypeptide wherein both polypeptides are members of the same protein superfamily. For example, the polypeptides can both be members of a protein superfamily is selected from the group consisting of the immunoglobulin superfamily, the TNF superfamily and the TNF receptor superfamily. Additionally or alternatively, the first polypeptide and said second polypeptide are both human polypeptides.

Generally X1, X2, Z1 and Z2 each, independently, consists of about 1 to about 200 amino acids. In some embodiments, the hybrid domain is about the size of an immunoglobulin variable domain or an immunoglobulin constant domain.

In more particular embodiments, the recombinant fusion protein comprises a hybrid immunoglobulin variable domain that is fused to an immunoglobulin constant domain. The hybrid immunoglobulin variable domain comprises a hybrid framework region (FR) that comprises a portion from a first immunoglobulin FR from a first immunoglobulin and a portion from a second immunoglobulin FR from a second immunoglobulin, the first immunoglobulin FR and the second immunoglobulin FR each comprise a conserved amino acid motif Y, and the hybrid immunoglobulin FR has the formula

$$(F^1\text{-}Y\text{-}F^2)$$

wherein Y is the conserved amino acid motif;

$F^1$ is the amino acid motif located adjacent to the amino-terminus of Y in the first immunoglobulin FR; and

$F^2$ is the amino acid motif located adjacent to the carboxy-terminus of Y in the second immunoglobulin FR.

Y can located in FR1, FR2, FR3 or FR4 of the first immunoglobulin and of the second immunoglobulin.

In some embodiments, Y is located in FR4, and $F^2$ is the amino acid sequence that is adj acent to (peptide bonded to) the amino-terminus of an immunoglobulin constant domain in a naturally occurring protein comprising said immunoglobulin constant domain. In some embodiments, the immunoglobulin constant domain is an antibody light chain constant domain and said second immunoglobulin FR is a FR4 from an antibody light chain variable domain. For example, the antibody constant domain is a Cκ or Cλ, and said second antibody FR4 is a Vκ FR4 or Vλ FR4, respectively.

In some embodiments, the first immunoglobulin is a non-human immunoglobulin, such as an immunoglobulin from a mouse, rat, shark, fish, possum, sheep, pig, *Camelid,* rabbit or non-human primate. In such embodiments, the second immunoglobulin can be a human immunoglobulin. Preferably, in such embodiments, the hybrid FR is bonded to a human immunoglobulin constant domain.

In particular embodiments, the hybrid immunoglobulin variable domain is a hybrid antibody variable domain, and Y is GlyXaaGlyThr (SEQ ID NO:386). In such embodiments, $F^1$ can be Phe and $F^2$ is (Leu/Met/Thr)ValThrValSerSer (SEQ ID NO:420). Preferably, the fusion protein of this embodiment comprises a human antibody constant domain, such as an IgG CH1 domain.

In particular embodiments, the hybrid immunoglobulin variable domain is a hybrid antibody variable domain, Y is GlyXaaGlyThr (SEQ ID NO:386), $F^1$ is Trp and $F^2$ is (Lys/Arg)(Val/Leu)(Glu/Asp)IleLys (SEQ ID NO:424) or (Lys/Gln/Glu)(Val/Leu)(Thr/Ile)(Val/Ile)Leu (SEQ ID NO:425). Preferably, the fusion protein of this embodiment comprises a human antibody light chain constant domain.

In particular embodiments, the hybrid immunoglobulin variable domain is a hybrid antibody variable domain, and Y is GlyXaaGlyThrXaa(Val/Leu) (SEQ ID NO:387). In such embodiments, $F^1$ can be Phe, and $F^2$ can be ThrValSerSer (SEQ ID NO:419). Preferably, the fusion protein of this embodiment comprises a human antibody heavy chain constant domain, such as an IgG1 or IgG4 CH1 domain or IgG1 or IgG4 CH2 domain.

In particular embodiments, the hybrid immunoglobulin variable domain is a hybrid antibody variable domain. Y is GlyXaaGlyThrXaa(Val/Leu) (SEQ ID NO:387), $F^1$ is Trp, and $F^2$ is (Glu/Asp)IleLys (SEQ ID NO:458) or (Thr/Ile)(Val/Ile)Leu (SEQ ID NO:459). Preferably, the fusion protein of this embodiment comprises a human antibody light chain constant domain.

If desired, the recombinant fusion protein can comprises a structure that has the formula $(F^1\text{-}Y\text{-}F^2)\text{-}C_L$, $(F^1\text{-}Y\text{-}F^2)\text{-}CH1$, $(F^1\text{-}Y\text{-}F^2)\text{-}CH2$, or $(F^1\text{-}Y\text{-}F^2)\text{-}Fc$. The recombinant fusion protein can further comprises a second immunoglobulin variable domain, that is amino terminal or carboxy-terminal to $(F^1\text{-}Y\text{-}F^2)$.

The invention also relates to improved fusion proteins that comprise a non-human antibody variable region fused to a human antibody constant domain, the improvement comprising a hybrid FR4 in the non-human variable region that has the formula

$$(F^1\text{-}Y\text{-}F^2)$$

wherein $F^1$ is Phe or Trp;

Y is GlyXaaGlyThr (SEQ ID NO:386), and $F^2$ is (Leu/Met/Thr)ValThrValSerSer (420), (Lys/Arg)(Val/Leu)(Glu/Asp)IleLys (SEQ ID NO:424) or

(Lys/Gln/Glu)(Val/Leu)(Thr/Ile)(Val/Ile)Leu (SEQ ID NO:425); or

Y is GlyXaaGlyThrXaa(Val/Leu) (SEQ ID NO:387), and $F^2$ is ThrValSerSer (SEQ ID NO:419), (Glu/Asp)IleLys (SEQ ID NO:458) or (Thr/Ile)(Val/Ile)Leu (SEQ ID NO:459).

The recombinant fusion protein can comprise an immunoglobulin variable domain fused to a hybrid immunoglobulin constant domain, The hybrid immunoglobulin constant domain comprises a portion from a first immunoglobulin constant domain and a portion from a second immunoglobulin constant domain, the first immunoglobulin constant domain and the second immunoglobulin constant domain each comprising a conserved amino acid motif Y. The hybrid immunoglobulin constant domain has the formula

$$C^1\text{-}Y\text{-}C^2$$

wherein Y is said conserved amino acid motif;

$C^1$ is the amino acid motif adjacent to the amino-terminus of Y in the first immunoglobulin constant region;

$C^2$ is the amino acid motif adjacent to the carboxy-terminus of Y in the second immunoglobulin constant region.

In some embodiments, the hybrid immunoglobulin constant domain is a hybrid antibody constant domain comprising a portion from a first antibody constant domain and a portion from a second antibody constant domain. the hybrid antibody constant domain can be a hybrid antibody CH1, a hybrid antibody hinge, a hybrid antibody CH2, or a hybrid antibody CH3.

In some embodiments, first antibody constant domain and said second antibody constant domain are from different species.

In other embodiments, the second antibody constant domain is a human antibody constant domain. Alternatively or additionally, first antibody constant domain is a mouse, rat, shark, fish, possum, sheep, pig, *Camelid,* rabbit or non-human primate constant domain.

In some embodiments, the fusion protein comprises an immunoglobulin variable domain that is a non-human antibody variable domain and the first constant domain is the corresponding non-human CH1 domain, Cλ domain or Cκ domain. In some embodiments, the first antibody constant domain is a light chain constant domain, and said second antibody constant domain is a heavy chain constant domain.

In other embodiments, the first antibody constant domain is a *Camelid* heavy chain constant domain, and said second antibody constant domain is a heavy chain constant domain. If desired, a VHH can be amino terminal to the hybrid constant domain.

In some embodiments, first antibody constant domain and said second antibody constant domain are of different isotypes. Preferably, the second antibody constant domain is an IgG constant domain.

In some embodiments, the fusion protein comprise an antibody variable domain that is a light chain variable domain and the first antibody constant domain is a light chain constant domain. In such embodiments, the second antibody constant domain can be a human antibody heavy chain constant domain or a human antibody light chain constant domain. In some embodiments, the human antibody heavy chain constant domain is a CH1, a hinge, a CH2, or a CH3.

In particular embodiments, Y is (Ser/Ala/Gly)Pro(Lys/Asp/Ser)Val (SEQ ID NO:391), (Ser/Ala/Gly)Pro (Lys/Asp/Ser)ValPhe (SEQ ID NO:392), LysValAspLys(Ser/Arg/Thr) (SEQ ID NO:393), or ValThrVal (SEQ ID NO:394). In some of these embodiments, the second antibody constant domain is a human antibody constant domain, such as Cκ, Cλ, a CH1, a hinge, a CH2 and a CH3.

In particular embodiments, the recombinant fusion protein comprises a human light chain variable domain that is fused to a hybrid human CH1 domain, and
$C^1$ is GlnProLysAla (SEQ ID NO:466) or ThrValAla (SEQ ID NO:467),
Y is (Ala/Gly)ProSerVal (SEQ ID NO:468), and
$C^2$ is the amino acid motif adjacent to the carboxy-terminus of Y in human IgG CH1.

In particular embodiments, the recombinant fusion protein comprises a human light chain variable domain that is fused to a hybrid human CH2, wherein:

$C^1$ is GlnProLysAla (SEQ ID NO:466) or ThrValAla (SEQ ID NO:467),
Y is (Ala/Gly)ProSerVal (SEQ ID NO:468), and
$C^2$ is the amino acid motif adjacent to the carboxy-terminus of Y in human IgG CH2.

In particular embodiments, the recombinant fusion protein comprises a human heavy chain variable domain that is fused to a hybrid human CH2, wherein
$C^1$ is SerThrLys (SEQ ID NO:469),
Y is (Ala/Gly)ProSerValPhe (SEQ ID NO:470), and
$C^2$ is the amino acid motif adjacent to the carboxy-terminus of Y in human IgG CH2.

In particular embodiments, the recombinant fusion protein comprises a human lambda chain variable domain that is fused to a hybrid human Cκ, and wherein
$C^1$ is GlnProLysAla (SEQ ID NO:466),
Y is (Ala/Gly)ProSerVal (SEQ ID NO:468), and
$C^2$ is the amino acid motif adjacent to the carboxy-terminus of Y in human Cκ.

In particular embodiments, the recombinant fusion protein comprises a human heavy chain variable domain that is fused to a hybrid human Cκ, wherein
$C^1$ is SerThrLys (SEQ ID NO:469),
Y is (Ala/Gly)ProSerValPhe (SEQ ID NO:470), and
$C^2$ is the amino acid motif adjacent to the carboxy-terminus of Y in human Cκ.

In particular embodiments, the recombinant fusion protein comprises a human kappa chain variable domain that is fused to a hybrid human Cλ, and wherein
$C^1$ is ThrValAla (SEQ ID NO:467),
Y is (Ala/Gly)ProSerVal (SEQ ID NO:468), and
$C^2$ is the amino acid motif adjacent to the carboxy-terminus of Y in human Cλ.

In particular embodiments, the recombinant fusion protein comprises a human heavy chain variable domain that is fused to a hybrid human Cλ, wherein
$C^1$ is SerThrLys (SEQ ID NO:469),
Y is (Ala/Gly)ProSerVal (SEQ ID NO:468), and
$C^2$ is the amino acid motif adjacent to the carboxy-terminus of Y in human Cλ.

The invention also relates to a recombinant fusion protein comprising a first portion derived from a first polypeptide and a second portion derived from a second polypeptide, wherein said first polypeptide comprises a structure having the formula (A)-L1, wherein (A) is an amino acid sequence present is said first polypeptide; and L1 is an amino acid motif comprising 1 to about 50 amino acids that are adjacent to the carboxy-terminus of (A) in said first polypeptide; wherein said fusion polypeptide has the formula

$$(A)\text{-}L1\text{-}(B);$$

wherein (B) is said portion derived from said second polypeptide; with the proviso that at least one of (A) and (B) is a domain, and when (A) and (B) are both antibody variable domains

> a) (A) and (B) are each human antibody variable domains;
> b) (A) and (B) are each antibody heavy chain variable domains;
> c) (A) and (B) are each antibody light chain variable domains;
> d) (A) is an antibody light chain variable domain and (B) is an antibody heavy chain variable domain; or
> e) (A) is a VHH and (B) is an antibody light chain variable domain; or with the proviso that when (A) and (B) are both antibody variable domains the following is excluded from the invention, (A)-L1-(B) where (A) is a mouse VH, (B) is a mouse VL and L1 is SerAlaLysThrThrPro (SEQ ID NO:537), SerAlaLysThrThrProLysLeuGlyGly (SEQ ID NO: 538), AlaLysThrThrProLysLeuGluGluGlyGluPheSerGluAlaArgVal (SEQ ID NO:539), or AlaLysThrThrProLys-LeuGluGlu (SEQ ID NO:540).

In some embodiments, the first polypeptide is an antibody variable domain. The second polypeptide can be an immunoglobulin constant region. In some embodiments, (B) comprises at least a portion of an antibody CH1, at least a portion of an antibody hinge, at least a portion of an antibody CH2, or at least a portion of an antibody CH3.

In some embodiments, (A) is an antibody light chain variable domain. In such embodiments, L1 comprises one to about 50 contiguous amino-terminal amino acids of Cκ or Cλ. In other embodiments, (A) is an antibody heavy chain variable domain, such as a VH or a VHH. In such embodiments, L1 can comprise one to about 50 contiguous amino-terminal amino acids of CH1.

In some embodiments, (A) is an antibody heavy chain variable domain and (B) is an antibody heavy chain variable domain, or (A) is an antibody light chain variable domain and (B) is an antibody heavy chain variable domain or an antibody light chain variable domain. For example, in certain embodiments (A) is a Vκ and (B) is a Vκ; (A) is a Vκ and (B) is a Vλ; (A) is a Vκ and (B) is a VH or a VHH; (A) is a Vλ and (B) is a Vκ; (A) is a Vλ and (B) is a Vλ; or(A) is a Vλ and (B) is a VH or a VHH.

In some embodiments (A) is a VH and L1 comprises the first 3 to about 12 amino acids of CH1; (A) is a Vκ and L1 comprises the first 3 to about 12 amino acids of Cκ; or (A) is a Vλ and L1 comprises the first 3 to about 12 amino acids of Cλ.

In certain embodiments (A) is an antibody variable domain comprising FR1, CDR1, FR2, CDR3, FR3 and CDR3 of a antibody light chain variable domain and FR4 comprising the amino acid sequence GlyGlnGlyThrLysValThrValSerSer (SEQ ID NO:472); and L1 comprises the first 3 to about 12 amino acids of CH1. In these embodiments, L1 can be AlaSerThr (SEQ ID NO:473), AlaSerThrLysGlyProSer (SEQ ID NO:474), or AlaSerThrLysGlyProSerGly (SEQ ID NO: 475).

In certain embodiments, (A) is an antibody variable domain comprising FR1, CDR1, FR2, CDR3, FR3 and CDR3 of a VH or Vκ domain and FR4 comprising the amino acid sequence GlyXaaGlyThr(Lys/Gln/Glu)(Val/Leu)(Thr/Ile) ValLeu (SEQ ID NO:476); and L1 comprises the first 3 to about 12 amino acids of Cλ. In other embodiments, (A) is an antibody variable domain comprising FR1, CDR1, FR2, CDR3, FR3 and CDR3 of a VH or Vλ domain and FR4 comprising the amino acid sequence GlyGlnGlyThrLysValGluIleLysArg (SEQ ID NO:477); and L1 comprises the first 3 to about 12 amino acids of Cκ.

In other embodiments, (A) is an immunoglobulin constant domain, such as an antibody constant domain. In other embodiments, (A) is a nonhuman immunoglobulin constant domain, and (B) is derived from a human polypeptide.

In some embodiments, the second polypeptide is selected from the group consisting of a cytokine, a cytokine receptor, a growth factor, a growth factor receptor, a hormone, a hormone receptor, an adhesion molecule, a haemostatic factor, a T cell receptor, a T cell receptor chain, a T cell receptor variable domain, enzyme, polypeptide comprising or consisting of an antibody variable domain, or a functional portion of any one of the foregoing.

In other embodiments, the first polypeptide is selected from the group consisting of a cytokine, a cytokine receptor, a growth factor, a growth factor receptor, a hormone, a hormone receptor, an adhesion molecule, a haemostatic factor, a T cell receptor, a T cell receptor chain, a T cell receptor variable domain, enzyme, polypeptide comprising or consisting of an antibody variable domain, or a functional portion of any one of the foregoing. In such embodiments, the second polypeptide can be an immunoglobulin constant region or Fc portion of an immunoglobulin constant region.

The invention relates to a recombinant fusion protein comprising a first portion that is an immunoglobulin variable domain and a second portion, wherein said first portion is bonded to said second portion through a linker, and the recombinant fusion protein has the formula

$$(A')\text{-L2-}(B)$$

wherein (A') is said immunoglobulin variable domain and comprises framework (FR) 4, L2 is said linker, wherein L2 comprises one to about 50 contiguous amino acids that are adjacent to the carboxy-terminus of said FR4 in a naturally occurring immunoglobulin that comprises said FR4; and (B) is said second portion;

with the proviso that L2-(B) is not a $C_L$ or CH1 domain that is peptide bonded to said FR4 in a naturally occurring antibody that comprises said FR4, and when (A) and (B) are both antibody variable domains

a) (A) and (B) are each human antibody variable domains;
b) (A) and (B) are each antibody heavy chain variable domains;
c) (A) and (B) are each antibody light chain variable domains;
d) (A) is an antibody light chain variable domain and (B) is an antibody heavy chain variable domain; or
e) (A) is a VHH and (B) is an antibody light chain variable domain; or with the proviso that when (A) and (B) are both antibody variable domains the following is excluded from the invention, (A)-L1-(B) where (A) is a mouse VH, (B) is a mouse VL and L1 is SerAlaLysThrThrPro (SEQ ID NO:537), SerAlaLysThrThrProLysLeuGlyGly (SEQ ID NO:538), AlaLysThrThrProLysLeuGluGluGlyGluPheSerGluAlaArgVal (SEQ ID NO:539), or AlaLysThrThrProLysLeuGlyGly (SEQ ID NO:540).

In some embodiments (A') is an antibody heavy chain variable domain or a hybrid antibody variable domain. In some embodiments antibody heavy chain variable domain or a hybrid antibody variable domain each comprise a FR4 that comprises the amino acid sequence GlyXaaGlyThr(Leu/Met/Thr)ValThrValSerSer (SEQ ID NO:478). In these embodiments, L2 can comprise one to about 50 contiguous amino acids from the amino-terminus of CH1. In particular embodiments L2 comprises AlaSerThr (SEQ ID NO:473), AlaSerThrLysGlyProSer (SEQ ID NO:474), or AlaSerThrLys-GlyProSerGly (SEQ ID NO:475).

In some embodiments (A') is a hybrid antibody variable domain or a Vκ that comprise a FR4 that comprises the amino acid sequence

GlyXaaGlyThr(Lys/Arg)(Val/Leu)(Glu/Asp)IleLysArg (SEQ ID NO: 485). In these embodiments, L2 can comprises one to about 50 contiguous amino acids from the amino-terminus of Cκ. In particular embodiments L2 comprises ThrValAla (SEQ ID NO:467), ThrValAlaAlaProSer (SEQ ID NO:490), or ThrValAlaAlaProSerGly (SEQ ID NO:491). In some embodiments (A') is a hybrid antibody variable domain or a Vλ that comprises a FR4 that comprises the amino acid sequence GlyXaaGlyThr(Lys/Gln/Glu)(Val/Leu)(Thr/Ile)(Val/Ile)Leu (SEQ ID NO:492).

In some embodiments, (B) comprises an antibody light chain variable domain or an antibody heavy chain variable domain. In other embodiments, (B) comprises at least a portion of an immunoglobulin constant region, for example at the amino-terminus of (B). The immunoglobulin constant region can be an IgG constant region, such as an IgG1 constant region or an IgG4 constant region. In some embodiments (B) comprises at least a portion of CH1, at least a portion of hinge, at least a portion of CH2 or at least a portion of CH3.

In particular embodiments, (B) comprises at least a portion of hinge that comprises ThrHisThrCysProProCysPro (SEQ ID NO:520). Additionally, (B) can further comprises CH2-CH3. In other embodiments, (B) comprises a portion of CH1-hinge-CH2-CH3, hinge-CH2-CH3, CH2-CH3, or CH3.

The invention relates to a recombinant fusion protein comprising a first portion and a second portion derived from an immunoglobulin constant region. The first portion is bonded to said second portion through a linker, and the recombinant fusion protein has the formula

$$(A)\text{-}L3\text{-}(C^3)$$

wherein (A) is said first portion, $(C^3)$ is said second portion derived from an immunoglobulin constant region; and L3 is said linker, wherein L3 comprises one to about 50 contiguous amino acids that are adjacent to the amino-terminus of $(C^3)$ in a naturally occurring immunoglobulin that comprises $(C^3)$, with the proviso that (A) is not an antibody variable domain found in said naturally occurring immunoglobulin.

In some embodiments, $(C^3)$ comprises at least on antibody constant domain, such as a human antibody constant domain. In some embodiments the antibody constant domain is an IgG constant domain, such as an IgG1 constant domain or an IgG4 constant domain.

In some embodiments, $(C^3)$ comprises CH3. In these embodiments, L3 comprises one to about 50 contiguous amino acids from the carboxy-terminus of CH2. In other embodiments, $(C^3)$ comprises CH2 or CH2-CH3. In these embodiments, L3 comprises one to about 34 contiguous amino acids from the carboxy-terminus of hinge. For example, L3 can comprise ThrHisThrCysProProCysPro (SEQ ID NO:520) or GlyThrHisThrCysProProCysPro (SEQ ID NO:521).

In some embodiments, $(C^3)$ comprises hinge. In these embodiments, L3 comprises one to about 50 contiguous amino acids from the carboxy-terminus of CH1. In some embodiments, $(C^3)$ comprises CH1. In these embodiments, L3 comprises one to about 50 contiguous amino acids from the carboxy-terminus of an antibody heavy chain V domain. For example, L3 comprises GlyXaaGlyThr(Leu/Met/Thr)ValThrValSerSer (SEQ ID NO:478).

In some embodiments, the antibody constant domain is a Cκ or a Cλ. In such embodiments, L3 comprises one to about 50 contiguous amino acids from the carboxy-terminus of an antibody light chain V domain. For example, when the antibody constant domain is a Cκ, L3 can comprises GlyXaaGlyThr(Lys/Arg)(Val/Leu)(Glu/Asp)IleLysArg (SEQ ID NO:485). When the antibody constant domain is a Cλ, L3 can comprises GlyXaaGlyThr(Lys/Gln/Glu)(Val/Leu)(Thr/Ile)(Val/Ile)Leu (SEQ ID NO:492).

In certain embodiments, (A) is selected from the group consisting of a cytokine, a cytokine receptor, a growth factor, a growth factor receptor, a hormone, a hormone receptor, an adhesion molecule, a haemostatic factor, a T cell receptor, a T cell receptor chain, a T cell receptor variable domain, enzyme, polypeptide comprising or consisting of an antibody variable domain, or a functional portion of any one of the foregoing.

The invention also relates to a recombinant fusion protein comprising a first portion derived from an antibody variable domain and a second portion derived from a second polypeptide, wherein said antibody variable domain comprises a structure having the formula (A)-L1, wherein (A) consists of CDR3; L1 consists of FR4, wherein said fusion polypeptide has the formula (A)-L1-(B), wherein (B) is said portion derived from said second polypeptide.

In some embodiments, the second polypeptide is an immunoglobulin constant region. In other embodiments, (B) comprises at least a portion of an antibody CH1, at least a portion of an antibody hinge, at least a portion of an antibody CH2, or at least a portion of an antibody CH3.

The invention also relates to an isolated recombinant nucleic acid molecule encoding a recombinant fusion protein comprising a natural junction as described herein, and to a host cell comprising a recombinant nucleic acid molecule encoding a recombinant fusion protein comprising a natural junction as described herein

The invention also relates to a method of producing a recombinant fusion protein comprising maintaining a host cell of the invention under conditions suitable for expression of a recombinant nucleic acid encoding the fusion protein comprising a natural junction, whereby said recombinant nucleic acid is expressed and said recombinant fusion protein is produced. In certain embodiments, the method further comprises isolating said recombinant fusion protein.

The invention also relates to recombinant fusion protein comprising a natural junction as described herein for use in therapy, diagnosis and/or prophylaxis. The invention also relates to the use of a recombinant fusion protein comprising a natural junction as described herein for the manufacture of a medicament for therapy, diagnosis and/or prophylaxis in a human, with reduced likelihood of inducing an immune response.

The invention also relates to a method of therapy, diagnosis and/or prophylaxis in a human comprising administering to said human an effective amount of a recombinant fusion protein comprising a natural junction as described herein, whereby the likelihood of inducing an immune response is reduced in comparison to a corresponding fusion protein that does not contain a natural junction.

The invention also relates to use of a natural junction for preparing a recombinant fusion protein for human therapy, diagnosis and/or prophylaxis, with reduced likelihood of inducing an immune response in comparison to a corresponding fusion protein that does not contain a natural junction.

The invention relates to use of a natural junction for preparing a recombinant fusion protein for human therapy, diagnosis and/or prophylaxis, with reduced propensity to aggregate in comparison to a corresponding fusion protein that does not contain a natural junction.

The invention relates to use of a natural junction for preparing a recombinant fusion protein for human therapy, diagnosis and/or prophylaxis, wherein said recombinant fusion protein is expressed at higher levels in comparison to a corresponding fusion protein that does not contain a natural junction.

The invention relates to use of a natural junction for preparing a recombinant fusion protein for human therapy, diagnosis and/or prophylaxis, wherein said recombinant fusion protein has enhanced stability in comparison to relative to a corresponding fusion protein that does not contain a natural junction.

The invention relates to use of a natural junction for preparing a recombinant fusion protein comprising a first portion (A) and a second portion (B), and at least one natural junction between (A) and (B), and wherein said recombinant fusion protein has reduced propensity to aggregate in comparison to a corresponding fusion protein comprising (A) and (B), wherein the interface of (A) and (B) is not a natural junction.

The invention relates to use of a natural junction for preparing a recombinant fusion protein comprising a first portion (A), a second portion (B), and at least one natural junction between (A) and (B), wherein said recombinant fusion protein is expressed at higher levels in comparison to a corresponding fusion protein comprising (A) and (B), wherein said corresponding fusion protein does not contain a natural junction between (A) and (B).

The invention relates to use of a natural junction for preparing a recombinant fusion protein comprising a first portion (A), a second portion (B), and at least one natural junction between (A) and (B), wherein said recombinant fusion protein has enhanced stability in comparison to a corresponding fusion protein comprising (A) and (B), wherein said corresponding fusion protein does not contain a natural junction between (A) and (B).

The invention relates to a pharmaceutical composition comprising a recombinant fusion protein comprising a natural junction as described herein and a physiologically acceptable carrier.

The invention relates to a method of designing or producing a fusion protein comprising a first portion and a second portion that are fused at a natural junction, wherein said first portion is derived from a first polypeptide and said second portion is derived from a second polypeptide. The method comprises analyzing the amino acid sequence of said first polypeptide or a portion thereof and the amino acid sequence of said second polypeptide or a portion thereof to identify a conserved amino acid motif present in both of the analyzed sequences; and preparing a fusion protein which has the formula

$$A\text{-}Y\text{-}B \; ;$$

wherein A is said first portion, Y is said conserved amino acid motif, B is said second portion, and wherein said first polypeptide comprises A-Y, and said second polypeptide comprises Y-B.

In some embodiments, the second polypeptide comprises an immunoglobulin constant domain, such as a human immunoglobulin constant domain or a nonhuman immunoglobulin constant domain. In particular embodiments, the second polypeptide comprises an antibody constant domain.

In some embodiments, the second polypeptide and B comprise an antibody heavy chain constant domain, such a hinge region, a portion of CH1-hinge-CH2-CH3, hinge-CH2-CH3, CH2-CH3, or CH3. Preferably, the constant domain is a human antibody heavy chain constant domain, such as an IgG (e.g., IgG1 constant domain or an IgG4 constant domain).

In some embodiments, the first polypeptide is selected from the group consisting of a cytokine, a cytokine receptor, a growth factor, a growth factor receptor, a hormone, a hormone receptor, an adhesion molecule, a haemostatic factor, a T cell receptor, a T cell receptor chain, a T cell receptor variable domain, enzyme, polypeptide comprising or consisting of an antibody variable domain, or a functional portion of any one of the foregoing

In some embodiments, the first polypeptide and A comprise an immunoglobulin variable domain, such as a human immunoglobulin variable domain or a nonhuman immunoglobulin variable domain. In certain embodiments, the first polypeptide comprises non-human antibody variable domain or a human antibody variable domain. In these embodiments, the second polypeptide can be selected from the group consisting of a cytokine, a cytokine receptor, a growth factor, a growth factor receptor, a hormone, a hormone receptor, an adhesion molecule, a haemostatic factor, a T cell receptor, a T cell receptor chain, a T cell receptor variable domain, enzyme, polypeptide comprising or consisting of an antibody variable domain, or a functional portion of any one of the foregoing.

In some embodiments, the first polypeptide is a first antibody chain, the second polypeptide is a second antibody chain. In these embodiments, Y is in the variable domain of said first antibody chain and the variable domain of said second antibody chain. In one embodiment, Y is in framework region (FR) 4. In these embodiments, Y can be GlyXaaGlyThr (SEQ ID NO:386) or GlyXaaGlyThrXaa(Val/Leu) (SEQ ID NO:387). In other embodiments, Y is in FR3. In these embodiments, Y can be GluAspThrAla (SEQ ID NO:388), ValTyrTyrCys (SEQ ID NO:389), or GluAspThrAlaVal-TyrTyrCys (SEQ ID NO:390). In other embodiments, Y is in a constant domain of said first antibody chain and a constant domain of said second antibody chain. In these embodiments, Y can be (Ser/Ala/Gly)Pro(Lys/Asp/Ser)Val (SEQ ID NO: 391), (Ser/Ala/Gly)Pro(Lys/Asp/Ser)ValPhe (SEQ ID NO:392), LysValAspLys(Ser/Arg/Thr) (SEQ ID NO:393) or ValThrVal (SEQ ID NO:394).

In some embodiments, the first antibody chain, and said second antibody chain are from different species. In other embodiments, the first antibody chain, and said second antibody chain are from the same species. In particular embodiments, the first antibody chain and said second antibody chain are human.

In some embodiment the fusion protein further comprises a third portion located amino terminally to A. In some embodiment, the third portion comprises an immunoglobulin variable domain.

In some embodiments, the first polypeptide and said second polypeptide are both members of the same protein superfamily. For example, the first polypeptide and the second polypeptide can be member of a protein superfamily selected from the group consisting of the immunoglobulin superfamily, the TNF superfamily and the TNF receptor superfamily.

BRIEF DESCRIPTION OF THE DRAWINGS

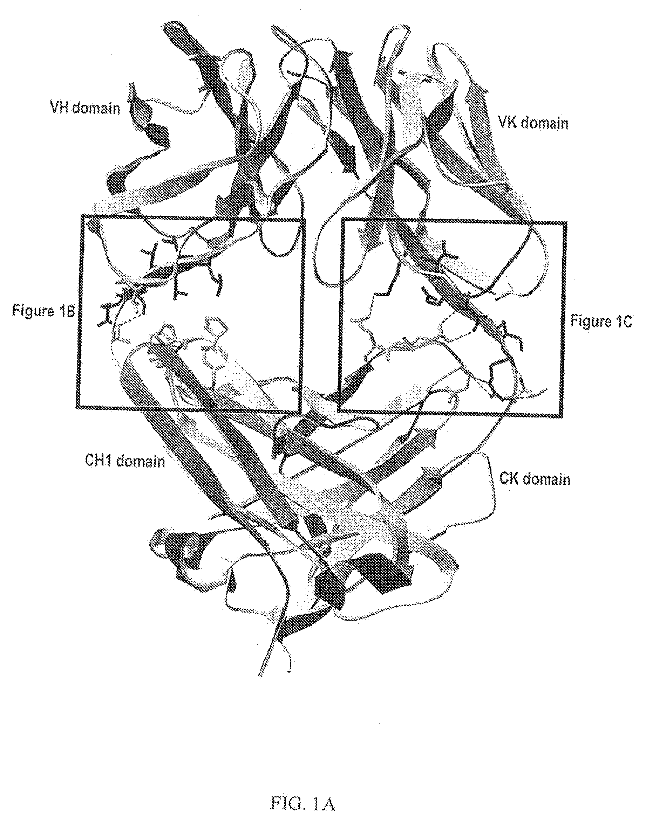

FIG. 1A illustrates the structure of a typical human Fab' fragment.

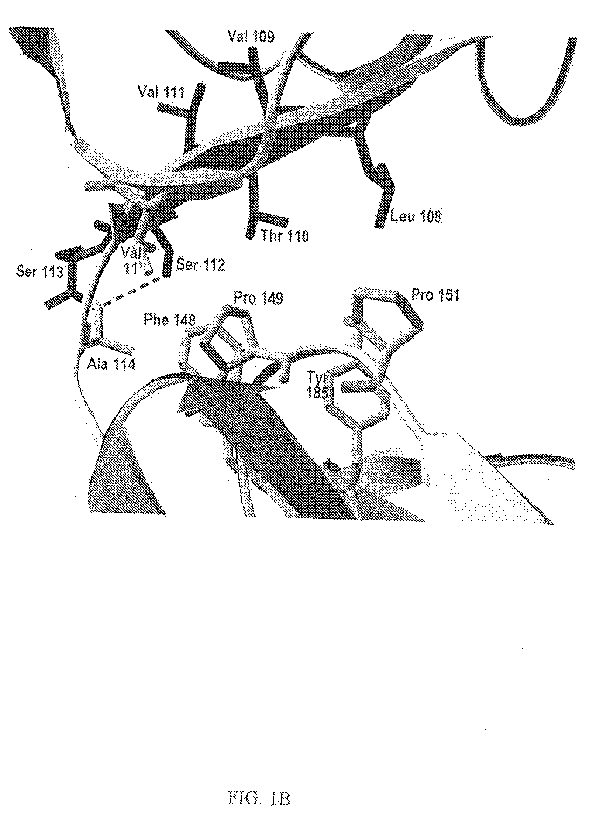

FIG. 1B illustrates a cluster of five residues in a typical human Fab' fragment (three highly conserved residues in VH (H11 [Leu or Val], H110 [Thr] and H112 [Ser]) and two highly conserved residues in CH1 (H148 [Phe] and H149 [Pro]). This cluster provides a degree of controlled flexibility that changes the orientation of Vκ-VH domains relative to Cκ-CH1 domains in immunoglobulins.

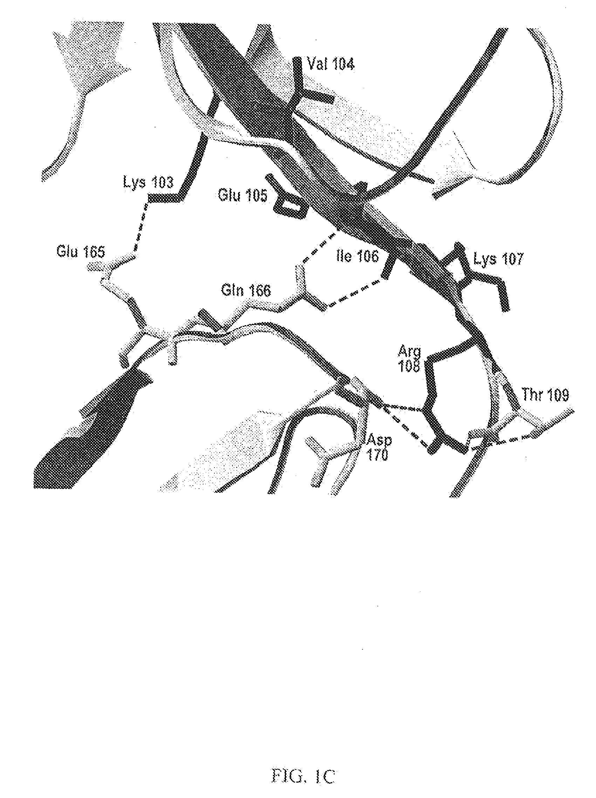

FIG. 1C illustrates the typical interactions found between Vκ and Cκ domains of a typical human Fab' fragment.

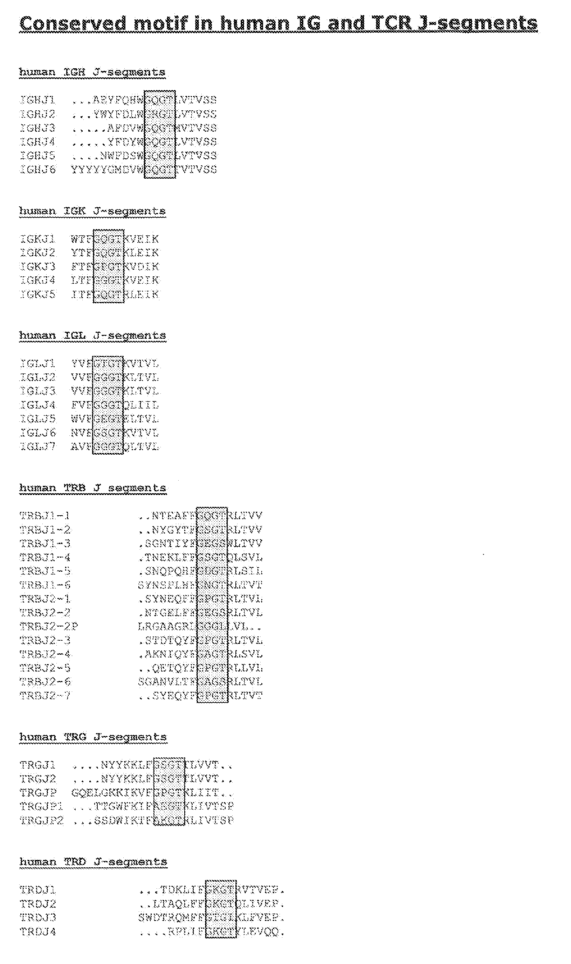

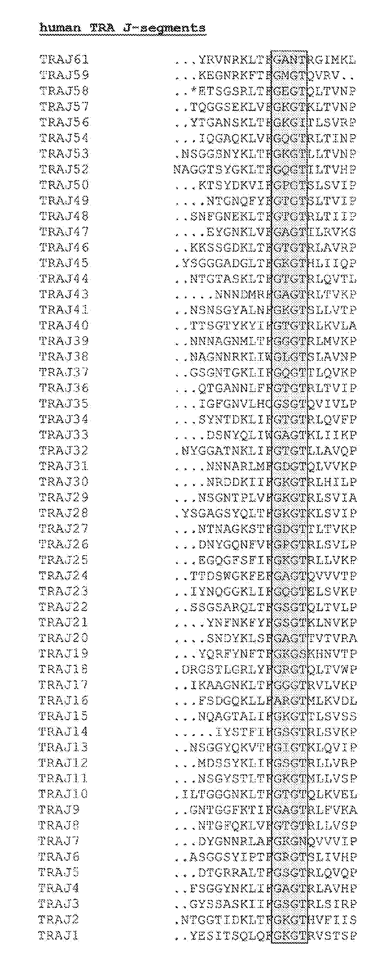

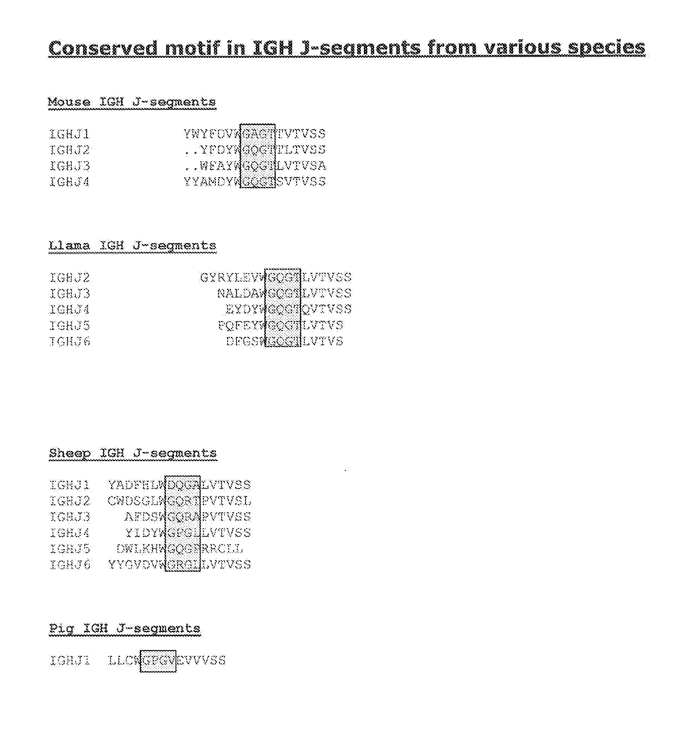

FIGS. 2A and 2B are alignments of the amino acid sequences in human antibody and TCR J-segments illustrating conserved motifs. The aligned amino acid sequences are from human IgH J-segments (SEQ ID NOS:1-6), human Igκ J-segments (SEQ ID NOS:7-11), human Igλ J-segments (SEQ ID NOS:12-18), human TCRβ J-segments (SEQ ID NOS:19-32), human TCRγ J-segments (SEQ ID NOS:33-37), human TCRδ J-segments (SEQ ID NOS:38-41) and human TCRα segments (SEQ ID NOS:42-98).

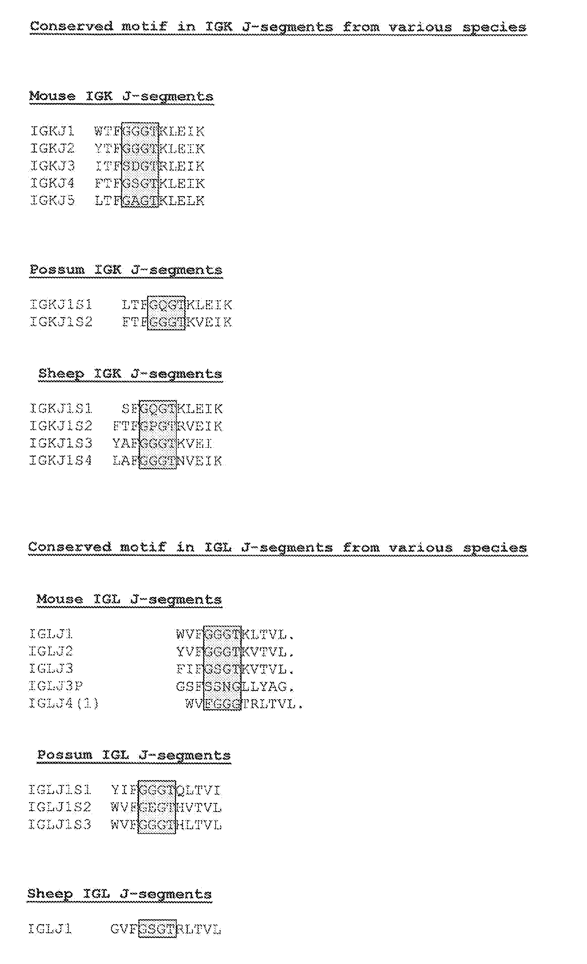

FIG. 3 illustrates a conserved motif in antibody heavy chain (IgH) J-segments from various species. Amino acid alignments of Mouse IgH J-segments (SEQ ID NOS:99-102), Llama IgH J-segments (SEQ ID NO:103-107), Sheep IgH J-segments (SEQ ID NOS:108-113) and a Pig IgH J-segment (SEQ ID NO: 114) are shown.

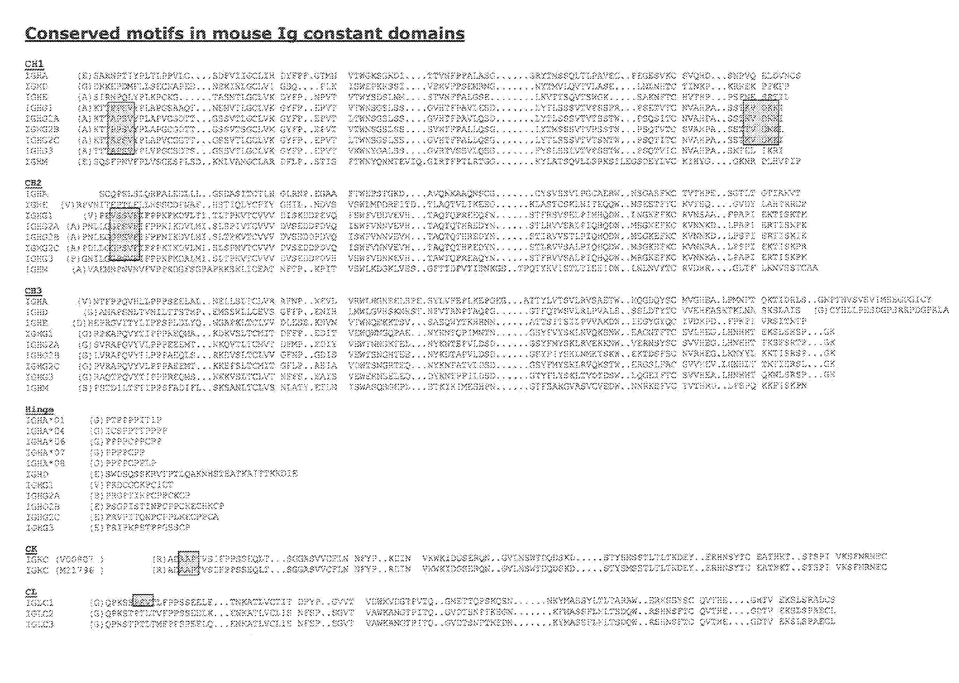

FIG. 4 illustrates a conserved motif in antibody κ chain (Igκ) J-segments from various species and a conserved motif in antibody λ chain (Igλ) J-segments from various species. Amino acid sequence alignments of Mouse Igκ J-segments (SEQ ID NOS:115-119) and Igλ J-segments (SEQ ID NOS:126-130), Possum Igκ J-segments (SEQ ID NOS:120-121) and Igλ J-segments (SEQ ID NOS:131-133), and Sheep Igκ J-segments (SEQ ID NOS:122-125) and Igλ J-segment (SEQ ID NO:134) are shown.

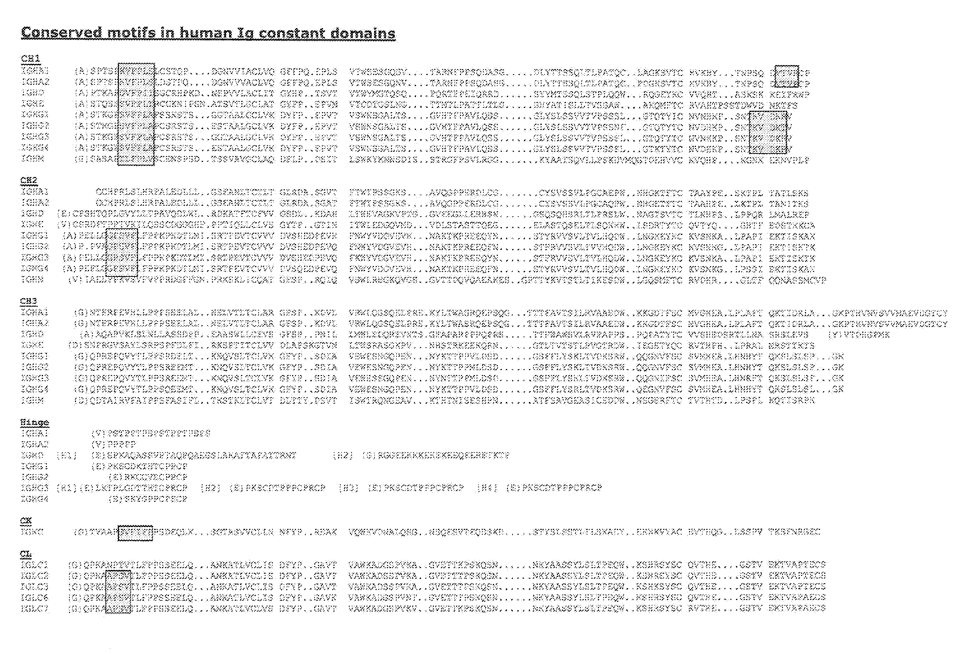

FIG. 5 illustrates the conserved motifs in mouse antibody constant domains. The amino acid sequence alignments show conserved motifs in CH1 (SEQ ID NOS:135-143), CH2 (SEQ ID NOS:144-151), CH3 (SEQ ID NOS:152-160), Hinge (SEQ ID NOS:161-171), Cκ (SEQ ID NOS:172-173), and Cλ regions (SEQ ID NOS:174-176) of mouse Ig.

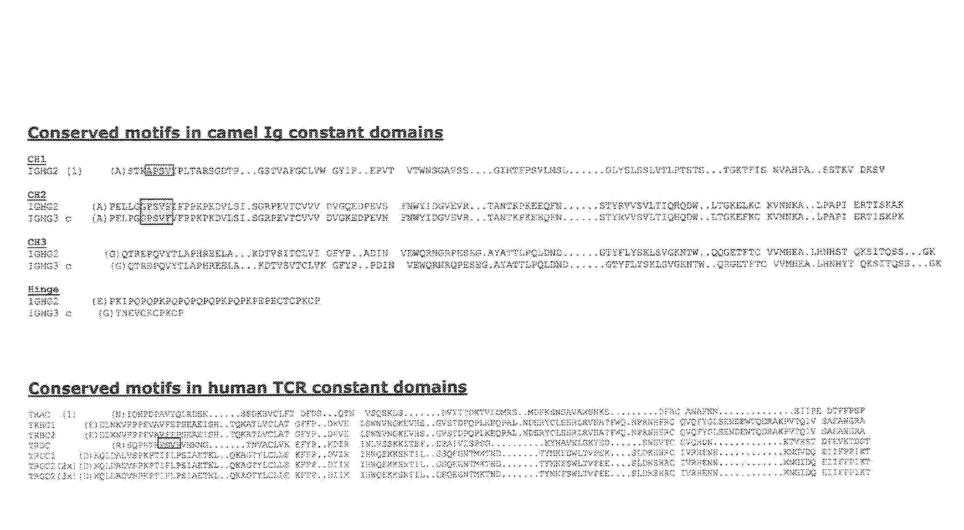

FIG. 6 illustrates the conserved motifs in human antibody constant domains. The amino acid sequence alignments show a conserved motifs in CH1 (SEQ ID NOS:177-185), CH2 (SEQ ID NOS:186-194), CH3 (SEQ ID NOS:195-203), Hinge (SEQ ID NOS:204-210), Cκ (SEQ ID NO:211), and Cλ regions (SEQ ID NOS:212-216) of human Ig.

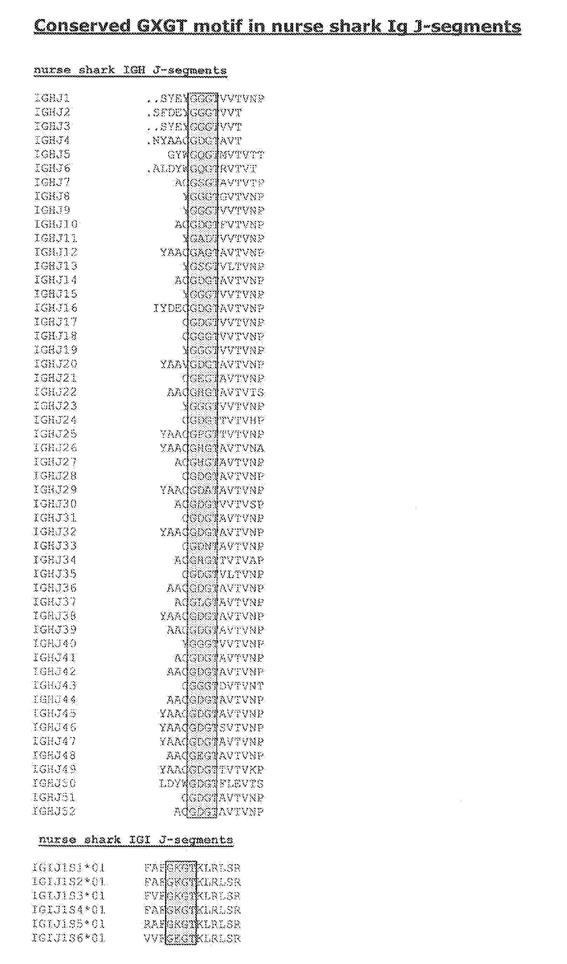

FIG. 7 illustrates the conserved motifs in camel antibody constant domains and human TCR constant domains. Amino acid sequence alignments show the conserved motifs in CH1 (SEQ ID NO:217), CH2 (SEQ ID NOS:218-219), CH3 (SEQ ID NOS:220-221) and Hinge (SEQ ID NOS:222-223) regions of camel antibody. An alignment of several human TCR constant domains is also shown (SEQ ID NOS:224-230).

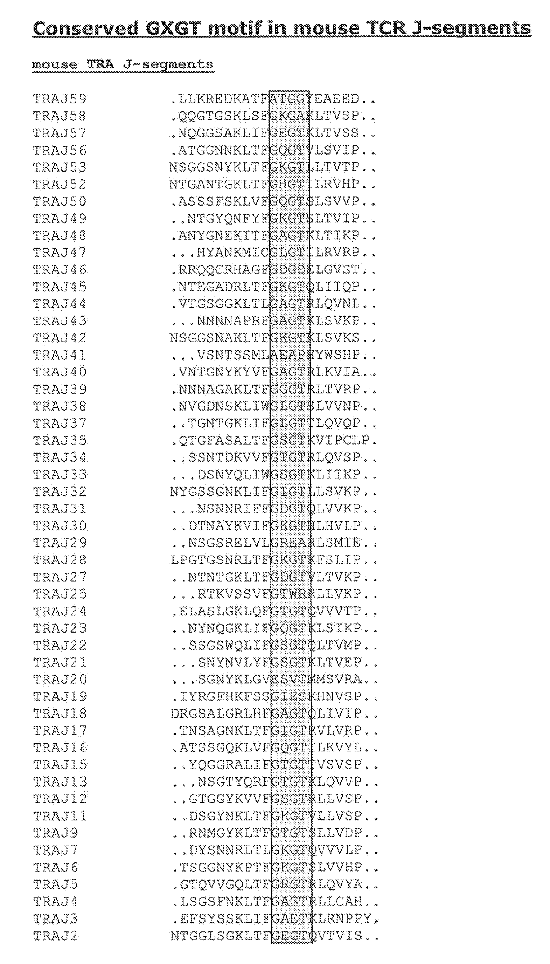

FIG. 8 illustrates the conserved motifs in nurse shark heavy chain (IgH) J-segments (SEQ ID NOS:231-282) and nurse shark IgI J-segments (SEQ ID NOS:283-288).

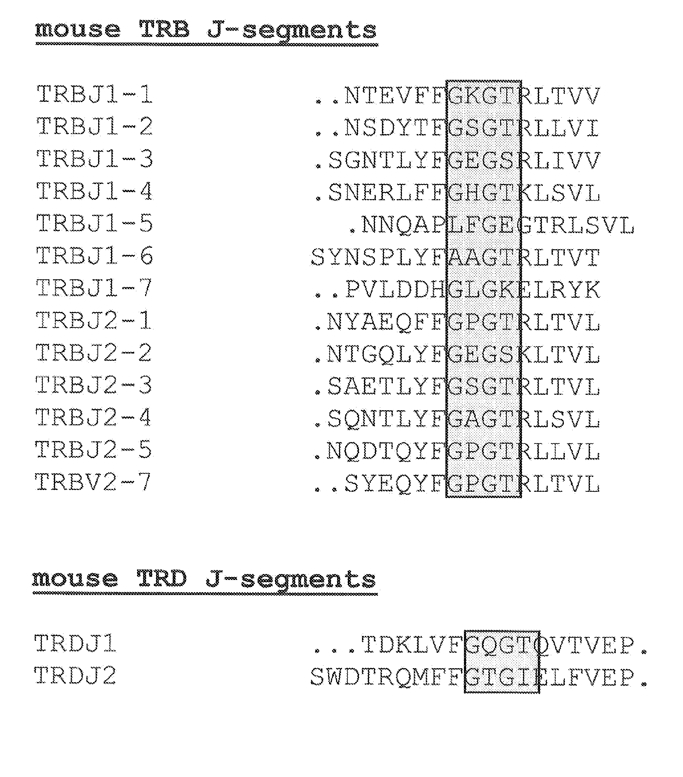

FIGS. 9A and 9B illustrate a conserved motif in mouse TCR J-segments. Amino acid sequence alignments of mouse TCRα J-segments (SEQ ID NOS:289-338), mouse TCRβ J-segments (SEQ ID NOS:339-351) and mouse TCRδ J-segments (SEQ ID NOS:352-353) are shown.

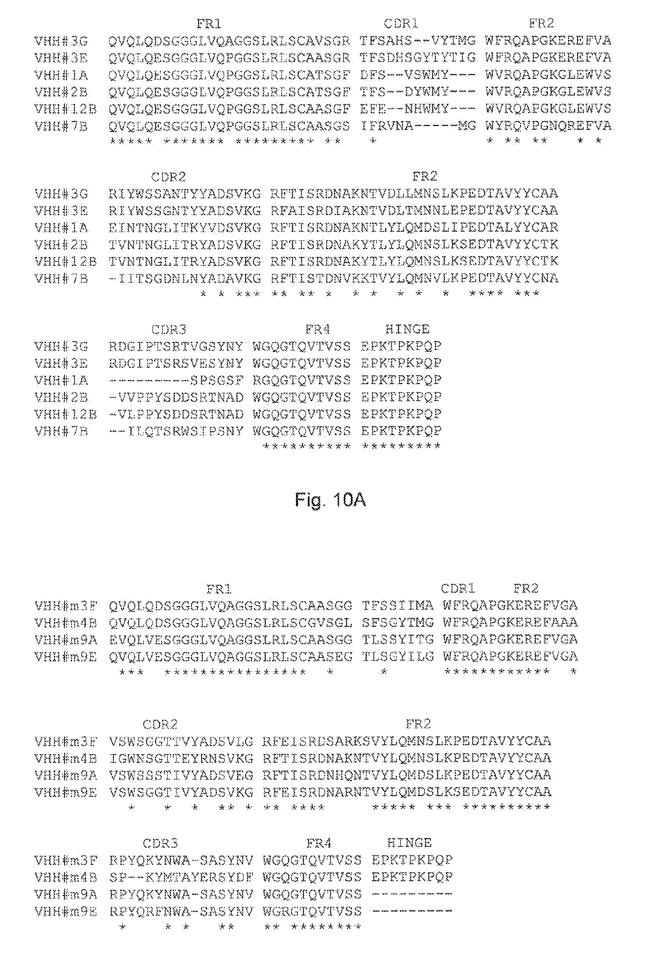

FIGS. 10A and 10B are alignments of the amino acid sequences of several Camelid VHHs (SEQ ID NOS:354-383), and show conserved motifs present in the VHHs (marked with *).

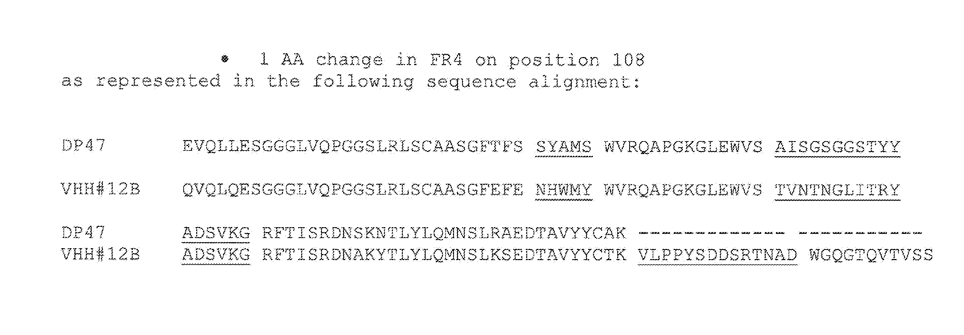

FIG. 11 is an alignment of the germline amino acid sequence of human DP-47 variable domain (SEQ ID NO:384), and the amino acid sequence of Camelid VHH#12B variable domain (SEQ ID NO:385). The alignments reveal that there are 4 amino acid differences in FR1 (positions 1, 5, 28 and 30), 5 amino acid differences in FR3 (positions 74, 76, 83, 84 and 93), and that there are amino acid motifs that are conserved in the sequences.

DETAILED DESCRIPTION OF THE INVENTION

Within this specification embodiments have been described in a way which enables a clear and concise specification to be written, but it is intended and will be appreciated that embodiments may be variously combined or separated without parting from the invention. To enable the invention to be described clearly and concisely, this specification contains formulae that represent partial structures of the disclosed fusion proteins. These formulae depict portions of the fusion protein that are located amino terminally to carboxy terminally (from left to right in the formulae) as is conventional in the art.

Within this specification, the term "about" is preferably interpreted to mean optionally plus or minus 50%, more preferably optionally plus or minus 20%, even more preferably optionally plus or minus 10%, even more preferably optionally plus or minus 5%, even more preferably optionally plus or minus 2%, even more preferably optionally plus or minus 1%.

"Fusion protein" is a term of art that refers to a continuous polypeptide chain that contains parts or portions that are derived from different parental amino acid sequences (*e.g.*, proteins). The portions of a fusion protein can be directly bonded to each other or indirectly bonded through, for example, a peptide linker. A fusion protein can contain two or more portions that are derived from two or more different polypeptides.

As used herein "junction" refers to the site at which two amino acid sequences that are derived from two different polypeptides are joined in a fusion protein.

As used herein a "natural junction" refers to a junction in a fusion protein that has an amino acid sequence that is the same as the amino acid sequence found at the corresponding position of one or both of the parental polypeptides.

For example, as illustrated herein in Scheme 1 using hypothetical parental proteins X and Y, a fusion protein can be prepared that contains the conceptual amino acid sequence XXXXX11111111111YYYYY, in which XXXXXX are amino acids derived from parental protein X, YYYYYY are amino acids derived from parental protein Y, and 11111111111 is a conserved amino acid motif present in both parental proteins. The fusion protein contains a natural junction because the amino acid sequence XXXXX11111111111 is the same as the amino acid sequence at the corresponding location in parental protein X. In this example, the fusion protein contains two natural junctions because the amino acid sequence 11111111111YYYYYY is also the same as the amino acid sequence at the corresponding location in parental protein Y.

As used herein, "immunoglobulin variable domain" refers to antibody variable domains and TCR variable domains. An immunoglobulin variable domain can be derived from an antibody or TCR of desired origin (e.g., of human origin) or from a library prepared using antibody variable region genes or TCR variable region genes, such as human antibody variable region genes or human TCR variable region genes. See, *e.g.,* Kabat, E.A. et al., Sequences of Proteins of Immunological Interest, Fifth Edition, U.S. Department of Health and Human Services, U.S. Government Printing Office (1991).

As used herein, "immunoglobulin constant domain" refers to antibody constant domains (*e.g.*, CH1, hinge, CH2, CH3) and TCR constant domains. An immunoglobulin constant domain can be derived from an antibody or TCR of desired origin (*e.g.*, of human origin) or by any suitable method using readily available antibody constant domain sequence information. See, *e.g.*, Kabat, E.A. et al., Sequences of Proteins of Immunological Interest, Fifth Edition, U.S. Department of Health and Human Services, U.S. GovernmentPrinting Office (1991).

As used herein, "human" refers to *Homo sapiens* and to polypeptides, and portions of polypeptides, of human origin. Such polypeptides or portions thereof are substantially non-immunogenic in humans. Human polypeptides and portions of human polypeptides include polypeptides or portions that contain the same amino acid sequence as a polypeptide or portion thereof that occurs naturally in a human. Human polypeptides or portions thereof can be produced using any suitable method, and include polypeptides or portions thereof that are isolated from a human (*e.g.*, of sample obtained from a human), and those that are produced recombinantly or synthetically.

As used herein, "human immunoglobulin variable domain," "human antibody variable domain" (*e.g.,* human $V_H$, human $V_L$, human $V_\kappa$, human $V_\lambda$, and the like), "human TCR variable domain" refer to variable domains in which one or more framework regions are encoded by a human germline immunoglobulin gene segment, or that have up to 5 amino acid differences relative to the amino acid sequence encoded by a human germline immunoglobulin gene segment. Immunoglobulin variable domains contain hypervariable regions (*e.g.*, CDR1, CDR2, CDR3) which by their nature contain diverse amino acid sequences. In accordance with accepted standards in the immunoglobulin arts, the presence of amino acids in hypervariable regions that are not encoded by the human germline does not render an immunoglobulin variable domain non-human. Human immunoglobulin variable domains can contain one or more CDRs that are not encoded by the human germline, and can additionally contain up to 10 additional amino acids that are not in the CDRs and are not encoded by the human germline. Preferably, the amino acid sequences of FW1, FW2, FW3 and FW4 are each encoded by a human germline immunoglobulin gene segment, or collectively contain up to 10 amino acid differences relative to the amino acid sequences of the corresponding framework regions encoded by the human germline immunoglobulin gene segment.

As used herein "hybrid domain" refers to a recombinant domain that comprises a portion from a first domain of the same type and a portion from a second domain of the same type. For example, a hybrid antibody variable domain can comprise FR1-CDR1-FR2-CDR2-FR3-CDR3 and a portion of FR4 from a Vκ, and a portion of FR4 from an antibody heavy chain variable domain. Domains of the same type include immunoglobulin variable domains (*e.g.*, antibody light and heavy chain variable domains, and TCR variable domains) and immunoglobulin constant domains (e.g., antibody light and heavy chain constant domains, TCR constant domains).

As used herein "conserved amino acid motif" refers to a region containing one to about 50 contiguous amino acids with conserved amino acid sequence that is present in one or more polypeptides, and in certain fusion proteins of the invention that contain portions derived from such polypeptides. The amino acid sequences of the conserved amino acid motif may or may not be identical in individual polypeptides that contain the conserved amino acid motif. As is known in the art, amino acid sequence motifs may differ in amino acid sequence to some degree, but the overall sequence diversity of an amino acid motif is limited by the presence of invariant amino acid residues, and of positions with limited variation, such as conservative amino acid substitutions. Conserved amino acid motifs, such as the GlyXaaGlyThr (SEQ ID NO:386) motif present in framework 4 of immunoglobulin variable domains from many species, can be identified in the conventional manner by alignment of amino acid sequences. Preferably, the amino acid sequences of the conserved amino acid motifs present in two or more polypeptides have at least about 50%, at least about 60%, at least about 70%, at least about 75%, at least about 80%, at least about 85%, at least about 90%, at least about 95%, at least about 96%, at least about 97%, at least about 98%, or at least about 99% amino acid sequence similarity or identity to each other over the length of the motif.

As used herein, a first amino acid, amino acid sequence or motif is "adjacent" to a second amino acid, amino acid sequence or motif when the first amino acid sequence or motif is peptide bonded directly to the second amino acid sequence or motif to create a continuous polypeptide chain.

Amino acid and nucleotide sequence alignments and homology, similarity or identity, as defined herein are preferably prepared and determined using the algorithm BLAST 2 Sequences, using default parameters (Tatusova, T. A. et al.., FEMS Microbiol Lett, 174:187-188 (1999)). Alternatively, the BLAST algorithm (version 2.0) is employed for sequence alignment, with parameters set to default values. BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul, Proc. Natl. Acad. Sci. USA 87(6):2264-8 (1990).

The invention relates to recombinant fusion proteins that contain natural junctions. The fusion proteins of the invention generally comprises a conserved amino acid sequence motif that is present in two polypeptides that are to be fused. The amino acid sequence that is adjacent to the amino-terminus of the conserved motif is the same as the amino sequence that is adjacent to the amino-terminus of the conserved motif in one of the original polypeptides, and the amino acid sequence that is adjacent to the carboxy-terminus of the conserved motif is the same as the amino acid sequence that is adjacent to the carboxy-terminus of the conserved motif in the other original polypeptide.

The fusion proteins of the invention provide several advantages over conventional fusion proteins. For example, domain interactions in proteins make important contributions to the stability (*e.g.*, aggregation resistance, protease resistance) of proteins. However, domain interaction in fusion proteins are frequently altered because the components of conventional fusion proteins are typically fused at domain boundaries. The resulting juxtaposition of domains from different parental proteins can result in low stability.

One feature of fusion proteins that contain natural junctions is that they generally are designed to preserve domain interactions, thereby improving stability and reducing immunogenicity of the fusion protein. Preferably, in some embodiments, the potential for domain repulsion is reduced in the fusion proteins of the invention, which also reduces susceptibility to proteolysis. A related common problem with conventional fusion proteins is that during production, a fraction of the recombinant protein usually forms soluble or insoluble aggregates, lowering the yield of desired soluble monomeric fusion proteins. The improved stability of the fusion proteins of the invention can also or alternatively result in less aggregation, improved expression and/or improved production yields. Fusion proteins that contain natural junctions also provide advantages for use as *in vivo* therapeutic or diagnostic agents, because they have reduced potential for immunogenicity when the parental polypeptides are from the same species as the patient.

Conventional fusion proteins contain non-self sequences due to the juxtaposition of amino acid sequences from different parental proteins. These sequences do not occur naturally and can be immunogenic (*e.g.*, form B cell epitopes, form T cell epitopes). Consequently, conventional fusion proteins can induce an immune response in patients. Immunogenicity is an important aspect that can limit or prevent *in vivo* use of fusion proteins. Immunogenicity occurs, for example, when epitopes on a recombinant fusion protein stimulate cellular (T cell) immune responses. T cell epitopes consist of linear peptides that are usually 8 to 11 amino acids in length. Thus, as described herein, recombinant fusion proteins can be designed and produced that have desired biological functions, but a reduced number of or no T epitopes in comparison to fusion proteins prepared using conventional methods.

In order to function as T cell epitopes, peptides derived from recombinant proteins must fulfill several requirements. They must survive intracellular proteolytic processing and must be able to bind to a host's major histocompatability molecules (e.g., human HLA molecules). Another factor that influences whether a peptide is recognized as a T cell epitope is the extent of self. Importantly, T cells directed at epitopes belonging to self proteins are tolerized or eliminated during thymic development (See, *e.g.*, Rosmalen et al., 2002). However, some auto-specific T cells persist in the periphery, where they are suppressed by CD4(+) CD25(+) regulatory T cells (See, e.g., Papiernik, 2001, and Shevach et al., 2001). When fusion proteins that contain T cell epitopes are administered tolerance can be breeched. In this situation, foreign and even self-peptides derived from a fusion protein can induce an immune response. It is therefore desirable to reduce the number of T cell epitopes in fusion proteins. As described herein, this can be accomplished by maximizing the extent of "self' within any given continuous peptide sequence found within a recombinant fusion protein.

Recombinant fusion proteins made up of two or more portions (*e.g.*, domains) that do not occur next to one another in naturally occuring proteins, comprise junctions that connect the portions. Since the portions are not connected in their native context, such junctions commonly comprise a non-self amino acid sequence motif at the junction (the site where the switch occurs from one native peptide sequence to another). This type of junction includes two amino acids that are not normally adjacent within their native context. Therefore, a peptide spanning such a junction is a non-self peptide and has the potential to act as an epitope for T cells. Using the approach described herein, the junction is designed to reduce or eliminate the potential to act as an epitope for T cells. The approach described herein is illustrated conceptually in the following schemes in which a fusion protein is produced that contains a portion derived from hypothetical protein X and a portion derived from hypothetical protein Y.

Protein X has the following sequence:

...XXXXXX11111111111XXXXXXX2XXXXXXXXX - XXXXXXX333X3XXXXXXXX...

Protein Y has the following sequence:

...YYYYYY11111111111YYYYYYY2YYYYYYYYY - YYYYYYYY333Y3YYYYYYYY...

In each of the conceptual protein sequences, "-" denotes the boundary between N-terminal and C-terminal domains within protein X an protein Y. A conventional fusion protein in which the amino terminal domain of protein X is fused to the carboxy-terminal domain of protein Y at the native domain boundary is illustrated in Scheme 1. This type of junction includes two amino acids that are not normally adjacent within their native contex (x - y)t. Therefore, a peptide spanning such a junction is a non-self peptide and has the potential to act as an epitope for T cells.

## Scheme 1

```
....XXXXXX11111111111XXXXXXX2XXXXXXXX  -  YYYYYYYYY333Y3YYYYYYYY....
```

As shown in the Schemes 2-4, one application of the invention involves fusion proteins in which a domain from a first polypeptide is to be fused to a domain from a second polypeptide. To prevent the creation of potential new T-cell epitopes, the junction is moved away from the native domain boundary by one or more amino acids (either N-terminally or C-terminally) to an amino acid sequence motif that is conserved in both domains that are to be fused. Since the conserved amino acid motif representing the new fusion site is found in both parental domains, peptides that could be produced *in vivo* that span the new junction have fewer or no amino acids that are not normally adjacent in the parental proteins, and consequently have reduced potential to function as T cell epitopes.

## Scheme 2

```
                          |      ≥1aa       |
....XXXXXX11111111111YYYYYYY2YYYYYYYYY  -  YYYYYYYYY333Y3YYYYYYYY....
```

For example, as illustrated in Scheme 2, a fusion protein comprising a domain from protein X and a domain from protein Y can be prepared. In this example, proteins X and Y each contain a conserved amino acid sequence motif (underlined). This shared motif is the fusion site, any peptide spanning the new domain fusion site that might potentially be a T cell epitope would be entirely self, with regard to the N-terminal domain and/or with regard to the C-terminal domain, thereby eliminating the possibility of being recognized as non-self by T cells.

## Scheme 3

```
                        | ≥1aa |
....XXXXXX11111111111XXXXXXX2YYYYYYYY  -  YYYYYYYYY333Y3YYYYYYYY....
```

In another example, the conserved amino acid motif representing the new domain fusion site could be 1 amino acid in length, so that any peptide spanning the boundaries of the two domains in the fusion protein that might potentially be a T cell epitope would not contain any amino acids that are not found adjacent in the native context of domain boundary in parental protein Y (Scheme 3).

## Scheme 4

```
                                                  |   ≥1aa   |
....XXXXXX11111111111XXXXXXX2XXXXXXX  -  XXXXXXXXX333Y3YYYYYYYY....
                            or
....XXXXXX11111111111XXXXXXX2XXXXXXX  -  XXXXXXXXX333X3YYYYYYYY....
```

As shown in Scheme 4, in some examples, the conserved amino acid motif is 2-10 amino acids in length and the amino acid sequence of the conserved amino acid motif is not identical in the two parental polypeptides.

APPLICATION TO FUSION OF A Vκ DOMAIN TO A CH1 DOMAIN

Additionally, domain interactions are important for the integrity and function of many proteins, including proteins and fusion proteins that contain an immunoglobulin fold. For example, the interactions between immunoglobulin variable and constant domains play an important role in the structure of IgGs (See, e.g., Rothlisberger et al., 2005). To produce fusion proteins that contain immunoglobulin domains, or portions of immunoglobulin domains, it is important to take into consideration the protein-protein interactions that these domains participate in within their native context. For example, the interactions between Vκ and Cκ differ from those between VH and CH1 in immunoglobulins, suggesting that it is potentially problematic to generate Vκ-CH1 or VH-Cκ fusion proteins. However, for some applications it is desirable to generate IgG-like molecules comprising 4 Vκ variable domains, Fab' fragments comprising 2 Vκ variable domains, or "inside-out" molecules similar to those described by Morrison et al. (1998) and Chan et al. (2004). Such work would require generating fusion proteins of Vκ and CH1 domains.

The structure of a typical human Fab' fragment is shown in FIG. 1A, which represents the structure 1VGE, published by Chacko et al. (1996). Lesk and Chothia reported (1988) that the interactions between domains VH and CH1 are determined significantly by 3 highly conserved residues in VH (H11 [Leu or Val], H110 [Thr] and H112 [Ser]) and 2 highly conserved residues in CH1 (H148 [Phe] and H149 [Pro]). This cluster of 5 residues, illustrated in FIG. 1B, provides a degree of controlled flexibility (termed elbow motion) that changes the orientation of Vκ or $V_H$ domains relative to Cκ or CH1 domains in immunoglobulins, respectively. This domain boundary can contribute to the functionality of some antibodies (Landolfi et al. 2001). In addition, the hydrophobic side chain of the conserved residue H108 (Leu) is located at the VH-CH1 interface and may participate in hydrophobic interactions between VH and CH1.

If a Vκ-CH1 fusion were prepared simply by joining an entire Vκ domain (up to residue L108 or L109) to a CH1 domain (from residue H114 [Ala]), 3 of the above 4 conserved residues that CH1 naturally interacts with would not be present in the new variable domain. Residue H11 (Leu / Val) is conserved between many VH and Vκ domains, but residues H108 (Leu), H110 (Thr) and H112 (Ser) are not. This would result in the loss of the conserved VH-CH1 domain interface at the variable domain constant domain boundary, in particular, the loss of hydrophobic interactions. Furthermore, this could result in the loss of a hydrogen bond that may exist between the side chain of residue H112 (Ser) and the backbone nitrogen of residue H114 (Ala), as it does in the example of the Fab structure 1VGE (FIG. 1A). In addition to the loss of residues that stabilize the VH-CH1 interface, new residues would be introduced that would potentially destabilize the fusion protein. Charged residues would be present in the C-terminal portion of the new variable domain, for example L103 (Lys), L107 (Lys) or L108 (Arg). These charged Vκ residues might cause repulsion between Vκ and CH1 at the variable domain-constant domain interface and prevent good domain packing.

Using Swiss-PdbViewer (version 3.7) and the GROMOS96 43B1 parameter set (van Gunsteren et al. 1996), it was determined that the C-terminal VH residues H108 to H113 (sequence LeuValThrValSerSer) in the human Fab structure 1VGE contribute -100.953 KJ/mol of to the total energy of the molecule. If these residues are replaced by the sequence LysValGluIleLysArg (a sequence commonly representing the C-terminal Vκ residues L103 to L108), the contribution to the total energy of the molecule would be +57.4 kJ/mol. This indicates that a Fab fragment could be significantly destabilized by replacing an entire VH domain with an entire Vκ domain which results in replacement of the C-terminal VH residues H108 to H113. Furthermore, any introduced charged Vκ residues would be prone to proteolysis in a context in which they are not accommodated by interactions with Cκ that they naturally participate in when found in their native context of a Vκ-Cκ junction.

In accordance with the invention, a Vκ-CH1 fusion protein can be generated by joining the N-terminal portion of a Vκ domain to the C-terminal portion of a VH domain in such a manner that the fusion site becomes the GlyXaaGlyThr (SEQ ID NO:386) motif that is conserved between Vκ (residues L99 - L102) and VH (residues H104 - H107). In this way, all 4 of the 4 conserved residues that CH1 naturally interacts with can be present in the new variable domain. Residue H11 (Leu / Val) is already conserved between many VH and Vκ domains, and residues H108 (Leu), H110 (Thr) and H112 (Ser) would also be present as the fusion site has been moved toward the N-terminus of Vκ, and residues H104 to H113 would be VH residues. This natural junction would preserve the VH-CH1 domain interface, including preservation of the elbow joint, and preservation of hydrophobic interactions and of hydrogen bonding, to a greater extent than if an entire Vκ domain (up to residue L108 / L109) were simply joined to a CH1 domain (from residue H114). In addition, the natural junction would avoid the repulsion and susceptibility to proteolysis potentially caused by the presence of charged Vκ residues in the region L103 - L108).

APPLICATION TO FUSION OF A VH DOMAIN TO A Cκ DOMAIN

Typical interactions found between Vκ and Cκ domains and also seen in 1VGE are highlighted in FIG. 1C. In particular, the Vκ-Cκ interface is stabilised by hydrogen bonding between the side chain of Vκ residue L103 (Lys) and Cκ residue L165 (Glu) and by hydrogen bonding between the side chain of Vκ residue L108 (Arg, in humans partially encoded by the Jκ exon and partially encoded by the Cκ exon) and Cκ residues L109 (Thr) and L170 (Asp). In addition, residue L106 (Ile) also participates, via its backbone nitrogen and oxygen, in hydrogen bonding with the side chain of Cκ residue L166 (Gln).

If a Vκ-CH1 fusion were prepared by simply joining an entire VH domain (up to residue H113 (Ser) to a Cκ domain (from residue L108 [Arg] or residue L 109 [Thr]), the above interactions would be lost (or could be modified, in the case of backbone interactions). Using Swiss-PdbViewer (version 3.7) and the GROMOS96 43B1 parameter set (van Gunsteren et al. 1996), it was determined that the C-terminal Vκ residues L103 to L108 (sequence LysValGluIleLysArg (SEQ ID NO:541)) in the human Fab structure 1VGE contributed -309.32 KJ/mol to the total energy of the molecule. If these residues are replaced by the sequence LeuValThrValSerSer (SEQ ID NO:421) (a sequence commonly representing the C-terminal $V_H$ residues H108 to H113), the contribution to the total energy of the molecule would be -5.202 kJ/mol. This indicates that a Fab fragment could be significantly destabilised by replacing an entire Vκ domain with an entire VH domain, which would result in replacement of C-terminal Vκ residues L103 to L108.

In accordance with the invention, a VH-Cκ fusion protein can be generated by joining the N-terminal portion of the VH domain to the C- terminal portion of the Vκ domain in such a manner that the fusion site becomes the GlyXaaGlyThr (SEQ ID NO:386) motif that is conserved between Vκ (residues L99 - L102) and VH (residues H104 - H107). In this way, the residues that Cκ naturally interacts with can be present in the new variable domain. This natural domain junction should result in a fusion protein with significantly better properties than the fusion protein with an unnatural domain junction.

FUSION PROTIENS

The fusion proteins of the invention comprise at least two portions derived from two different polypeptides, and at least one natural junction between the two portions. If desired, the fusion protein can contain three or more portions, and some of the junctions between portions can be non-natural. In one aspect, the recombinant fusion protein comprises a hybrid domain. The hybrid domain comprises a first portion (amino acid sequence) that is derived from a first polypeptide, a second portion (amino acid sequence) that is derived from a second polypeptide, and a conserved amino acid motif that is present in the first polypeptide and the second polypeptide. The first polypeptide will comprise a domain that has the formula (X1-Y-X2), and the second polypeptide will comprise a domain that has the formula (Z1-Y-Z2), and the fusion protein will comprise a hybrid domain that has the formula (X1-Y-Z2).

In the above formulae,

Y is a conserved amino acid motif;

X1 and Z1 are the amino acid motifs that are located adjacent to the amino-terminus of Y in the first polypeptide and the second polypeptide, respectively;

X2 and Z2 are the amino acid motifs that are located adjacent to the carboxy-terminus of Y in the first polypeptide and the second polypeptide, respectively;

with the proviso that when the amino acid sequences of X1 and Z1 are the same, the amino acid sequences of X2 and Z2 are not the same; and when the amino acid sequences of X2 and Z2 are the same, the amino acid sequences of X1 and Z1 are not the same.

The number of amino acids represented by X1, X2, Z1 and Z2 is dependent on the size of the hybrid domain, and the size of the domains in the parental polypeptides. Generally, X1, X2, Z1 and Z2 each, independently, consist of about 1 to about 400, about 1 to about 200, about 1 to about 100, about 1 to about 50, about 1 to about 40, about 1 to about 30, about 1 to about 20, about 1 to about 15, about 1 to about 10, about 1 to about 6, about 15, about 14, about 13, about 12, about 11, about 10, about 9, about 8, about 7, about 6, about 5, about 4, about 3, about 2 or 1 amino acid. Similarly, the size of the hybrid domain can vary, and is depend on the size of the domains that contain Y in the parental proteins. The overall size of the hybrid domain can be about 75 to about 400, about 75 to about 350, about 75 to about 300, about 75 to about 250, about 75 to about 150, about 75 to about 125, about 75 to about 100 or about 75 amino acids. In particular embodiments, the hybrid domain is about the size of an immunoglobulin variable domain or immunoglobulin constant domain. In some embodiments, the hybrid domain is about 1 kDa to about 25 kDa, about 5 kDa to about 25 kDa, about 5 kDa to about 20 kDa, about 5 kDa to about 15 kDa, about 6 kDa, about 7 kDa, about 8 kDa, about 9 kDa, about 10 kDa, about 11 kDa, about 12 kDa, about 13 kDa or about 14 kDa.

The conserved amino acid motif Y can consist of one to about 50 amino acid residues. In certain embodiments, Y consists of about 3 to about 50 amino acids, about 3 to about 40 amino acids, about 3 to about 30 amino acids, about 3 to about 20 amino acids, about 3 to about 15 amino acids, about 3 to about 14 amino acids, about 3 to about 13 amino acids, about 3 to about 12 amino acids, about 3 to about 11 amino acids, about 3 to about 10 amino acids, about 3 to about 9 amino acids, about 3 to about 8 amino acids, about 3 to about 7 amino acids, about 3 to about 6 amino acids, about 3 to about 5 amino acids, at least about 8 amino acids, up to about 11 amino acids, or about 8 to about 11 amino acids. In other embodiments, Y consists of about 1 to about 11 amino acids, about 15 amino acids, about 14 amino acids, about 13 amino acids, about 12 amino acids, about 11 amino acids, about 10 amino acids, about 9 amino acids, about 8 amino acids, about 7 amino acids, about 6 amino acids, about 5 amino acids, about 4 amino acids, about 3 amino acids, about 2 amino acids, or about 1 amino acid.

The conserved amino acid motif Y is found in two or more parental polypeptides, of which at least a portion is incorporated into a fusion protein of the invention. The fusion protein of the invention, and the hybrid domain in the fusion protein, can contain portions from any desired parental polypeptides provided that each parental protein contains a conserved amino acid motif. For example, the parental polypeptides can be unrelated (e.g., from different protein superfamilies) or related (e.g., from the same protein superfamily). In certain embodiments, the fusion protein and hybrid domain contains portions derived from parental polypeptides from the same protein superfamily, such as the immunoglobulin superfamily, the tumor necrosis factor (TNF) superfamily or the TNF receptor superfamily.

The parental proteins can be from the same species or from different species. For example, the parental polypeptides can independently be from a human (*Homo sapiens*), or from a non-human species such as mouse, chicken, pig, torafugu, frog, cow (*e.g., Bos taurus*), rat, shark (*e.g.*, bull shark, sandbar shark, nurse shark, homed shark, spotted wobbegong shark), skate (*e.g.*, clearnose skate, little skate), fish (*e.g.*, atlantic salmon, channel catfish, lady fish, spotted ratfish, atlantic cod, chinese perch, rainbow trout, spotted wolf fish, zebrafish), possum, sheep, *Camelid* (*e.g.*, llama, guanaco, alpaca, vicunas, dromedary camel, bactrian camel), rabbit, non-human primate (*e.g.*, new world monkey, old world monkey, cynomolgus monkey (*Macaca fascicularis*), *Callithricidae* (*e.g.*, marmosets)), or any other desired non-human species. In particular embodiments, both parental proteins are human, or one parental protein is human and the other is from a non-human species.

Conserved amino acid motifs can be readily identified using any suitable method, such as by aligning two or more amino acid sequences and identifying regions of conserved amino acid sequence. (See, *e.g.*, FIGS. 2A and 2B/) For example, as described herein, conserved amino acid motifs that are present in immunoglobulin proteins have been identified by alignment of immunoglobulin amino acid sequences. Particular examples of conserved amino acid motifs include: GlyXaaGlyThr (SEQ ID NO:386) or GlyXaaGlyThrXaa(Val/Leu) (SEQ ID NO:387) in framework region (FR) 4 of antibody variable domains; GluAspThrAla (SEQ ID NO:388), ValTyrTyrCys (SEQ ID NO:389), or GluAspThrAlaVal-TyrTyrCys (SEQ ID NO:390) in FR3 of antibody variable domains; (Ser/Ala/Gly)Pro(Lys/Asp/Ser)Val (SEQ ID NO:391), (Ser/Ala/Gly)Pro(Lys/Asp/Ser)ValPhe (SEQ ID NO:392), LysValAspLys(Ser/Arg/Thr) (SEQ ID NO:393), or ValThrVal (SEQ ID NO:394) in antibody constant regions.

The hybrid domain in the fusion protein of the invention can be a hybrid immunoglobulin domain, such as a hybrid immunoglobulin variable domain or a hybrid immunoglobulin constant domain. For example, the fusion protein of the invention can comprise a hybrid T cell receptor variable domain or a hybrid antibody variable domain.

In some embodiments, the hybrid domain is a hybrid immunoglobulin variable domain (*e.g.*, a hybrid antibody variable domain), and Y is located in a framework region (FR), such as FR1, FR2, FR3 or FR 4. In particular examples, Y is in FR4 and is GlyXaaGlyThr (SEQ ID NO:386) or GlyXaaGlyThrXaa(Val/Leu) (SEQ ID NO:387). For example, Y can be GlyXaaGlyThrXaaVal (SEQ ID NO:395) or GlyXaaGlyThrXaaLeu (SEQ ID NO:396). In these embodiments, X1 can be a portion of an antibody variable domain comprising FR1, complementarity determining region (CDR) 1, FR2, CDR2, FR3, and CDR3.

In other particular examples, the hybrid domain is a hybrid immunoglobulin variable domain (*e.g.*, a hybrid antibody variable domain), Y is located in FR3 and is GluAspThrAla (SEQ ID NO:388), ValTyrTyrCys (SEQ ID NO:389), or GluAspThrAlaValTyrTyrCys (SEQ ID NO:390). In these embodiments, X1 can be a portion of an antibody variable domain comprising FR1, CDR1, FR2, and CDR2.

The hybrid domain in the fusion protein of the invention can be a hybrid a immunoglobulin constant domain, such as a hybrid T cell receptor constant domain or a hybrid antibody constant domain. In some embodiments, the hybrid domain is a hybrid immunoglobulin constant domain (*e.g.*, a hybrid antibody constant domain), and Y is located in a constant domain, such as an antibody light chain constant domain (*e.g.*, Cκ, Cλ), or an antibody heavy chain constant domain (*e.g.*, CH1, hinge, CH2, CH3). For example, the hybrid domain can be a hybrid immunoglobulin CH1, CH2, Cκ or Cλ wherein Y is (Ser/Ala/Gly)Pro(Lys/Asp/Ser)Val(SEQ ID NO:391); a hybrid CH1, CH2, or Cκ wherein Y is (Ser/Ala/Gly)Pro(Lys/Asp/Ser)ValPhe (SEQ ID NO:392); a hybrid CH1 wherein Y is LysValAspLys(Ser/Arg/Thr) (SEQ ID NO:393) or ValThrVal (SEQ ID NO:394); or a hybrid TCR constant domain wherein Y is ProSerValPhe (SEQ ID NO:397). In particular embodiments of these examples, Y can be SerProLysVal (SEQ ID NO:398), SerProAspVal (SEQ ID NO:399), SerProSerVal (SEQ ID NO:400), AlaProLysVal (SEQ ID NO:401), AlaProAspVal (SEQ ID NO:402), AlaProSerVal (SEQ ID NO:403), GlyProLysVal (SEQ ID NO:404), GlyProAspVal (SEQ ID NO:405), GlyProSerVal (SEQ ID NO:406), SerProLysValPhe (SEQ ID NO:407), SerProAspValPhe (SEQ ID NO:408), SerProSerValPhe (SEQ ID NO:409), AlaProLysValPhe (SEQ ID NO:410), AlaProAspValPhe (SEQ ID NO:411), AlaProSerValPhe (SEQ ID NO:412), GlyProLysValPhe (SEQ ID NO:413), GlyProAspValPhe (SEQ ID NO:414), GlyProSerValPhe (SEQ ID NO:415), LysValAspLysSer (SEQ ID NO:416), LysValAspLysArg (SEQ ID NO:417), LysValAspLysThr (SEQ ID NO:418), or ValThrVal (SEQ ID NO:394).

The hybrid domain in the fusion protein of the invention can be bonded to an adjacent amino-terminal amino acid sequence, D, and/or be bonded to an adjacent carboxy-terminal amino acid sequence E, such that the recombinant fusion protein comprises a partial structure that has the formula

$$D\text{-}(X1\text{-}Y\text{-}Z2)\text{-}E,$$

wherein D is absent or is an amino acid sequence that is adjacent to the amino-terminus of (X1-Y-X2) in the first polypeptide, and E is absent or is an amino acid sequence that adjacent to the carboxy-terminus of (Z1-Y-Z2) in the second polypeptide.

For example, the fusion protein of the invention can comprise D-(X1-Y-Z2), wherein D is an immunoglobulin variable domain and (X1-Y-Z2) is a hybrid immunoglobulin constant domain. If desired, the fusion proteins can further comprise E and have the formula D-(X1-Y-Z2)-B, wherein D is an immunoglobulin variable domain, (X1-Y-Z2) is a hybrid immunoglobulin constant domain, and E is an immunoglobulin constant domain. As described above, the components of the fusion protein can be derived from parental proteins from any desired species. In this example of the fusion proteins of the invention, D can be an antibody variable region of non-human origin (e.g., from shark, mouse, Camelid), E can comprise a human immunoglobulin constant domain, and the hybrid constant domain (X1-Y-Z2) contains a portion (X1) of a non-human constant domain, a portion (Z2) of a human constant domain, and a conserved amino acid motif (Y) that is present in the non-human constant domain and the human constant domain. In other embodiments, D is absent and the fusion protein comprises a further domain that is amino terminal to (X1-Y-Z2). The further amino terminal domain can be bonded to (X1-Y-Z2) directly or indirectly through a natural junction or a non-natural junction.

In another example, the fusion protein of the invention comprises D-(X1-Y-Z2), wherein D is an immunoglobulin constant domain, and (X1-Y-Z2) is a hybrid immunoglobulin constant domain. If desired, the fusion protein of this example can contain additional components that are amino terminal to (X1-Y-Z2). ). For example, in one embodiment the fusion protein comprises an immunoglobulin variable domain, such as a $V_L$, $V_H$ or $V_{HH}$, that is amino terminal to D. Thus, the fusion protein can have the structure: antibody variable domain-D-(X1-Y-Z2), wherein D is an immunoglobulin constant domain (e.g., an antibody constant domain), and (X1-Y-Z2) is a hybrid immunoglobulin constant domain (e.g., a hybrid antibody constant domain).

In another example, the fusion protein of the invention comprises (X1-Y-Z2)-E, wherein (X1-Y-Z2) is a hybrid immunoglobulin variable domain, and E is an immunoglobulin constant domain. If desired, the fusion protein of this example can contain additional components that are amino terminal to (X1-Y-Z2). For example, in one embodiment the fusion protein comprises another immunoglobulin variable domain, such as a $V_L$, $V_H$ or $V_{HH}$, that is amino terminal to (X1-Y-Z2). Thus, the fusion protein can have the structure: antibody variable domain-(X1-Y-Z2)-E, wherein (X1-Y-Z2) is a hybrid immunoglobulin variable domain (e.g., a hybrid antibody variable domain) and E is an immunoglobulin constant domain (e.g., an antibody constant domain).

In another example, the fusion protein of the invention comprises (X1-Y-Z2)-E, wherein (X1-Y-Z2) is a hybrid immunoglobulin constant domain, and B is an immunoglobulin constant domain. If desired, the fusion proteins can contain additional components that are amino terminal to (X1-Y-Z2). For example, in one embodiment the fusion protein comprises an immunoglobulin variable domain, such as a $V_L$, $V_H$ or $V_{HH}$, that is amino terminal to (X1-Y-Z2). Thus, the fusion protein can have the structure: antibody variable domain-(X1-Y-Z2)-E, wherein (X1-Y-Z2) is a hybrid immunoglobulin constant domain (e.g., a hybrid antibody CH1 domain) and E comprises an immunoglobulin constant domain (e.g., hinge, hinge-CH2, hinge-CH2-CH3).

Some of the fusion proteins of the invention comprises a hybrid immunoglobulin variable domain that is fused to an immunoglobulin constant domain, wherein said hybrid immunoglobulin variable domain comprises a hybrid framework region (FR) that comprises a portion from a first immunoglobulin FR from a first immunoglobulin and a portion from a second immunoglobulin FR from a second immunoglobulin, the first and second immunoglobulins each comprising a conserved amino acid motif. The hybrid FR has the formula

$$(F^1\text{-}Y\text{-}F^2)$$

wherein Y is a conserved amino acid motif;

$F^1$ is the amino acid motif located adjacent to the amino-terminus of Y in the first immunoglobulin FR; and

$F^2$ is the amino acid motif located adjacent to the carboxy-terminus of Y in the second immunoglobulin FR.

The hybrid FR can be a hybrid FR1, hybrid FR2, hybrid FR3 or hybrid FR4. In one example, the first immunoglobulin is an antibody heavy chain, the second immunoglobulin is an antibody light chain, $F^1$ is derived from FR1, FR2, FR3 or FR4 of the antibody heavy chain variable domain, and $F^2$ is derived from the corresponding FR of the antibody light chain variable domain. Thus, the hybrid immunoglobulin domain can comprise FR1, CDR1, FR2, CDR2, FR3, CDR3 and a portion of FR4 ($F^1$) of an antibody heavy chain variable domain, a portion of FR4 ($F^2$) of an antibody light chain variable domain, and a conserved amino acid motif (Y) that is present in FR4 of both the heavy chain and light chain variable domains. In other embodiments, the hybrid immunoglobulin domain can comprise FR1, CDR1, FR2, CDR2, and a portion of FR3 ($F^1$) of an antibody heavy chain variable domain, a portion of FR3, CDR3 and FR4 ($F^2$) of an antibody light chain variable domain, and a conserved amino acid motif (Y) that is present in FR3 of both the heavy chain and light chain variable domains. Similarly, the hybrid immunoglobulin domain can comprise FR1, CDR1, and a portion of FR2 ($F^1$) of an antibody heavy chain variable domain, a portion of FR2 ($F^2$), CDR2, FR3, CDR3 and FR4) of an antibody light chain variable domain, and a conserved amino acid motif (Y) that is present in FR2 both the heavy chain and light chain variable domains. The hybrid immunoglobulin domain can comprise a portion of FR1 ($F^1$) of an antibody heavy chain variable domain, a portion of FR1 ($F^2$), CDR1, FR2, CDR2, FR3, CDR3 and FR4 of an antibody light chain variable domain, and a conserved amino acid motif (Y) that is present in FR1 both the heavy chain and light chain variable domains.

In another example, the first immunoglobulin is an antibody light chain, the second immunoglobulin is an antibody heavy chain, $F^1$ is derived from FR1, FR2, FR3 or FR4 of the antibody light chain variable region, and $F^2$ is derived from the corresponding FR of the antibody heavy chain variable region. Thus, the hybrid immunoglobulin domain can comprise FR1, CDR1, FR2, CDR2, FR3, CDR3 and a portion of FR4 ($F^1$) of an antibody light chain variable domain, a portion of FR4 ($F^2$) of an antibody heavy chain variable domain, and a conserved amino acid motif (Y) that is present in FR4 both the light chain and heavy chain variable domains. In other embodiments, the hybrid immunoglobulin domain can comprise FR1, CDR1, FR2, CDR2, and a portion of FR3 ($F^1$) of an antibody light chain variable domain, a portion of FR3 ($F^2$), CDR3 and FR4 of an antibody heavy chain variable domain, and a conserved amino acid motif (Y) that is present in FR3 both the light chain and heavy chain variable domains. Similarly, the hybrid immunoglobulin domain can comprise FR1, CDR1, and a portion of FR2 ($F^1$) of an antibody light chain variable domain, a portion of FR2 ($F^2$), CDR2, FR3, CDR3 and FR4 of an antibody heavy chain variable domain, and a conserved amino acid motif (Y) that is present in FR2 both the light chain and heavy chain variable domains. The hybrid immunoglobulin domain can comprise a portion of FR1 ($F^1$) of an antibody light chain variable domain, a portion of FR1 ($F^2$), CDR1, FR2, CDR2, FR3, CDR3 and FR4 of an antibody heavy chain variable domain, and a conserved amino acid motif (Y) that is present in FR1 both the light chain and heavy chain variable domains.

The hybrid immunoglobulin variable domain can be fused to any desired immunoglobulin constant domain. Generally, the carboxy-terminus of the hybrid immunoglobulin variable domain is fused directly to the amino terminus of an immunoglobulin constant domain. The fusion protein can comprise additional immunoglobulin constant domains and/or variable domains if desired. For example, a hybrid immunoglobulin variable domain can be fused to C$\lambda$, C$\kappa$, CH1, CH2, CH3, CH1-hinge-CH2-CH3, hinge-CH2-CH3, CH2-CH3, or a T cell receptor constant domain.

In preferred embodiments, the amino acid sequence $F^2$ is adjacent to the amino-terminus of the immunoglobulin constant domain to which the hybrid immunoglobulin variable domain is fused in a naturally occurring protein comprising said immunoglobulin constant domain. For example, when the second polypeptide is a TCR chain and $F^2$ is derived from a TCR FR4, the hybrid immunoglobulin domain is peptide bonded to the amino-terminus of a TCR constant domain. Similarly, when the second polypeptide is an antibody light chain and $F^2$ is derived from an antibody light chain variable region FR4, the hybrid immunoglobulin domain can be peptide bonded to the amino-terminus of an antibody light chain constant domain. In particular embodiments, the second polypeptide is a $\kappa$ or $\lambda$ light chain, $F^2$ is derived from a V$\kappa$ or V$\lambda$ FR4, and the hybrid immunoglobulin domain is bonded to the amino-terminus of C$\kappa$ or C$\lambda$, respectively. When the second polypeptide is an antibody heavy chain and $F^2$ is derived from an antibody heavy chain variable domain FR4, the hybrid immunoglobulin domain can be bonded to the amino-terminus of an antibody heavy chain constant domain. In particular embodiments, the second polypeptide is an antibody heavy chain, $F^2$ is derived from an antibody heavy chain variable domain FR4 (*e.g.*, V$_H$ FR4, V$_{HH}$ FR4), and the hybrid immunoglobulin domain is bonded to the amino-terminus of CH1.

In particular embodiments, the hybrid immunoglobulin variable domain is a hybrid antibody variable domain and Y is GlyXaaGlyThr (SEQ ID NO:386) or GlyXaaGlyThrXaa(Val/Leu) (SEQ ID NO:387). For example, the fusion protein can comprise a hybrid antibody variable domain in which $F^1$ is Phe, Y is GlyXaaGlyThr (SEQ ID NO:386), and $F^2$ is (Leu/Met/Thr)ValThrValSerSer (SEQ ID NO:420). In particular embodiments, $F^2$ is LeuValThrValSerSer (SEQ ID NO:421), MetValThrValSerSer (SEQ ID NO:422), or ThrValThrValSerSer (SEQ ID NO:423). In other examples, the fusion protein can comprise a hybrid antibody variable domain, in which $F^1$ is Phe, Y is GlyXaaGlyThrXaa(Val/Leu) (SEQ ID NO:387), and $F^2$ is ThrValSerSer (SEQ ID NO:419). In particular embodiments, Y is GlyXaaGlyThrXaaVal (SEQ ID NO:395) or GlyXaaGlyThrXaaLeu (SEQ ID NO:396). Preferably the carboxy-terminus of these types of hybrid antibody variable domains is bonded directly to an antibody heavy chain constant domain, such as an IgG (*e.g.*, IgG1, IgG2, IgG3, IgG4) constant domain. Preferably, the antibody heavy chain constant domain is a human antibody heavy chain constant domain. In particular embodiments, the carboxy-terminus of the hybrid antibody variable domain is bonded directly to IgG CH1 or IgG CH2 (*e.g.*, IgG1 CH1, IgG4 CH1, IgG1 CH2, IgG4 CH2).

In other embodiments, the fusion protein comprises a hybrid variable domain in which $F^1$ is Trp, Y is GlyXaaGlyThr (SEQ ID NO:386), and $F^2$ is (Lys/Arg)(Val/Leu)(Glu/Asp)IleLys (SEQ ID NO:424) or (Lys/Gln/Glu)(Val/Leu)(Thr/Ile)(Val/Ile)Leu (SEQ ID NO:425). In particular embodiments, $F^2$ is LysValGluIleLys (SEQ ID NO:426), LysValAspIleLys (SEQ ID NO:427), LysLeuGluIleLys (SEQ ID NO:428), LysLeuAspIleLys (SEQ ID NO:429), ArgValGluIleLys (SEQ ID NO:430), ArgValAspIleLys (SEQ ID NO:431), ArgLeuGluIleLys (SEQ ID NO:432), ArgLeuAspIleLys (SEQ ID NO:433), LysValThrValLeu (SEQ ID NO:434), LysValThrIleLeu (SEQ ID NO:435), LysValIleValLeu (SEQ ID NO:436), LysValIleIleLeu (SEQ ID NO:437), LysLeuThrValLeu (SEQ ID NO:438), LysLeuThrIleLeu (SEQ ID NO:439), LysLeuIleValLeu (SEQ ID NO:440), LysLeuIleIleLeu (SEQ ID NO:441), GlnValThrValLeu (SEQ ID NO:442), GlnValThrIleLeu (SEQ ID NO:443), GlnValIleValLeu (SEQ ID NO:444), GlnValIleIleLeu (SEQ ID NO:445), GlnLeuThrValLeu (SEQ ID NO:446), GlnLeuThrIleLeu (SEQ ID NO:447), GlnLeuIleValLeu (SEQ ID NO:448), GlnLeuIleIleLeu (SEQ ID NO:449), GluValThrValLeu (SEQ ID NO:450), GluValThrIleLeu (SEQ ID NO:451), GluValIleValLeu (SEQ ID NO:452), GluValIleIleLeu (SEQ ID NO:453), GluLeuThrValLeu (SEQ ID NO:454), GluLeuThrIleLeu (SEQ ID NO:455), GluLeuIleValLeu (SEQ ID NO:456), or GluLeuIleIleLeu (SEQ ID NO:457).

In other examples, the fusion protein can comprise a hybrid antibody variable domain, in which $F^1$ is Trp, Y is GlyXaaGlyThrXaaVal (SEQ ID NO:395), and $F^2$ is (Glu/Asp)IleLys (SEQ ID NO:458) or (Thr/Ile)(Val/Ile)Leu (SEQ ID NO:459). In particular embodiments, $F^2$ is GluIleLys (SEQ ID NO:460), AspIleLys (SEQ ID NO:461), ThrValLeu (SEQ ID NO:462), ThrIleLeu (SEQ ID NO:463), IleValLeu (SEQ ID NO:464), or IleIleLeu (SEQ ID NO:465). Preferably the carboxy-terminus of these types of hybrid antibody variable domains is bonded directly to an antibody light chain constant domain, such as Cκ or Cλ Preferably, the antibody light chain constant domain is a human antibody light chain constant domain.

In certain embodiments, the fusion protein that comprises a hybrid immunoglobulin variable domain that is fused to an immunoglobulin constant domain comprises a partial structure that has the formula $(F^1-Y-F^2)$-Cκ, $(F^1-Y-F^2)$-Cλ, $(F^1-Y-F^2)$-CH1, $(F^1-Y-^2)$-CH2 or $(F^1-Y-F^2)$-Fc (*e.g.*, $F^1-Y-F^2$)-Fc-V, wherein the hybrid domain is a heavy chain V domain (*e.g.*, human VH, VHH or camelized VH) and V is a heavy chain V domain (*e.g.*, human VH, VHH or camelized VH), preferably both the hybrid domain and V are both human, both VHH or both camelized VH). The invention also provides dimers of such structures.

In certain embodiments, the fusion protein that comprises a hybrid immunoglobulin variable domain that is fused to an immunoglobulin constant domain further comprises a second immunoglobulin variable domain (e.g., antibody variable domain). The second immunoglobulin domain can be amino terminal or carboxy terminal to the hybrid immunoglobulin variable domain. Preferably, the second immunoglobulin variable domain is amino-terminal to the hybrid immunoglobulin variable domain in the fusion protein.

In some embodiments, the fusion protein of the invention comprises a non-human antibody variable region that is fused to a human antibody constant domain, wherein the non-human antibody variable region contains a hybrid FR4. The fusion protein contains a natural junction between the non-human antibody variable domain and the human antibody constant domain because the fusion site is in FR4 and not at the boundary between the variable domain and human constant domain. The hybrid FR4 has the formula $(F^1-Y-F^2)$.

In some embodiments, $F^1$ is Phe or Trp; Y is GlyXaaGlyThr (SEQ ID NO:386), and $F^2$ is (Leu/Met/Thr)ValThrSerSer (SEQ ID NO:420), (Lys/Arg)(Val/Leu)(Glu/Asp)IleLys (SEQ ID NO:424) or (Lys/Gln/Glu)(Val/Leu)(Thr/Ile)(Val/Ile)Leu (SEQ ID NO:425).

In other embodiments, $F^1$ is Phe or Trp, Y is GlyXaaGlyThrXaa(Val/Leu) (SEQ ID NO:387), and $F^2$ is ThrValSerSer (SEQ ID NO:419), (Glu/Asp)IleLys (SEQ ID NO:458) or (Thr/Ile)(Val/Ile)Leu (SEQ ID NO:459).

In some embodiments, the human antibody constant domain is a CH1 domain, Y is GlyXaaGlyThr (SEQ ID NO:386), and $F^2$ is (Leu/Met/Thr)ValThrValSerSer (SEQ ID NO:420). For example, in particular embodiments, $F^2$ is LeuValThrValSerSer (SEQ ID NO:421), MetValThrValSerSer (SEQ ID NO:422), or ThrValThrValSerSer (SEQ ID NO:423). In other embodiments, the human antibody constant domain is a CH1 domain, Y is GlyXaaGlyThrXaa(Val/Leu) (SEQ ID NO:387), and $F^2$ is ThrValSerSer (SEQ ID NO:418).

In some embodiments, the human antibody constant domain is a light chain constant domain, Y is GlyXaaGlyThr (SEQ ID NO:386), and $F^2$ is (Lys/Arg)(Val/Leu)(Glu/Asp)IleLys (SEQ ID NO:424) or (Lys/Gln/Glu)(Val/Leu)(Thr/Ile)(Val/Ile)Leu (SEQ ID NO:425). For example, in particular embodiments, $F^2$ is LysValGluIleLys (SEQ ID NO:426), LysValAspIleLys (SEQ ID NO:427), LysLeuGluIleLys (SEQ ID NO:428), LysLeuAspIleLys (SEQ ID NO:429), ArgValGluIleLys (SEQ ID NO:430), ArgValAspIleLys (SEQ ID NO:431), ArgLeuGluIleLys (SEQ ID NO:432), ArgLeuAspIleLys (SEQ ID NO:433), LysValThrValLeu (SEQ ID NO:434), LysValThrIleLeu (SEQ ID NO:435), LysValIleValLeu (SEQ ID NO:

436), LysValIleIleLeu (SEQ ID NO:437), LysLeuThrValLeu (SEQ ID NO:438), LysLeuThrIleLeu (SEQ ID NO:439), Lys-LeuIleValLeu (SEQ ID NO:440), LysLeuIleIleLeu (SEQ ID NO:441), GlnValThrValLeu (SEQ ID NO:442), GlnValThrIleLeu (SEQ ID NO:443), GlnValIleValLeu (SEQ ID NO:444), GlnValIleIleLeu (SEQ ID NO:445), GlnLeuThrValLeu (SEQ ID NO:446), GlnLeuThrIleLeu (SEQ ID NO:447), GlnLeuIleValLeu (SEQ ID NO:448), GlnLeuIleIleLeu (SEQ ID NO:449), GluValThrValLeu (SEQ ID NO:450), GluValThrIleLeu (SEQ ID NO:451), GluValIleValLeu (SEQ ID NO:452), GluValIleIleLeu (SEQ ID NO:453), GluLeuThrValLeu (SEQ ID NO:454), GluLeuThrIleLeu (SEQ ID NO:455), GluLeuIleValLeu (SEQ ID NO:456), or GluLeuIleIleLeu (SEQ ID NO:457).

In other embodiments, the human antibody constant domain is a light chain constant domain, Y is GlyXaaGlyThrXaa(Val/Leu) (SEQ ID NO:387), and $F^2$ is (Glu/Asp)IleLys (SEQ ID NO:458) or (Thr/Ile)(Val/Ile)Leu (SEQ ID NO:459). For example, in particular embodiments, Y is GlyXaaGlyThrXaaVal (SEQ ID NO:395) or GlyXaaGlyThrXaaLeu (SEQ ID NO:396); and $F^2$ is GluIleLys (SEQ ID NO:460), AspIleLys (SEQ ID NO:461), ThrValLeu (SEQ ID NO:462), ThrIleLeu (SEQ ID NO:463), IleValLeu (SEQ ID NO:464), or IleIleLeu (SEQ ID NO:465).

Some of the fusion proteins of the invention comprise an immunoglobulin variable domain that is fused to a hybrid immunoglobulin constant domain, wherein said hybrid immunoglobulin constant domain comprises a portion from a first immunoglobulin constant domain and a portion from a second immunoglobulin constant domain, the first and second immunoglobulin constant domains each comprising a conserved amino acid motif. The hybrid immunoglobulin constant domain has the formula

$$(C^1\text{-}Y\text{-}C^2)$$

wherein Y is a conserved amino acid motif;
$C^1$ is the amino acid motif located adjacent to the amino-terminus of Y in the first immunoglobulin constant domain; and
$C^2$ is the amino acid motif located adjacent to the carboxy-terminus of Y in the second immunoglobulin constant domain.

The hybrid immunoglobulin constant domain can comprise portions from any two immunoglobulin constant domains that contain a conserved amino acid motif. In certain embodiments, the hybrid immunoglobulin constant domain is a hybrid antibody constant domain that comprises a portion from a first antibody constant domain and a portion from a second antibody constant domain. For example, the hybrid antibody constant domain can be a hybrid CH1, hybrid hinge, hybrid CH2 or hybrid CH3, wherein portions of the hybrid domain are derived from antibody constant domains from different species (*e.g.*, human and non-human, such as *Camelid* or nurse shark) or different isotypes (*e.g.,* IgA, IgD, IgM, IgE, IgG (IgG1, IgG2, IgG3, IgG4)). The hybrid immunoglobulin constant domain can also comprise portions from two different constant domains, such as a portion from a CH1 domain and a portion from a CH2 domain.

In some embodiments, the hybrid antibody constant domain comprises portions that are derived from antibody constant domains of different species. For example, the first antibody constant domain can be a non-human antibody constant domain and the second antibody constant domain can be a human antibody constant domain. Suitable non-human antibody constant domains include those from mouse, chicken, pig, torafugu, frog, cow (*e.g., Bos taurus*), rat, shark (*e.g.*, bull shark, sandbar shark, nurse shark, homed shark, spotted wobbegong shark), skate (*e.g.*, clearnose skate, little skate), fish (*e.g.,* atlantic salmon, channel catfish, lady fish, spotted ratfish, atlantic cod, chinese perch, rainbow trout, spotted wolf fish, zebrafish), possum, sheep, *Camelid* (*e.g.,* llama, guanaco, alpaca, vicunas, dromedary camel, bactrian camel), rabbit, non-human primate (*e.g.*, new world monkey, old world monkey, cynomolgus monkey (*Macaca fascicularis*), *Callithricidae* (*e.g.,* marmosets)), or any other desired non-human species. Preferably, the amino terminus of a hybrid antibody constant domain is directly fused to the carboxy-terminus of an antibody variable domain that is from the same species as the amino terminal $C^1$ of the hybrid antibody constant domain. Preferably, the carboxy-terminal $C^2$ of the hybrid antibody constant domain is derived from a human antibody constant domain. For example, the fusion protein can comprise a partial structure having the formula: non-human V domain-($C^1$-Y- $C^2$), wherein $C^1$ is derived from a non-human constant domain (*e.g.*, Cκ,Cλ, CH1) from the same species as the non-human V domain, Y is a conserved amino acid motif, and $C^2$ is derived from a human antibody constant domain.

In some embodiments, the hybrid antibody constant domain comprises a portion from a first antibody constant domain and a portion from a second antibody constant domain that are from antibodies of different isotypes. For example, in this type of the hybrid antibody constant domain, $C^1$ is a portion from an IgA, IgD, IgM, IgE, or IgG (*e.g.*, IgG1, IgG2, IgG3, IgG4), and $C^2$ is a portion from an antibody constant domain of a different isotype than $C^1$. Preferably, $C^2$ is a portion from an IgG (*e.g.*,IgG1, IgG2, IgG3, IgG4) constant domain. In a particular embodiment, the hybrid antibody constant domain comprises a portion from an IgG1 constant domain and a portion from an IgG4 constant domain. In such embodiments, $C^1$ is from an IgG1 constant domain and $C^2$ is from and IgG4 constant domain, or $C^2$ is from and IgG4 constant domain and $C^2$ is from an IgG1 constant domain.

In some embodiments, the hybrid immunoglobulin constant domain comprises a portion from a first antibody constant domain that is a light chain constant domain, and a portion from a second antibody constant domain that is a heavy chain constant domain. For example, the fusion protein can comprise a light chain antibody variable domain that is fused directly to a hybrid antibody constant domain, wherein the first antibody constant domain is a light chain constant domain and $C^1$ is derived from said light chain constant domain, the second antibody constant domain is a heavy chain constant domain and $C^2$ is derived from said heavy chain constant domain. For example, $C^2$ can be derived from an IgG (*e.g.*, IgG1, IgG2, IgG3, IgG4) constant domain, such as an IgG CH1 (*e.g.,* IgG1 CH1, IgG4 CH1), IgG hinge (*e.g.,* IgG1 hinge, IgG4 hinge), IgG CH2 (*e.g.,* IgG1 CH2, IgG4 CH2), IgG CH3 (*e.g.,* IgG1 CH3 or IgG4 CH3).

In other embodiments, the hybrid immunoglobulin constant domain comprises a portion from a first antibody constant domain that is a heavy chain constant domain, and a portion from a second antibody constant domain that is a light chain constant domain. For example, the fusion protein can comprise a heavy chain antibody variable domain that is fused directly to a hybrid antibody constant domain, wherein the first antibody constant domain is a heavy chain constant domain and $C^1$ is derived from said heavy chain constant domain, and the second antibody constant domain is a light chain constant domain and $C^2$ is derived from said light chain constant domain. In particular embodiments, the first antibody constant domain is a CH1 domain and $C^1$ is derived from said CH1 domain.

In particular embodiments, the hybrid immunoglobulin constant domain comprises a portion from a first antibody constant domain that is a *Camelid* heavy chain constant domain, and a portion from a second antibody constant domain that is a heavy chain constant domain. For example, in some embodiments, the carboxy-terminal ($C^2$) of the hybrid antibody constant domain is derived from a human heavy chain constant domain. If desired, the fusion protein can comprise a *Camelid* $V_{HH}$ that is amino-terminal to the hybrid antibody constant domain. For example, in some embodiments, the fusion protein comprises a partial structure having the formula: *Camelid* $V_{HH}$-($C^1$-Y-$C^2$), wherein $C^1$ is derived from a *Camelid* heavy chain constant domain (*e.g., Camelid* CH1), Y is a conserved amino acid motif, and $C^2$ is derived from an antibody heavy chain constant domain (*e.g.*, a human antibody constant domain, such as human CH1).

Some of fusion proteins of the invention comprise an immunoglobulin variable domain (e.g., antibody variable domain) that is fused directly to a hybrid antibody constant domain, wherein said hybrid antibody constant domain comprises a portion from a first antibody constant domain and a portion from a second antibody constant domain, the first and second antibody constant domains each comprising a conserved amino acid motif. The hybrid antibody constant domain has the formula

$$(C^1\text{-}Y\text{-}C^2)$$

wherein Y is a conserved amino acid motif;
$C^1$ is the amino acid motif located adjacent to the amino-terminus of Y in the first antibody constant domain; and
$C^2$ is the amino acid motif located adjacent to the carboxy-terminus of Y in the second antibody constant domain. Preferably, the immunoglobulin variable domain is located amino-terminally to the hybrid antibody constant domain such that the fusion protein comprises a partial structure having the formula: antibody variable domain-($C^1$-Y-$C^2$).

In some embodiments, Y is (Ser/Ala/Gly)Pro(Lys/Asp/Ser)Val (SEQ ID NO:391), (Ser/Ala/Gly)Pro(Lys/Asp/Ser)ValPhe (SEQ ID NO:392), LysValAspLys(Ser/Arg/Thr) (SEQ ID NO:393), or ValThrVal (SEQ ID NO:394). For example, in particular embodiments, Y is SerProLysVal (SEQ ID NO:398), SerProAspVal (SEQ ID NO:399), SerProSerVal (SEQ ID NO:400), AlaProLysVal (SEQ ID NO:401), AlaProAspVal (SEQ ID NO:402), AlaProSerVal (SEQ ID NO:403), Gly-ProLysVal (SEQ ID NO:404), GlyProAspVal (SEQ ID NO:405), GlyProSerVal (SEQ ID NO:406), SerProLysValPhe (SEQ ID NO:407), SerProAspValPhe (SEQ ID NO:408), SerProSerValPhe (SEQ ID NO:409), AlaProLysValPhe (SEQ ID NO: 410), AlaProAspValPhe (SEQ ID NO:411), AlaProSerValPhe (SEQ ID NO:412), GlyProLysValPhe (SEQ ID NO:413), GlyProAspValPhe (SEQ ID NO:414), GlyProSerValPhe (SEQ ID NO:415), LysValAspLysSer (SEQ ID NO:416), Lys-ValAspLysArg (SEQ ID NO:417), LysValAspLysThr (SEQ ID NO:418), or ValThrVal(SEQ ID NO:394). Preferably, the second antibody constant domain is a human antibody constant domain, and $C^2$ is derived from said human antibody constant domain. For example, the human antibody constant domain can be a human Cκ, a human Cλ or a human heavy chain constant domain, such as a human CH1, a human hinge, a human CH2 or a human CH3. In particular preferred embodiments, the human antibody constant domain is an IgG CH1 (*e.g.,* IgG1 CH1, IgG4 CH1), IgG hinge (*e.g.,* IgG1 hinge, IgG4 hinge), IgG CH2 (*e.g.,* IgG1 CH2, IgG4 CH2), or IgG CH3 (*e.g.,* IgG1 CH3 or IgG4 CH3), and $C^2$ is derived from said human antibody constant domain.

In particular embodiments, the fusion protein comprises an antibody light chain variable domain, such as a human light chain variable domain, that is fused to a hybrid antibody CH2 domain, wherein $C^1$ is GlnProLysAla (SEQ ID NO:466) or ThrValAla (SEQ ID NO:467), and Y is (Ala/Gly)ProSerVal (SEQ ID NO:468). In these embodiments, $C^2$ is the amino acid sequence that is adjacent to carboxy-terminus of Y in IgG CH1, such as human IgG CH1 (*e.g.,* IgG1 CH1, IgG4 CH1).

In particular embodiments, the fusion protein comprises an antibody light chain variable domain, such as a human light chain variable domain, that is fused to a hybrid antibody CH2 domain, wherein $C^1$ is GlnProLysAla (SEQ

ID NO:466) or ThrValAla (SEQ ID NO:467), and Y is (Ala/Gly)ProSerVal (SEQ ID NO:468). In these embodiments, $C^2$ is the amino acid sequence that is adjacent to carboxy-terminus of Y in IgG CH2, such as human IgG CH2 (*e.g.*, IgG1 CH2, IgG4 CH2).

In particular embodiments, the fusion protein comprises an antibody heavy chain variable domain, such as a human heavy chain variable domain, that is fused to a hybrid antibody CH2 domain, wherein $C^1$ is SerThrLys (SEQ ID NO:469), and Y is (Ala/Gly)ProSerValPhe (SEQ ID NO:470). In these embodiments, $C^2$ is the amino acid sequence that is adjacent to the carboxy-terminus of Y in IgG CH2, such as human IgG CH2 (*e.g.*, IgG1 CH2, IgG4 CH2).

In particular embodiments, the fusion protein comprises an antibody light chain variable domain, such as a human λ chain variable domain, that is fused to a hybrid antibody Cκ domain, wherein $C^1$ is GlnProLysAla (SEQ ID NO: 466), and Y is (Ala/Gly)ProSerValPhe (SEQ ID NO:470). In these embodiments, $C^2$ is the amino acid sequence that is adjacent to the carboxy-terminus of Y in Cκ, such as human Cκ.

In particular embodiments, the fusion protein comprises an antibody heavy chain variable domain, such as a human heavy chain variable domain, that is fused to a hybrid antibody Cκ domain, wherein $C^1$ is SerThrLys (SEQ ID NO:469), and Y is (Ala/Gly)ProSerValPhe (SEQ ID NO:470). In these embodiments, $C^2$ is the amino acid sequence that is adjacent to the carboxy-terminus of Y in Cκ, such as human Cκ.

In particular embodiments, the fusion protein comprises an antibody light chain variable domain, such as a human κ chain variable domain, that is fused to a hybrid antibody Cλ domain, wherein $C^1$ is ThrValAla (SEQ ID NO: 467), and Y is (Ala/Gly)ProSerVal (SEQ ID NO:468). In these embodiments, $C^2$ is the amino acid sequence that is adjacent to the carboxy-terminus of Y in Cλ, such as human Cλ.

In particular embodiments, the fusion protein comprises an antibody heavy chain variable domain, such as a human heavy chain variable domain, that is fused to a hybrid antibody Cλ domain, wherein $C^1$ is SerThrLys (SEQ ID NO:469), and Y is (Ala/Gly)ProSerVal (SEQ ID NO:468). In these embodiments, $C^2$ is the amino acid sequence that is adjacent to the carboxy-terminus of Y in Cλ, such as human Cλ.

In another aspect, the first portion and the second portion of the recombinant fusion protein of the invention are fused through a linker. The linker can be selected or designed to provide a natural junction between the first portion and the linker, the second portion and the linker or both the first and second portions and the linker. For example, when it is desired that a fusion protein of the invention contain portion (A) from a first polypeptide and portion (B) from a second polypeptide, the fusion protein can comprise a partial structure having the formula (A)-linker-(B), wherein a natural junction exists between (A) and the linker, between the linker and (B), or between (A) and the linker and the linker and (B). When a portion of a polypeptide that is to be included in a fusion protein of the invention is a domain, the linker used in the fusion protein can consist of the one to about 50 contiguous amino acids that are adjacent to the domain in a naturally occurring polypeptide that contains the domain. For example, the linker can consist of 1 to about 40, 1 to about 30, 1 to about 20, 1 to about 15, 1 to about 10, 1 to about 5, about 20, about 19, about 18, about 17, about 16, about 15, about 14, about 13, about 12, about 11, about 10, about 9, about 8, about 7, about 6, about 5, about 4, about 3, about 2 or about 1 amino acids that are adjacent to the domain in a naturally occurring polypeptide that contains the domain. This approach results in improved preservation of domain interactions in the fusion protein, thereby improving stability of the fusion protein.

In this aspect, the fusion protein generally comprises a first portion derived from a first polypeptide and a second portion derived from a second polypeptide, wherein said first polypeptide comprises a structure having the formula (A)-L1, wherein (A) is an amino acid sequence present in said first polypeptide; and L1 is an amino acid motif comprising 1 to about 50 amino acids that are adjacent to the carboxy-terminus of (A) in said first polypeptide. The fusion protein has the formula

$$(A)\text{-}L1\text{-}(B) ;$$

wherein (A) is the portion derived from the first polypeptide; L1 is an amino acid motif comprising 1 to about 50 contiguous amino acids that are adjacent to the carboxy-terminus of (A) in said first polypeptide and provides a linker that connects (A) and (B), and (B) is the portion derived from the second polypeptide. Preferably, (A) is a domain derived from the first polypeptide.

In some embodiments, the first polypeptide comprises (A) and the second polypeptide comprises a structure having the formula L1-(B) wherein L1 is an amino acid motif comprising 1 to about 50 amino acids that are adjacent to the amino-terminus of (B) in the second polypeptide. The fusion protein has the formula

$$(A)\text{-}L1\text{-}(B) ;$$

wherein (A) is the portion derived from the first polypeptide; L1 is an amino acid motif comprising 1 to about 50 contiguous amino acids that are adjacent to the amino-terminus of (B) in said second polypeptide and provides a linker that connects (A) and (B), and (B) is the portion derived from the second polypeptide. Preferably (B) is a domain derived from the second polypeptide.

In preferred embodiments, this aspect includes the proviso that at least one of (A) and (B) is a domain (e.g., (A) is a domain, (B) is a domain, (A) and (B) are both a domain). In other preferred embodiments, this aspect includes the further proviso that when (A) and (B) are both antibody variable domains 1) (A) and (B) are each human antibody variable domains; 2) (A) and (B) are each antibody heavy chain variable domains; 3) (A) and (B) are each antibody light chain variable domains; 4) (A) is an antibody light chain variable domain and (B) is an antibody heavy chain variable domain (e.g, VHH or VH); or 5) (A) is a VHH and (B) is an antibody light chain variable domain. Additionally or alternatively, preferred embodiments of this aspect include the proviso that when (A) is a $V_H$ and (B) is a $V_L$, L1 does not consist of one to five or one to six contiguous amino acids from the amino-terminus of CH1. Additionally or alternatively, when (A) and (B) are both antibody variable domains the following is excluded from the invention, (A)-L1-(B) where (A) is a mouse VH, (B) is a mouse VL and L1 is SerAlaLysThrThrPro (SEQ ID NO:537), SerAlaLysThrThrProLysLeuGlyGly (SEQ ID NO:538), AlaLysThrThrProLysLeuGluGluGlyGluPheSerGluAlaArgVal (SEQ ID NO:539), or AlaLysThrThrProLysLeuGluGlu (SEQ ID NO:540). Additionally or alternatively, (A)-L1-(B) is not a fusion protein wherein (A) is a mouse VH, (B) is a mouse VL and L1 is a linker as disclosed in Le Gall et al., Protein Engineering, Design & Selection, 17:357-366 (2004), Kipriyanov et al., Int. J. Cancer, 77:763-772 (1998); Le Gall et al., J. Immunol. Methods, 285:111-127 (2004); Le Gall et al., FEBS Letters, 453:164-168 (1995); or Kipriyanov et al., Protein Engineering, 10:445-453 (1997).

In particular embodiments, the first polypeptide comprises (A)-L1, and the fusion protein comprises (A)-L1-(B), wherein (A) consists of complementarity determining region (CDR) 3, and L1 consists of framework 4. In other embodiments (A) comprises CDR1 and L1 comprises FR2; (A) comprises CDR2 and L1 comprises FR3; (A) comprises CDR1 and CDR2 (e.g., CDR1-FR2-CDR2) and L1 comprises FR3; (A) comprises CDR2 and CDR3 and L1 comprises FR4; or (A) comprises CDR1, CDR2 and CDR3 (e.g., CDR1-FR2-CDR2-FR3-CDR3) and L1 comprises FR4.

In other embodiments, the first polypeptide comprises (A), the second polypeptide comprises L1-(B) and the fusion protein comprises (A)-L1-(B), wherein (B) consists of CDR 3, and L1 consists of framework 3. In other embodiments (B) comprises CDR1 and L1 comprises FR1; (B) comprises CDR2 and L1 comprises FR2; (B) comprises CDR1 and CDR2 (e.g., CDR1-FR2-CDR2) and L1 comprises FR1; (B) comprises CDR2 and CDR3 and L1 comprises FR2; or (B) comprises CDR1, CDR2 and CDR3 (e.g., CDR1-FR2-CDR2-FR3-CDR3) and L1 comprises FR1.

In some embodiments, (A) is an immunoglobulin variable domain, such as an antibody variable domain. For example, (A) can be an antibody light chain variable domain (e.g., Cκ, Cλ) or an antibody heavy chain variable domain (e.g., $V_H$, $V_{HH}$). In such embodiments, L1 is 1 to about 50 contiguous amino acids that are adjacent to the carboxy-terminus of (A) in a naturally occurring polypeptide that comprises the variable domain A. For example, when (A) is Vκ (e.g., human Vκ), L1 is 1 to about 50 contiguous N-terminal amino acids of Cκ (e.g., human Cκ); when (A) is Vλ (e.g., human Vλ), L1 is 1 to about 50 contiguous N-terminal amino acids of Cλ (e.g., human Cλ), and when (A) is a heavy chain variable domain (e.g., human $V_H$, Camelid $V_{HH}$), L1 is 1 to about 50 contiguous N-terminal amino acids of CH1 (e.g., human CH1, Camelid $V_{HH}$). In some embodiments, (A) is a VH and L1 comprises the first 3 to about 12 N-terminal amino acids of CH1; (A) is a Vκ and 11 comprises the first 3 to about 12 N-terminal amino acids of Cκ; or (A) is a Vλ and L1 comprises the first 3 to about 12 N-terminal amino acids of Cλ.

In some embodiments, the second polypeptide comprises an immunoglobulin constant region, and (B) is derived from the immunoglobulin constant region. For example, (B) can comprise at least a portion of an antibody CH1, at least a portion of an antibody hinge, at least a portion of an antibody CH2, or at least a portion of an antibody CH3.

In some embodiments, (A) is an antibody variable domain, and (B) is an antibody variable domain. In these embodiments, the antibody variable domains (A) and (B) can be the same or different. For example, (A) can be an antibody heavy chain variable domain and (B) can be the same or a different antibody heavy chain variable domain; A) can be an antibody light chain variable domain and (B) can be the same or a different antibody light chain variable domain; A) can be an antibody heavy chain variable domain and (B) can be an antibody light chain variable domain, or A) can be an antibody light chain variable domain and (B) can be an antibody heavy chain variable domain. In exemplary embodiments (A) is a Vκ and (B) is a Vκ; (A) is a Vκ and (B) is a Vλ; (A) is a Vκ and (B) is a VH or a VHH; (A) is a Vλ and (B) is a Vκ; (A) is a Vλ and (B) is a Vλ; or (A) is a Vλ and (B) is a VH or a VHH. In preferred embodiments, this aspect additional or alternatively includes the proviso that when (A) and (B) are both antibody variable domains 1) (A) and (B) are each human antibody variable domains; 2) (A) and (B) are each antibody heavy chain variable domains; 3) (A) and (B) are each antibody light chain variable domains; 4) (A) is an antibody light chain variable domain and (B) is an antibody heavy chain variable domain; or 5) (A) is a VHH and (B) is an antibody light chain variable domain. Additionally or alternatively, preferred embodiments of this aspect include the proviso that when (A) is a $V_H$ and (B) is a $V_L$, L1 does not consist of one to five or one to six contiguous amino acids from the amino-terminus of CH1.

In some embodiments, (A) is an antibody variable domain comprising FR1, CDR1, FR2, CDR3, FR3 and CDR3 of a antibody light chain variable domain and FR4 comprising the amino acid sequence GlyGlnGlyThrLysValThrValSerSer (SEQ ID NO:472); and L1 comprises the first 3 to about 12 amino acids of CH1. In particular embodiments, L1 is AlaSerThr (SEQ ID NO:473), AlaSerThrLysGlyProSer (SEQ ID NO:474), or AlaSerThrLysGlyProSerGly (SEQ ID NO:475).

In other embodiments, (A) is an antibody variable domain comprising FR1, CDR1, FR2, CDR3, FR3 and CDR3 of a $V_H$ or Vκ domain and FR4 comprising the amino acid sequence GlyXaaGlyThr(Lys/Gln/Glu)(Val/Leu)(Thr/Ile)ValLeu (SEQ ID NO:476); and L1 comprises the first 3 to about 12 amino acids of Cλ.

In other embodiments, (A) is an antibody variable domain comprising FR1, CDR1, FR2, CDR3, FR3 and CDR3 of a $V_H$ or Vλ domain and FR4 comprising the amino acid sequence GlyGlnGlyThrLysValGluIleLysArg (SEQ ID NO: 477); and L1 comprises the first 3 to about 12 amino acids of Cκ.

In some embodiments, (A) is an immunoglobulin constant domain, such as an antibody constant domain or a TCR constant domain. In particular embodiments, (A) is an antibody heavy chain constant domain, such as CH1, hinge, CH2, or CH3. In some embodiments (A) is a non-human antibody heavy chain constant domain, such as an antibody constant domain from mouse, chicken, pig, torafugu, frog, cow (*e.g., Bos taurus*), rat, shark (*e.g.,* bull shark, sandbar shark, nurse shark, homed shark, spotted wobbegong shark), skate (*e.g.,* clearnose skate, little skate), fish (*e.g.,* atlantic salmon, channel catfish, lady fish, spotted ratfish, atlantic cod, chinese perch, rainbow trout, spotted wolf fish, zebrafish), possum, sheep, *Camelid (e.g.,* llama, guanaco, alpaca, vicunas, dromedary camel, bactrian camel), rabbit, non-human primate (*e.g.,* new world monkey, old world monkey, cynomolgus monkey (*Macaca fascicularis*), *Callithricidae (e.g.,* marmosets)), or any other desired non-human species. In more particular embodiments, (A) is a non-human constant domain and (B) is derived from a human polypeptide.

In particular embodiments, (B) is derived from the second polypeptide, wherein the second polypeptide is selected from, for example, a cytokine, a cytokine receptor (*e.g.,* an interleukin receptor, such as IL-1R, IL1R Type, a tumor necrosis factor receptor, such as TNF1, TNFR2), a growth factor (*e.g.,* VEGF, EGF, CSF-1), a growth factor receptor (*e.g.,* VEGF-R1, VEGF-R2, EGFR, CSF-1R), a hormone (*e.g.,* insulin), a hormone receptor (*e.g.,* insulin receptor), an adhesion molecule, a haemostatic factor, a T cell receptor, a T cell receptor chain, a T cell receptor variable domain, an enzyme, a polypeptide comprising or consisting of an antibody variable domain, or a functional portion of any one of the foregoing. For example, in some fusion proteins (A) is an immunoglobulin variable domain (*e.g.* antibody variable domain), L1 is 1 to about 50 contiguous amino acids that are adjacent to the carboxy-terminus of (A) in a naturally occurring polypeptide that comprises the variable domain A, and (B) is derived from the second polypeptide, wherein the second polypeptide is selected from, for example, a cytokine, a cytokine receptor (*e.g.,* an interleukin receptor, such as IL-1R, IL1R Type, a tumor necrosis factor receptor, such as TNF1, TNFR2), a growth factor (e.g., VEGF, EGF, CSF-1), a growth factor receptor (e.g., VEGF-R1, VEGF-R2, EGFR, CSF-1R), a hormone (e.g., insulin), a hormone receptor (e.g., insulin receptor), an adhesion molecule, a haemostatic factor, a T cell receptor, a T cell receptor chain, a T cell receptor variable domain, an enzyme, a polypeptide comprising or consisting of an antibody variable domain, or a functional portion of any one of the foregoing.

In other fusion proteins, (A) is derived from the first polypeptide, wherein the first polypeptide is selected from, for example, a cytokine, a cytokine receptor (*e.g.,* an interleukin receptor, such as IL-1R, IL1R Type, a tumor necrosis factor receptor, such as TNFR1, TNFR2), a growth factor (e.g., VEGF, EGF, CSF-1), a growth factor receptor (e.g., VEGF-R1, VEGF-R2, EGFR, CSF-1R), a hormone (e.g., insulin), a hormone receptor (e.g., insulin receptor), an adhesion molecule, a haemostatic factor, a T cell receptor, a T cell receptor chain, a T cell receptor variable domain, an enzyme, a polypeptide comprising or consisting of an antibody variable domain, or a functional portion of any one of the foregoing, L1 is 1 to about 50 contiguous amino acids that are adjacent to the carboxy-terminus of (A) in a naturally occurring polypeptide that comprises (A), and B is an immunoglobulin constant domain. Alternatively, L1 is 1 to about 50 contiguous amino acids that are adjacent to the amino-terminus of (B) in a naturally occurring polypeptide that comprises (B), and (B) is an immunoglobulin constant domain. If desired, the recombinant fusion protein can comprise one or more additional immunoglobulin constant domain that are carboxyl to (B). For example, the fusion protein can comprise an antibody Fc (e.g., optional hinge-CH2-CH3). In further examples, the fusion protein has the structure (A)-L1-CH1-hinge-CH2-CH3; (A)-L1-hinge-CH2-CH3; (A)-L1-CH2-CH3; or (A)-L1-CH3. The constant domains are preferably IgG constant domains, such as IgG1 or IgG4 constant domains.

In particular embodiments, the recombinant fusion protein comprises a first portion derived from an immunoglobulin and a second portion, wherein said first portion is bonded to said second portion through a linker, and the recombinant fusion protein has the formula

$$(A')\text{-}L2\text{-}(B)$$

wherein (A') is an immunoglobulin variable domain and (A') comprises framework (FR) 4 of said immunoglobulin variable domain; L2 is said linker, wherein L2 comprises one to about 50 contiguous amino acids that are adjacent to the carboxy-terminus of said FR4 in a naturally occurring immunoglobulin that comprises said FR4; and (B) is said second portion.

In preferred embodiments, this aspect includes the proviso that (A') is an antibody variable domain, and L2-B is not a $C_L$ or CH1 domain that is peptide bonded to the FR4 of the variable domain (A') in a naturally occurring antibody that contains the FR4,and when (A') and (B) are both antibody variable domains 1) (A') and (B) are each human antibody variable domains; 2) (A') and (B) are each antibody heavy chain variable domains; 3) (A1) and (B) are each antibody light chain variable domains; 4) (A') is an antibody light chain variable domain and (B) is an antibody heavy chain variable domain (e.g, VH, VHH); or 5) (A') is a VHH and (B) is an antibody light chain variable domain. Additionally or alternatively, preferred embodiments of this aspect include the proviso that when (A') is a $V_H$ and (B) is a $V_L$, L2 does not consist of one to five or one to six contiguous amino acids from the amino-terminus of CH1. Additionally or alternatively, preferred embodiments of this aspect include the proviso that (B) is a domain but is not an antibody variable domain. Additionally or alternatively, preferred embodiments of this aspect include the proviso that (B) is, or is derived from, a polypeptide selected from, for example, a cytokine, a cytokine receptor (*e.g.*, an interleukin receptor, such as IL-1R, IL1R Type, a tumor necrosis factor receptor, such as TNFR1, TNFR2), a growth factor (e.g.,VEGF, EGF, CSF-1), a growth factor receptor (e.g.,VEGF-R1, VEGF-R2. EGFR, CSF-1R), a hormone (e.g., insulin), a hormone receptor (e.g., insulin receptor), an adhesion molecule, a haemostatic factor, a T cell receptor, a T cell receptor chain, a T cell receptor variable domain, an enzyme, or a functional portion of any one of the foregoing. Additionally or alternatively, when (A) and (B) are both antibody variable domains the following is excluded from the invention, (A)-L2-(B) where (A) is a mouse VH, (B) is a mouse VL and L2 is SerAlaLysThrThrPro (SEQ ID NO:537), SerAlaLysThrThrProLysLeuGlyGly (SEQ ID NO:538), AlaLysThrThrProLysLeuGluGluGlyGluPheSerGluAlaArgVal (SEQ ID NO:539), or AlaLysThrThrProLysLeuGluGlu (SEQ ID NO:540). Additionally or alternatively, (A)-L2-(B) is not a fusion protein wherein (A) is a mouse VH, (B) is a mouse VL and L1 is a linker as disclosed in Le Gall et al., Protein Engineering, Design & Selection, 17:357-366 (2004), Kipriyanov et al., Int. J. Cancer, 77:763-772 (1998); Le Gall et al., J. Immunol. Methods, 285:111-127 (2004); Le Gall et al., FEBS Letters, 453:164-168 (1995); or Kipriyanov et al., Protein Engineering, 10:445-453 (1997).

In some embodiments, (A') is an antibody heavy chain variable domain or a hybrid antibody variable domain, for example, an antibody heavy chain variable domain or a hybrid antibody variable domain that comprises a FR4 that comprises the amino acid sequence GlyXaaGlyThr(Leu/Met/Thr)ValThrValSerSer (SEQ ID NO:478). In particular embodiments, the FR4 comprises GlyXaaGlyThrLeuValThrValSerSer (SEQ ID NO:479), GlyXaaGlyThrMetValThrValSerSer (SEQ ID NO:480), or GlyXaaGlyThrThrValThrValSerSer (SEQ ID NO:481). In such embodiments, L2 comprises one to about 50 contiguous amino acids from the amino-terminus of CH1. For example, L2 can comprise AlaSerThr (SEQ ID NO:473), AlaSerThrLysGlyProSer (SEQ ID NO:474), or AlaSerThrLysGlyProSerGly (SEQ ID NO:475).

In other embodiments, (A') is a hybrid antibody heavy chain variable domain or a Vk that comprises a FR4 that comprises the amino acid sequence
GlyXaaGlyThr(Lys/Arg)(Val/Leu)(Glu/Asp)IleLysArg (SEQ ID NO:485). For example, FR4 can comprise GlyXaaGlyThrLysValGluIleLysArg (SEQ ID NO:486),
GlyXaaGlyThrLysLeuGluIleLysArg (SEQ ID NO:487), GlyXaaGlyThrLysValAspIleLysArg (SEQ ID NO:488), or GlyXaaGlyThrArgLysGluIleLysArg (SEQ ID NO:489). In such embodiments, L2 comprises one to about 50 contiguous amino acids from the amino-terminus of Cκ. For example, L2 can comprise ThrValAla (SEQ ID NO:467), ThrValAlaAlaProSer (SEQ ID NO:490), or ThrValAlaAlaProSerGly (SEQ ID NO:491).

In other embodiments, (A') is a hybrid antibody variable domain or a Vλ that comprises a FR4 that comprises the amino acid sequence
GlyXaaGlyThr(Lys/Gln/Glu)(Val/Leu)(Thr/Ile)(Val/Ile)Leu (SEQ ID NO:492). For example FR4 can comprise GlyXaaGlyThrLysValThrValLeu(SEQ ID NO:493),
GlyXaaGlyThrLysValThrIleLeu(SEQ ID NO:494), GlyXaaGlyThrLysValIleValLeu(SEQ ID NO:495), GlyXaaGlyThrLysValIleIleLeu(SEQ ID NO:496),
GlyXaaGlyThrLysLeuThrValLeu(SEQ ID NO:497), GlyXaaGlyThrLysLeuThrIleLeu(SEQ ID NO:498), GlyXaaGlyThrLysLeuIleValLeu(SEQ ID NO:499),
GlyXaaGlyThrLysLeuIleIleLeu(SEQ ID NO:500), GlyXaaGlyThrGlnValThrValLeu(SEQ ID NO:501), GlyXaaGlyThrGlnValThrIleLeu(SEQ ID NO:502),
GlyXaaGlyThrGlnValIleValLeu(SEQ ID NO:503), GlyXaaGlyThrGlnValIleIleLeu(SEQ ID NO:504), GlyXaaGlyThrGlnLeuThrValLeu(SEQ ID NO:505),
GlyXaaGlyThrGlnLeuThrIleLeu(SEQ ID NO:506), GlyXaaGlyThrGlnLeuIleValLeu(SEQ ID NO:507), GlyXaaGlyThrGlnLeuIleIleLeu(SEQ ID NO:508),
GlyXaaGlyThrGluValThrValLeu(SEQ ID NO:509), GlyXaaGlyThrGluValThrIleLeu(SEQ ID NO:510), GlyXaaGlyThrGluValIleValLeu(SEQ ID NO:511),
GlyXaaGlyThrGluValIleIleLeu(SEQ ID NO:512), GlyXaaGlyThrGluLeuThrValLeu(SEQ ID NO:513), GlyXaaGlyThrGluLeuThrIleLeu(SEQ ID NO:514),
GlyXaaGlyThrGluLeuIleValLeu(SEQ ID NO:515), and GlyXaaGlyThrGluLeuIleIleLeu(SEQ ID NO:516). Preferably, FR4 comprises GlyXaaGlyThrLysValThrValLeu(SEQ ID NO:493), GlyXaaGlyThrLysLeuThrValLeu(SEQ ID NO:497), GlyXaaGlyThrGlnLeuIleIleLeu(SEQ ID NO:508), GlyXaaGlyThrGluLeuThrValLeu(SEQ ID NO:513), or GlyXaaGlyThrGlnLeuThrValLeu(SEQ ID NO:505). In such embodiments, L2 comprises one to about 50 contiguous amino acids from the amino-terminus of Cλ.

In some embodiments, (B) comprises an immunoglobulin variable domain. Preferably, the immunoglobulin variable domain (e.g., antibody variable domain) is at the amino terminus of (B) and is directly bonded to the carboxy-terminus of L2. In particular examples, the immunoglobulin variable domain is an antibody light chain variable domain or an antibody heavy chain variable domain (e.g., $V_H$, $V_{HH}$).

In some embodiments, (B) comprises at least a portion of an immunoglobulin constant region. Preferably, said at least a portion immunoglobulin constant region is at the amino terminus of (B) and is directly bonded to the carboxy-terminus of L2. In particular examples, (B) comprises at least a portion of an IgG constant region, such as an IgG1 constant region, an IgG2 constant region, an IgG3 constant region, or an IgG4 constant region. For example, (B) can comprise at least a portion of CH1, at least a portion of hinge, at least a portion of CH2 or at least a portion of CH3. In particular embodiments, (B) comprises at least a portion of hinge, such as a portion of hinge that comprises ThrHisThr-CysProProCysPro (SEQ ID NO:520). In other embodiments, (B) comprises at least a portion of hinge and further comprises CH2-CH3. In other embodiments, (') comprises a portion of CH1-hinge-CH2-CH3, hinge-CH2-CH3, CH2-CH3, or CH3.

In another aspect, the recombinant fusion protein comprises a first portion derived from a first polypeptide and a second portion derived from an immunoglobulin constant region, wherein said first portion is bonded to said second portion through a linker, and the recombinant fusion protein has the formula

$$(A)\text{-}L3\text{-}(C^3)$$

wherein (A) is said first portion; ($C^3$) is said second portion derived from an immunoglobulin constant region; and L3 is said linker, wherein L3 comprises one to about 50 contiguous amino acids that are adjacent to the amino-terminus of ($C^3$) in a naturally occurring immunoglobulin that comprises ($C^3$). In certain embodiments of this aspect, the invention includes the proviso that (A) is not a variable domain peptide bonded to L3 in a naturally occuring immunoglobulin comprising L3-($C^3$).

In preferred embodiments, the first polypeptide is a cytokine, a cytokine receptor (*e.g.*, an interleukin receptor, such as IL-1R, IL1R Type, a tumor necrosis factor receptor, such as TNF1, TNFR2), a growth factor (e.g., VEGF, EGF, CSF-1), a growth factor receptor (e.g., VEGF-R1, VEGF-R2, EGFR, CSF-1R), a hormone (e.g., insulin), a hormone receptor (e.g., insulin receptor), an adhesion molecule, a haemostatic factor, a T cell receptor, a T cell receptor chain, a T cell receptor variable domain, an enzyme, a polypeptide comprising or consisting of an antibody variable domain, or a functional portion of any one of the foregoing. Thus, in preferred embodiments, (A) is derived from or is a cytokine, a cytokine receptor (*e.g.*, an interleukin receptor, such as IL-1R, IL1R Type, a tumor necrosis factor receptor, such as TNF1, TNFR2), a growth factor (e.g., VEGF, EGF, CSF-1), a growth factor receptor (e.g.,VEGF-R1, VEGF-R2, EGFR, CSF-1R), a hormone (e.g., insulin), a hormone receptor (e.g., insulin receptor), an adhesion molecule, a haemostatic factor, a T cell receptor, a T cell receptor chain, a T cell receptor variable domain, an enzyme, a polypeptide comprising or consisting of an antibody variable domain, or a functional portion of any one of the foregoing.

In some embodiments, ($C^3$) comprises at least one antibody constant domain, such as a human antibody constant domain. Preferably, the antibody constant domain is a human IgG constant domain (e.g., IgG1 constant domain, IgG2 constant domain, IgG3 constant domain, IgG4 constant domain). In some embodiments, ($C^3$) comprises CH3. In these example, 3 can comprise one to about 50 contiguous amino acids from the carboxy-terminus of CH2.

In other embodiments, ($C^3$) comprises CH2 or CH2-CH3, e.g., IgG1 or IgG4 CH2 or CH2-CH3. In these embodiments, L3 can comprise one to about 34 contiguous amino acids from the carboxy-terminus of hinge. For example, L3 can comprise ThrHisThrCysProProCysPro (SEQ ID NO:520) or GlyThrHisThrCysProProCysPro (SEQ ID NO:521). In other embodiments, ($C^3$) comprises hinge. In these embodiments, L3 can comprise one to about 50 contiguous amino acids from the carboxy-terminus of CH1.

In other embodiments, ($C^3$) comprises CH1. In these embodiments, L3 comprises one to about 50 contiguous amino acids from the carboxy-terminus of an antibody heavy chain V domain. For example, L3 can comprise GlyXaaGlyThr(Leu/Met/Thr)ValThrValSerSer (SEQ ID NO:478). In particular embodiments, L3 comprises GlyXaaGlyThrLeu-ValThrValSerSer (SEQ ID NO:479), GlyXaaGlyThrMetValThrValSerSer (SEQ ID NO:480), or GlyXaaGlyThrThrValThr-ValSerSer (SEQ ID NO:481).

In some embodiments, ($C^3$) comprises at least a portion of an antibody light chain constant domain. In particular embodiments, ($C^3$) is a Cκ. In such embodiments, L3 comprises one to about 50 contiguous amino acids from the carboxy-terminus of an antibody light chain V domain. For example, L3 can comprise GlyXaaGlyThr(Lys/Arg)(Val/Leu)(Glu/Asp)IleLysArg (SEQ ID NO:485). In particular embodiments, L3 comprises

GlyXaaGlyThrLysValGluIleLysArg (SEQ ID NO:486), GlyXaaGlyThrLysLeuGluIleLysArg (SEQ ID NO:487), GlyXaaGlyThrLysValAspIleLysArg (SEQ ID NO:488), or
GlyXaaGlyThrArgLysGluIleLysArg (SEQ ID NO:489).

In other embodiments, (C³) is a Cλ. In such embodiments, L3 comprises one to about 50 contiguous amino acids from the carboxy-terminus of an antibody light chain V domain. For example, L3 can comprise GlyXaaGlyThr (Lys/Gln/Glu)(Val/Leu)(Thr/Ile)(Val/Ile)Leu (SEQ ID NO:492). In particular embodiments, L3 comprises GlyXaaGlyThr-LysValThrValLeu(SEQ ID NO:493), GlyXaaGlyThrLysValThrIleLeu(SEQ ID NO:494),
GlyXaaGlyThrLysValIleValLeu(SEQ ID NO:495), GlyXaaGlyThrLysValIleIleLeu(SEQ ID NO:496), GlyXaaGlyThrLys-LeuThrValLeu(SEQ ID NO:497),
GlyXaaGlyThrLysLeuThrIleLeu(SEQ ID NO:498), GlyXaaGlyThrLysLeuIleValLeu(SEQ ID NO:499), GlyXaaGlyThrLys-LeuIleIleLeu(SEQ ID NO:500),
GlyXaaGlyThrGlnValThrValLeu(SEQ ID NO:501), GlyXaaGlyThrGlnValThrIleLeu(SEQ ID NO:502), GlyXaaGlyThr-GlnValIleValLeu(SEQ ID NO:503),
GlyXaaGlyThrGlnValIleIleLeu(SEQ ID NO:504), GlyXaaGlyThrGlnLeuThrValLeu(SEQ ID NO:505), GlyXaaGlyThr-GlnLeuThrIleLeu(SEQ ID NO:506),

GlyXaaGlyThrGlnLeuIleValLeu(SEQ ID NO:507), GlyXaaGlyThrGlnLeuIleIleLeu(SEQ ID NO:508), GlyXaaGlyThrGlu-ValThrValLeu(SEQ ID NO:509),
GlyXaaGlyThrGluValThrIleLeu(SEQ ID NO:510), GlyXaaGlyThrGluValIleValLeu(SEQ ID NO:511), GlyXaaGlyThrGlu-ValIleIleLeu(SEQ ID NO:512),
GlyXaaGlyThrGluLeuThrValLeu(SEQ ID NO:513), GlyXaaGlyThrGluLeuThrIleLeu(SEQ ID NO:514), GlyXaaGlyThrGlu-LeuIleValLeu(SEQ ID NO:515), and
GlyXaaGlyThrGluLeuIleIleLeu(SEQ ID NO:516). Preferably, L3 comprises
GlyXaaGlyThrLysValThrValLeu(SEQ ID NO:493), GlyXaaGlyThrLysLeuThrValLeu(SEQ ID NO:497), GlyXaaGlyThr-GlnLeuIleIleLeu(SEQ ID NO:508),
GlyXaaGlyThrGluLeuThrValLeu(SEQ ID NO:513), or GlyXaaGlyThrGlnLeuThrValLeu(SEQ ID NO:505).

METHODS FOR PRODUCING FUSION PROTEINS

The invention relates to methods for producing fusion proteins that contain one or more natural junctions. The method generally comprises identifying a conserved amino acid sequence motif that is present in two polypeptides or portions thereof that are to be fused. A fusion protein is then prepared that contains the conserved amino acid motif, and in which the amino acid sequence that is adj acent to the amino-terminus of the conserved motif is the same as the amino sequence that is adj acent to the amino-terminus of the conserved motif in one of the original polypeptides, and the amino acid sequence that is adjacent to the carboxy-terminus of the conserved motif is the same as the amino acid sequence that is adjacent to the carboxy-terminus of the conserved motif in the other original polypeptide. Generally, the amino acid sequences of two polypeptides or portions of polypeptides are analyzed to identify a conserved amino acid sequence motif that is present in both of the polypeptides of portions. The analysis can be performed using any suitable method. In one example, the amino acid sequences of a first polypeptide and of a second polypeptide are provided (*e.g.*, from a database) and a conserved amino acid sequence motif present in each polypeptide is identified (*e.g.*, manually or using a suitable sequence analysis software package).

The invention provides a method for producing a fusion protein that comprises at least two portions derived from two different polypeptides, and at least one natural junction between the two portions. If desired, the fusion protein can contain three or more portions, and some of the junctions between portions can be non-natural.

In a general aspect, the invention provides a method of producing a fusion protein comprising a first portion and a second portion that are fused at a natural junction, wherein said first portion is derived from a first polypeptide and said second portion is derived from a second polypeptide. The method comprise analyzing the amino acid sequence of a first polypeptide or a portion thereof and the amino acid sequence of a second polypeptide or a portion thereof to identify a conserved amino acid motif present in the analyzed sequences (the first polypeptide or portion thereof and the second polypeptide or portion thereof); and preparing a fusion protein which has the formula

$$A\text{-}Y\text{-}B\,;$$

wherein, A is said first portion; Y is said conserved amino acid motif; B is said second portion; and wherein said first polypeptide comprises A-Y, and said second polypeptide comprises Y-B.

The invention also relates to an improved method for making a fusion protein, such as a fusion protein described herein. For example, in some embodiments, the invention relates to an improved method of producing a fusion protein comprising a first portion and a second portion that linked by at least one natural junction, wherein said first portion is derived from a first polypeptide and said second portion is derived from a second polypeptide, the improvement comprising, analyzing the amino acid sequence of said first polypeptide or a portion thereof and the amino acid sequence of said second polypeptide or a portion thereof to identify a conserved amino acid motif present in both of the analyzed sequences; and preparing a fusion protein which has the formula

$$A\text{-}Y\text{-}B\ ;$$

wherein, A is said first portion, Y is said conserved amino acid motif; B is said second portion; and wherein said first polypeptide comprises A-Y, and said second polypeptide comprises Y-B.

The conserved amino acid motif Y can consist of one to about 50 amino acid residues. In certain embodiments, Y consists of about 3 to about 50 amino acids, about 3 to about 40 amino acids, about 3 to about 30 amino acids, about 3 to about 20 amino acids, about 3 to about 15 amino acids, about 3 to about 14 amino acids, about 3 to about 13 amino acids, about 3 to about 12 amino acids, about 3 to about 11 amino acids, about 3 to about 10 amino acids, about 3 to about 9 amino acids, about 3 to about 8 amino acids, about 3 to about 7 amino acids, about 3 to about 6 amino acids, about 3 to about 5 amino acids, at least 8 amino acids, up to about 11 amino acids, or about 8 to about 11 amino acids. In other embodiments, Y consists of about 15 amino acids, about 14 amino acids, about 13 amino acids, about 12 amino acids, about 11 amino acids, about 10 amino acids, about 9 amino acids, about 8 amino acids, about 7 amino acids, about 6 amino acids, about 5 amino acids, about 4 amino acids, about 3 amino acids, about 2 amino acids, or about 1 amino acid.

The conserved amino acid motif Y is found in the first and second polypeptides (parental polypeptides) of which at least a portion is incorporated into a fusion protein of the invention. The fusion protein of the invention, and the hybrid domain in the fusion protein, can contain portions from any desired parental polypeptides provided that each parental protein contains a conserved amino acid motif. For example, the first and second polypeptides (parental polypeptides) can be unrelated (e.g., from different protein superfamilies) or related (e.g., from the same protein superfamily). In certain embodiments, the fusion protein and hybrid domain contains portions derived from first and second polypeptides (parental polypeptides) from the same protein superfamily, such as the immunoglobulin superfamily, the tumor necrosis factor (TNF) superfamily or the TNF receptor superfamily.

The first and second polypeptides (parental polypeptides) can be from the same species or from different species. For example, the first and second polypeptides can independently be from a human (*Homo sapiens*), or from a non-human species such as mouse, chicken, pig, torafugu, frog, cow (*e.g., Bos taurus*), rat, shark (*e.g.,* bull shark, sandbar shark, nurse shark, homed shark, spotted wobbegong shark), skate (*e.g.*, clearnose skate, little skate), fish (*e.g.,* atlantic salmon, channel catfish, lady fish, spotted ratfish, atlantic cod, chinese perch, rainbow trout, spotted wolf fish, zebrafish), possum, sheep, *Camelid* (*e.g.,* llama, guanaco, alpaca, vicunas, dromedary camel, bactrian camel), rabbit, non-human primate (*e.g.*, new world monkey, old world monkey, cynomolgus monkey (*Macaca fascicularis*), *Callithricidae* (*e.g.,* marmosets)), or any other desired non-human species. In particular embodiments, the first and second polypeptides are both human, or one is human and the other is from a non-human species.

The first and second polypeptides (parental polypeptides) can be any desired polypeptides. Suitable examples of first and second polypeptides include a cytokine, a cytokine receptor (*e.g.*, an interleukin receptor, such as IL-1R, IL1R Type, a tumor necrosis factor receptor, such as TNF1, TNFR2), a growth factor (e.g., VEGF, EGF, CSF-1), a growth factor receptor (*e.g.*,VEGF-R1, VEGF-R2, EGFR, CSF-1R), a hormone (e.g., insulin), a hormone receptor (e.g., insulin receptor), an adhesion molecule, a haemostatic factor, a T cell receptor, a T cell receptor chain, a T cell receptor variable domain, an enzyme, a polypeptide comprising or consisting of an antibody variable domain, or a functional portion of any one of the foregoing.

Conserved amino acid motifs can be readily identified using any suitable method, such as by aligning two or more amino acid sequences and identifying regions of conserved amino acid sequence. This can be accomplished manually or by using any other suitable method, such as using a suitable sequence analysis algorithm or software package (e.g., CLUSTAL (Thompson et al.. Nucleic Acids Research, 25:4876-4882(1997); Chenna R, et al., Nucleic Acids Res, 31:3497-3500. (2003)), BLAST (Altschul, et al., J. Mol. Biol., 215:403-410 (1990), Gish, W. & States, D.J., Nature Genet., 3:266-272 (1993), Madden, et al., Meth. Enzymol., 266:131-141 (1996), Altschul, et al., Nucleic Acids Res., 25:3389-3402 (1997), Zhang et al., J Comput Biol; 7(1-2):203-14 (2000), Zhang, J. & Madden, T.L., Genome Res., 7:649-656 (1997), MOTIF available online from Genomenet, Bioinformatics Center Institute for Chemical Research, Kyoto University (www.genome.jp). For example, as described herein, conserved amino acid motifs that are present in immunoglobulin proteins have been identified by alignment of immunoglobulin amino acid sequences. Particular examples of conserved amino acid motifs include: GlyXaaGlyThr (SEQ ID NO:386) or GlyXaaGlyThrXaa(Val/Leu) (SEQ ID NO:387) in framework region (FR) 4 of antibody variable domains; GluAspThrAla (SEQ ID NO:388), ValTyrTyrCys (SEQ ID NO:389), or GluAspThrAlaValTyrTyrCys (SEQ ID NO:390) in FR3 of antibody variable domains; (Ser/Ala/Gly)Pro (Lys/Asp/Ser)Val (SEQ ID NO:391), (Ser/Ala/Gly)Pro(Lys/Asp/Ser)ValPhe (SEQ ID NO:392), LysValAspLys (Ser/Arg/Thr) (SEQ ID NO:393), or ValThrVal (SEQ ID NO:394) in antibody constant regions.

In some embodiments, the second polypeptide comprises an immunoglobulin constant domain, such as a TCR constant domain or an antibody constant domain. The immunoglobulin constant domain can be a human immunoglobulin constant domain or a nonhuman immunoglobulin constant domain. In one example, the second polypeptide comprises a T cell receptor constant domain.

In certain embodiments, the second polypeptide comprises an antibody light chain constant domain or an antibody heavy chain constant domain, preferably, a human light chain constant domain or a human heavy chain constant domain. In particular embodiments, B comprises an antibody hinge region, a portion of CH1-hinge-CH2-CH3, Fc (hinge-CH2-CH3 or CH2-CH3), or CH3. Preferably, the human antibody heavy chain constant domain is an IgG (IgG1, IgG2, IgG3, IgG4) constant domain. For example, in some embodiments, the IgG constant domain is an IgG1 constant domain or an IgG4 constant domain.

In particular embodiments, the first polypeptide is a cytokine, a cytokine receptor (*e.g.,* an interleukin receptor, such as IL-1R, IL1R Type, a tumor necrosis factor receptor, such as TNF1, TNFR2), a growth factor (e.g., VEGF, EGF, CSF-1), a growth factor receptor (e.g., VEGF-R1, VEGF-R2, EGFR, CSF-1R), a hormone (e.g., insulin), a hormone receptor (e.g., insulin receptor), an adhesion molecule, a haemostatic factor, a T cell receptor, a T cell receptor chain, a T cell receptor variable domain, an enzyme, a polypeptide comprising or consisting of an antibody variable domain, or a functional portion of any one of the foregoing, and the second polypeptide and B comprise an immunoglobulin constant domain.

In some embodiments, the first polypeptide and A comprise an immunoglobulin variable domain, such as a TCR constant domain or an antibody constant domain. The immunoglobulin variable domain can be a human immunoglobulin variable domain or a nonhuman immunoglobulin variable domain. In one example, the first polypeptide comprises a T cell receptor variable domain.

In certain embodiments, the first polypeptide comprises an antibody light chain variable domain (e.g., Vκ, Vλ) or an antibody heavy chain variable domain (e.g., $V_H$, $V_{HH}$). In some embodiment, the antibody variable domain is a non-human light chain variable domain or a non-human heavy chain variable domain. For example, the non-human antibody variable domain can be a *Camelid* antibody variable domain or a nurse shark antibody variable domain. In other embodiments, the antibody variable domain is a human antibody variable domain, such as a human Vk, human Vλ. or human $V_H$.

In particular embodiments, the first polypeptide and A comprise an immunoglobulin variable domain (*e.g.*, antibody variable domain) and said second polypeptide is a cytokine, a cytokine receptor (*e.g.*, an interleukin receptor, such as IL-1R, IL1R Type, a tumor necrosis factor receptor, such as TNF1, TNFR2), a growth factor (e.g., VEGF, EGF, CSF-1), a growth factor receptor (e.g.,VEGF-R1, VEGF-R2, EGFR, CSF-1R), a hormone (e.g., insulin), a hormone receptor (e.g., insulin receptor), an adhesion molecule, a haemostatic factor, a T cell receptor, a T cell receptor chain, a T cell receptor variable domain, an enzyme, a polypeptide comprising or consisting of an antibody variable domain, or a functional portion of any one of the foregoing.

In other embodiments, the first polypeptide is a first antibody chain, and the second polypeptide is a second antibody chain. In such embodiments, Y can be in the variable domain of the first and second antibody chains, or in a constant domain of said first and second antibody chains. For example, Y can be in a framework region of the variable domain of the first and second antibody chains. In a particular embodiment, Y is in FR 4. For example, Y can be GlyXaaGlyThr (SEQ ID NO:386) or GlyXaaGlyThrXaa(Val/Leu) (SEQ ID NO:387). In such embodiments, A comprises a portion of an antibody variable domain comprising FR1, complementarity determining region (CDR) 1, FR2, CDR2, FR3, and CDR3.

In other particular embodiments, Y is in FR3. For example, Y can be GluAspThrAla (SEQ ID NO:388), ValTyr-TyrCys (SEQ ID NO:389), or GluAspThrAlaValTyrTyrCys (SEQ ID NO:390). In such embodiments, A comprises a portion of an antibody variable domain comprising FR1, CDR1, FR2, and CDR2.

In other embodiments, Y is in a constant domain (*e.g.*, CH1, hinge, CH2, CH3) of said first antibody chain and a constant domain of said second antibody chain. For example, Y can be (Ser/Ala/Gly)Pro(Lys/Asp/Ser)Val (SEQ ID NO:391), (Ser/Ala/Gly)Pro(Lys/Asp/Ser)ValPhe (SEQ ID NO:392), LysValAspLys(Ser/Arg/Thr) (SEQ ID NO:393) or ValThrVal (SEQ ID NO:394). In particular embodiments, Y is SerProLysVal (SEQ ID NO:398), SerProAspVal (SEQ ID NO:399), SerProSerVal (SEQ ID NO:400), AlaProLysVal (SEQ ID NO:401), AlaProAspVal (SEQ ID NO:402), AlaProSerVal (SEQ ID NO:403), GlyProLysVal (SEQ ID NO:404), GlyProAspVal (SEQ ID NO:405), GlyProSerVal (SEQ ID NO: 406), SerProLysValPhe (SEQ ID NO:407), SerProAspValPhe (SEQ ID NO:408), SerProSerValPhe (SEQ ID NO:409), AlaProLysValPhe (SEQ ID NO:410), AlaProAspValPhe (SEQ ID NO:411), AlaProSerValPhe (SEQ ID NO:412), GlyPro-LysValPhe (SEQ ID NO:413), GlyProAspValPhe (SEQ ID NO:414), GlyProSerValPhe (SEQ ID NO:415), LysValAsp-LysSer (SEQ ID NO:416), LysValAspLysArg (SEQ ID NO:417), LysValAspLysThr (SEQ ID NO:418), or ValThrVal (SEQ ID NO:394).

When the first polypeptide is a first antibody chain, and the second polypeptide is a second antibody chain, the antibody chains can be from the same or different species. For example, in some embodiments, the first antibody chain and said second antibody chain are both human. In other embodiments, the first antibody chain is human and the second antibody chain is non-human, or the first antibody chain is non-human and the second antibody chain is human.

The recombinant fusion proteins prepared by the methods described herein comprise a partial structure depicted in the formulae presented herein. As described herein, the fusion proteins can comprise additional portions or components that are directly or indirectly fused to the portions specified in the formulae through a natural junction or non-natural junction. For example, if desired the fusion protein of the invention can further comprises a third portion located amino terminally to A. The third portion can be derived from any desired polypeptide. In certain embodiments, the third portion located amino terminally to A is an immunoglobulin variable domain (e.g., antibody variable domain).

The recombinant fusion protein can comprise a hybrid domain, wherein said hybrid domain comprises a first portion derived from a first polypeptide and a second portion derived from a second polypeptide, and a conserved motif that is present in said first polypeptide and in said second polypeptide. This type of recombinant fusion protein can be prepared by a method that comprises analyzing the amino acid sequence of a first domain from a first polypeptide and the amino acid sequence of a second domain from a second polypeptide to identify a conserved amino acid motif present in said first domain and in said second domain, wherein said first domain has the formula (X1-Y-Z1) and said second domain has the formula (X2-Y-Z2), and preparing a fusion protein comprising a hybrid domain that has the formula (X1-Y-Z2), wherein Y is said conserved amino acid motif;

X1 and Z1 are the amino acid motifs that are located adjacent to the amino-terminus of Y in said first polypeptide and said second polypeptide, respectively.

X2 and Z2 are the amino acid motifs that are located adjacent to the carboxy-terminus of Y in said first polypeptide and said second polypeptide, respectively.

In some embodiments, the first polypeptide and the second polypeptide are both members of the same protein superfamily, such as the immunoglobulin superfamily, the TNF superfamily and the TNF receptor superfamily. The first and second polypeptides can both be human polypeptides, or one can be a human polypeptide and the other a non-human polypeptide.

The number of amino acids represented by X1, X2, Z1 and Z2 is dependent on the size of the hybrid domain, and the size of the domains in the parental polypeptides. Generally, X1, X2, Z1 and Z2 each, independently, consist of about 1 to about 400, about 1 to about 200, about 1 to about 100, or about 1 to about 50 amino acids. Similarly, the size of the hybrid domain can vary, and is depend on the size of the domains that contain Y in the parental proteins. In particular embodiments, the hybrid domain is about the size of an immunoglobulin variable domain or immunoglobulin constant domain. In some embodiments, the hybrid domain is about 1 kDa to about 25 kDa, about 5 kDa to about 25 kDa, about 5 kDa to about 20 kDa, about 5 kDa to about 15 kDa, about 6 kDa, about 7 kDa, about 8 kDa, about 9 kDa, about 10 kDa, about 11 kDa, about 12 kDa, about 13 kDa or about 14 kDa.

In some embodiments, the first polypeptide comprises an immunoglobulin variable domain that contains Y, the second polypeptide comprises an immunoglobulin variable domain that contains Y, and (X1-Y-Z2) is a hybrid immunoglobulin variable domain. For example, the first polypeptide can comprises an antibody variable domain, the second polypeptide can comprises an antibody variable domain and Y can be in a framework region (FR), such as FR1, FR2, FR3 or FR 4. In particular examples, Y is in FR4 and is GlyXaaGlyThr (SEQ ID NO:386) or GlyXaaGlyThrXaa(Val/Leu) (SEQ ID NO:387). For example, Y can be GlyXaaGlyThrXaaVal (SEQ ID NO:395) or GlyXaaGlyThrXaaLeu (SEQ ID NO:396). In these embodiments, X1 can be a portion of the antibody variable domain of the first polypeptide that comprises FR1, CDR 1, FR2, CDR2, FR3, and CDR3. In other examples, Y is in FR3 and is GluAspThrAla (SEQ ID NO:388), ValTyrTyrCys (SEQ ID NO:389), or GluAspThrAlaValTyrTyrCys (SEQ ID NO:390). In these embodiments, X1 can be a portion of the antibody variable domain of the first polypeptide that comprises FR1, CDR1, FR2, and CDR2.

In other embodiments, the first polypeptide comprises an immunoglobulin constant domain that contains Y, the second polypeptide comprises an immunoglobulin constant domain, that contains Y and (X1-Y-Z2) is a hybrid immunoglobulin constant domain. For example, Y can be located in an antibody light chain constant domain (*e.g.*, Ck, Cl), or an antibody heavy chain constant domain (*e.g.*, CH1, hinge, CH2, CH3). For example, in an antibody constant domain Y can be (Ser/Ala/Gly)Pro(Lys/Asp/Ser)Val (SEQ ID NO:391), (Ser/Ala/Gly)Pro(Lys/Asp/Ser)ValPhe (SEQ ID NO:392), LysValAspLys(Ser/Arg/Thr) (SEQ ID NO:393) or ValThrVal (SEQ ID NO:394), and in a TCR constant domain Y can be ProSerValPhe (SEQ ID NO:397). In particular embodiments, Y is in an antibody constant domain and is SerProLysVal (SEQ ID NO:398), SerProAspVal (SEQ ID NO:399), SerProSerVal (SEQ ID NO:400), AlaProLysVal (SEQ ID NO:401), AlaProAspVal (SEQ ID NO:402), AlaProSerVal (SEQ ID NO: 403), GlyProLysVal (SEQ ID NO:404), GlyProAspVal (SEQ ID NO:405), GlyProSerVal (SEQ ID NO:406), SerProLysValPhe (SEQ ID NO:407), SerProAspValPhe (SEQ ID NO:408), SerProSerValPhe (SEQ ID NO:409), AlaProLysValPhe (SEQ ID NO:410), AlaProAspValPhe (SEQ ID NO:411), AlaProSerValPhe (SEQ ID NO:412), GlyProLysValPhe (SEQ ID NO:413), Gly-

ProAspValPhe (SEQ ID NO:414), GlyProSerValPhe (SEQ ID NO:415), LysValAspLysSer (SEQ ID NO:416), LysValAspLysArg (SEQ ID NO:417), LysValAspLysThr (SEQ ID NO:418), or ValThrVal (SEQ ID NO:394)

In some embodiments, (X1-Y-Z2) is a hybrid immunoglobulin constant domain, and A is an immunoglobulin variable domain. In other embodiments, (X1-Y-Z2) is a hybrid immunoglobulin constant domain, and B is an immunoglobulin constant domain.

In some embodiments the recombinant fusion protein comprises a hybrid immunoglobulin variable domain that is fused to an immunoglobulin constant domain, wherein said hybrid immunoglobulin variable domain comprises a hybrid framework region (FR) that comprises a portion from a first immunoglobulin FR from a first immunoglobulin and a portion from a second immunoglobulin FR from a second immunoglobulin. This type of recombinant fusion protein can be prepared by a method that comprises analyzing the amino acid sequence of a first immunoglobulin FR from a first immunoglobulin and the amino acid sequence of a second immunoglobulin FR from a second immunoglobulin to identify a conserved amino acid motif present in said first immunoglobulin FR and in said second immunoglobulin FR; and preparing a fusion protein comprising a hybrid immunoglobulin FR that has the formula

$$(F^1\text{-}Y\text{-}F^2),$$

wherein Y is said conserved amino acid motif; $F^1$ is the amino acid sequence located adjacent to the amino-terminus of Y in said first immunoglobulin FR; and $F^2$ is the amino acid sequence located adjacent to the carboxy-terminus of Y in said second immunoglobulin FR.

The hybrid FR can be a hybrid FR1, hybrid FR2, hybrid FR3 or hybrid FR4. In one example, the first immunoglobulin is an antibody heavy chain, the second immunoglobulin is an antibody light chain, $F^1$ is derived from FR1, FR2, FR3 or FR4 of the antibody heavy chain variable region, and $F^2$ is derived from the corresponding FR of the antibody light chain variable region. In another example, the first immunoglobulin is an antibody light chain, the second immunoglobulin is an antibody heavy chain, $F^1$ is derived from FR1, FR2, FR3 or FR4 of the antibody light chain variable region, and $F^2$ is derived from the corresponding FR of the antibody heavy chain variable region.

In some embodiments, the second immunoglobulin comprises a variable domain containing Y and $F^2$ in FR4, and a constant domain. For example, the second polypeptide can be a TCR chain in which Y and $F^2$ are in TCR FR4. In this example, the recombinant fusion protein contains a hybrid immunoglobulin domain that is bonded to the amino-terminus of the TCR constant domain. Similarly, the second polypeptide can be an antibody light chain in which Y and $F^2$ are in FR4, and the recombinant fusion protein contains a hybrid immunoglobulin domain that is bonded to the amino-terminus of an antibody light chain constant domain. In particular embodiments, the second polypeptide is a κ or λ light chain, $F^2$ is derived from a Vκ or Vλ FR4, and the hybrid immunoglobulin domain is bonded to the amino-terminus of Cκ or Cλ, respectively. When the second polypeptide is an antibody heavy chain and $F^2$ is derived from an antibody heavy chain variable domain FR4, the hybrid immunoglobulin domain can be bonded to the amino-terminus of an antibody heavy chain constant domain. In particular embodiments, the second polypeptide is an antibody heavy chain, $F^2$ is derived from an antibody heavy chain variable domain FR4 (*e.g.*, $V_H$ FR4, $V_{HH}$ FR4), and the hybrid immunoglobulin domain is bonded to the amino-terminus of CH1.

In particular embodiments, Y is in FR4 and is GlyXaaGlyThr (SEQ ID NO:386) or GlyXaaGlyThrXaa(Val/Leu) (SEQ ID NO:387). For example, the first immunoglobulin can comprise antibody light chain variable domain comprising an FR4 in which $F^1$ is Phe and Y is GlyXaaGlyThr (SEQ ID NO:386), and the second immunoglobulin can comprise an antibody heavy chain variable comprising an FR4 domain in which Y is GlyXaaGlyThr (SEQ ID NO:386), and $F^2$ is (Leu/Met/Thr)ValThrValSerSer (SEQ ID NO:420). In particular embodiments, $F^2$ can be LeuValThrValSerSer (SEQ ID NO:421), MetValThrValSerSer (SEQ ID NO:422), or ThrValThrValSerSer (SEQ ID NO:423).

In other examples, the first immunoglobulin comprises antibody light chain variable domain comprising an FR4 in which $F^1$ is Phe and Y is GlyXaaGlyThrXaa(Val/Leu) (SEQ ID NO:387), and the second immunoglobulin comprises an antibody heavy chain variable domain comprising an FR4 in which Y is GlyXaaGlyThrXaa(Val/Leu) (SEQ ID NO:387), and $F^2$ is ThrValSerSer (SEQ ID NO:419). In particular embodiments, Y is GlyXaaGlyThrXaaVal (SEQ ID NO:395) or GlyXaaGlyThrXaaLeu (SEQ ID NO:396). Preferably the carboxy-terminus of these types of hybrid antibody variable domains is bonded directly to an antibody heavy chain constant domain, such as an IgG (*e.g.*, IgG1, IgG2, IgG3, IgG4) constant domain. Preferably, the antibody heavy chain constant domain is a human antibody heavy chain constant domain. In particular embodiments, the carboxy-terminus of the hybrid antibody variable domain is bonded directly to IgG CH1 or IgG CH2 (*e.g.*, IgG1 CH1, IgG4 CH1, IgG1 CH2, IgG4 CH2).

In other embodiments, the first immunoglobulin comprises antibody heavy chain variable domain comprising an FR4 in which X is Trp, Y is GlyXaaGlyThr (SEQ ID NO:386), and the second immunoglobulin comprises an antibody light chain variable domain comprising an FR4 in which Y is GlyXaaGlyThr (SEQ ID NO:386) and $F^2$ is (Lys/Arg)(Val/Leu)(Glu/Asp)IleLys (SEQ ID NO:424) or (Lys/Gln/Glu)(Val/Leu)(Thr/Ile)(Val/Ile)Leu (SEQ ID NO:425). In particular embod-

iments, F$^2$ is LysValGluIleLys (SEQ ID NO:426), LysValAspIleLys (SEQ ID NO:427), LysLeuGluIleLys (SEQ ID NO: 428), LysLeuAspIleLys (SEQ ID NO:429), ArgValGluIleLys (SEQ ID NO:430), ArgValAspIleLys (SEQ ID NO:431), ArgLeuGluIleLys (SEQ ID NO:432), ArgLeuAspIleLys (SEQ ID NO:433), LysValThrValLeu (SEQ ID NO:434), LysValThrIleLeu (SEQ ID NO:435), LysValIleValLeu (SEQ ID NO:436), LysValIleIleLeu (SEQ ID NO:437), LysLeuThrValLeu (SEQ ID NO:438), LysLeuThrIleLeu (SEQ ID NO:439), LysLeuIleValLeu (SEQ ID NO:440), LysLeuIleIleLeu (SEQ ID NO:441), GlnValThrValLeu (SEQ ID NO:442), GlnValThrIleLeu (SEQ ID NO:443), GlnValIleValLeu (SEQ ID NO:444), GlnValIleIleLeu (SEQ ID NO:445), GlnLeuThrValLeu (SEQ ID NO:446), GlnLeuThrIleLeu (SEQ ID NO:447), GlnLeuIleValLeu (SEQ ID NO:448), GlnLeuIleIleLeu (SEQ ID NO:449), GluValThrValLeu (SEQ ID NO:450), GluValThrIleLeu (SEQ ID NO:451), GluValIleValLeu (SEQ ID NO:452), GluValIleIleLeu (SEQ ID NO:453), GluLeuThrValLeu (SEQ ID NO:454), GluLeuThrIleLeu (SEQ ID NO:455), GluLeuIleValLeu (SEQ ID NO:456), or GluLeuIleIleLeu (SEQ ID NO:457).

In other examples, the first immunoglobulin comprises antibody heavy chain variable domain comprising a FR4 in which F$^1$ is Trp and Y is GlyXaaGlyThrXaaVal (SEQ ID NO:395), and the second immunoglobulin comprises an antibody light chain variable domain comprising an FR4 in which Y is GlyXaaGlyThrXaaVal (SEQ ID NO:395) and F$^2$ is (Glu/Asp)IleLys (SEQ ID NO:458) or (Thr/Ile)(Val/Ile)Leu (SEQ ID NO:459). In particular embodiments, F$^2$ is GluIleLys (SEQ ID NO:460), AspIleLys (SEQ ID NO:461), ThrValLeu (SEQ ID NO:462), ThrIleLeu (SEQ ID NO:463), IleValLeu (SEQ ID NO:464), or IleIleLeu (SEQ ID NO:465). Preferably the carboxy-terminus of these types of hybrid antibody variable domains is bonded directly to an antibody light chain constant domain, such as Cκ or Cλ. Preferably, the antibody light chain constant domain is a human antibody light chain constant domain.

In certain embodiments, the fusion protein produced by this method comprises a hybrid immunoglobulin variable domain that is fused to an immunoglobulin constant domain comprises a partial structure that has the formula (F$^1$-Y-F$^2$)-Cκ, (F$^1$-Y-F$^2$)-Cλ, (F$^1$-Y-F$^2$)-CH1, (F$^1$-Y-F$^2$)-CH2 or (F$^1$-Y-F$^2$)-Fc. In certain embodiments, the fusion protein produced by this method comprises a hybrid immunoglobulin variable domain that is fused to an immunoglobulin constant domain further comprises a second immunoglobulin variable domain (*e.g.*, antibody variable domain). Preferably, the second immunoglobulin variable domain is amino-terminal to the hybrid immunoglobulin variable domain in the fusion protein.

In particular embodiments, the recombinant fusion protein comprises a non-human antibody variable region directly fused to a human antibody constant domain, wherein the non-human antibody variable region comprises a hybrid FR4 having the formula

$$(F^1\text{-}Y\text{-}F^2)$$

wherein F$^1$ is Phe or Trp;
Y is GlyXaaGlyThr (SEQ ID NO:386), and F$^2$ is (Leu/Met/Thr)ValThrValSerSer (SEQ ID NO:420), (Lys/Arg)(Val/Leu)(Glu/Asp)IleLys (SEQ ID NO:424) or (Lys/Gln/Glu)(Val/Leu)(Thr/Ile)(Val/Ile)Leu (SEQ ID NO:425); or
Y is GlyXaaGlyThrXaa(Val/Leu) (SEQ ID NO:387), and F$^2$ is ThrValSerSer (SEQ ID NO:419), (Glu/Asp)IleLys (SEQ ID NO:458) or (Thr/Ile)(Val/Ile)Leu (SEQ ID NO:459).

This type of recombinant fusion protein can be prepared by a method that comprises analyzing the amino acid sequence of a first polypeptide that comprises a non-human antibody variable region and the amino acid sequence of and a second polypeptide comprising a human antibody variable domain to identify a conserved amino acid motif Y in FR4 of said non-human antibody variable domain and in FR4 of said human antibody variable domain, and preparing a fusion protein comprising a hybrid FR4 having the formula

$$(F^1\text{-}Y\text{-}F^2)$$

wherein F$^1$ is Phe or Trp;
Y is GlyXaaGlyThr (SEQ ID NO:386), and F$^2$ is (Leu/Met/Thr)ValThrValSerSer (SEQ ID NO:420), (Lys/Arg)(Val/Leu)(Glu/Asp)IleLys (SEQ ID NO:424) or (Lys/Gln/Glu)(Val/Leu)(Thr/Ile)(Val/Ile)Leu (SEQ ID NO:425); or
Y is GlyXaaGlyThrXaa(Val/Leu) (SEQ ID NO:387), and F$^2$ is ThrValSerSer (SEQ ID NO:419), (Glu/Asp)IleLys (SEQ ID NO:458) or (Thr/Ile)(Val/Ile)Leu (SEQ ID NO:459).

The non-human antibody variable region can be from any desired species, such as mouse, chicken, pig, torafugu, frog, cow (*e.g.*, *Bos taurus*), rat, shark *(e.g.,* bull shark, sandbar shark, nurse shark, homed shark, spotted wobbegong shark), skate (*e.g.*, clearnose skate, little skate), fish (*e.g.*, atlantic salmon, channel catfish, lady fish, spotted ratfish, atlantic cod, chinese perch, rainbow trout, spotted wolf fish, zebrafish), possum, sheep, *Camelid* (*e.g.*, llama, guanaco, alpaca, vicunas, dromedary camel, bactrian camel), rabbit, non-human primate (*e.g.*, new world monkey, old world monkey, cynomolgus monkey (*Macaca fascicularis*), *Callithricidae* (*e.g.*, marmosets)), or any other desired non-human species. In certain embodiments, the non-human variable region is a mouse variable region, *Camelid* variable region, or nurse shark variable region) The second polypeptide can comprise a human heavy chain or light chain variable domain.

In particular examples, the non-human antibody variable domain is a light chain variable domain or a heavy chain variable domain comprising FR4 in which $F^1$ is Phe or Trp and Y is GlyXaaGlyThr (SEQ ID NO:368), and the second polypeptide comprises a human antibody light chain variable domain comprising FR4 in which $F^2$ is (Lys/Arg) (Val/Leu)(Glu/Asp)IleLys (SEQ ID NO:424) or (Lys/Gln/Glu)(Val/Leu)(Thr/Ile)(Val/Ile)Leu (SEQ ID NO:425). Preferably the carboxy-terminus of this type of non-human variable domains that contain a hybrid FR4 is bonded directly to a human antibody light chain constant domain, such as Cκ or Cλ. In other examples, the non-human antibody variable domain is a light chain variable domain or a heavy chain variable domain comprising FR4 in which $F^1$ is Phe or Trp and Y is GlyXaaGlyThr (SEQ ID NO:386), and the second polypeptide comprises a human antibody light chain variable domain comprising FR4 in which $F^2$ is (Leu/Met/Thr)ValThrValSerSer (SEQ ID NO:420). Preferably the carboxy-terminus of this type of non-human variable domains that contain a hybrid FR4 is bonded directly to a human antibody heavy chain constant domain. Preferably, the antibody heavy chain constant domain is a human antibody heavy chain constant domain, such as an IgG (*e.g.*, IgG1, IgG2, IgG3, IgG4) constant domain. In particular embodiments, the human antibody heavy chain constant domain is IgG CH1 or IgG CH2 (*e.g.*, IgG1 CH1, IgG4 CH1, IgG1 CH2, IgG4 CH2).

In particular examples, the non-human antibody variable domain is a light chain variable domain or a heavy chain variable domain comprising FR4 in which $F^1$ is Phe or Trp and Y is GlyXaaGlyThrXaa(Val/Leu) (SEQ ID NO:387), and the second polypeptide comprises a human antibody light chain variable domain comprising FR4 in which $F^2$ is ((Glu/Asp)IleLys (SEQ ID NO:458) or (Thr/Ile)(Val/Ile)Leu (SEQ ID NO:459). Preferably the carboxy-terminus of this type of non-human variable domains that contain a hybrid FR4 is bonded directly to a human antibody light chain constant domain, such as Cκ or Cλ. In other examples, the non-human antibody variable domain is a light chain variable domain or a heavy chain variable domain comprising FR4 in which $F^1$ is Phe or Trp and Y is GlyXaaGlyThrXaa(Val/Leu) (SEQ ID NO:387), and the second polypeptide comprises a human antibody light chain variable domain comprising FR4 in which $F^2$ is ThrValSerSer (SEQ ID NO:419). Preferably the carboxy-terminus of this type of non-human variable domains that contain a hybrid FR4 is bonded directly to a human antibody heavy chain constant domain. Preferably, the antibody heavy chain constant domain is a human antibody heavy chain constant domain, such as an IgG (*e.g.*, IgG1, IgG2, IgG3, IgG4) constant domain. In particular embodiments, the human antibody heavy chain constant domain is IgG CH1 or IgG CH2 (*e.g.*, IgG1 CH1, IgG4 CH1, IgG1 CH2, IgG4 CH2).

In certain embodiments, the fusion protein produced by this method comprises a hybrid immunoglobulin variable domain that is fused to an immunoglobulin constant domain comprises a partial structure that has the formula $(F^1\text{-}Y\text{-}F^2)\text{-}C\kappa$, $(F^1\text{-}Y\text{-}F^2)\text{-}C\lambda$, $(F^1\text{-}Y\text{-}F^2)\text{-}CH1$, $(F^1\text{-}Y\text{-}F^2)\text{-}CH2$ or $(F^1\text{-}Y\text{-}F^2)\text{-}Fc$. In certain embodiments, the fusion protein produced by this method comprises a hybrid immunoglobulin variable domain that is fused to an immunoglobulin constant domain further comprises a second immunoglobulin variable domain (*e.g.*, antibody variable domain). Preferably, the second immunoglobulin variable domain is amino-terminal to the hybrid immunoglobulin variable domain in the fusion protein.

In some embodiments the recombinant fusion protein an immunoglobulin variable domain fused to a hybrid immunoglobulin constant domain, wherein said hybrid immunoglobulin constant domain comprises a portion from a first immunoglobulin constant domain and a portion from a second immunoglobulin constant domain. This type of recombinant fusion protein can be prepared by a method that comprises analyzing the amino acid sequences of a first immunoglobulin constant domain and a second immunoglobulin constant domain to identify a conserved amino acid motif present in said first immunoglobulin constant domain and in said second immunoglobulin constant domain; and preparing a fusion protein comprising a hybrid immunoglobulin constant domain having the formula

$$C^1\text{-}Y\text{-}C^2$$

wherein Y is said conserved amino acid motif;

$C^1$ is the amino acid sequence adjacent to the amino-terminus of Y in said first immunoglobulin constant domain, and $C^2$ is the amino acid sequence adjacent to the carboxy-terminus of Y in said second immunoglobulin constant domain. The hybrid immunoglobulin constant domain can comprise portions from any two immunoglobulin constant domains that contain a conserved amino acid motif. In certain embodiments, the hybrid immunoglobulin constant domain is a hybrid antibody constant domain that comprises a portion from a first antibody constant domain and a portion from a second antibody constant domain. For example, the hybrid antibody constant domain can be a hybrid CH1, hybrid hinge, hybrid CH2 or hybrid CH3, wherein portions of the hybrid domain are derived from antibody constant domains from different species (*e.g.*, human and non-human, such as *Camelid* or nurse shark) or different isotypes (*e.g.*, IgA, IgD, IgM, IgE, IgG (IgG1, IgG2, IgG3, IgG4)). The hybrid immunoglobulin constant domain can also comprise portions from two different constant domains, such as a portion from a CH1 domain and a portion from a CH2 domain, or from constant domains of different isotypes (*e.g.*, IgG1 and IgG4).

In some embodiments, the method comprises analyzing the sequences of a first immunoglobulin constant domain and a second immunoglobulin constant domain that are from different species. For example, the first immunoglobulin domain can be a non-human antibody constant domain (*e.g.*, *Camelid* or nurse shark constant domain) and the second immunoglobulin constant domain is a human antibody constant domain. In certain embodiments, the first immunoglobulin constant domain is a *Camelid* antibody constant domain (e.g., *Camelid* CH1). In such embodiments, a *Camelid* VHH can be located amino-terminally to the hybrid constant domain in the fusion protein. For example, the carboxy-terminus of the VHH can be bonded to $C^1$.

In other embodiments, the method comprises analyzing the sequences of a first immunoglobulin constant domain and a second immunoglobulin constant domain or antibody constant domains of different isotypes. Preferably, the second antibody constant domain is an IgG constant domain (IgG1, IgG2, IgG3, IgG4).

In certain embodiments, the fusion protein comprises an antibody variable domain that is directly bonded to $C^1$. In such embodiments, the first immunoglobulin constant domain can be the antibody constant domain that is bonded to the variable domain in a naturally occurring antibody. Such constant domains correspond to the variable domain. For example, if the variable domain is a Vκ or Vλ, the first immunoglobulin domain can be a corresponding Cκ or Cλ, respectively. Similarly, if the variable domain is an antibody heavy chain variable domain, the first immunoglobulin variable domain can be a corresponding CH1 domain.

In some embodiments, the method comprises analyzing the amino acid sequence of a first immunoglobulin constant domain that is an antibody light chain constant domain, and the amino acid sequence of a second immunoglobulin constant domain that is an antibody heavy chain constant domain, preferably a human antibody heavy chain constant domain. In some embodiments, the human antibody heavy chain constant domain is a CH1, hinge, CH2 or CH3 domain. Preferably, the human antibody heavy chain constant domain is an IgG (e.g., IgG1, IgG2, IgG3, IgG4) constant domain such as an IgG1 CH1, IgG4 CH1, IgG1 hinge, IgG4 hinge, IgG1 CH2, IgG4 CH2, IgG1 CH3, IgG4 CH3.

In other embodiments, the fusion protein comprises an antibody heavy chain variable domain and the method comprises analyzing the amino acid sequence of a first immunoglobulin constant domain that is a CH1 domain. In such embodiments, the second immunoglobulin constant domain can be an antibody CH1 domain from a different isotype or species, or a different antibody constant domain (e.g., CH2). In a particular embodiment, the second immunoglobulin constant domain is an antibody light chain constant domain.

In some embodiments, the method comprises analyzing the amino acid sequences of a first antibody constant domain and a second antibody constant domain that both contain a conserved amino acid motif (Y) selected (Ser/Ala/Gly) Pro(Lys/Asp/Ser)Val (SEQ ID NO:391), (Ser/Ala/Gly)Pro(Lys/Asp/Ser)ValPhe (SEQ ID NO:392), LysValAspLys (Ser/Arg/Thr) (SEQ ID NO:393), or ValThrVal (SEQ ID NO:394). For example, in particular embodiments, Y is SerProLysVal (SEQ ID NO:398), SerProAspVal (SEQ ID NO:399), SerProSerVal (SEQ ID NO:400), AlaProLysVal (SEQ ID NO:401), AlaProAspVal (SEQ ID NO:402), AlaProSerVal (SEQ ID NO:403), GlyProLysVal (SEQ ID NO:404), GlyProAspVal (SEQ ID NO:405), GlyProSerVal (SEQ ID NO:406), SerProLysValPhe (SEQ ID NO:407), SerProAspValPhe (SEQ ID NO:408), SerProSerValPhe (SEQ ID NO:409), AlaProLysValPhe (SEQ ID NO:410), AlaProAspValPhe (SEQ ID NO:411), AlaProSerValPhe (SEQ ID NO:412), GlyProLysValPhe (SEQ ID NO:413), GlyProAspValPhe (SEQ ID NO:414), GlyProSerValPhe (SEQ ID NO:415), LysValAspLysSer (SEQ ID NO:416), LysValAspLysArg (SEQ ID NO:417), LysValAspLysThr (SEQ ID NO:418), or ValThrVal(SEQ ID NO:394). Preferably, the second antibody constant domain is a human antibody constant domain, and $C^2$ is derived from said human antibody constant domain. For example, the human antibody constant domain can be a human Cκ, a human Cλ or a human heavy chain constant domain, such as a human CH1, a human hinge, a human CH2 or a human CH3. In particular preferred embodiments, the human antibody constant domain is an IgG CH1 (*e.g.,* IgG1 CH1, IgG4 CH1), IgG hinge (*e.g.,* IgG1 hinge, IgG4 hinge), IgG CH2 (*e.g.,* IgG1 CH2, IgG4 CH2), or IgG CH3 (*e.g.,* IgG1 CH3 or IgG4 CH3), and Z' is derived from said human antibody constant domain.

Some fusion proteins comprise an antibody light chain variable domain, such as a human light chain variable domain, that is fused to a hybrid antibody CH1 domain, wherein $C^1$ is GlnProLysAla (SEQ ID NO:466) or ThrValAla (SEQ ID NO:467), and Y is (Ala/Gly)ProSerVal (SEQ ID NO:468). In these embodiments, $C^2$ is the amino acid sequence that is adjacent to carboxy-terminus ofY in IgG CH1, such as human IgG CH1 (*e.g.*, IgG1 CH1, IgG4 CH1). This type of fusion protein can be prepared using the methods described herein wherein the amino acid sequence of a Cκ or Cλ domain, and the amino acid sequence of a CH1 domain, are provided.

Some fusion protein comprise an antibody light chain variable domain, such as a human light chain variable domain, that is fused to a hybrid antibody CH2 domain, wherein $C^1$ is GlnProLysAla (SEQ ID NO:466) or ThrValAla (SEQ ID NO:467), and Y is (Ala/Gly)ProSerVal (SEQ ID NO:468). In such fusion proteins, $C^2$ is the amino acid sequence that is adjacent to carboxy-terminus of Y in CH2, such as human IgG CH2 (*e.g.*, IgG1 CH2, IgG4 CH2). This type of fusion protein can be prepared using the methods described herein wherein the amino acid sequence of a Cκ or Cλ domain, and the amino acid sequence of a CH2 domain, are provided.

Some fusion protein comprise an antibody heavy chain variable domain, such as a human heavy chain variable domain, that is fused to a hybrid antibody CH2 domain, wherein $C^1$ is SerThrLys (SEQ ID NO:469), and Y is (Ala/Gly) ProSerValPhe (SEQ ID NO:470). In these embodiments, $C^2$ is the amino acid sequence that is adjacent to the carboxy-terminus of Y in IgG CH2, such as human IgG CH2 (e.g., IgG1 CH2, IgG4 CH2). This type of fusion protein can be prepared using the methods described herein wherein the amino acid sequence of a CH1 domain, and the amino acid sequence of a CH2 domain, are provided.

Some fusion protein comprise an antibody light chain variable domain, such as a human λ chain variable domain, that is fused to a hybrid antibody Cκ domain, wherein $C^1$ is GlnProLysAla (SEQ ID NO:466), and Y is (Ala/Gly) ProSerVal (SEQ ID NO:468). In these embodiments, Z' is the amino acid sequence that is adjacent to the carboxy-terminus of Y in Cκ, such as $C^2$ as human Cκ. This type of fusion protein can be prepared using the methods described herein wherein the amino acid sequence of a Cλ domain, and the amino acid sequence of a Cκ domain, are provided.

Some fusion protein comprise an antibody heavy chain variable domain, such as a human heavy chain variable domain, that is fused to a hybrid antibody Cκ domain, wherein $C^1$ is SerThrLys (SEQ ID NO:469), and Y is (Ala/Gly) ProSerValPhe (SEQ ID NO:470). In these embodiments, $C^2$ is the amino acid sequence that is adjacent to the carboxy-terminus of Y in Cκ, such as human Cκ. This type of fusion protein can be prepared using the methods described herein wherein the amino acid sequence of a CH1 domain, and the amino acid sequence of a Cκ domain, are provided.

Some fusion protein comprise an antibody light chain variable domain, such as a human κ chain variable domain, that is fused to a hybrid antibody Cλ domain, wherein $C^1$ is ThrValAla (SEQ ID NO:467), and Y is (Ala/Gly) ProSerVal (SEQ ID NO:468). In these embodiments, $C^2$ is the amino acid sequence that is adjacent to the carboxy-terminus of Y in Cλ, such as human Cλ. This type of fusion protein can be prepared using the methods described herein wherein the amino acid sequence of a Cκ domain, and the amino acid sequence of a Cλ domain, are provided.

Some fusion protein comprise an antibody heavy chain variable domain, such as a human heavy chain variable domain, that is fused to a hybrid antibody Cλ domain, wherein $C^1$ is SerThrLys (SEQ ID NO:469), and Y is (Ala/Gly) ProSerVal (SEQ ID NO:468). In these embodiments, $C^2$ is the amino acid sequence that is adjacent to the carboxy-terminus of Y in Cλ, such as human Cλ. This type of fusion protein can be prepared using the methods described herein wherein the amino acid sequence of a CH1 domain, and the amino acid sequence of a Cλ domain, are provided.

The fusion proteins of the invention can be produced using any suitable method. For example, expression of a nucleic acid that encodes the fusion protein or by chemical synthesis. For expression, a nucleic acid encoding the fusion protein can be expressed using any suitable method, (e.g., in vitro expression, in vivo expression). For example, a nucleic acid that encodes a fusion protein of the invention can be inserted into a suitable expression vector. The resulting construct is then introduced into a suitable host cell for expression. Upon expression, fusion protein can be isolated or purified from a cell lysate or preferably from the culture media or periplasm using any suitable method. (See e.g., Current Protocols in Molecular Biology (Ausubel, F.M. et al., eds., Vol. 2, Suppl. 26, pp. 16.4.1-16.7.8 (1991)).

Suitable expression vectors can contain a number of components, for example, an origin of replication, a selectable marker gene, one or more expression control elements, such as a transcription control element (e.g., promoter, enhancer, terminator) and/or one or more translation signals, a signal sequence or leader sequence, and the like. Suitable expression vectors include, for example, pTT (National Research Council Canada), pcDNA3.1 (Invitrogen), pIRES (Clontech), pEAK8 (EdgeBioSystems), pCEP4 (Invitrogen). Expression control elements and a signal sequence, if present, can be provided by the vector or other source. For example, the transcriptional and/or translational control sequences of a cloned nucleic acid encoding an antibody chain can be used to direct expression.

A promoter can be provided for expression in a desired host cell. Promoters can be constitutive or inducible. For example, a promoter can be operably linked to a nucleic acid encoding a fusion protein of the invention, such that it directs transcription of the nucleic acid. A variety of suitable promoters for prokaryotic (e.g., lac, tac, T3, T7 promoters for E. coli) and eukaryotic (e.g., simian virus 40 early or late promoter, Rous sarcoma virus long terminal repeat promoter, cytomegalovirus promoter, adenovirus late promoter) hosts are available.

In addition, expression vectors typically comprise a selectable marker for selection of host cells carrying the vector, and, in the case of a replicable expression vector, an origin or replication. Genes encoding products which confer antibiotic or drug resistance are common selectable markers and may be used in prokaryotic (e.g., lactamase gene (ampicillin resistance), Tet gene for tetracycline resistance) and eukaryotic cells (e.g., neomycin (G418 or geneticin), gpt (mycophenolic acid), ampicillin, or hygromycin resistance genes). Dihydrofolate reductase marker genes permit selection with methotrexate in a variety of hosts. Genes encoding the gene product of auxotrophic markers of the host (e.g., LEU2, URA3, HIS3) are often used as selectable markers in yeast. Use of viral (e.g., baculovirus) or phage vectors, and vectors which are capable of integrating into the genome of the host cell, such as retroviral vectors, are also contemplated. Suitable expression vectors for expression in mammalian cells and prokaryotic cells (E. coli), insect cells (Drosophila Schnieder S2 cells, Sf9) and yeast (P. methanolica, P. pastoris, S. cerevisiae) are well-known in the art.

Recombinant host cells that express a fusion protein of the invention and a method of preparing a fusion protein as described herein are provided. The recombinant host cell comprises a recombinant nucleic acid encoding a recombinant fusion protein. Recombinant fusion proteins can be produced by the expression of a recombinant nucleic acid encoding the protein in a suitable host cell, or using other suitable methods. For example, the expression constructs described herein can be introduced into a suitable host cell, and the resulting cell can be maintained (*e.g.*, in culture, in an animal) under conditions suitable for expression of the constructs. Suitable host cells can be prokaryotic, including bacterial cells such as *E. coli*, *B. subtilis* and or other suitable bacteria, eukaryotic, such as fungal or yeast cells (*e.g.*, *Pichia pastoris*, *Aspergillus* species, *Saccharomyces cerevisiae, Schizosaccharomyces pombe, Neurospora crassa*), or other lower eukaryotic cells, and cells of higher eukaryotes such as those from insects (*e.g.*, Sf9 insect cells (WO 94/26087 (O'Connor)) or mammals (*e.g.*, COS cells, such as COS-1 (ATCC Accession No. CRL-1650) and COS-7 (ATCC Accession No. CRL-1651), CHO (e.g., ATCC Accession No. CRL-9096), 293 (ATCC Accession No. CRL-1573), HeLa (ATCC Accession No. CCL-2), CV1 (ATCC Accession No. CCL-70), WOP (Dailey et al., J. Virol. 54:739-749 (1985)), 3T3, 293T (Pear et al., Proc. Natl. Acad. Sci. U.S.A., 90:8392-8396 (1993)), 293-6E cells (National Research Council Canada), NSO cells, SP2/0, HuT 78 cells, and the like (see, e.g., Ausubel, F.M. et al., eds. Current Protocols in Molecular Biology, Greene Publishing Associates and John Wiley & Sons Inc., (1993)).

The invention also includes a method of producing a recombinant fusion protein, comprising maintaining a recombinant host cell of the invention under conditions appropriate for expression of a recombinant fusion protein. The method can further comprise the step of isolating or recovering the recombinant fusion protein, if desired. In another embodiment, the components of the recombinant fusion protein are chemically assembled to create a continuous polypeptide chain.

The invention also provides an isolated recombinant nucleic acid encoding the novel fusion proteins described herein, and a recombinant vector (e.g., expression vector) that contain a recombinant nucleic acid encoding the novel fusion proteins described herein. The invention also relates to an isolated host cell (e.g., non-human host cell) that contains such a nucleic acid or recombinant vector.

The invention also relates to a method for producing a recombinant fusion protein of the invention comprising maintaining host cell (e.g., non-human host cell) that contains a recombinant nucleic acid encoding the novel fusion proteins described herein, or a recombinant vector (e.g., expression vector) that contain a recombinant nucleic acid encoding the novel fusion proteins described herein, under conditions suitable for expression, whereby a recombinant fusion protein is produced. In some embodiments, the method further comprises isolating the recombinant fusion protein (e.g., from the host cell, or the culture medium in which the host cell is maintained.)

COMPOSITIONS AND THERAPEUTIC AND DIAGNOSTIC METHODS

Compositions comprising fusion proteins of the invention including pharmaceutical or physiological compositions (e.g., for human and/or veterinary administration) are provided. Pharmaceutical or physiological compositions comprise one or more fusion protein and a pharmaceutically or physiologically acceptable carrier. Typically, these carriers include aqueous or alcoholic/aqueous solutions, emulsions or suspensions, including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in suspension, may be chosen from thickeners such as carboxymethylcellulose, polyvinylpyrrolidone, gelatin and alginates. Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition).

The compositions can comprise a desired amount of fusion protein. For example the compositions can comprise about 5% to about 99% fusion protein by weight. In particular embodiments, the composition can comprise about 10% to about 99%, or about 20% to about 99%, or about 30% to about 99% or about 40% to about 99%, or about 50% to about 99%, or about 60% to about 99%, or about 70% to about 99%, or about 80% to about 99%, or about 90% to about 99%, or about 95% to about 99% fusion protein, by weight. In one example, the composition is freeze dried (lyophilized).

The drug compositions described herein will typically find use in preventing, suppressing or treating disease states, such as inflammatory states, cancer, pain, and the like. The drug compositions (*e.g.*, drug conjugates, noncovalent drug conjugates, drug fusions), described herein can also be administered for diagnostic purposes.

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

Animal model systems which can be used to screen the effectiveness of drug compositions in protecting against or treating the disease are available. Methods for the testing of systemic lupus erythematosus (SLE) in susceptible mice are known in the art (Knight et al. (1978) J. Exp. Med., 147: 1653; Reinersten et al. (1978) New Eng. J. Med., 299: 515). Myasthenia Gravis (MG) is tested in SJL/J female mice by inducing the disease with soluble AchR protein from another species (Lindstrom et al. (1988) Adv. Immunol., 42: 233). Arthritis is induced in a susceptible strain of mice by injection of Type II collagen (Stuart et al. (1984) Ann. Rev. Immunol., 42: 233). A model by which adjuvant arthritis is induced in susceptible rats by injection of mycobacterial heat shock protein has been described (Van Eden et al. (1988) Nature, 331: 171). Effectiveness for treating osteoarthritis can be assessed in a murine model in which arthritis is induced by intra-articular injection of collagenase (Blom, A.B. et al., Osteoarthritis Cartilage 12:627-635 (2004). Thyroiditis is induced in mice by administration of thyroglobulin as described (Maron et al. (1980) J. Exp. Med., 152: 1115). Insulin dependent diabetes mellitus (IDDM) occurs naturally or can be induced in certain strains of mice such as those described by Kanasawa et al. (1984) Diabetologia, 27: 113. EAE in mouse and rat serves as a model for MS in human. In this model, the demyelinating disease is induced by administration of myelin basic protein (see Paterson (1986) Textbook of Immunopathology, Mischer et al., eds., Grune and Stratton, New York, pp. 179-213; McFarlin et al. (1973) Science, 179: 478: and Satoh et al. (1987) J. Immunol., 138: 179).

The drug compositions of the present invention may be used as separately administered compositions or in conjunction with other agents. Pharmaceutical compositions can include "cocktails" of various cytotoxic or other agents in conjunction with the drug composition of the present invention, or combinations of drug compositions (*e.g.*, fusion proteins) according to the present invention comprising different drugs.

The drug compositions can be administered to any individual or subject in accordance with any suitable techniques. A variety of routes of administration are possible including, for example, oral, dietary, topical, transdermal, rectal, parenteral (*e.g.*, intravenous, intraarterial, intramuscular, subcutaneous, intradermal, intraperitoneal, intrathecal, intraarticular injection), and inhalation (*e.g.*, intrabronchial, intranasal or oral inhalation, intranasal drops) routes of administration, depending on the drug composition and disease or condition to be treated. Administration can be local or systemic as indicated. The preferred mode of administration can vary depending upon the fusion protein chosen, and the condition (*e.g.*, disease) being treated. The dosage and frequency of administration will depend on the age, sex and condition of the patient, concurrent administration of other drugs, counter-indications and other parameters to be taken into account by the clinician. A therapeutically effective amount of a drug composition (*e.g.*, fusion protein) is administered. A therapeutically effective amount is an amount sufficient to achieve the desired therapeutic effect, under the conditions of administration.

The term "subject" or "individual" is defined herein to include animals such as mammals, including, but not limited to, primates (*e.g.*, humans), cows, sheep, goats, horses, dogs, cats, rabbits, guinea pigs, rats, mice or other bovine, ovine, equine, canine, feline, rodent or murine species.

The drug composition (*e.g.*, fusion protein) can be administered as a neutral compound or as a salt. Salts of compounds (*e.g.*, fusion proteins) containing an amine or other basic group can be obtained, for example, by reacting with a suitable organic or inorganic acid, such as hydrogen chloride, hydrogen bromide, acetic acid, perchloric acid and the like. Compounds with a quaternary ammonium group also contain a counteranion such as chloride, bromide, iodide, acetate, perchlorate and the like. Salts of compounds containing a carboxylic acid or other acidic functional group can be prepared by reacting with a suitable base, for example, a hydroxide base. Salts of acidic functional groups contain a countercation such as sodium, potassium and the like.

The invention also provides a kit for use in administering a drug composition (*e.g.*, fusion protein) to a subject (*e.g.*, patient), comprising a drug composition (*e.g.*, fusion protein), a drug delivery device and, optionally, instructions for use. The drug composition (*e.g.*, fusion protein) can be provided as a formulation, such as a freeze dried formulation. In certain embodiments, the drug delivery device is selected from the group consisting of a syringe, an inhaler, an intranasal or ocular administration device (*e.g.*, a mister, eye or nose dropper), and a needleless injection device.

The drug composition (*e.g.*, fusion protein) of this invention can be lyophilized for storage and reconstituted in a suitable carrier prior to use. Any suitable lyophilization method (*e.g.*, spray drying, cake drying) and/or reconstitution techniques can be employed. It will be appreciated by those skilled in the art that lyophilisation and reconstitution can lead to varying degrees of antibody activity loss (*e.g.*, with conventional immunoglobulins, IgM antibodies tend to have greater activity loss than IgG antibodies) and that use levels may have to be adjusted to compensate. In a particular embodiment, the invention provides a composition comprising a lyophilized (freeze dried) drug composition (*e.g.*, fusion protein) as described herein. Preferably, the lyophilized (freeze dried) drug composition (*e.g.*, fusion protein) loses no more than about 20%, or no more than about 25%, or no more than about 30%, or no more than about 35%, or no more than about 40%, or no more than about 45%, or no more than about 50% of its activity when rehydrated. Activity is the amount of drug composition (*e.g.*, fusion protein) required to produce the effect of the drug composition before it was lyophilized. For example, the amount of fusion protein needed to achieve and maintain a desired serum concentration for a desired period of time. The activity of the drug composition (*e.g.*, fusion protein) can be determined using any suitable method before lyophilization, and the activity can be determined using the same method after rehydration to determine amount of lost activity.

Compositions containing the drug composition (*e.g.*, fusion protein) or a cocktail thereof can be administered for prophylactic and/or therapeutic treatments. In certain therapeutic applications, an amount sufficient to achieve the desired therapeutic or prophylactic effect, under the conditions of administration, such as at least partial inhibition, suppression, modulation, killing, or some other measurable parameter, of a population of selected cells is defined as a "therapeutically-effective amount or dose." Amounts needed to achieve this dosage will depend upon the severity of the disease and the general state of the patient's own immune system and general health, but generally range from about 0.005 to 10.0 mg of fusion protein per kilogram of body weight, with doses of 0.05 to 2.0 mg/kg/dose being more commonly used. For prophylactic applications, compositions containing the drug composition (*e.g.*, fusion protein) or cocktails thereof may also be administered in similar or slightly lower dosages. A composition containing a drug composition (*e.g.*, fusion protein) according to the present invention may be utilized in prophylactic and therapeutic settings to aid in the alteration, inactivation, killing or removal of a select target cell population in a mammal.

The invention also relates to a drug delivery device comprising the composition (*e.g.*, pharmaceutical composition) or fusion protein of the invention. In some embodiments, the drug delivery device is selected from the group consisting of parenteral delivery device, intravenous delivery device, intramuscular delivery device, intraperitoneal delivery device, transdermal delivery device, pulmonary delivery device, intraarterial delivery device, intrathecal delivery device, intraarticular delivery device, subcutaneous delivery device, intranasal delivery device, vaginal delivery device, rectal delivery device, syringe, a transdermal delivery device, a capsule, a tablet, a nebulizer, an inhaler, an atomizer, an aerosolizer, a mister, a dry powder inhaler, a metered dose inhaler, a metered dose sprayer, a metered dose mister, a metered dose atomizer, and a catheter.

It is expected that the conservation of structural features and avoidance of exposure of charged residues, that could be achieved by natural junctions in some circumstances, could be demonstrated in a proteolysis assay. The assay can be carried out as follows. A solution of the recombinant protein (1mg/mL in phosphate buffered saline) is supplemented with 0.04 mg/mL of sequencing grade trypsin (available from Promega) and incubated at 30°C. At intervals, aliquots of the protein solution are withdrawn, mixed with a stop solution (containing SDS loading buffer and protease inhibitors) and snap frozen. Aliquots are withdrawn after times ranging from, for example, 5 minutes to 24 hours. After completion of the time course, the extent of proteolysis is assessed, for example by separation of samples on SDFS-PAGE gels and visualization with a protein stain such as Coomassie Blue. It is expected that fusion proteins with natural junctions would be more resistant to fragmentation that corresponding fusion proteins that contain non-natural junctions.

EXAMPLES

EXAMPLE 1: GENERAL METHODS

Construction of expression vectors

IgGs were expressed using a vector based on the Invitrogen pBudCE4.1 backbone. The backbone was modified by deleting a unique NheI restriction site, which was achieved by NheI restriction digestion, fill-in using Klenow enzyme, and self-ligation, using standard protocols. IgG heavy and light chain expression cassettes comprising a Kozak sequence, murine V-J2-C signal peptide cDNA and constant region cDNA were prepared. The heavy chain expression cassette encoding a human IgG heavy chain constant domain was digested using HindIII and BglII restriction enzymes and sub-cloned into the modified vector backbone that was digested using HindIII and BamHI restriction enzymes, thereby deleting an internal BamHI restriction site in the vector backbone. Light chain expression cassettes encoding human kappa or lambda constant region genes were sub-cloned into the vector backbone using NotI and MluI restriction enzymes.

Sub-cloning of variable domain genes

IgG variable domain genes were sub-cloned into the expression vectors described above using standard molecular biology protocols. IgG variable domain genes used for expression as part of the heavy chain were sub-cloned using a BamHI restriction site in the heavy chain signal peptide cDNA and a XhoI restriction site or NheI restriction site in the cDNA encoding the mature heavy chain protein. IgG variable domain genes used for expression as part of the light chain were subcloned in one of two ways. The IgG variable domain genes were either joined to light chain cDNA using PCR overlap extension, and subsequently sub-cloned using a SalI restriction site in the light chain signal peptide cDNA and the MluI restriction site located downstream of the light chain expression cassette, or they were sub-cloned directly using a SalI restriction site in the cDNA encoding the light chain signal peptide and a BsiWI restriction site in cDNA encoding the mature light chain peptide.

Expression, purification and quantification of IgGs

Following DNA sequence verification, vector DNAs were produced using the Qiagen EndoFree Plasmid Mega kit, according to manufacturer's instructions. The vector DNAs were then used to transfect HEK293T cells (ATCC®). For each construct, cells were typically cultured in 5 or 10 cell culture flasks with a 175cm² surface area (T175, Nunc) until they reached approximately 70%-80% confluency. Cells were then transfected using 34 microgram of DNA per flask, using FuGENE® 6 Transfection Reagent (lipid-based transfection reagent, Roche), according to manufacturer's instructions. Transfected cells were grown in DMEM with glutamine and high glucose (Invitrogen) supplemented with 1% non-essential amino acids and 4% foetal bovine serum (FBS). The FBS was prepared from Invitrogen ultra-low IgG FBS by removing residual bovine IgG, using PROSEP®-G resin (recombinant protein G resin, Millipore), followed by sterile filtration. Culture supernatants were harvested by centrifugation after 4 or 5 days of expression. Secreted IgG was affinity purified using protein A resin (Streamline A, GE Healthcare) in the case of IgG molecules comprising 2 VH and 2 Vκ domains or 4 Vκ domains, or using protein G resin engineered without Fab binding sites (protein G agarose, Sigma Aldrich) in the case of IgG molecules comprising 4 VH domains. Resins were typically washed using 20-50 bed volumes of 2xPBS followed by 10-20 bed volumes of 150 mM NaCl, 10 mM Tris HCl, pH 7.4. IgGs were typically eluted using either 100 mM glycine pH 2.0 and neutralized to pH 8.0 using Tris, or they were eluted using 10 mM citrate, 50% ethylene glycol, pH 3.5. Eluted proteins were quantified by absorbance reading at 280 nm, using a spectrophotometer.

Size exclusion chromatography

IgGs were analyzed by HPLC size exclusion chromatography, using CHROMELEON® software (chromatography management software, Dionex Corporation). Analysis parameters most typically included using a Tosoh G3000 SWXL column, with 1xPBS supplemented with 10% ethanol as running buffer at a 1 mL/min flow rate, and an acquisition period of 20 minutes following injection. Absorbance was recorded at 225, 280 and 300 nm wavelengths.

EXAMPLE 2: PROTEIN EXPRESSION AND FORMATION OF SOLUBLE OLIGOMERS AND AGGREGATES

Two Vκ variable domains, designated DOM9-155-25 and DOM10-176-535 were paired into IgGs containing a total of 4 Vκ variable domains per molecule. The Vκ domain DOM10-176-535 was expressed as part of a native light chain while the Vκ domain DOM9-155-25 was fused to CH1 on the heavy chain, using three different junctions.

| Kabat number: | 97 | 114 |
| --- | --- | --- |
| Unnatural junction 1: | TFGQGTKVEIK__ | ASTKGPS |
| Unnatural junction 2: | TFGQGTKVEIKR_ | ASTKGPS |
| Natural junction: | TF<u>GQGT</u>LVTVSS | ASTKGPS |

For each junction the fusion is underlined
Unnatural junction 1 (SEQ ID NO:522) represents the direct fusion of a Vκ domain comprising Kabat residues 1-112 with CH1, while unnatural junction 2 (SEQ ID NO:523) represents the direct fusion of a Vκ domain also comprising Kabat residue 113 (partially encoded by the Jκ exon and partially by the Cκ exon in humans) with CH1. In IgGs with the natural junction (SEQ ID NO:524) the conserved GlyXaaGlyThr motif (SEQ ID NO:386) (residues H104-H107 in VH domains and L99-L102 in Vκ domains) was used as the fusion site.
Expression yields were compared using absorbance reading at 280 nm wavelength and confirmed by size exclusion HPLC and SDS-PAGE. The yields are summarized in Table 1. The expression yield was significantly higher with the natural junction (SEQ ID NO:524) than with either unnatural junction 1 (SEQ ID NO:522) or unnatural junction 2 (SEQ ID NO:523).
The use of natural junctions reduced the proportion of soluble oligomers and aggregates compared to using unnatural junctions for some antibodies. For example, three IgGs were expressed which comprised the same Vκ domain, designated VκDUM-1, as part of a native light chain and fused to CH1 on the heavy chain using different junctions. The three IgGs were analyzed by size exclusion HPLC (Table 2). The fraction of oligomers and aggregates was 9% for the IgG with unnatural junction 1 (SEQ ID NO:522) and 10% for the IgG with unnatural junction 2 (SEQ ID NO:523), but only 7% for the IgG with the natural junction (SEQ ID NO:524), indicating that fewer oligomers and aggregates were expressed and purified when the natural junction was used. A reduction in oligomers and aggregates by a few percent provides advantages and reduces the costs and time required to produce the fusion proteins, especially for industrial scale production.

Table 1

| Variable domain fused to Cκ | Variable domain fused to CH1 | Junction | Expression yield (mg/L) |
| --- | --- | --- | --- |
| DOM10-176-535 | DOM9-155-25 | Natural junction | 1.4 |
| DOM10-176-535 | DOM9-155-25 | Unnatural junction 1 | 1.0 |
| DOM10-176-535 | DOM9-155-25 | Unnatural junction 2 | 0.4 |

Table 2

| Variable domain fused to Cκ | Variable domain fused to CH1 | Junction | Percentage of oligomers and aggregates purified on protein A |
|---|---|---|---|
| VHDUM-1 | VκDUM-1 | Natural junction | 7 |
| VHDUM-1 | VκDUM-1 | Unnatural junction 1 | 9 |
| VHDUM-1 | VκDUM-1 | Unnatural junction 2 | 10 |

EXAMPLE 3: PROTEIN SOLUBILITY

A VH variable domain, designated VHDUM-1, was expressed in IgG molecules containing 4 copies of this variable domain. The solubility of three molecules was compared, two of the molecules had an unnatural junction between VHDUM-1 and Cκ and one domain had a natural junction between VHDUM-1 and Cκ.

```
Kabat number:        100              109
Unnatural junction 1:   FDYWGQGTLVTVSS__TVAAPS
Unnatural junction 2:   FDYWGQGTLVTVSS_RTVAAPS
Natural junction:       FDYWGQGTKVEIK R TVAAPS
```

For each junction the fusion site is underlined

Following elution from protein G resin and neutralization, strong precipitation was observed for the IgG with unnatural junction 1 (SEQ ID NO:525), while only traces of precipitation were observed for the IgG with unnatural junction 2 (SEQ ID NO:526) and for the IgG with the natural junction (SEQ ID NO:527). The concentration of soluble protein remaining in solution after neutralization (100 mM glycine, 130 mM Tris pH8) was 0.16 mg/mL for the IgG with unnatural junction 1, 1.37 mg/mL for the IgG with unnatural junction 2, and 1.20 mg/mL for the IgG with the natural junction. The data demonstrated that the IgG with unnatural junction 1 had significantly lower solubility in 100 mM glycine, 130 mM Tris pH 8 than the other IgGs. This result suggested that residue L108 (Arg) that is part of the natural junction between Vκ and Cκ domains played an important role in the structure and solubility of the tested IgGs. This residue is partially encoded by the Jκ exon and partially by the Cκ exon in humans and was absent in the poorly soluble IgG with unnatural junction 1. The observed differences in solubility demonstrated the benefit of moving the domain fusion site to the GlyXaaGlyThr (SEQ ID NO:386) motif that is conserved between Vκ (residues L99 - L102) and VH (residues H104 - H107), thus preserving the remaining structurally important Vκ residues encoded by the Jκ exon downstream of the conserved motif.

EXAMPLE 4: CLONING, EXPRESSION AND CHARACTERIZATION OF DOM15/16 INLINE FUSIONS

Domain antibodies that bind VEGF or EGFR were incorporated into fusion polypeptides that contained an anti-VEGFR dAb and an anti-EGFR dAb in a single polypeptide chain. Some of the fusion polypeptides also included an antibody Fc region (-CH2-CH3 of human IgG1). Specific examples of the fusion polypeptides that were cloned and expressed include TAR15-10 fused to DOM16-39-206 and to Fc; DOM16-39-206 fused to TAR15-10 and to Fc; DOM16-39-206 fused to TAR15-26-501 and to Fc; TAR15-26-501 fused to DOM16-39-206 and to Fc; TAR15-10 fused to DOM16-39-206; DOM16-39-206 fused to TAR15-10; DOM16-39-206 fused to TAR15-26-501; and TAR15-26-501 fused to DOM16-39-206. The positions of the foregoing fusions are listed as they appear in the fusion proteins from amino terminus to carboxy terminus. Polypeptides that are referred to using the prefix TAR or DOM are antibody variable domains.

DNA encoding dAbs was PCR amplified and cloned into expression vectors using standard methods. Inline fusion polypeptides were produced by expressing the expression vectors in *Pichia* (fusion that did not contain an Fc region) or in HEK 293T cells (Fc region containing fusions). Inline fusions were batch bound and affinity purified on streamline protein A and streamline protein L resins for HEK 293T cells (Fc-tagged) and *Pichia* expressed constructs respectively.

The portions of several fusions that contain Fc are listed in Table 3 as they appear in the fusion proteins, from amino terminus to carboxy terminus. Accordingly, the structure of the fusion proteins can be appreciated by reading the table from left to right. The first fusion protein presented in Table 3 has the structure, from amino terminus to carboxy terminus, DOM15-10-Linker 1-DOM16-39-206-Linker 2- Fc.

General robustness and resistance to degradation were tested by subj ecting the inline fusions to proteolysis with trypsin. A solution of dual specific ligand and trypsin (1/25 (w/w) trypsin to ligand) was prepared and incubated at 30°C. Samples were taken at 0 minutes (*i.e.*, before addition of trypsin), 60 minutes, 180 minutes, and 24 hours. At the given time points, the reaction was stopped by the addition of complete protease inhibitor cocktail at 2X final concentration (Roche code: 11 836 145 001) with PAGE loading dye, followed by flash freezing the samples in liquid nitrogen. Samples were analyzed by SDS-PAGE, and protein bands were visualized to reveal a time course for the protease degradation of the fusions.

These experiments showed that inline fusions having a "natural" linker (KVEIKRTVAAPS (SEQ ID NO:528), which contains the carboxy-terminal amino acids of Vκ and amino-terminal amino acids of Cκ, were susceptible proteolysis, with degradation evident at the 10 minute time point. SDS-PAGE analysis revealed that degradation occurred at the linkers between dAbs and at the linkers between dAb and Fc.

New linkers were designed that contain fewer Lys and Arg residues, which are cleavage points for trypsin and are abundant in the natural linker. Fusions that contained the engineered linkers (LVTVSSAST (SEQ ID NO:529)) or (LVTVSSGGGGSGGGS (SEQ ID NO:530)) showed much improved resistance to trypsin proteolysis.

Additional binding assays were performed to assess the potency of the inline fusions that contained the engineered linkers. The results revealed engineered linkers did not have any substantial adverse effect on potency.

Table 3: Fusion polypeptides that contain Fc

| dAb1 | Linker 1 | dAb2 | Linker 2 | Assay dAb1 (nM) | Assay dAb2 (nM) |
|---|---|---|---|---|---|
| DOM15-10 (Vκ) | KVEIKRTVAAPS | DOM16-39-206 (Vκ) | KVEIKRTVAAPS | 0.45 | 23.8 |
| DOM16-39-206 (Vκ) | KVEIKRTVAAPS | DOM15-10 (Vκ) | KVEIKRTVAAPS | 3.7 | 0.88 |
| DOM16-39-206 (Vκ) | KVEIKRTVAAPS | DOM15-26-501 (V$_H$) | LVTVSSASTKGPS | 20.7 | 21.3 |
| DOM15-26-501 (V$_H$) | LVTVSSASTKGPS | DOM16-39-206 (Vκ) | KVEIKRTVAAPS | 5.7 | 7.7 |
| DOM16-39-601 (Vκ) | LVTVSSAST | DOM15-10 (Vκ) | LVTVSSAST | 0.68 | 10.8 |
| DOM16-39-601 (Vκ) | KVEIKRTVAAPS | DOM15-10 (Vκ) | KVEIKRTVAAPS | 0.77 | 2.9 |
| DOM15-10 (VK) | LVTVSSAST | DOM16-39-601 (Vκ) | LVTVSSAST | 1.2 | 4.2 |
| DOM16-39-601 (Vκ) | LVTVSSGGGGSGGGS | DOM15-10 (Vκ) | LVTVSSGGGGSGGGS | 5.7 | 0.2 |
| DOM15-10 (Vκ) | LVTVSSGGGGSGGGS | DOM16-39-601 (Vκ) | LVTVSSGGGGSGGGS | 0.8 | 3.1 |
| DOM15-10 (Vκ) | KVEIKRTVAAPS | DOM16-39-601 (Vκ) | KVEIKRTVAAPS | 0.2 | 2.9 |

EXAMPLE 5. ADDITIONAL ENGINEERED LINKERS

Several designed mutations were introduced to the C-terminal region of Vκ dAbs expressed on the light chain of IgG-like formats to reduce protease sensitivity. The "natural linker" was GQGTKVEIKRTVAAPS (SEQ ID NO:531) which contains the carboxy-terminal amino acids of Vκ and amino-terminal amino acids of Ck). Variant linkers 1-3 were designed with amino acid replacements that replaced some or all of the positively charged residues in the natural linker with the most conservative substitutions that are not positively charged at physiological pH. It is likely that the arginine residue in the natural linker is less amenable to alteration due to ionic interactions it forms within the CL domain.

Variant linker 1 (GQGTNVEINRTVAAPS (SEQ ID NO:532)) substitutes both lysines in the natural linker with asparagines. Variant linker 1, and variant linker 2 (GQGTNVEINQTVAAPS (SEQ ID NO:533)), which additionally changes the arginine in the natural linker to glutamine, introduce an N-glycosylation site (NxT) into the linker. SDS-PAGE analysis of IgG-like formats containing variant linker 1 or variant linker 2 showed that the light chain had a higher molecular weight, consistent with an N-glycosylation event. Variant linker 3 (GQGTNVEIQRTVAAPS (SEQ ID NO:534) removes the N-glycosylation site while leaving the arginine in the natural linker in place. Variant linker 4 (GQGTLVTVSSTVAAPS (SEQ ID NO:535)) replaces the six C-terminal amino acids of the Vκ domain with the corresponding residues from a VH domain, and is devoid of positive charges.

Protease resistance (trypsin resistance assessed as described in Example 4) of IgG-like formats that contain variant linkers 1-4 revealed that IgG-like formats that contained engineered variant linkers were more protease resistant than an IgG-like format that contained the natural linker.

EXAMPLE 6: CLONING, EXPRESSION AND CHARACTERIZATION OF DOM9/10 INLINE FUSIONS

A. Fusion Proteins

Cloning and production of anti-IL-4 and anti-IL-13 dual specificity dimer

Nucleic acids encoding the anti-IL-4 dAb DOM9-112 and anti-IL-13 dAb DOM10-53-343 were cloned into a construct that encoded an in-line fusion protein with a C-terminal cysteine. The amino acid sequence AST was present between the two dAbs, this sequence is the natural CH sequence present in natural antibodies. The construct was cloned in the *Pichia pastoris* vector pPICZα (Invitrogen). Electrocompetent cells (X-33 or KM71H) were transformed with the construct and transformants were selected on 100 μg/ml Zeocin. 500ml cultures were grown on BMGY media at 30° C, 250 rpm for 24 hrs until the $OD_{600}$ had reached ~15-20. The cells were then spun down and resuspended in BMMY media (containing 0.5% (v/v) methanol) to induce protein expression. The cultures were maintained at 30°C with shaking at 250 rpm. At 24 hour intervals the cultures were fed with the following incremental increase in the methanol concentration; 1%, 1.5% and 2% (v/v) using a 50% methanol solution. The cultures were then harvested by centrifugation and the supernatant containing the expressed protein stored at 4°C until required. The protein was purified from the supernatant using PrA streamline using the standard purification protocol.

The PrA purified protein was found to contain both dimer and monomer species. Therefore chromatofocusing was used to separate the two proteins. A Mono P 5/20 column was used (GE Healthcare) for the separation, using a pH gradient of 6 to 4. The poly-buffers used were as described by the manufacturer to make the 6 to 4 pH range. The sample was applied at pH6 and the pH gradient generated by using 100% buffer B over 35 column volumes run at 1ml/min. Dimer containing fractions were identified using SDS-PAGE and pooled for PEGylation.

The protein was then PEGylated using 40K PEG2-MAL using the method outlined above. This material was purified using anion exchange chromatography up to a purity >95%. The potency of the resulting dual specific ligand (PEGylated DOM9-112 (AST) DOM10-53-344) was determined in an IL-4 RBA and an IL-13 RBA. The potency of the anti-IL-4 arm of the dual specific ligand (13 nM) was slightly reduced compared with the potency of the dAb DOM9-112 monomer (3.5 nM), whereas the potency of the anti-IL-13 arm was maintained (310 pM for the dual specific ligand vs 230pM for the dAb monomer).

The anti-IL-4 and anti-IL-13 dAbs DOM9-112 and DOM10-53-344 were also cloned as an in-line fusion with the amino acid sequence ASTKGPS (SEQ ID NO:535) present between the two dAbs, this sequence is the start of the CH sequence present in natural antibodies. The potency of the resulting purified dual specific ligand (DOM9-112 (ASTKGPS) DOM10-53-344) was determined in an IL-4 RBA and an IL-13 sandwich ELISA. The potency of the anti-IL-4 arm was maintained (~1 nM) whereas the potency of the anti-IL-13 arm was only slightly reduced compared with the dAb monomer (40pM for the dAb monomer vs 120 pM for the dual specific ligand).

Additional dual targeting in-line fusions for IL-4 and IL-13.

To further understand the behaviour of dual targeting in-line fusions of IL4 and IL13 binding dAbs, a series of new in-line fusions and in-line fusion libraries were constructed. The DOM10-53 lineage was affinity matured using phage display using libraries diversifying triplet residues of FR1, CDR1, CDR2 and CDR3. The libraries were cloned in a phage vector and displayed as fusion protein to the gene3 protein as an (dAb 1 linker dAb2) in-line fusion with dAb1 being DOM9-112-210, the linker being amino acid residues ASTKGPS (SEQ ID NO:535) and dAb2 being the DOM10-53 library. The selection method, subcloning and expression in E coli and screening method were essentially performed as described above, except that in-line fusion constructs were used instead of single dAbs. Outputs were cloned into vector pDOM5 and expression supernatants were screened for improved expression by binding to a protein A coated Biacore chip.

In-line fusions with improved expression levels were expressed, purified and tested in a IL-13 sandwich ELISA and cell assay. A number of variants were selected (including DOM9-112-210 - ASTKGPS - DOM10-53-566). The most potent clones were DOM10-53-531 and DOM10-53-546 (see Table 4). Different protein preparations were made from these clones and these were tested in the IL-4 RBA and IL-13 sandwich assay as described above.

Table 4

| Clone name | Expression level (mg/l) | IL-13 Sanwich ELISA (EC50 nM) | IL-4 RBA (IC50 nM) |
|---|---|---|---|
| DOM9-112-210-DOM10-53-531 | | | |
| Prep 1 | 9.3 | 1.1/1.9 | 3.5/4.8 |
| Prep 2 | 11.5 | 4.9 | n.d. |
| Prep 3 | 4.5 | 2/2.8 | 13.9 |
| Prep 4 | 10 | 1 | 5.4 |
| DOM9-112-210-DOM10-53-546 | | | |
| Prep 1 | 2.2 | 0.62/0.77 | 4.3 |
| Prep 2 | 7.7 | 1 | 6 |

Further in-line fusions were constructed by SOE PCR of the DNA fragments encoding a dAb linker which is either ASTKGPS (SEQ ID NO:535), if the first dAb was a Vh, or TVAAPS (SEQ ID NO:536) if the first dAb was a Vκ. This PCR product was digested with SalI/NotI and ligated in the *E. coli* expression vector pDOM5. After transformation to MACH1 (Invitrogen) cells, the clones were sequence verified and the in-line fusions were expressed. Expression was done by growing *E. coli* in 2TY supplemented with Onex media (Novagen) for 2 nights at 30°C, the cells were centrifuged and the supernatant was incubated with either Protein-L or Protein-A resin. After elution from the resin, the quality and quantity of produced in-line fusion product was verified on SDS-PAGE. The vast majority of product formed had the molecular mass of an in-line fusion with only limited free monomer. Therefore, no additional purification steps were required and the material could be tested directly.

Using the above described method the following IL-4/IL-13 in-line fusions were expressed, purified and characterised:

    DOM9-112-210 - ASTKGPS - DOM10-208
    DOM9-112-210 - ASTKGPS - DOM10-212
    DOM9-112-210 - ASTKGPS - DOM10-213
    DOM9-112-210 - ASTKGPS - DOM10-215
    DOM9-112-210 - ASTKGPS - DOM10-224
    DOM9-112-210 - ASTKGPS - DOM10-270
    DOM9-112-210 - ASTKGPS - DOM10-416
    DOM9-112-210 - ASTKGPS - DOM10-236
    DOM9-112-210 - ASTKGPS - DOM10-273
    DOM9-112-210 - ASTKGPS - DOM10-275
    DOM9-112-210 - ASTKGPS - DOM10-276
    DOM9-112-210 - ASTKGPS - DOM10-277
    DOM10-208 - TVAAPS - DOM9-155-78
    DOM10-212 - TVAAPS - DOM9-155-78
    DOM10-213 - TVAAPS - DOM9-155-78
    DOM10-215 - TVAAPS - DOM9-155-78
    DOM10-224 - TVAAPS - DOM9-155-78
    DOM10-270 - TVAAPS - DOM9-155-78
    DOM10-416 - ASTKGPS - DOM9-155-78
    DOM10-236 - ASTKGPS - DOM9-155-78
    DOM10-273 - ASTKGPS - DOM9-155-78
    DOM10-275 - ASTKGPS - DOM9-155-78
    DOM10-276 - ASTKGPS - DOM9-155-78
    DOM10-277 - ASTKGPS - DOM9-155-78

Once purified, the expression levels were determined (mg/l) and the activities were tested in an RBA for IL-4 binding and in a sandwich ELISA for IL-13 binding. The amino acid sequences of the listed variable domains are disclosed in the International Patent Application by Domantis Limited, entitled Ligands that Bind IL-4 and/of IL-13, which was filed in the UK receiving office on January 24, 2007, and are incorporated herein by reference for the purpose of providing examples of variable domains that can be used to make fusion proteins that contain natural junctions. The table below (Table 5) summarizes the data for these in-line fusions:

Table 5

| clone name | Expression mg/ml | | | IL-13 Biacore | Dom10 RBA (EC50 nM) | DOM9 RBA (IC50 nM) |
|---|---|---|---|---|---|---|
| DOM9-112-210-DOM10-208 | 0.3 | 19.2 | 37.4 | √ | - | 2.48 |
| DOM9-112-210-DOM10-212 | 3.7 | | 6.3 | √ | 999.9 | 3.21 |
| DOM9-112-210-DOM10-213 | 5.6 | 0.2 | 4.9 | √ | 1152 | 4.29 |
| DOM9-112-210-DOM10-215 | 0.1 | 8.8 | 2.2 | √ | - | 14.19 |
| DOM9-112-210-DOM10-224 | 4.6 | | 13.4 | √ | 4575 | 2.75 |
| DOM9-112-210-DOM10-270 | 3.7 | | 2.7 | √ | 397.5 | 2.79 |
| DOM9-112-210-DOM10-416 | 6.9 | | 0.0 | √ | 34420 | 7.27 |
| DOM9-112-210-DOM10-236 | 0.2 | 0.1 | 2.2 | √ | - | >20 |
| DOM9-112-210-DOM10-273 | 1.2 | | 0.3 | √ | 4553 | 10.51 |
| DOM9-112-210-DOM10-275 | 4.9 | 0.2 | 0.0 | √ | - | 10.89 |
| DOM9-112-210-DOM10-276 | 6.9 | | 0.1 | √ | - | 10.20 |
| DOM9-112-210-DOM10-277 | 1.3 | 3.7 | 0.2 | √ | 4385 | 11.74 |
| DOM10-208-DOM9-155-78 | | | 41.0 | √ | 4243 | 8.18 |
| DOM10-212-DOM9-155-78 | | | 0.5 | √ | - | >20 |
| DOM10-213-DOM9-155-78 | | | 16.9 | √ | 62.04 | 6.91 |
| DOM10-215-DOM9-155-78 | | | 22.6 | √ | 10.82 | 6.65 |
| DOM10-224-DOM9-155-78 | | | 3.6 | √ | - | 12.49 |
| DOM10-270-DOM9-155-78 | | | 2.9 | √ | 37.23 | 8.60 |
| DOM10-416-DOM9-155-78 | | 1.1 | 26.3 | √ | 443.7 | 5.88 |

(continued)

| clone name | Expression mg/ml | | IL-13 Biacore | Dom10 RBA (EC50 nM) | DOM9 RBA (IC50 nM) |
|---|---|---|---|---|---|
| DOM10-236-DOM9-155-78 | 3.6 | 10.8 | √ | 372 | 2.54 |
| DOM10-273-DOM9-155-78 | 6.4 | 16.2 | √ | 185.2 | 2.25 |
| DOM10-275-DOM9-155-78 | 0.2 | 0.0 | - | - | - |
| DOM10-276-DOM9-155-78 | 0.2 | 20.0 | √ | - | 5.02 |
| DOM10-277-DOM9-155-78 | 1.1 | 1.3 | √ | 648 | 9.45 |
| | | | | DOM9-112 | 3.60 |
| | | | | DOM9-155-78 | 0.41 |

Furthermore, an affinity matured variant of DOM 10-275, *i.e.* DOM10-275-1, was specifically chosen to be paired with both DOM9-112-210 and DOM9-155-78. These in-line fusions were constructed and expressed as described above using a natural linker. In addition to testing in the mentioned IL-4 RBA and IL-13 sandwich ELISA, these in-line fusions were also tested for functionality in a TF-1 cell proliferation assay. In these assays the dAb was preincubated with a fixed amount of either IL-4 or IL-13, this mixture was added to the TF-1 cells and the cells were incubated for 72 hours. After this incubation, the level of cell proliferation was determined. The results of this assay are summarized below (Table 6) and demonstrate that both arms of the in-line fusion were active in the cell assay.

Table 6

| Sample | DOM9 RBA IC50 (nM) | DOM10 RBA IC50 (nM) | Il-4 cell assay IC50 (nM) | IL-13 cell assay IC50 (nM) |
|---|---|---|---|---|
| DOM9-112-210 | 0.391 | - | | |
| DOM9-155-78 | 0.456 | - | | |
| DOM10-275-1-DOM9-155-78 | 6.238 | 39.17 | 5.1 - 7.6 | 31 - 46 |
| DOM9-112-210-DOM10-275-1 | 4.189 | 44.88 | 6.8 - 10.2 | 27 - 40 |
| IDOM10-275-1 | - | 31.30 | | |

Table 7

| IgGs including 4 VH variable domains expressed with natural junctions | | | | |
|---|---|---|---|---|
| Number | Heavy chain variable domain | Light chain variable domain | Junction between GQGT in JH-segment and non-native constant domain | Non-native constant domain |
| 1. | VHDUM-1 | VHDUM-1 | KVEIKR (SEQ ID NO: 471) | CK |
| 2. | VHDUM-1 | VHDUM-1 | KVTVL (SEQ ID NO: 482) | CL2 |
| 3. | VHDUM-1 | VHDUM-1 | LVTVL (SEQ ID NO: 483) | CL2 |

(continued)

| IgGs including 4 VH variable domains expressed with natural junctions | | | | |
|---|---|---|---|---|
| Number | Heavy chain variable domain | Light chain variable domain | Junction between GQGT in JH-segment and non-native constant domain | Non-native constant domain |
| 4. | VHDUM-1 | DOM10-53-345 | KVEIKR (SEQ ID NO: 471) | CK |
| 5. | VHDUM-1 | DOM10-53-345 | KVTVL (SEQ ID NO: 482) | CL2 |
| 6. | HEL-4 | HEL-4 | KVEIKR (SEQ ID NO: 471) | CK |
| 7. | DOM9-112 | DOM10-53-285 | KVEIKR (SEQ ID NO: 471) | CK |
| 8. | DOM9-112 | DOM10-53-347 | KVEIKR (SEQ ID NO: 471) | CK |
| 9. | DOM9-112 | DOM10-53-337 | LVTVL (SEQ ID NO: 483) | CL2 |
| 10. | DOM9-112 | DOM10-53-343 | KVTVL (SEQ ID NO: 482) | CL2 |
| 11. | DOM9-112 | DOM10-53-343 | LVTVL LVTVL (SEQ ID NO: 483) | CL2 |
| 12. | DOM10-53-285 | DOM9-112 | KVEIKR (SEQ ID NO: 471) | CK |
| 13. | DOM10-53-338 | DOM9-112 | KVTVL (SEQ ID NO: 482) | CL2 |
| 14. | DOM10-53-338 | DOM9-112 | LVTVL | CL2 |
| 15. | DOM10-53-345 | VHDUM-1 | KVEIKR (SEQ ID NO: 471) | CK |
| 16. | DOM10-53-345 | VHDUM-1 | KVTVL (SEQ ID NO: 482) | CL2 |
| 17. | DOM10-53-347 | DOM9-112 | KVEIKR (SEQ ID NO: 471) | CK |
| 18. | DOM10-53-347 | DOM9-112 | KVTVL (SEQ ID NO: 482) | CL2 |
| 19. | DOM15-26 | DOM16-201 | KVEIKR (SEQ ID NO: 471) | CK |
| 20. | DOM15-26 | DOM15-26 | KVEIKR (SEQ ID NO: 471) | CK |

Table 8

| | IgGs including 4 VK variable domains expressed with natural junctions | | | |
|---|---|---|---|---|
| Number | Heavy chain variable domain | Light chain variable domain | Junction between GQGT in J-segment and non-native constant domain | Non-native constant domain |
| 21. | VKDUM-1 | VKDUM-1 | LVTVSS (SEQ ID NO: 484) | CH (IgG1) |
| 22. | DOM2-100-206 | DOM15-10 | LVTVSS (SEQ ID NO: 484) | CH (IgG1) |
| 23. | DOM4-122-24 | DOM4-130-54 | LVTVSS (SEQ ID NO: 484) | CH (IgG1) |
| 24. | DOM4-130-54 | DOM4-122-24 | LVTVSS (SEQ ID NO: 484) | CH (IgG1) |
| 25. | DOM4-130-54 | DOM4-130-54 | LVTVSS (SEQ ID NO: 484) | CH (IgG1) |
| 26. | DOM9-155-25 | DOM10-176-511 | LVTVSS (SEQ ID NO: 484) | CH (IgG1) |
| 27. | DOM9-155-25 | DOM10-176-535 | LVTVSS (SEQ ID NO: 484) | CH (IgG1) |
| 28. | DOM9-155-25 | DOM10-176 | LVTVSS (SEQ ID NO: 484) | CH (IgG1) |
| 29. | DOM9-155-25 | DOM10-176-535 | LVTVSS (SEQ ID NO: 484) | CH (IgG1) |
| 30. | DOM9-155-25 | DOM10-176-535 | LVTVSS (SEQ ID NO: 484) | CH (IgG1) |
| 31. | DOM9-155-29 | DOM10-176 | LVTVSS (SEQ ID NO: 484) | CH (IgG1) |
| 32. | DOM9-155-29 | DOM10-176-535 | LVTVSS (SEQ ID NO: 484) | CH (IgG1) |
| 33. | DOM9-44-502 | DOM10-176-511 | LVTVSS (SEQ ID NO: 484) | CH (IgG1) |
| 34. | DOM9-44-502 | DOM10-176 | LVTVSS (SEQ ID NO: 484) | CH (IgG1) |
| 35. | DOM10-176-511 | DOM9-44-502 | LVTVSS (SEQ ID NO: 484) | CH (IgG1) |
| 36. | DOM10-176-535 | DOM9-155-25 | LVTVSS (SEQ ID NO: 484) | CH (IgG1) |
| 37. | DOM15-10 | DOM15-10 | LVTVSS (SEQ ID NO: 484) | CH (IgG1) |
| 38. | DOM15-10 | DOM16-200 | LVTVSS (SEQ ID NO: 484) | CH (IgG1) |
| 39. | DOM15-10 | DOM16-32 | LVTVSS (SEQ ID NO: 484) | CH (IgG1) |
| 40. | DOM15-10 | DOM16-72 | LVTVSS (SEQ ID NO: 484) | CH (IgG1) |

(continued)

| IgGs including 4 VK variable domains expressed with natural junctions | | | | |
|---|---|---|---|---|
| Number | Heavy chain variable domain | Light chain variable domain | Junction between GQGT in J-segment and non-native constant domain | Non-native constant domain |
| 41. | DOM15-10 | DOM16-39 | LVTVSS (SEQ ID NO: 484) | CH (IgG1) |
| 42. | DOM15-10 | DOM2-100-206 | LVTVSS (SEQ ID NO: 484) | CH (IgG1) |
| 43. | DOM16-200 | DOM16-200 | LVTVSS (SEQ ID NO: 484) | CH (IgG1) |
| 44. | DOM16-32 | DOM15-10 | LVTVSS (SEQ ID NO: 484) | CH (IgG1) |
| 45. | DOM16-39 | DOM16-39 | LVTVSS (SEQ ID NO: 484) | CH (IgG1) |
| 46. | DOM16-39 | DOM15-10 | LVTVSS (SEQ ID NO: 484) | CH (IgG1) |
| 47. | DOM16-72 | DOM15-10 | LVTVSS (SEQ ID NO: 484) | CH (IgG1) |

Table 9

| "inside-out" IgGs expressed with natural junctions | | | | |
|---|---|---|---|---|
| Number | Heavy chain variable domain | Light chain variable domain | Junction between GQGT in J-segment and non-native constant domain | Non-native constant domain |
| 48. | DOM15-10 | DOM15-26 | LVTVSS (SEQ ID NO: 484 & KVEIKR (SEQ ID NO:471) | CH (IgG1) & CK |

In Tables 7-9, the non-native constant domain referred to in the right column is CH (IgG1) for IgGs comprising 2 Vκ variable domains, and either Cκ or Cλ2 for IgGs comprising 2 VH variable domains. For IgG 50 both constant domain sequences are non-native as this was an inside-out IgG with a VH variable domain fused to Cκ via the sequence KVEIKR and a Vκ variable domain fused to CH (IgG1) via the sequence LVTVSS.

Sequences of non-native constant domains:

CH (IgG1) (SEQ ID NO:517):

ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDK
SRWQQGNVFSCSVMHEALHNHYTQKSLSLSPGK

CK (SEQ ID NO:518):

TVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC

CL2 (SEQ ID NO:519):

GQPKAAPSVTLFPPSSEELQANKATLVCLISDFYPGAVTVAWKADSSPVKAGVETTTPS
KQSNNKYAASSYLSLTPEQWKSHRSYSCQVTHEGSTVEKTVAPTECS

Representative embodiments of the invention include:

1. A recombinant fusion protein comprising a hybrid domain, wherein said hybrid domain comprises a first portion derived from a first polypeptide and a second portion derived from a second polypeptide, said first polypeptide comprising a domain that has the formula (X1-Y-X2), and said second polypeptide comprising a domain that has the formula (Z1-Y-Z2), wherein

Y is a conserved amino acid motif;

X1 and Z1 are the amino acid motifs that are located adjacent to the amino-terminus of Y in said first polypeptide and said second polypeptide, respectively;

X2 and Z2 are the amino acid motifs that are located adjacent to the carboxy-terminus of Y in said first polypeptide and said second polypeptide, respectively;

with the proviso that when the amino acid sequences of X1 and Z1 are the same, the amino acid sequences of X2 and Z2 are not the same; and when the amino acid sequences of X2 and Z2 are the same, the amino acid sequences of X1 and Z1 are not the same;

wherein said hybrid domain has the formula

$$(X1\text{-}Y\text{-}Z2).$$

2. The recombinant fusion protein of embodiment 1, wherein said hybrid domain is bonded to an amino-terminal amino acid sequence D, and/or bonded to a carboxy-terminal amino acid sequence E, such that the recombinant fusion protein comprises a structure that has the formula

$$D\text{-}(X1\text{-}Y\text{-}Z2)\text{-}E;$$

wherein D is absent or is an amino acid sequence that is adjacent to the amino-terminus of (X1-Y-X2) in said first polypeptide; and

E is absent or is an amino acid sequence that adjacent to the carboxy-terminus of (Z1-Y-Z2) in said second polypeptide.

3. The recombinant fusion protein of embodiment 2, wherein D is present.

4. The recombinant fusion protein of embodiment 2, wherein E is present.

5. The recombinant fusion protein of embodiment 2, wherein D and E are present.

6. The recombinant fusion protein of embodiment 1 or embodiment 2, wherein (X1-Y-Z2) is a hybrid immunoglobulin variable domain.

7. The recombinant fusion protein of embodiment 6, wherein said hybrid immunoglobulin variable domain is a hybrid antibody variable domain.

8. The recombinant fusion protein of embodiment 7, wherein Y is in framework region (FR) 4.

9. The recombinant fusion protein of embodiment 8, wherein Y is GlyXaaGlyThr or GlyXaaGlyThrXaa(Val/Leu).

10. The recombinant fusion protein of embodiment 8, wherein X1 is a portion of an antibody variable domain comprising FR1, complementarity determining region (CDR) 1, FR2, CDR2, FR3, and CDR3.

11. The recombinant fusion protein of embodiment 7, wherein Y is in FR3.

12. The recombinant fusion protein of embodiment 11, wherein Y is GluAspThrAla, ValTyrTyrCys, or GluAspThrAlaValTyrTyrCys.

13. The recombinant fusion protein of embodiment 11, wherein X1 is a portion of an antibody variable domain comprising FR1, CDR1, FR2, and CDR2.

14. The recombinant fusion protein of embodiment 1, wherein (X1-Y-Z2) is a hybrid immunoglobulin constant domain.

15. The recombinant fusion protein of embodiment 14, wherein said hybrid immunoglobulin constant domain is a hybrid antibody constant domain.

16. The recombinant fusion protein of embodiment 15, wherein Y is (Ser/Ala/Gly)Pro(Lys/Asp/Ser)Val, (Ser/Ala/Gly)Pro(Lys/Asp/Ser)ValPhe, LysValAspLys(Ser/Arg/Thr) or ValThrVal.

17. The recombinant fusion protein of embodiment 16, wherein Y is selected from the group consisting of SerProLysVal, SerProAspVal, SerProSerVal, AlaProLysVal, AlaProAspVal, AlaProSerVal, GlyProLysVal, GlyProAspVal, GlyProSerVal, SerProLysValPhe, SerProAspValPhe, SerProSerValPhe, AlaProLysValPhe, AlaProAspValPhe, AlaProSerValPhe, GlyProLysValPhe, GlyProAspValPhe, GlyProSerValPhe, LysValAspLysSer, LysValAspLysArg, LysValAspLysThr, and or ValThrVal.

18. The recombinant fusion protein of embodiment 2, wherein D is absent, (X1-Y-Z2) is a hybrid immunoglobulin variable domain, and E is an immunoglobulin constant domain.

19. The recombinant fusion protein of embodiment 18, further comprising a second immunoglobulin variable domain that is amino terminal to (X1-Y-Z2).

20. The recombinant fusion protein of embodiment 2, wherein D is an immunoglobulin variable domain, and (X1-Y-Z2) is a hybrid immunoglobulin constant domain.

21. The recombinant fusion protein of embodiment 2, wherein (X1-Y-Z2) is a hybrid immunoglobulin constant domain, and E is an immunoglobulin constant domain.

22. The recombinant fusion protein of embodiment 21, wherein D is absent and the fusion protein comprises a further domain that is amino terminal to (X1-Y-Z2).

23. The recombinant fusion protein of embodiment 2, wherein D is an immunoglobulin constant domain, and (X1-Y-Z2) is a hybrid immunoglobulin constant domain.

24. The recombinant fusion protein of embodiment 1, wherein said first polypeptide and said second polypeptide are both members of the same protein superfamily.

25. The recombinant fusion protein of embodiment 1, wherein said protein superfamily is selected from the group consisting of the immunoglobulin superfamily, the TNF superfamily and the TNF receptor superfamily.

26. The recombinant fusion protein of embodiment 1, wherein said first polypeptide and said second polypeptide are both human polypeptides.

27. The recombinant fusion protein of embodiment 1, wherein X1, X2, Z1 and Z2 each, independently, consists of about 1 to about 200 amino acids.

28. The recombinant fusion protein of embodiment 1, wherein said hybrid domain is about the size of an immunoglobulin variable domain.

29. The recombinant fusion protein of embodiment 1, wherein said hybrid domain is about the size of an immunoglobulin constant domain.

30. The recombinant fusion protein of embodiment 1, wherein said hybrid domain is about 8 kDa to about 20 kDa.

31. An isolated recombinant nucleic acid molecule encoding the recombinant fusion protein of any one of embodiments 1-30.

32. A host cell comprising a recombinant nucleic acid molecule encoding the recombinant fusion protein of any one of embodiments 1-30.

33. A method of producing a recombinant fusion protein comprising maintaining the host cell of embodiment 32 under conditions suitable for expression of said recombinant nucleic acid, whereby said recombinant nucleic acid is expressed and said recombinant fusion protein is produced.

34. The method of embodiment 33, further comprising isolating said recombinant fusion protein.

35. A recombinant fusion protein comprising a hybrid immunoglobulin variable domain that is fused to an immunoglobulin constant domain, wherein said hybrid immunoglobulin variable domain comprises a hybrid framework region (FR) that comprises a portion from a first immunoglobulin FR from a first immunoglobulin and a portion from a second immunoglobulin FR from a second immunoglobulin, said first immunoglobulin FR and said second immunoglobulin FR each comprising a conserved amino acid motif Y, and said hybrid immunoglobulin FR has the formula

$$(F^1\text{-}Y\text{-}F^2)$$

wherein Y is said conserved amino acid motif;
$F^1$ is the amino acid motif located adjacent to the amino-terminus of Y in said first immunoglobulin FR; and
$F^2$ is the amino acid motif located adjacent to the carboxy-terminus of Y in said second immunoglobulin FR.

36. The recombinant fusion protein of embodiment 35, wherein Y is located in framework region (FR) 1, FR2 or FR3 of said first immunoglobulin and of said second immunoglobulin.

37. The recombinant fusion protein of embodiment 35, wherein Y is located in FR4 of said first immunoglobulin and of said second immunoglobulin.

38. The recombinant fusion protein of embodiment 35, wherein said hybrid FR is a hybrid FR4, and $F^2$ is adjacent to the amino-terminus of said immunoglobulin constant domain in a naturally occurring protein comprising said immunoglobulin constant domain.

39. The recombinant fusion protein of embodiment 35, wherein said immunoglobulin constant domain is a T cell receptor constant domain and said second immunoglobulin FR is a FR4 from a T cell receptor variable domain.

40. The recombinant fusion protein of embodiment 39, wherein $F^2$ is amino terminal to said immunoglobulin constant domain in a naturally occurring immunoglobulin.

41. The recombinant fusion protein of embodiment 35, wherein said immunoglobulin constant domain is an antibody light chain constant domain and said second immunoglobulin FR is a FR4 from an antibody light chain variable domain.

42. The recombinant fusion protein of embodiment 41, wherein $F^2$ is amino terminal to said antibody light chain constant domain in a naturally occurring antibody light chain.

43. The recombinant fusion protein of embodiment 41, wherein said antibody constant domain is a Cκ or Cλ, and said second antibody FR4 is a Vκ FR4 or Vλ FR4, respectively.

44. The recombinant fusion protein of embodiment 43, wherein said first antibody variable domain is an antibody heavy chain variable domain.

45. The recombinant fusion protein of embodiment 35, wherein said immunoglobulin constant domain is an antibody heavy chain constant domain and said second immunoglobulin FR is a FR4 from an antibody heavy chain variable domain.

46. The recombinant fusion protein of embodiment 35, wherein said first immunoglobulin is a non-human immunoglobulin.

47. The recombinant fusion protein of embodiment 46, wherein said non-human immunoglobulin is an immunoglobulin from a mouse, rat, shark, fish, possum, sheep, pig, *Camelid,* rabbit or non-human primate.

48. The recombinant fusion protein of embodiment 47, wherein said non-human immunoglobulin is a *Camelid* or nurse shark heavy chain antibody.

49. The recombinant fusion protein of embodiment 46, wherein said second immunoglobulin is a human immunoglobulin.

50. The recombinant fusion protein of embodiment 35, wherein said immunoglobulin constant domain is a human immunoglobulin constant domain.

51. The recombinant fusion protein of embodiment 35, wherein said hybrid immunoglobulin variable domain is a hybrid antibody variable domain.

52. The recombinant fusion protein of embodiment 51, wherein Y is GlyXaaGlyThr.

53. The recombinant fusion protein of embodiment 52, wherein $F^1$ is Phe and $F^2$ is (Leu/Met/Thr)ValTheValSerSer.

54. The recombinant fusion protein of embodiment 53, wherein $F^2$ is selected from the group consisting of LeuValTheValSerSer, MetValTheValSerSer; and ThrValTheValSerSer.

55. The recombinant fusion protein of embodiment 53, wherein said immunoglobulin constant domain is a human antibody constant domain.

56. The recombinant fusion protein of embodiment 55, wherein said human antibody constant domain is an IgG CH1 domain.

57. The recombinant fusion protein of embodiment 52, wherein said hybrid antibody variable domain is a hybrid heavy chain variable domain, $F^1$ is Trp and $F^2$ is (Lys/Arg)(Val/Leu)(Glu/Asp)IleLys or (Lys/Gln/Glu)(Val/Leu)(Thr/Ile)(Val/Ile)Leu.

58. The recombinant fusion protein of embodiment 57, wherein $F^2$ is selected from the group consisting of LysValGluIleLys, LysValAspIleLys, LysLeuGluIleLys, LysLeuAspIleLys, ArgValGluIleLys, ArgValAspIleLys, ArgLeuGluIleLys, ArgLeuAspIleLys, LysValThrValLeu, LysValThrIleLeu, LysValIleValLeu, LysValIleIleLeu, LysLeuThrValLeu, LysLeuThrIleLeu, LysLeuIleValLeu, LysLeuIleIleLeu, GlnValThrValLeu, GlnValThrIleLeu, GlnValIleValLeu, GlnValIleIleLeu, GlnLeuThrValLeu, GlnLeuThrIleLeu, GlnLeuIleValLeu, GlnLeuIleIleLeu, GluValThrValLeu, GluValThrIleLeu, GluValIleValLeu, GluValIleIleLeu, GluLeuThrValLeu, GluLeuThrIleLeu, GluLeuIleValLeu, and GluLeuIleIleLeu.

59. The recombinant fusion protein of embodiment 57, wherein said antibody constant domain is a human antibody light chain constant domain.

60. The recombinant fusion protein of embodiment 51, wherein Y is GlyXaaGlyThrXaa(Val/Leu).

61. The recombinant fusion protein of embodiment 60, wherein $F^1$ is Phe and $F^2$ is ThrValSerSer.

62. The recombinant fusion protein of embodiment 61, wherein said antibody constant domain is a human antibody constant domain.

63. The recombinant fusion protein of embodiment 62, wherein said human antibody constant domain is an IgG CH1 domain or an IgG CH2 domain.

64. The recombinant fusion protein of embodiment 63, wherein said IgG is IgG1 or IgG4.

65. The recombinant fusion protein of embodiment 60, wherein $F^1$ is Trp and $F^2$ is (Glu/Asp)IleLys or (Thr/Ile)(Val/Ile)Leu.

66. The recombinant fusion protein of embodiment 65, wherein $F^2$ is selected from the group consisting of GluIleLys, AspIleLys, ThrValLeu, ThrIleLeu, IleValLeu, and IleIleLeu.

67. The recombinant fusion protein of embodiment 65, wherein said antibody constant domain is a human antibody light chain constant domain.

68. The recombinant fusion protein of any one of embodiments 31-67, wherein said recombinant fusion protein comprises a partial structure that has the formula $(F^1$-Y-$F^2)$-$C_L$, $(F^1$-Y-$F^2)$-CH1, $(F^1$-Y-$F^2)$-CH2, or $(F^1$-Y-$F^2)$-$F_c$.

69. The recombinant fusion protein of embodiment 68, wherein said recombinant fusion protein further comprises a second immunoglobulin variable domain.

70. The recombinant fusion proteins of embodiment 69, wherein said second immunoglobulin variable domain is amino-terminal of $(F^1$-Y-$F^2)$.

71. The recombinant fusion proteins of embodiment 69, wherein said second immunoglobulin variable domain is carboxy-terminal of $(F^1$-Y-$F^2)$.

72. An isolated recombinant nucleic acid molecule encoding the recombinant fusion protein of any one of embodiments 35-71.

73. A host cell comprising a recombinant nucleic acid molecule encoding the recombinant fusion protein of any one of embodiments 35-71.

74. A method of producing a recombinant fusion protein comprising maintaining the host cell of embodiment 73 under conditions suitable for expression of said recombinant nucleic acid, whereby said recombinant nucleic acid is expressed and said recombinant fusion protein is produced.

75. The method of embodiment 74, further comprising isolating said recombinant fusion protein.

76. In a recombinant fusion protein comprising a non-human antibody variable region fused to a human antibody constant domain, the improvement comprising:

said non-human antibody variable region comprising a hybrid FR4 having the formula

$$(F^1\text{-}Y\text{-}F^2)$$

wherein $F^1$ is Phe or Trp;

Y is GlyXaaGlyThr, and $F^2$ is (Leu/Met/Thr)ValThrValSerSer, (Lys/Arg)(Val/Leu)(Glu/Asp)IleLys or (Lys/Gln/Glu)(Val/Leu)(Thr/Ile)(Val/Ile)Leu; or

Y is GlyXaaGlyThrXaa(Val/Leu), and $F^2$ is ThrValSerSer, (Glu/Asp)IleLys or (Thr/Ile)(Val/Ile)Leu.

77. The recombinant fusion protein of embodiment 76, wherein said human antibody constant domain is a CH1 domain, Y is GlyXaaGlyThr, and $F^2$ is (Leu/Met/Thr)ValThrValSerSer.

78. The recombinant fusion protein of embodiment 77, wherein $F^2$ is selected from the group consisting of LeuValThrValSerSer, MerValThrValSerSer, and ThrValThrValSerSer.

79. The recombinant fusion protein of embodiment 76, wherein said human antibody constant domain is a CH1 domain, Y is GlyXaaGlyThrXaa(Val/Leu), and $F^2$ is ThrValSerSer.

80. The recombinant fusion protein of embodiment 76, wherein said human antibody constant domain is a light chain constant domain, Y is GlyXaaGlyThr, and $F^2$ is (Lys/Arg)(Val/Leu)(Glu/Asp)IleLys or (Lys/Gln/Glu)(Val/Leu)(Thr/Ile)(Val/Ile)Leu.

81. The recombinant fusion protein of embodiment 80, wherein $F^2$ is selected from the group consisting of LeuValThrValSerSer, MetValThrValSerSer, ThrValThrValSerSer, LysValGluIleLys, LysValAspIleLys, LysLeuGluIleLys, LysLeuAspIleLys, ArgValGluIleLys, ArgValAspIleLys, ArgLeuGluIleLys, ArgLeuAspIleLys, LysValThrValLeu, LysValThrIleLeu, LysValIleValLeu, LysValIleIleLeu, LysLeuThrValLeu, LysLeuThrIleLeu, LysLeuIleValLeu, LysLeuIleIleLeu, GlnValThrValLeu, GlnValThrIleLeu, GlnValIleValLeu, GlnValIleIleLeu, GlnLeyThrValLeu, GlnLeuThrIleLeu, GlnLeuIleValLeu, GlnLeuIleIleLeu, GluValThrValLeu, GluValThrIleLeu, GluValIleValLeu, GluValIleIleLeu, GluLeuThrValLeu, GluLeuThrIleLeu, GluLeuIleValLeu, and GluLeuIleIleLeu.

82. The recombinant fusion protein of embodiment 76, wherein said human antibody constant domain is a light chain constant domain, Y is GlyXaaGlyThrXaa(Val/Leu), and $F^2$ is (Glu/Asp)IleLys or (Thr/Ile)(Val/Ile)Leu.

83. The recombinant fusion protein of embodiment 82 wherein Y is GlyXaaGlyThrXaaVal or GlyXaaGlyThrXaaLeu; and $F^2$ is selected from the group consisting of GluIleLys, AspIleLys, ThrValLeu, ThrIleLeu, IleValLeu, and IleIleLeu.

84. An isolated recombinant nucleic acid molecule encoding the recombinant fusion protein of any one of embodiments 76-83.

85. A host cell comprising a recombinant nucleic acid molecule encoding the recombinant fusion protein of any one of embodiments 76-83.

86. A method of producing a recombinant fusion protein comprising maintaining the host cell of embodiment 85 under conditions suitable for expression of said recombinant nucleic acid, whereby said recombinant nucleic acid is expressed and said recombinant fusion protein is produced.

87. The method of embodiment 86, further comprising isolating said recombinant fusion protein.

88. A recombinant fusion protein comprising an immunoglobulin variable domain fused to a hybrid immunoglobulin constant domain, wherein said hybrid immunoglobulin constant domain comprises a portion from a first immunoglobulin constant domain and a portion from a second immunoglobulin constant domain, said first immunoglobulin constant domain and said second immunoglobulin constant domain each comprising a conserved amino acid motif Y, said hybrid immunoglobulin constant domain having the formula

$$C^1\text{-}Y\text{-}C^2$$

wherein Y is said conserved amino acid motif;

$C^1$ is the amino acid motif adjacent to the amino-terminus of Y in said first immunoglobulin constant region;

$C^2$ is the amino acid motif adjacent to the carboxy-terminus of Y in said second immunoglobulin constant region.

89. The recombinant fusion protein of embodiment 88, wherein said hybrid immunoglobulin constant domain is a hybrid antibody constant domain comprising a portion from a first antibody constant domain and a portion from a second antibody constant domain.

90. The recombinant fusion protein of embodiment 89, wherein said hybrid antibody constant domain is a hybrid antibody CH1, a hybrid antibody hinge, a hybrid antibody CH2, or a hybrid antibody CH3.

91. The recombinant fusion protein of embodiment 90, wherein said antibody is an IgG.

92. The recombinant fusion protein of embodiment 88, wherein said first antibody constant domain and said second antibody constant domain are from different species.

93. The recombinant fusion protein of embodiment 88, wherein said second antibody constant domain is a human antibody constant domain.

94. The recombinant fusion protein of embodiment 93, wherein said first antibody constant domain is a mouse, rat, shark, fish, possum, sheep, pig, *Camelid,* rabbit or non-human primate constant domain.

95. The recombinant fusion protein of embodiment 88, wherein said immunoglobulin variable domain is a non-human antibody variable domain and said first constant domain is the corresponding non-human CH1 domain, Cλ domain or Cκ domain.

96. The recombinant fusion protein of embodiment 88, wherein said first antibody constant domain is a light chain constant domain, and said second antibody constant domain is a heavy chain constant domain.

97. The recombinant fusion protein of embodiment 88, wherein said first antibody constant domain is a *Camelid* heavy chain constant domain, and said second antibody constant domain is a heavy chain constant domain.

98. The recombinant fusion protein of embodiment 97, wherein a VHH is amino terminal to the hybrid constant domain.

99. The recombinant fusion protein of embodiment 88, wherein said first antibody constant domain and said second antibody constant domain are of different isotypes.

100. The recombinant fusion protein of embodiment 99, wherein said second antibody constant domain is an IgG constant domain.

101. The recombinant fusion protein of embodiment 88, wherein said antibody variable domain is a light chain variable domain and said first antibody constant domain is a light chain constant domain.

102. The recombinant fusion protein of embodiment 101, wherein said second antibody constant domain is a human antibody heavy chain constant domain.

103. The recombinant fusion protein of embodiment 101, wherein said second antibody constant domain is a human antibody light chain constant domain.

104. The recombinant fusion protein of embodiment 102, wherein said human antibody heavy chain constant domain is a CH1, a hinge, a CH2, or a CH3.

105. The recombinant fusion protein of embodiment 102, wherein said human antibody heavy chain constant domain is an IgG CH1 or an IgG CH2.

106. The recombinant fusion protein of embodiment 88, wherein said antibody variable domain is a heavy chain variable domain and said first antibody constant domain is a CH1 domain.

107. The recombinant fusion protein of embodiment 106, wherein said second antibody constant domain is a human antibody light chain constant domain.

108. The recombinant fusion protein of embodiment 106, wherein said second antibody constant domain is a human antibody heavy chain constant domain.

109. The recombinant fusion protein of embodiment 88, wherein Y is (Ser/Ala/Gly)Pro(Lys/Asp/Ser)Val, (Ser/Ala/Gly)Pro(Lys/Asp/Ser)ValPhe, LysValAspLys(Ser/Arg/Thr), or ValThrVal.

110. The recombinant fusion protein of embodiment 109, wherein Y is selected from the group consisting of Ser-ProLysVal, SerProAspVal, SerProSerVal, AlaProLysVal, AlaProAspVal, AlaProSerVal, GlyProLysVal, Gly-ProAspVal, GlyProSerVal, SerProLysValPhe, SerProAspValPhe, SerProSerValPhe, AlaProLysValPhe, AlaProAspValPhe, AlaProSerValPhe, GlyProLysValPhe, GlyProAspValPhe, GlyProSerValPhe, LysValAspLysSer, LysValAspLysArg, LysValAspLysThr, and ValThrVal.

111. The recombinant fusion protein of embodiment 109, wherein said second antibody constant domain is a human antibody constant domain.

112. The recombinant fusion protein of embodiment 111, wherein said human antibody constant domain is selected from the group consisting of Cκ, Cλ, a CH1, a hinge, a CH2 and a CH3.

113. The recombinant fusion protein of embodiment 111, wherein said human antibody constant domain is an IgG CH1, or an IgG CH2.

114. The recombinant fusion protein of embodiment 88, wherein said recombinant fusion protein comprises a human light chain variable domain that is fused to a hybrid human CH1 domain, and wherein:

$C^1$ is GlnProLysAla or ThrValAla,
Y is (Ala/Gly)ProSerVal, and
$C^2$ is the amino acid motif adjacent to the carboxy-terminus of Y in human IgG CH1.

115. The recombinant fusion protein of embodiment 88, wherein

A) said recombinant fusion protein comprises a human light chain variable domain that is fused to a hybrid human CH2, wherein:

$C^1$ is GlnProLysAla or ThrValAla,
Y is (Ala/Gly)ProSerVal, and
$C^2$ is the amino acid motif adjacent to the carboxy-terminus of Y in human IgG CH2; or

B) said recombinant fusion protein comprises a human heavy chain variable domain that is fused to a hybrid human CH2, wherein
$C^1$ is SerThrLys,
Y is (Ala/Gly)ProSerValPhe; and
$C^2$ is the amino acid motif adjacent to the carboxy-terminus of Y in human IgG CH2.

116. The recombinant fusion protein of embodiment 88, wherein

A) said recombinant fusion protein comprises a human lambda chain variable domain that is fused to a hybrid human Cκ, and wherein
$C^1$ is GlnProLysAla,
Y is (Ala/Gly)ProSerVal, and
$C^2$ is the amino acid motif adjacent to the carboxy-terminus of Y in human Cκ; or
B) said recombinant fusion protein comprises a human heavy chain variable domain that is fused to a hybrid human Cκ, wherein
$C^1$ is SerThrLys,
Y is (Ala/Gly)ProSerValPhe; and
$C^2$ is the amino acid motif adjacent to the carboxy-terminus of Y in human Cκ.

117. The recombinant fusion protein of embodiment 88, wherein

A) said recombinant fusion protein comprises a human kappa chain variable domain that is fused to a hybrid human Cλ, and wherein
$C^1$ is ThrValAla,

Y is (Ala/Gly)ProSerVal, and

$C^2$ is the amino acid motif adjacent to the carboxy-terminus of Y in human Cλ; or

B) said recombinant fusion protein comprises a human heavy chain variable domain that is fused to a hybrid human Cλ, wherein

$C^1$ is SerThrLys,

Y is (Ala/Gly)ProSerVal; and

$C^2$ is the amino acid motif adjacent to the carboxy-terminus of Y in human Cλ.

118. An isolated recombinant nucleic acid molecule encoding the recombinant fusion protein of any one of embodiments 88-117.

119. A host cell comprising a recombinant nucleic acid molecule encoding the recombinant fusion protein of any one of embodiments 88-117.

120. A method of producing a recombinant fusion protein comprising maintaining the host cell of embodiment 119 under conditions suitable for expression of said recombinant nucleic acid, whereby said recombinant nucleic acid is expressed and said recombinant fusion protein is produced.

121. The method of embodiment 120, further comprising isolating said recombinant fusion protein.

122. A recombinant fusion protein comprising a first portion derived from a first polypeptide and a second portion derived from a second polypeptide, wherein said first polypeptide comprises a structure having the formula (A)-L1, wherein

(A) is an amino acid sequence present is said first polypeptide; and

L1 is an amino acid motif comprising 1 to about 50 amino acids that are adjacent to the carboxy-terminus of (A) in said first polypeptide;

wherein said fusion polypeptide has the formula

$$(A)\text{-}L1\text{-}(B);$$

wherein (B) is said portion derived from said second polypeptide;

with the proviso that at least one of (A) and (B) is a domain, and when (A) and (B) are both antibody variable domains

a) (A) and (B) are each human antibody variable domains;

b) (A) and (B) are each antibody heavy chain variable domains;

c) (A) and (B) are each antibody light chain variable domains;

d) (A) is an antibody light chain variable domain and (B) is an antibody heavy chain variable domain; or

e) (A) is a VHH and (B) is an antibody light chain variable domain; or with the proviso that when (A) and (B) are both antibody variable domains the following

is excluded from the invention, (A)-L1-(B) where (A) is a mouse VH, (B) is a mouse VL and L1 is SerAlaLysThrThrPro, SerAlaLysThrThrProLysLeuGlyGly, AlaLysThrThrProLysLeuGluGluGlyGluPheSerGluAlaArgVal, or AlaLysThrThrProLysLeuGluGlu.

123. The recombinant fusion protein of embodiment 122, with the proviso that when (A) is a $V_H$ and (B) is a $V_L$, L1 does not consist of one to five or one to six contiguous amino acids from the amino-terminus of CH1.

124. The recombinant fusion protein of embodiment 122, wherein said first polypeptide is an immunoglobulin variable domain.

125. The recombinant fusion protein of embodiment 124 wherein said immunoglobulin variable domain is an antibody variable domain.

126. The recombinant fusion protein of embodiment 124, wherein said second polypeptide is an immunoglobulin constant region.

127. The recombinant fusion protein of embodiment 126, wherein (B) comprises at least a portion of an antibody CH1, at least a portion of an antibody hinge, at least a portion of an antibody CH2, or at least a portion of an antibody CH3.

128. The recombinant fusion protein of embodiment 122, wherein (A) is an immunoglobulin variable domain.

129. The recombinant fusion protein of embodiment 128, wherein said immunoglobulin variable domain is an antibody variable domain.

130. The recombinant fusion protein of embodiment 129, wherein said antibody variable domain is an antibody light chain variable domain.

131. The recombinant fusion protein of embodiment 130, wherein L1 comprises one to about 50 contiguous amino-terminal amino acids of Cκ or Cλ.

132. The recombinant fusion protein of embodiment 129, wherein said antibody variable domain is an antibody heavy chain variable domain.

133. The recombinant fusion protein of embodiment 132, wherein said antibody heavy chain variable domain is a VH or a VHH.

134. The recombinant fusion protein of embodiment 132 wherein L1 comprises one to about 50 contiguous amino-terminal amino acids of CH1.

135. The recombinant fusion protein of embodiment 129, wherein (A) is an antibody heavy chain variable domain and (B) is an antibody heavy chain variable domain.

136. The recombinant fusion protein of embodiment 128, wherein (A) is an antibody light chain variable domain and (B) is an antibody heavy chain variable domain or an antibody light chain variable domain.

137. The recombinant fusion protein of embodiment 136, wherein

(A) is a Vκ and (B) is a Vκ;
(A) is a Vκ and (B) is a Vλ;
(A) is a Vκ and (B) is a VH or a VHH;
(A) is a Vλ and (B) is a Vκ;
(A) is a Vλ and (B) is a Vλ; or
(A) is a Vλ and (B) is a VH or a VHH.

138. The recombinant fusion protein of embodiment 122, wherein: (A) is a VH and L1 comprises the first 3 to about 12 amino acids of CH1; (A) is a Vκ and L1 comprises the first 3 to about 12 amino acids of Cκ; or (A) is a Vλ and L1 comprises the first 3 to about 12 amino acids of Cλ.

139. The recombinant fusion protein of embodiment 122, wherein (A) is an antibody variable domain comprising FR1, CDR1, FR2, CDR3, FR3 and CDR3 of a antibody light chain variable domain and FR4 comprising the amino acid sequence GlyGlnGlyThrLysValThrValSerSer; and L1 comprises the first 3 to about 12 amino acids of CH1.

140. The recombinant fusion protein of embodiment 139, wherein L1 is AlaSerThr, AlaSerThrLysGlyProSer, or AlaSerThrLysGlyProSerGly.

141. The recombinant fusion protein of embodiment 122, wherein (A) is an antibody variable domain comprising FR1, CDR1, FR2, CDR3, FR3 and CDR3 of a VH or Vκ domain and FR4 comprising the amino acid sequence GlyXaaGlyThr(Lys/Gln/Glu)(Val/Leu)(Thr/Ile)ValLeu; and L1 comprises the first 3 to about 12 amino acids of Cλ.

142. The recombinant fusion protein of embodiment 122, wherein (A) is an antibody variable domain comprising FR1, CDR1, FR2, CDR3, FR3 and CDR3 of a VH or Vλ domain and FR4 comprising the amino acid sequence GlyGlnGlyThrLysValGluIleLysArg; and L1 comprises the first 3 to about 12 amino acids of Cκ.

143. The recombinant fusion protein of embodiment 122, wherein (A) is an immunoglobulin constant domain.

144. The recombinant fusion protein of embodiment 143, wherein said immunoglobulin constant domain is an antibody constant domain.

145. The recombinant fusion protein of embodiment 144, wherein said antibody constant domain is an antibody heavy chain constant domain.

146. The recombinant fusion protein of embodiment 145, wherein (A) is a nonhuman immunoglobulin constant domain, and (B) is derived from a human polypeptide.

147. The recombinant fusion protein of any one of embodiments 122-124 and 128-146 wherein said second polypeptide is selected from the group consisting of a cytokine, a cytokine receptor, a growth factor, a growth factor receptor, a hormone, a hormone receptor, an adhesion molecule, a haemostatic factor, a T cell receptor, a T cell receptor chain, a T cell receptor variable domain, enzyme, polypeptide comprising or consisting of an antibody variable domain, or a functional portion of any one of the foregoing.

148. The recombinant fusion protein of embodiment 122 wherein said first polypeptide is selected from the group consisting of a cytokine, a cytokine receptor, a growth factor, a growth factor receptor, a hormone, a hormone receptor, an adhesion molecule, a haemostatic factor, a T cell receptor, a T cell receptor chain, a T cell receptor variable domain, enzyme, polypeptide comprising or consisting of an antibody variable domain, or a functional portion of any one of the foregoing.

149. The recombinant fusion protein of embodiment 148, wherein said second polypeptide is an immunoglobulin constant region or Fc portion of an immunoglobulin constant region.

150. An isolated recombinant nucleic acid molecule encoding the recombinant fusion protein of any one of embodiments 122-149.

151. A host cell comprising the a recombinant nucleic acid molecule encoding the recombinant fusion protein of any one of embodiments 122-149.

152. A method of producing a recombinant fusion protein comprising maintaining the host cell of embodiment 151 under conditions suitable for expression of said recombinant nucleic acid, whereby said recombinant nucleic acid is expressed and said recombinant fusion protein is produced.

153. The method of embodiment 152, further comprising isolating said recombinant fusion protein.

154. A recombinant fusion protein comprising a first portion that is an immunoglobulin variable domain and a second portion, wherein said first portion is bonded to said second portion through a linker, and the recombinant fusion protein has the formula

$$(A')\text{-}L2\text{-}(B)$$

wherein (A') is said immunoglobulin variable domain and comprises framework (FR) 4;

L2 is said linker, wherein L2 comprises one to about 50 contiguous amino acids that are adjacent to the carboxy-terminus of said FR4 in a naturally occurring immunoglobulin that comprises said FR4; and

(B) is said second portion;

with the proviso that L2-(B) is not a $C_L$ or CH1 domain that is peptide bonded to said FR4 in a naturally occurring antibody that comprises said FR4, and when (A) and (B) are both antibody variable domains

    a) (A) and (B) are each human antibody variable domains;
    b) (A) and (B) are each antibody heavy chain variable domains;
    c) (A) and (B) are each antibody light chain variable domains;
    d) (A) is an antibody light chain variable domain and (B) is an antibody heavy chain variable domain; or
    e) (A) is a VHH and (B) is an antibody light chain variable domain or with the proviso that when (A) and (B) are both antibody variable domains the following is excluded from the invention, (A)-L1-(B) where (A) is a mouse VH, (B) is a mouse VL and L1 is SerAlaLysThrThrPro, SerAlaLysThrThrProLysLeuGlyGly, AlaLysThrThrProLysLeuGluGluGlyGluPheSerGluAlaArgVal, or AlaLysThrThrProLysLeuGlyGly.

155. The recombinant fusion protein of embodiment 154, wherein (A') is an antibody heavy chain variable domain or a hybrid antibody light chain variable domain.

156. The recombinant fusion protein of embodiment 155, wherein said antibody heavy chain variable domain and said hybrid light chain variable domain each comprise a FR4 that comprises the amino acid sequence GlyXaaGlyThr(Leu/Met/Thr)ValThrValSerSer.

157. The recombinant fusion protein of embodiment 156, wherein FR4 comprises GlyXaaGlyThrLeuValThrValSerSer, GlyXaaGlyThrMetValThrValSerSer, or GlyXaaGlyThrThrValThrValSerSer.

158. The recombinant fusion protein of embodiment 157, wherein X4 comprises one to about 50 contiguous amino acids from the amino-terminus of CH1.

159. The recombinant fusion protein of embodiment 158, wherein L2 comprises AlaSerThr, AlaSerThrLysGlyProSer, or AlaSerThrLysGlyProSerGly.

160. The recombinant fusion protein of embodiment 154, wherein (A') is a hybrid antibody variable domain or a Vκ.

161. The recombinant fusion protein of embodiment 160, wherein said hybrid variable domain and Vκ, each comprise a FR4 that comprises the amino acid sequence GlyXaaGlyThr(Lys/Arg)(Val/Leu)(Glu/Asp)IleLysArg.

162. The recombinant fusion protein of embodiment 161, wherein FR4 comprises GlyXaaGlyThrLysValGluIleLysArg, GlyXaaGlyThrLysLeuGluIleLysArg, GlyXaaGlyThrLysValAspIleLysArg, or GlyXaaGlyThrArgLysGluIleLysArg.

163. The recombinant fusion protein of embodiment 161, wherein L2 comprises one to about 50 contiguous amino acids from the amino-terminus of Cκ.

164. The recombinant fusion protein of embodiment 163, wherein L2 comprises ThrValAla, ThrValAlaAlaProSer, or ThrValAlaAlaProSerGly.

165. The recombinant fusion protein of embodiment 154, wherein (A') is a hybrid antibody variable domain or a Vλ.

166. The recombinant fusion protein of embodiment 165, wherein said hybrid antibody variable domain and Vλ each comprise a FR4 that comprises the amino acid sequence GlyXaaGlyThr(Lys/Gln/Glu)(Val/Leu)(Thr/Ile)(Val/Ile)Leu.

167. The recombinant fusion protein of embodiment 166, wherein FR4 comprises GlyXaaGlyThrLysValThrValLeu, GlyXaaGlyThrLysLeuThrValLeu, GlyXaaGlyThrGlnLeuIleIleLeu, GlyXaaGlyThrGluLeuThrValLeu, or GlyXaaGlyThrGlnLeuThrValLeu.

168. The recombinant fusion protein of embodiment 154, wherein (B) comprises an immunoglobulin variable domain

169. The recombinant fusion protein of embodiment 168, wherein said immunoglobulin variable domain is amino-terminal of (B).

170. The recombinant fusion protein of embodiment 168, wherein (B) comprises an antibody light chain variable domain or an antibody heavy chain variable domain.

171. The recombinant fusion protein of embodiment 154, wherein (B) comprises at least a portion of an immunoglobulin constant region.

172. The recombinant fusion protein of embodiment 171, wherein said at least a portion of an immunoglobulin constant region domain is at the amino-terminus of (B).

173. The recombinant fusion protein of embodiment 171, wherein said immunoglobulin constant region is an IgG constant region.

174. The recombinant fusion protein of embodiment 173, wherein said immunoglobulin constant region is an IgG1 constant region or an IgG4 constant region.

175. The recombinant fusion protein of embodiment 174, wherein (B) comprises at least a portion of CH1, at least a portion of hinge, at least a portion of CH2 or at least a portion of CH3.

176. The recombinant fusion protein of embodiment 175, wherein (B) comprises at least a portion of hinge.

177. The recombinant fusion protein of embodiment 176, wherein said portion of hinge comprises ThrHisThrCysProProCysPro.

178. The recombinant fusion protein of embodiment 177, wherein (B) further comprises CH2-CH3.

179. The recombinant fusion protein of embodiment 175, wherein (B) comprises a portion of CH1-hinge-CH2-CH3.

180. The recombinant fusion protein of embodiment 175, wherein (B) comprises hinge-CH2-CH3.

181. The recombinant fusion protein of embodiment 175, wherein (B) comprises CH2-CH3.

182. The recombinant fusion protein of embodiment 175, wherein (B) comprises CH3.

183. A recombinant fusion protein comprising a first portion and a second portion derived from an immunoglobulin constant region, wherein said first portion is bonded to said second portion through a linker, and the recombinant fusion protein has the formula

$$(A)\text{-}L3\text{-}(C^3)$$

wherein (A) is said first portion;

$(C^3)$ is said second portion derived from an immunoglobulin constant region; and

L3 is said linker, wherein L3 comprises one to about 50 contiguous amino acids that are adjacent to the amino-terminus of $(C^3)$ in a naturally occurring immunoglobulin that comprises $(C^3)$;

with the proviso that (A) is not an antibody variable domain found in said naturally occurring immunoglobulin.

184. The recombinant fusion protein of embodiment 183, wherein $(C^3)$ comprises at least on antibody constant domain.

185. The recombinant fusion protein of embodiment 184, wherein said antibody constant domain is a human antibody constant domain.

186. The recombinant fusion protein of embodiment 185, wherein said antibody constant domain is an IgG constant domain.

187. The recombinant fusion protein of embodiment 186, wherein said antibody constant domain is an IgG1 constant domain or an IgG4 constant domain.

188. The recombinant fusion protein of embodiment 187, wherein $(C^3)$ comprises CH3.

189. The recombinant fusion protein of embodiment 188, wherein L3 comprises one to about 50 contiguous amino acids from the carboxy-terminus of CH2.

190. The recombinant fusion protein of embodiment 189, wherein $(C^3)$ comprises CH2 or CH2-CH3.

191. The recombinant fusion protein of embodiment 190, wherein L3 comprises one to about 34 contiguous amino acids from the carboxy-terminus of hinge.

192. The recombinant fusion protein of embodiment 191, wherein L3 comprises ThrHisThrCysProProCysPro or GlyThrHisThrCysProProCysPro.

193. The recombinant fusion protein of embodiment 187, wherein $(C^3)$ comprises hinge.

194. The recombinant fusion protein of embodiment 193, wherein L3 comprises one to about 50 contiguous amino acids from the carboxy-terminus of CH1.

195. The recombinant fusion protein of embodiment 186, wherein $(C^3)$ comprises CH1.

196. The recombinant fusion protein of embodiment 195, wherein L3 comprises one to about 50 contiguous amino acids from the carboxy-terminus of an antibody heavy chain V domain.

197. The recombinant fusion protein of embodiment 196, wherein L3 comprises GlyXaaGlyThr(Leu/Met/Thr)Val-ThrValSerSer.

198. The recombinant fusion protein of embodiment 197, wherein L3 comprises GlyXaaGlyThrLeuValThrValSerSer, GlyXaaGlyThrMetValThrValSerSer, or GlyXaaGlyThrThrValThrValSerSer.

199. The recombinant fusion protein of embodiment 184, wherein said antibody constant domain is an antibody light chain constant domain.

200. The recombinant fusion protein of embodiment 199, wherein said antibody light chain constant domain is a Cκ.

201. The recombinant fusion protein of embodiment 200, wherein L3 comprises one to about 50 contiguous amino acids from the carboxy-terminus of an antibody light chain V domain.

202. The recombinant fusion protein of embodiment 201, wherein L3 comprises GlyXaaGlyThr(Lys/Arg)(Val/Leu)(Glu/Asp)IleLysArg.

203. The recombinant fusion protein of embodiment 202, wherein L3 is GlyXaaGlyThrLysValGluIleLysArg, GlyXaaGlyThrLysLeuGluIleLysArg, GlyXaaGlyThrLysValAspIleLysArg, or GlyXaaGlyThrArgLysGluIleLysArg.

204. The recombinant fusion protein of embodiment 199, wherein said antibody light chain constant domain is a Cλ.

205. The recombinant fusion protein of embodiment 204, wherein L3 comprises one to about 50 contiguous amino acids from the carboxy-terminus of an antibody light chain V domain.

206. The recombinant fusion protein of embodiment 205, wherein L3 comprises GlyXaaGlyThr(Lys/Gln/Glu)(Val/Leu)(Thr/Ile)(Val/Ile)Leu.

207. The recombinant fusion protein of embodiment 206 wherein L3 is GlyXaaGlyThrLysValThrValLeu, GlyXaaGlyThrLysLeuThrValLeu, GlyXaaGlyThrGlnLeuIleIleLeu, GlyXaaGlyThrGluLeuThrValLeu, or GlyXaaGlyThrGlnLeuThrValLeu.

208. The recombinant fusion protein of any one of embodiments 183-207 wherein (A) is selected from the group consisting of a cytokine, a cytokine receptor, a growth factor, a growth factor receptor, a hormone, a hormone receptor, an adhesion molecule, a haemostatic factor, a T cell receptor, a T cell receptor chain, a T cell receptor variable domain, enzyme, polypeptide comprising or consisting of an antibody variable domain, or a functional portion of any one of the foregoing.

209. A recombinant fusion protein comprising a first portion derived from an antibody variable domain and a second portion derived from a second polypeptide, wherein said antibody variable domain comprises a structure having the formula (A)-L1, wherein

(A) consists of CDR3; and
L1 consists of FR4;
wherein said fusion polypeptide has the formula (A)-L1-(B);
wherein
(B) is said portion derived from said second polypeptide.

210. The recombinant fusion protein of embodiment 209, wherein said second polypeptide is an immunoglobulin constant region.

211. The recombinant fusion protein of embodiment 210, wherein (B) comprises at least a portion of an antibody CH1, at least a portion of an antibody hinge, at least a portion of an antibody CH2, or at least a portion of an antibody CH3.

212. An isolated recombinant nucleic acid molecule encoding the recombinant fusion protein of any one of embodiments 154-211.

213. A host cell comprising a recombinant nucleic acid molecule encoding the recombinant fusion protein of any one of embodiments 154-211.

214. A method of producing a recombinant fusion protein comprising maintaining the host cell of embodiment 213 under conditions suitable for expression of said recombinant nucleic acid, whereby said recombinant nucleic acid is expressed and said recombinant fusion protein is produced.

215. The method of embodiment 214, further comprising isolating said recombinant fusion protein.

216. A recombinant fusion protein according to any one of embodiments 1-30, 35-71, 76-83, 88-117, 122-149, and 154-211, for use in therapy, diagnosis and/or prophylaxis.

217. Use of a recombinant fusion protein according to any one of embodiments 1-30, 35-71, 76-83, 88-117, 122-149, and 154-211, for the manufacture of a medicament for therapy, diagnosis and/or prophylaxis in a human, with reduced likelihood of inducing an immune response.

218. A method of therapy, diagnosis and/or prophylaxis in a human comprising administering to said human an effective amount of a recombinant fusion protein of any one of embodiments 1-30, 35-71, 76-83, 88-117, 122-149, and 154-211, whereby the likelihood of inducing an immune response is reduced in comparison to a corresponding fusion protein that does not contain a natural junction.

219. Use of a natural junction for preparing a recombinant fusion protein for human therapy, diagnosis and/or prophylaxis, with reduced likelihood of inducing an immune response in comparison to a corresponding fusion protein that does not contain a natural junction.

220. Use of a natural junction for preparing a recombinant fusion protein for human therapy, diagnosis and/or prophylaxis, with reduced propensity to aggregate in comparison to a corresponding fusion protein that does not contain a natural junction.

221. Use of a natural junction for preparing a recombinant fusion protein for human therapy, diagnosis and/or prophylaxis, wherein said recombinant fusion protein is expressed at higher levels in comparison to a corresponding fusion protein that does not contain a natural junction.

222. Use of a natural junction for preparing a recombinant fusion protein for human therapy, diagnosis and/or prophylaxis, wherein said recombinant fusion protein has enhanced stability in comparison to relative to a corresponding fusion protein that does not contain a natural junction.

223. Use of a natural junction for preparing a recombinant fusion protein comprising a first portion (A) and a second portion (B), and at least one natural junction between (A) and (B), and wherein said recombinant fusion protein has reduced propensity to aggregate in comparison to a corresponding fusion protein comprising (A) and (B), wherein the interface of (A) and (B) is not a natural junction.

224. Use of a natural junction for preparing a recombinant fusion protein comprising a first portion (A), a second portion (B), and at least one natural junction between (A) and (B), wherein said recombinant fusion protein is expressed at higher levels in comparison to a corresponding fusion protein comprising (A) and (B), wherein said corresponding fusion protein does not contain a natural junction between (A) and (B).

225. Use of a natural junction for preparing a recombinant fusion protein comprising a first portion (A), a second portion (B), and at least one natural junction between (A) and (B), wherein said recombinant fusion protein has enhanced stability in comparison to a corresponding fusion protein comprising (A) and (B), wherein said corresponding fusion protein does not contain a natural junction between (A) and (B).

226. A pharmaceutical composition comprising a recombinant fusion protein of any one of embodiments 1-30, 35-71, 76-83, 88-117, 122-149 and 154-211 and a physiologically acceptable carrier.

227. A method of producing a fusion protein comprising a first portion and a second portion that are fused at a natural junction, wherein said first portion is derived from a first polypeptide and said second portion is derived from a second polypeptide, the method comprising, analyzing the amino acid sequence of said first polypeptide or a portion thereof and the amino acid sequence of said second polypeptide or a portion thereof to identify a conserved amino acid motif present in both of the analyzed sequences; and preparing a fusion protein which has the formula

$$A\text{-}Y\text{-}B\ ;$$

wherein:

A is said first portion;
Y is said conserved amino acid motif;
B is said second portion; and

wherein said first polypeptide comprises A-Y, and said second polypeptide comprises Y-B.

228. The method of embodiment 227, wherein Y consists of 1 to about 50 amino acids.

229. The method of embodiment 227, wherein Y consists of 3 to 10 amino acids.

230. The method of embodiment 227, wherein said second polypeptide comprises an immunoglobulin constant domain.

231. The method of embodiment 230, wherein said immunoglobulin constant domain is a human immunoglobulin constant domain.

232. The method of embodiment 230, wherein said immunoglobulin constant domain is a nonhuman immunoglobulin constant domain.

233. The method of any one of embodiments 230-232, wherein said second polypeptide comprises a T cell receptor constant domain.

234. The method of any one of embodiments 230-232, wherein said second polypeptide comprises an antibody constant domain.

235. The method of embodiment 234, wherein said antibody constant domain is a light chain constant domain or a heavy chain constant domain.

236. The method of embodiment 234, wherein said antibody constant domain is a human antibody heavy chain constant domain.

237. The method of embodiment 234, wherein said second polypeptide and B comprise an antibody hinge region.

238. The method of embodiment 234, wherein said second polypeptide and B comprise a portion of CH1-hinge-CH2-CH3.

239. The method of embodiment 234, wherein said second polypeptide and B comprise hinge-CH2-CH3.

240. The method of embodiment 234, wherein said second polypeptide and B comprise CH2-CH3.

241. The method of embodiment 234, wherein said second polypeptide and B comprise CH3.

242. The method of embodiment 236, wherein said human antibody heavy chain constant domain is an IgG constant domain.

243. The method of embodiment 242, wherein said IgG constant domain is an IgG1 constant domain or an IgG4 constant domain.

244. The method of any one of embodiments 227-243, wherein said first polypeptide is selected from the group consisting of a cytokine, a cytokine receptor, a growth factor, a growth factor receptor, a hormone, a hormone receptor, an adhesion molecule, a haemostatic factor, a T cell receptor, a T cell receptor chain, a T cell receptor variable domain, enzyme, polypeptide comprising or consisting of an antibody variable domain, or a functional portion of any one of the foregoing

245. The method of embodiment 227, wherein said first polypeptide and A comprise an immunoglobulin variable domain.

246. The method of embodiment 245, wherein said immunoglobulin variable domain is a human immunoglobulin variable domain.

247. The method of embodiment 246, wherein said immunoglobulin variable domain is a nonhuman immunoglobulin variable domain.

248. The method of any one of embodiments 245-247, wherein said first polypeptide and A comprise a T cell receptor variable domain.

249. The method of any one of embodiments 245-247, wherein said first polypeptide and A comprise an antibody variable domain.

250. The method of embodiment 249, wherein said antibody variable domain is a non-human antibody variable domain.

251. The method of embodiment 250, wherein said non-human antibody variable domain is a *Camelid* antibody variable domain or a nurse shark antibody variable domain..

252. The method of embodiment 249, wherein said antibody variable domains is a human antibody variable domain.

253. The method of embodiment 252, wherein said human antibody variable domain is a Vκ, Vλ.or $V_H$.

254. The method of any one of embodiments 227-229 and 244-253, wherein said second polypeptide is selected from the group consisting of a cytokine, a cytokine receptor, a growth factor, a growth factor receptor, a hormone, a hormone receptor, an adhesion molecule, a haemostatic factor, a T cell receptor, a T cell receptor chain, a T cell receptor variable domain, enzyme, polypeptide comprising or consisting of an antibody variable domain, or a functional portion of any one of the foregoing.

255. The method of embodiment 227, wherein said first polypeptide is a first antibody chain, said second polypeptide is a second antibody chain.

256. The method of embodiment 255, wherein Y is in the variable domain of said first antibody chain and the variable domain of said second antibody chain.

257. The method of embodiment 256 wherein Y is in framework region (FR) 4.

258. The method of embodiment 257, wherein Y is GlyXaaGlyThr or GlyXaaGlyThrXaa(Val/Leu).

259. The method of embodiment 257 or 258, wherein A comprises a portion of an antibody variable domain comprising FR1, complementarity determining region (CDR) 1, FR2, CDR2, FR3, and CDR3.

260. The method of embodiment 255, wherein Y is in FR3.

261. The method of embodiment 260, wherein Y is GluAspThrAla, ValTyrTyrCys, or GluAspThrAlaValTyrTyrCys.

262. The method of embodiment 260 or 261, wherein A comprises a portion of an antibody variable domain comprising FR1, CDR1, FR2, and CDR2.

263. The method of embodiment 255, wherein Y is in a constant domain of said first antibody chain and a constant domain of said second antibody chain.

264. The method of embodiment 263, wherein Y is (Ser/Ala/Gly)Pro(Lys/Asp/Ser)Val, (Ser/Ala/Gly)Pro(Lys/Asp/Ser)ValPhe, LysValAspLys(Ser/Arg/Thr) or ValThrVal.

265. The method of embodiment 264, wherein Y is selected from the group consisting of SerProLysVal, Ser-ProAspVal, SerProSerVal, AlaProLysVal, AlaProAspVal, AlaProSerVal, GlyProLysVal, GlyProAspVal, GlyProSer-Val, SerProLysValPhe, SerProAspValPhe, SerProSerValPhe, AlaProLysValPhe, AlaProAspValPhe, AlaProSerVal-

Phe, GlyProLysValPhe, GlyProAspValPhe, GlyProSerValPhe, LysValAspLysSer, LysValAspLysArg, LysValAsp-LysThr, and ValThrVal.

266. The method of any one of embodiments 255-265, wherein said first antibody chain, and said second antibody chain are from different species.

267. The method of embodiment 266, wherein said first antibody chain is human and said second antibody chain is non-human.

268. The method of embodiment 266, wherein said first antibody chain is non-human and said second antibody chain is human.

269. The method of any one of embodiments 255-265, wherein said first antibody chain, and said second antibody chain are from the same species.

270. The method of embodiment 269, wherein said first antibody chain and said second antibody chain are human.

271. The method of any one of embodiments 227-270, wherein said fusion protein further comprises a third portion located amino terminally to A.

272. The method of embodiment 271, wherein said third portion comprises an immunoglobulin variable domain.

273. The method of embodiment 227, wherein said first polypeptide and said second polypeptide are both members of the same protein superfamily.

274. The method of embodiment 273, wherein said protein superfamily is selected from the group consisting of the immunoglobulin superfamily, the TNF superfamily and the TNF receptor superfamily.

275. The method of embodiment 227, wherein said first polypeptide and said second polypeptide are both human polypeptides.

276. A recombinant fusion protein comprising a first portion that is an immunoglobulin variable domain and a second portion that is an immunoglobulin variable domain, wherein said first portion is bonded to said second portion through a linker, and the recombinant fusion protein has the formula

$$(A')-L2-(B)$$

wherein (A') is said immunoglobulin variable domain and comprises framework FR4; L2 is said linker, wherein L2 comprises one to about 50 contiguous amino acids that are adjacent to the carboxy-terminus of said FR4 in a naturally occurring immunoglobulin that comprises said FR4; and wherein L2 comprises AlaSerThr, AlaSerThrLys-GlyProSer, or AlaSerThrLysGlyProSerGly; and

(B) is said second portion;

with the proviso that L2-(B) is not a $C_L$ or CH1 domain that is peptide bonded to said FR4 in a naturally occurring antibody that comprises said FR4, and when (A') and (B) are both antibody variable domains

    a) (A') and (B) are each human antibody variable domains;
    b) (A') and (B) are each antibody heavy chain variable domains;
    c) (A') and (B) are each antibody light chain variable domains;
    d) (A') is an antibody light chain variable domain and (B) is an antibody heavy chain variable domain; or
    e) (A') is a VHH and (B) is an antibody light chain variable domain.

277. The recombinant fusion protein of embodiment 276, wherein (A') is an antibody heavy chain variable domain or a hybrid antibody light chain variable domain.

278. The recombinant fusion protein of embodiment 277, wherein said antibody heavy chain variable domain and said hybrid light chain variable domain each comprise a FR4 that comprises the amino acid sequence GlyXaaGlyThr(Leu/Met/Thr)ValThrValSerSer.

279. The recombinant fusion protein of embodiment 278, wherein FR4 comprises GlyXaaGlyThrLeuValThrValSerSer, GlyXaaGlyThrMetValThrValSerSer, or GlyXaaGlyThrThrValThrValSerSer.

280. The recombinant fusion protein of embodiment 276, wherein (A') is a hybrid antibody variable domain or a Vκ.

281. The recombinant fusion protein of embodiment 280, wherein said hybrid variable domain and Vκ, each comprise a FR4 that comprises the amino acid sequence GlyXaaGlyThr(Lys/Arg)(Val/Leu)(Glu/Asp)IleLysArg.

282. The recombinant fusion protein of embodiment 281, wherein FR4 comprises GlyXaaGlyThrLysValGluIleLysArg, GlyXaaGlyThrLysLeuGluIleLysArg, GlyXaaGlyThrLysValAspIleLysArg, or GlyXaaGlyThrArgLysGluIleLysArg.

283. The recombinant fusion protein of embodiment 276, wherein (A') is a hybrid antibody variable domain or a Vλ.

284. The recombinant fusion protein of embodiment 283, wherein said hybrid antibody variable domain and Vλ each comprise a FR4 that comprises the amino acid sequence GlyXaaGlyThr(Lys/Gln/Glu)(Val/Leu)(Thr/Ile)(Val/Ile)Leu.

285. The recombinant fusion protein of embodiment 284, wherein FR4 comprises GlyXaaGlyThrLysValThrValLeu, GlyXaaGlyThrLysLeuThrValLeu, GlyXaaGlyThrGlnLeuIleIleLeu, GlyXaaGlyThrGluLeuThrValLeu, or GlyXaaGlyThrGlnLeuThrValLeu.

286. The recombinant fusion protein of embodiment 276, wherein (B) comprises an immunoglobulin variable domain

287. The recombinant fusion protein of embodiment 286, wherein said immunoglobulin variable domain (A') is amino-terminal of (B).

288. The recombinant fusion protein of embodiment 286, wherein (B) comprises an antibody light chain variable domain or an antibody heavy chain variable domain.

289. The recombinant fusion protein of embodiment 276, wherein (B) comprises at least a portion of an immunoglobulin constant region.

290. The recombinant fusion protein of embodiment 289, wherein said at least a portion of an immunoglobulin constant region domain is at the amino-terminus of (B).

291. The recombinant fusion protein of embodiment 289, wherein said immunoglobulin constant region is an IgG constant region.

292. The recombinant fusion protein of embodiment 291, wherein said immunoglobulin constant region is an IgG1 constant region or an IgG4 constant region.

293. The recombinant fusion protein of embodiment 292, wherein (B) comprises at least a portion of CH1, at least a portion of hinge, at least a portion of CH2 or at least a portion of CH3.

294. The recombinant fusion protein of embodiment 293, wherein (B) comprises at least a portion of hinge, which comprises ThrHisThrCysProProCysPro.

295. The recombinant fusion protein of embodiment 294, wherein (B) further comprises CH2-CH3.

296. The recombinant fusion protein of embodiment 293, wherein (B) comprises a portion of CH1-hinge-CH2-CH3.

297. The recombinant fusion protein of embodiment 293, wherein (B) comprises hinge-CH2-CH3.

298. The recombinant fusion protein of embodiment 293, wherein (B) comprises CH2-CH3.

299. The recombinant fusion protein of embodiment 293, wherein (B) comprises CH3.

300. An isolated recombinant nucleic acid molecule encoding the recombinant fusion protein of any one of embodiments 276-299.

301. A host cell comprising a recombinant nucleic acid molecule encoding the recombinant fusion protein of any one of embodiments 276-299.

302. A method of producing a recombinant fusion protein comprising maintaining the host cell of embodiment 301 under conditions suitable for expression of said recombinant nucleic acid, whereby said recombinant nucleic acid is expressed and said recombinant fusion protein is produced.

303. The method of embodiment 302, further comprising isolating said recombinant fusion protein.

304. A recombinant fusion protein according to any one of embodiments 276-299, for use in therapy, diagnosis and/or prophylaxis.

305. Use of a recombinant fusion protein according to any one of embodiments 276-299, for the manufacture of a medicament for therapy, diagnosis and/or prophylaxis in a human, with reduced likelihood of inducing an immune response.

306. A pharmaceutical composition comprising a recombinant fusion protein of any one of embodiments 276-299 and a physiologically acceptable carrier.

307. A polypeptide linker selected from LVTVSSAST (SEQ ID NO: 529), LVTVSSGGGGSGGGS (SEQ ID NO: 530), LVTVSSASTKGPS, GQGTNVEINRTVAAPS (SEQ ID NO: 532), GQGTNVEINQTVAAPS (SEQ ID NO: 533), GQGTNVEIQRTVAAPS (SEQ ID NO: 534) and GQGTLVTVSSTVAAPS (SEQ ID NO: 535)

The teachings of all patents, published applications and references cited herein are incorporated by reference in their entirety.

While this invention has been particularly shown and described with references to example embodiments thereof, it will be understood by those skilled in the art that various changes in form and details may be made therein without departing from the scope of the invention encompassed by the appended claims.

**Claims**

1. A recombinant fusion protein comprising a first portion that is an immunoglobulin variable domain and a second portion that is an immunoglobulin variable domain, wherein said first portion is bonded to said second portion through a linker, and the recombinant fusion protein has the formula

$$(A')-L2-(B)$$

wherein (A') is said immunoglobulin variable domain and comprises framework FR4;
L2 is said linker, wherein L2 comprises one to about 50 contiguous amino acids that are adjacent to the carboxy-terminus of said FR4 in a naturally occurring immunoglobulin that comprises said FR4; and wherein L2 comprises AlaSerThr, AlaSerThrLysGlyProSer, or AlaSerThrLysGlyProSerGly; and
(B) is said second portion;
with the proviso that L2-(B) is not a $C_L$ or CH1 domain that is peptide bonded to said FR4 in a naturally occurring antibody that comprises said FR4, and when (A') and (B) are both antibody variable domains

   (a) (A') and (B) are each human antibody variable domains;
   (b) (A') and (B) are each antibody heavy chain variable domains;
   (c) (A') and (B) are each antibody light chain variable domains;
   (d) (A') is an antibody light chain variable domain and (B) is an antibody heavy chain variable domain; or
   (e) (A') is a VHH and (B) is an antibody light chain variable domain.

2. A recombinant fusion protein comprising a hybrid domain, wherein said hybrid domain comprises a first portion derived from a first polypeptide and a second portion derived from a second polypeptide, said first polypeptide comprising a domain that has the formula (X1-Y-X2), and said second polypeptide comprising a domain that has the formula (Z1-Y-Z2), wherein
Y is a conserved amino acid motif;
X1 and Z1 are the amino acid motifs that are located adjacent to the amino-terminus of Y in said first polypeptide and said second polypeptide, respectively;
X2 and Z2 are the amino acid motifs that are located adjacent to the carboxy-terminus of Y in said first polypeptide and said second polypeptide, respectively;

with the proviso that when the amino acid sequences of X1 and Z1 are the same, the amino acid sequences of X2 and Z2 are not the same; and when the amino acid sequences of X2 and Z2 are the same, the amino acid sequences of X1 and Z1 are not the same;
wherein said hybrid domain has the formula

$$(X1\text{-}Y\text{-}Z2).$$

3. A recombinant fusion protein comprising a hybrid immunoglobulin variable domain that is fused to an immunoglobulin constant domain, wherein said hybrid immunoglobulin variable domain comprises a hybrid framework region (FR) that comprises a portion from a first immunoglobulin FR from a first immunoglobulin and a portion from a second immunoglobulin FR from a second immunoglobulin, said first immunoglobulin FR and said second immunoglobulin FR each comprising a conserved amino acid motif Y, and said hybrid immunoglobulin FR has the formula

$$(F^{1}\text{-}Y\text{-}F^{2})$$

wherein Y is said conserved amino acid motif;
$F^1$ is the amino acid motif located adjacent to the amino-terminus of Y in said first immunoglobulin FR; and
$F^2$ is the amino acid motif located adjacent to the carboxy-terminus of Y in said second immunoglobulin FR.

4. A recombinant fusion protein comprising a non-human antibody variable region fused to a human antibody constant domain, the improvement comprising:

said non-human antibody variable region comprising a hybrid FR4 having the formula

$$(F^{1}\text{-}Y\text{-}F^{2})$$

wherein $F^1$ is Phe or Trp;
Y is GlyXaaGlyThr, and $F^2$ is (Leu/Met/Thr)ValThrValSerSer,
(Lys/Arg)(Val/Leu)(Glu/Asp)IleLys or (Lys/Gln/Glu)(Val/Leu)(Thr/Ile)(Val/Ile)Leu; or
Y is GlyXaaGlyThrXaa(Val/Leu), and $F^2$ is ThrValSerSer, (Glu/Asp)IleLys or (Thr/Ile)(Val/Ile)Leu.

5. A recombinant fusion protein comprising an immunoglobulin variable domain fused to a hybrid immunoglobulin constant domain, wherein said hybrid immunoglobulin constant domain comprises a portion from a first immunoglobulin constant domain and a portion from a second immunoglobulin constant domain, said first immunoglobulin constant domain and said second immunoglobulin constant domain each comprising a conserved amino acid motif Y, said hybrid immunoglobulin constant domain having the formula

$$C^{1}\text{-}Y\text{-}C^{2}$$

wherein Y is said conserved amino acid motif;
$C^1$ is the amino acid motif adjacent to the amino-terminus of Y in said first immunoglobulin constant region;
$C^2$ is the amino acid motif adjacent to the carboxy-terminus of Y in said second immunoglobulin constant region.

6. A recombinant fusion protein comprising a first portion derived from a first polypeptide and a second portion derived from a second polypeptide, wherein said first polypeptide comprises a structure having the formula (A)-L1, wherein

(A) is an amino acid sequence present is said first polypeptide; and
L1 is an amino acid motif comprising 1 to about 50 amino acids that are adjacent to the carboxy-terminus of (A) in said first polypeptide;
wherein said fusion polypeptide has the formula

$$(A)\text{-}L1\text{-}(B);$$

wherein (B) is said portion derived from said second polypeptide;

with the proviso that at least one of (A) and (B) is a domain, and when (A) and (B) are both antibody variable domains

    a) (A) and (B) are each human antibody variable domains;
    b) (A) and (B) are each antibody heavy chain variable domains;
    c) (A) and (B) are each antibody light chain variable domains;
    d) (A) is an antibody light chain variable domain and (B) is an antibody heavy chain variable domain; or
    e) (A) is a VHH and (B) is an antibody light chain variable domain; or
    with the proviso that when (A) and (B) are both antibody variable domains the following is excluded from the invention, (A)-L1-(B) where (A) is a mouse VH, (B) is a mouse VL and L1 is SerAlaLysThrThrPro, SerAlaLysThrThrProLysLeuGlyGly, AlaLysThrThrProLysLeuGluGluGlyGluPheSerGluAlaArgVal, or Ala-LysThrThrProLysLeuGluGlu.

7. A recombinant fusion protein comprising a first portion that is an immunoglobulin variable domain and a second portion, wherein said first portion is bonded to said second portion through a linker, and the recombinant fusion protein has the formula

$$(A')\text{-}L2\text{-}(B)$$

wherein (A') is said immunoglobulin variable domain and comprises framework (FR) 4;
L2 is said linker, wherein L2 comprises one to about 50 contiguous amino acids that are adjacent to the carboxy-terminus of said FR4 in a naturally occurring immunoglobulin that comprises said FR4; and
(B) is said second portion;
with the proviso that L2-(B) is not a $C_L$ or CH1 domain that is peptide bonded to said FR4 in a naturally occurring antibody that comprises said FR4, and when (A) and (B) are both antibody variable domains

    a) (A) and (B) are each human antibody variable domains;
    b) (A) and (B) are each antibody heavy chain variable domains;
    c) (A) and (B) are each antibody light chain variable domains;
    d) (A) is an antibody light chain variable domain and (B) is an antibody heavy chain variable domain; or
    e) (A) is a VHH and (B) is an antibody light chain variable domain or
    with the proviso that when (A) and (B) are both antibody variable domains the following is excluded from the invention, (A)-L1-(B) where (A) is a mouse VH, (B) is a mouse VL and L1 is SerAlaLysThrThrPro, SerAlaLys-ThrThrProLysLeuGlyGly, AlaLysThrThrProLysLeuGluGluGlyGluPheSerGluAlaArgVal, or AlaLysThrThrPro-LysLeuGlyGly.

8. A recombinant fusion protein comprising a first portion and a second portion derived from an immunoglobulin constant region, wherein said first portion is bonded to said second portion through a linker, and the recombinant fusion protein has the formula

$$(A)\text{-}L3\text{-}(C^3)$$

wherein (A) is said first portion;
$(C^3)$ is said second portion derived from an immunoglobulin constant region; and
L3 is said linker, wherein L3 comprises one to about 50 contiguous amino acids that are adjacent to the amino-terminus of $(C^3)$ in a naturally occurring immunoglobulin that comprises $(C^3)$; with the proviso that (A) is not an antibody variable domain found in said naturally occurring immunoglobulin.

9. A recombinant fusion protein comprising a first portion derived from an antibody variable domain and a second portion derived from a second polypeptide, wherein said antibody variable domain comprises a structure having the formula (A)-L1, wherein

    (A) consists of CDR3; and
    L1 consists of FR4;

wherein said fusion polypeptide has the formula (A)-L1-(B);
wherein
(B) is said portion derived from said second polypeptide.

10. An isolated recombinant nucleic acid molecule encoding the recombinant fusion protein of any of claims 1 to 9.

11. A host cell comprising a recombinant nucleic acid molecule encoding the recombinant fusion protein of any of claims 1 to 9.

12. A method of producing a recombinant fusion protein comprising maintaining the host cell of claim 11 under conditions suitable for expression of said recombinant nucleic acid, whereby said recombinant nucleic acid is expressed and said recombinant fusion protein is produced, optionally further comprising isolating said recombinant fusion protein.

13. A recombinant fusion protein according to any of claims 1 to 9, for use in therapy, diagnosis and/or prophylaxis, such as for use with the reduced likelihood of inducing an immune response.

14. A pharmaceutical composition comprising a recombinant fusion protein of any of claims 1 to 9 and a physiologically acceptable carrier.

15. A polypeptide linker selected from LVTVSSAST (SEQ ID NO: 529), LVTVSSGGGGSGGGS (SEQ ID NO: 530), LVTVSSASTKGPS, GQGTNVEINRTVAAPS (SEQ ID NO: 532), GQGTNVEINQTVAAPS (SEQ ID NO: 533), GQGTNVEIQRTVAAPS (SEQ ID NO: 534) AND GQGTLVTVSSTVAAPS (SEQ ID NO: 535).

VH domain

VK domain

Figure 1B

Figure 1C

CH1 domain

CK domain

FIG. 1A

FIG. 1B

FIG. 1C

# Conserved motif in human IG and TCR J-segments

### human IGH J-segments

```
IGHJ1    ...AEYFQHWGQGTLVTVSS
IGHJ2    ...YWYFDLWGRGTLVTVSS
IGHJ3    .....AFDVWGQGTMVTVSS
IGHJ4    .....YFDYWGQGTLVTVSS
IGHJ5    ....NWFDSWGQGTLVTVSS
IGHJ6    YYYYYGMDVWGQGTTVTVSS
```

### human IGK J-segments

```
IGKJ1    WTFGQGTKVEIK
IGKJ2    YTFGQGTKLEIK
IGKJ3    FTFGPGTKVDIK
IGKJ4    LTFGGGTKVEIK
IGKJ5    ITFGQGTRLEIK
```

### human IGL J-segments

```
IGLJ1    YVFGTGTKVTVL
IGLJ2    VVFGGGTKLTVL
IGLJ3    VVFGGGTKLTVL
IGLJ4    FVFGGGTQLIIL
IGLJ5    WVFGEGTELTVL
IGLJ6    NVFGSGTKVTVL
IGLJ7    AVFGGGTQLTVL
```

### human TRB J segments

```
TRBJ1-1      ..NTEAFFGQGTRLTVV
TRBJ1-2      ..NYGYTFGSGTRLTVV
TRBJ1-3      .SGNTIYFGEGSWLTVV
TRBJ1-4      .TNEKLFFGSGTQLSVL
TRBJ1-5      .SNQPQHFGDGTRLSIL
TRBJ1-6      SYNSPLHFGNGTRLTVT
TRBJ2-1      .SYNEQFFGPGTRLTVL
TRBJ2-2      .NTGELFFGEGSRLTVL
TRBJ2-2P     LRGAAGRLGGGLLVL..
TRBJ2-3      .STDTQYFGPGTRLTVL
TRBJ2-4      .AKNIQYFGAGTRLSVL
TRBJ2-5      ..QETQYFGPGTRLLVL
TRBJ2-6      SGANVLTFGAGSRLTVL
TRBJ2-7      ..SYEQYFGPGTRLTVT
```

### human TRG J-segments

```
TRGJ1    ....NYYKKLFGSGTTLVVT..
TRGJ2    ....NYYKKLFGSGTTLVVT..
TRGJP    GQELGKKIKVFGPGTKLIIT..
TRGJP1   ...TTGWFKIFAEGTKLIVTSP
TRGJP2   ...SSDWIKTFAKGTRLIVTSP
```

### human TRD J-segments

```
TRDJ1    ...TDKLIFGKGTRVTVEP.
TRDJ2    ..LTAQLFFGKGTQLIVEP.
TRDJ3    SWDTRQMFFGTGTKLFVEP.
TRDJ4    ....RPLIFGKGTYLEVQQ.
```

216

**human TRA J-segments**

```
TRAJ61        ...YRVNRKLTFGANTRGIMKL
TRAJ59        ...KEGNRKFTFGMGTQVRV..
TRAJ58        ..*ETSGSRLTFGEGTQLTVNP
TRAJ57        ..TQGGSEKLVFGKGTKLTVNP
TRAJ56        ..YTGANSKLTFGKGITLSVRP
TRAJ54        ...IQGAQKLVFGQGTRLTINP
TRAJ53        .NSGGSNYKLTFGKGTLLTVNP
TRAJ52        NAGGTSYGKLTFGQGTILTVHP
TRAJ50        ...KTSYDKVIFGPGTSLSVIP
TRAJ49        ....NTGNQFYFGTGTSLTVIP
TRAJ48        ..SNFGNEKLTFGTGTRLTIIP
TRAJ47        ....EYGNKLVFGAGTILRVKS
TRAJ46        ..KKSSGDKLTFGTGTRLAVRP
TRAJ45        .YSGGGADGLTFGKGTHLIIQP
TRAJ44        ..NTGTASKLTFGTGTRLQVTL
TRAJ43        .....NNNDMRFGAGTRLTVKP
TRAJ41        ..NSNSGYALNFGKGTSLLVTP
TRAJ40        ..TTSGTYKYIFGTGTRLKVLA
TRAJ39        ..NNNAGNMLTFGGGTRLMVKP
TRAJ38        ..NAGNNRKLIWGLGTSLAVNP
TRAJ37        ..GSGNTGKLIFGQGTTLQVKP
TRAJ36        ...QTGANNLFFGTGTRLTVIP
TRAJ35        ...IGFGNVLHCGSGTQVIVLP
TRAJ34        ...SYNTDKLIFGTGTRLQVFP
TRAJ33        ....DSNYQLIWGAGTKLIIKP
TRAJ32        .NYGGATNKLIFGTGTLLAVQP
TRAJ31        ....NNNARLMFGDGTQLVVKP
TRAJ30        ....NRDDKIIFGKGTRLHILP
TRAJ29        ...NSGNTPLVFGKGTRLSVIA
TRAJ28        .YSGAGSYQLTFGKGTKLSVIP
TRAJ27        ...NTNAGKSTFGDGTTLTVKP
TRAJ26        ...DNYGQNFVFGPGTRLSVLP
TRAJ25        ...EGQGFSFIFGKGTRLLVKP
TRAJ24        ..TTDSWGKFEFGAGTQVVVTP
TRAJ23        ..IYNQGGKLIFGQGTELSVKP
TRAJ22        ..SSGSARQLTFGSGTQLTVLP
TRAJ21        ....YNFNKFYFGSGTKLNVKP
TRAJ20        ....SNDYKLSFGAGTTVTVRA
TRAJ19        ...YQRFYNFTFGKGSKHNVTP
TRAJ18        .DRGSTLGRLYFGRGTQLTVWP
TRAJ17        ..IKAAGNKLTFGGGTRVLVKP
TRAJ16        ...FSDGQKLLFARGTMLKVDL
TRAJ15        ...NQAGTALIFGKGTTLSVSS
TRAJ14        .....IYSTFIFGSGTRLSVKP
TRAJ13        ..NSGGYQKVTFGIGTKLQVIP
TRAJ12        ...MDSSYKLIFGSGTRLLVRP
TRAJ11        ...NSGYSTLTFGKGTMLLVSP
TRAJ10        .ILTGGGNKLTFGTGTQLKVEL
TRAJ9         ..GNTGGFKTIFGAGTRLFVKA
TRAJ8         ...NTGFQKLVFGTGTRLLVSP
TRAJ7         ...DYGNNRLAFGKGNQVVVIP
TRAJ6         ..ASGGSYIPTFGPGTSLIVHP
TRAJ5         ...DTGRRALTFGSGTRLQVQP
TRAJ4         ..FSGGYNKLIFGAGTRLAVHP
TRAJ3         ..GYSSASKIIFGSGTRLSIRP
TRAJ2         .NTGGTIDKLTFGKGTHVFIIS
TRAJ1         ..YESITSQLQFGKGTRVSTSP
```

## Conserved motif in IGH J-segments from various species

### Mouse IGH J-segments

```
IGHJ1          YWYFDVW GAG  TVTVSS
IGHJ2          ..YFDYW GQG  TLTVSS
IGHJ3          ..WFAYW GQG  LVTVSA
IGHJ4          YYAMDYW GQG  SVTVSS
```

### Llama IGH J-segments

```
IGHJ2          GYRYLEVW GQG  LVTVSS
IGHJ3            NALDAW GQG  LVTVSS
IGHJ4             EYDYW GQG  QVTVSS
IGHJ5            FQFEYW GQG  LVTVS
IGHJ6            DFGSW  GQG  LVTVS
```

### Sheep IGH J-segments

```
IGHJ1   YADFHLW DQGA LVTVSS
IGHJ2   CWDSGLW GQRT PVTVSL
IGHJ3     AFDSW GQRA PVTVSS
IGHJ4     YIDYW GPGL LVTVSS
IGHJ5    DWLKHW GQGF RRCLL
IGHJ6   YYGVDVW GRGL LVTVSS
```

### Pig IGH J-segments

```
IGHJ1   LLCW GPGV EVVVSS
```

Conserved motif in IGK J-segments from various species

Mouse IGK J-segments

```
IGKJ1    WTFGGGTKLEIK
IGKJ2    YTFGGGTKLEIK
IGKJ3    ITFSDGTRLEIK
IGKJ4    FTFGSGTKLEIK
IGKJ5    LTFGAGTKLELK
```

Possum IGK J-segments

```
IGKJ1S1    LTFGQGTKLEIK
IGKJ1S2    FTFGGGTKVEIK
```

Sheep IGK J-segments

```
IGKJ1S1    SFGQGTKLEIK
IGKJ1S2    FTFGPGTRVEIK
IGKJ1S3    YAFGGGTKVEI
IGKJ1S4    LAFGGGTNVEIK
```

Conserved motif in IGL J-segments from various species

Mouse IGL J-segments

```
IGLJ1       WVFGGGTKLTVL.
IGLJ2       YVFGGGTKVTVL.
IGLJ3       FIFGSGTKVTVL.
IGLJ3P      GSFSSNGLLYAG.
IGLJ4(1)    WVFGGGTRLTVL.
```

Possum IGL J-segments

```
IGLJ1S1    YIFGGGTQLTVI
IGLJ1S2    WVFGEGTHVTVL
IGLJ1S3    WVFGGGTHLTVL
```

Sheep IGL J-segments

```
IGLJ1    GVFGSGTRLTVL
```

# Conserved motifs in mouse Ig constant domains

## CH1

```
IGHA    (E)SARNPTIYPLTLPPVLC....SDEVIIGCLIR  DYFPP.GTMN   VTWSKSGKDI....TTVNFPALASG......GRYTNSSQLTLPAVEC..FEGESVKC SVQRC...SNPVQ ELDVNCS
IGHD    (G)DKKEPDMPLLSKCNAPSS...NEKINLGCLVI GBQ....PLK   ISWEPKKSSI....VEKVFPSEHRNG......NYTMVLQVTVLASE..LRLNHTC TIDKPL..KRREK PFKIP
IGHE    (A)SIRNPDLYPLKPCKG.....TASNTLGCLVK DYFP.NPVT   VTWYSDSLNM....STVNFPALGSE.......LKVTTSQVTSRGK...SAKNFTC HVTHP...PSFNE SRTIL
IGHG1   (A)KTTPPSVYPLAPGSAAQT...NSMVTLGCLVK GYFP.EPVT   VTWNSGSLSS....GVHTFPAVLQSG......LYTLSSSVTVPGSPR..FSETVTC NVAHPA..SSTTV DKKI
IGHG2A  (A)KTTPPSVYPLAPVCGSTT..GSSVTLGCLVK GYFP.EPVT   LTWNSGSLSS....GVHTFPAVLQSD......LYTLSSSVTVTSSTW..PSQSITC NVAHPA..SSTTV DKKI
IGHG2B  (A)KTTPPSVYPLAPGCGSTT..GSSVTSGCLVK GYFP.EAVT   VTWNSGSLSS....SVHTFPALLQSG......LYTMSSSVTVPSSTW..PSQTVTC SVAHPA..SSTTV DKI
IGHG2C  (A)KTTPPSVYPLAPVCGSTT..GSSVTLGCLVK GYFP.EPVT   LTWNSGSLSS....GVHTFPALLQSG......LYTLSSSVTVTSNTW..PSQTITC NVAHPA..SSTTV DKK
IGHG3   (A)TTTPPSVYPLVPGCSDTS..GSSVTLGCLVK GYFP.EPVT   VKWNYGALSS....GVRTVSSVLQSS......FYSLSSBIVTVFSSTW..PSQTVIC NVAHPA..SKTEL IKRI
IGHM    (E)SQSFPNVFPLVSCCESPLSD..KNLVRNGCLAR DFLP.STIS   FTWNYQNNTEVIQ.GIRTFPTLRTGG......KYLATSQVLLSPKSILEGSDSYLVC KIHYG...GKNR DLHVPIP
```

## CH2

```
IGHA        SCQPSLSLQRPALEDLLL..GSDASICTLN GLRNP.ESAA   FTWEPSTGKD....AVQKAAQNSCG.......CYSVSSVLPGCAERW..NSGASFKC TVTHPE..SGTIT GPTAKVT
IGHE    (V)RPGVNITFTPILELLSSCDFNAF..HSTIQLYCFIY GHIL..NDVS   VSRLMPDREITD..TLAQTVLIKESG......KLASTCSKLNITEQQW..MSESTFTC KVTSQ....GVEV LAHTKRCP
IGHG3      (V)PEVSSVFIFPPKPKDVLTI.TLTPKVTCVVV  DISKEDPEVQ   FSWFVDDVEVH...TAQTQPREDQSN......STFRSVSELPIMHQDW..LNGKEFKC RVNSAA..FPAPI EKTISKTK
IGHG2A  (A)PNLLGGPSVFIFPPKIKDVLMI.SLSPIVTCVVV  DVGEDDPDVQ   ISWFVNNVEVH...TAQTQTHREDYN......STLRVVSALPIQHQDW..MSGKEFKC KVNNKD..LPSPI ERTISKIK
IGHG2B  (A)PNLEGGPSVFIFPNIKDVLMI.SLEPKVTCVVV  DVGEDDPDVQ   ISKFVNNVEVH...TAQTQTHREDYN......STIRVVSTLPIQHQDW..MSGKEFKC KVNNKD..LPSPI ERTISKIK
IGHG2C  (A)PNLEGGPSVFIFPPKIKGVLMI.SLSPMVTCVVV  DVSEDDPDVQ   ISKFVNNVEVH...TAQTQTHREDYN......STIRVVSALPIQHQDW..MSGKEFKC KVNNKA..LPSPI EKTISKPR
IGHG3   (P)GNILGGPSVFIFPPKPKDRLMI.SLTPKVTCVVV  DVSEEHPDVH   VSWFVDNKEVH...TAWTQPREAQYN......STFRVVSALPIQHQDW..MRGKEFKC KVNNKA..LPAPI ERTISKPK
IGHM    (A)VAENPNVNVFVPPRDGFSGPAPRKSKLICSAT NFTP..KPIT   VSWLKDGKLVES..GFTTDPVTIENKGS..TPQFKVISTLTFISE(DW..LNLNVYTC RVDHR....GLTF LKNVSSTCAA
```

## CH3

```
IGHA    (V)NTFPPQVHLFPPSEELAL..NELLSLTCLVR AFNP..NEVL   VSRLRGNEKLSPE.SYLVFESFLKEPGGS..ATTYLVTSVLRVSAETW..KQGDQYSC MVGHEA.LPMNFP QKTIQRLS..GKFTNVSVSVIMSEGCGICY
IGHD    (G)ANAPSMLTVNILTTSTKP..SMSSWLLCEVS QFFP..ENIB   LRWLGVESKNRST.NFVTRNPTAQPG......GTFQTWSVLRLPVALE..SGLDTYTC VVRHEASKTKLNA SKSLAIS  (G)CYHLLPESGCPSRRPDGPALA
IGHE    (D)HEPRGVITYLIPPSPLDIYQ..NGAPKLTCLVV DLESE.KNVN   VTWSQEKKTSV...SASQWYTKHHN......ATTSITSILPVVAKDW..IEGYGYQC IVDKPD..SFKPI VRSITKNFP
IGHG1   (G)RPKAPQVYTIFPPKEQMA...KDKVSLTCMIT DFFPP.EDIT   VEWQWNGQPAE...NYKNTPIMNTN......GSYFVYSKLNVQKSNW..EAGNTFTC SVLHEG.LHNHHT EKSLSHSP...GK
IGHG2A  (G)SVRAPQVYVLPPPEEEMT...KKQVTLTCMVT DFMP..EDIY   VEWTNNGKTEL...NYKNTEPVLDSD......GSYFMYSKLRVEKKNW..VERNSYSC SVVHEG..LHNHHT TKSFSRTP..GK
IGHG2B  (G)LVRAPQVYTLPPSFAEQLS...RKDVSLTCLVV GFNP..GDIS   VEWTSNGHTEE...NYKDTAPVLDSD......GSYFIYSKLNMKTSKW..EKTDSFSC NVRHEG.LKNHYL KKTISRSP...GK
IGHG2C  (G)PVRAPQVYVLPPPAEEMT...KREFSLTCMIT GFLP..AEIA   VDWTSNGHTEQ...NYKNTFATVLQSD......GSYFMYSKLRVQKSTW..ERGSLFAC SVVHEV.LHNHHT TKNFSRSL...GK
IGHG3   (G)PAQTPQVYTIFPPKEQMS...KKKVSLTCLVT NFFS..EAIS   VEWERNGELEQ...DYKNTPPILDSD......GTYFLYSKLTVDTDSW..IQGEIFTC SVVHEA.LHNHHT QKNLSRSP...GK
IGHM    (S)PSTDILTFTIPPSFADIFL..SKSANLTCLVS NLATY.ETLR   ISWASQSGEPL...ETKIK IMESHPN.......GTFSAKGVASVCVEDW..DNRKEFVC TVTHRD..LPSPQ KKFISKPN
```

## Hinge

```
IGHA*01   (G)PTPPPITIP
IGHA*04   (G)IOSPPTTPPPP
IGHA*06   (G)PPPPCPPCPP
IGHA*07   (S)PPPPCPP
IGHA*08   (G)PPPPCPPLP
IGHD      (E)SWDEQSSKRVTPTIQAKNHSTEATKAIFTKRDIS
IGHG1     (V)PRDCGCKPCICT
IGHG2A    (E)PRGPTIKPCPPCKCP
IGHG2B    (E)PSGPISTINPCPPCKECHKCP
IGHG2C    (E)PRVPITQNPCPPLKECPPCA
IGHG3     (E)PRIPKPSTPPGSSCP
```

## CK

```
IGKC (V00807 )     (R)ADAAPTVSIFPPSSEQLT...SGGASVVCFLN NFYP..KDIN  VKWKIDGSERQN..GVLNSWTQDSKD.....STYSMSSTLTLTKDEY..ERHNSYTC EATHKT..STSPI VKSFNRNEC
IGKC (M21798 )     (R)ADAAPTVSIFPPSSEQLT...SGGASVVCFLN NFYP..RDIN  VKWKIDGSERQN..GVLNSWTQDQSKD.....STYSMSSTLTLTKDEY..ERHNSYTC EATHKT..STSPI VKSFNRNEC
```

## CL

```
IGLC1    (G)QPKSSPSVTLFPPSSEELE...TNKATLVCTIP DFYP..SGVT  VDWKVDGTFVIQ..GMRTTQPSKQSN......NKYMASSYLTLTARAW..ERHSSYSC QVTHE....GHTV EKSLSRADCS
IGLC2    (G)QPKSTPTLTVFPPSSEELK...ENKATLVCLIS NFSP..SGVT  VAWKANGTPITQ..GVDTSNPTKEDN......KFMASSFLHLTSDQW..RSHNSFTC QVTHE....GGTV EKSLSPAECL
IGLC3    (S)QPASTPTLTMFPPSPEELQ...ENKATLVCLIS NFSP..SGVT  VAWKANGTPITQ..GVDTSNPTKEDN......KYMASSFLHLTSDQW..RSHNSFTC QVTHE....GGTV EKSLSPAECL
```

## Conserved motifs in human Ig constant domains

**CH1**
```
IGHA1   (A)SPTSPKVFPLSLCSTQP....DGNVVIACLVQ GFFPQ.EPLS  VTWSESGQSV....TARNFPPSQDASG.....DLYTTSSQLTLPATQC.LAGKSVTC RVKHY...TNPSQ D...CP
IGHA2   (A)SPTSPKVFPLSLDSTPQ....DGNVVACLVQ GFFPQ.EPLG  VTWSESGQNV....TARNFPPSQDASS.....DLYTTSSQLTLPATQC.PDGKSVTC HVKHY...TNPSQ D...CP
IGHD    (A)PTKAPDVFPIISGCRHPKD.NSPVVLACLIT GYHP.TSVT   VTWYKGTQSQ...PQRTFPEIQRAD.....SVYMTSSQLSTPLQQW.SDGEYKC VVQHT...ASKSK KEIFRWP
IGHE    (A)STQSPSVFPLTRCCKNIPSN.ATSVTLGCLAT GYFP.EPVM   VTCDTSSLNG....FTMNFPATFLTLS.....GHYATISLLTVSGAW....AKQMFTC RVAHTPSSTDWVD NKTFS
IGHG1   (A)STKGPSVFPLAPSSKSTS...SGTAALGCLVK DYFP.EPVT   VSWNSGALTS....GVHTFPAVLQSS.....GLYSLSSVVTVPSSSL...GTQTYIC NVNHKP..SNTKV DKKV
IGHG2   (A)STKGPSVFPLAPCSRSTS...ESTAALGCLVK DYFP.EPVT   VSWNSGALTS....GVHTFPAVLQSS.....GLYSLSSVVTVPSSNF...GTQTYTC NVDHKP..SNTKV DKTV
IGHG3   (A)STKGPSVFPLAPCSRSTS...GGTAALGCLVK DYFP.EPVT   VSWNSGALTS....GVHTFPAVLQSS.....GLYSLSSVVTVPSSSL...GTQTYTC NVNHKP..SNTKV DKTV
IGHG4   (A)STKGPSVFPLAPCSRSTS...ESTAALGCLVK DYFP.EPVT   VSWNSGALTS....GVHTFPAVLQSS.....GLYSLSSVVTVPSSSL...GTKTYTC NVDHKP..SNTKV DKKV
IGHM    (G)SASAPTLFPLVSCENSPSD.TSSVAVGCLAQ DFLP.DSIT    LSWKYKNNSDIS..STRGFPSVLRGG......KYAATSQVLLPSKDVMQGTDEHVVC KVQHP...NGNK EKNVPLP
```

**CH2**
```
IGHA1       CCHPRLSLHRPALEDLLL..GSEANLTCTLT GLRDA.SGVT   FTWTPSSGKS....AVQGPPERDLCG......CYSVSSVLPGCAEPW.NHGKTFTC TAAYPE..SKTPL TATLSKS
IGHA2       CCKPRLSLHRPALEDLLL..GSEANLTCTLT GLRDA.SGAT   FTWTPSSGKS....AVQGPPERDLCG......CYSVSSVLPGCAQPW.NHGKTFTC TAAHPE..LKTPL TANITKS
IGHD    (E)CPSHTQPLGVYLLTPAVQDLWL..RDKATFTCFVV GSDL..KDAH  LTRWEVAGKVPTS..GVEKGLLERHSN......GSQSQHSRLTLPRSLW..NAGTSVTC TLNHPS..LPPQR LMALREP
IGHE    (V)CSRDFTPPTVKILQSSCDGGGHF.PPTIQLLCLVS GYTP.GTIN   ITWLEDGQVMD...VDLSTASTTQEG....ELASTQSELTLSQKHW...LSDRTYTC QVTYQ...GHTF EDSTKKCA
IGHG1   (A)PELLGGPSVFLFPPKPKDTLMI.SRTPEVTCVVV DVSHEDPEVK    FNWYVDGVEVK...NAKTKPREEQYN......STYRVVSVLTVLHQDW.LNGKEYKC KVSNKA.LPAPI EKTISKAK
IGHG2   (A)P.FVKGGPSVFLFPPKPKDTLMI.SRTPEVTCVVV DVSHEDPEVQ    FNWYVDGVEVH...NAKTKPREEQFN......STFRVVSVLTVVHQDW.LNGKEYKC KVSNKG.LPAPI EKTISKTK
IGHG3   (A)PELLGGPSVFLFPPKPKDTLMI.SRTPEVTCVVV DVSHEDPEVQ    FKNYVGGVEVH...NAKTKPREEQYN......STFRVVSVLTVLHQDW.LNGKEYKC KVSNKA.LPAPI EKTISKTK
IGHG4   (A)PEFLGGPSVFLFPPKPKDTLMI.SRTPEVTCVVV DVSQEDPEVQ    FNWYVDGVEVH...NAKTKPREEQFN......STYRVVSVLTVLHQDW.LNGKEYKC KVSNKG.LPSSI EKTISKAK
IGHM    (V)IAELPPKVSVFVPPRDGFFGN.PRKSKLICQAT GFSP..RQIQ     VSWLREGKQVGS..GVTTDQVQAEAKES..GPTTYKVTSTLTIKESDW..LGQSMFTC RVDHR...GLTF QQNASSMCVP
```

**CH3**
```
IGHA1   (G)NTFRPEVHLLPPPSEELAL..NELVTLTCLAR GFSP..KDVL   VRWLQGSQELPRE.KYLTWASRQEPSQG...TTTFAVTSILRVAAEDW..KKGDTFSC MVGHEA.LPLAFT QKTIDRLA...GKPTHVNVSVVMAEVDGTCY
IGHA2   (G)NTFRPEVHLLPPPSEELAL..NELVTLTCLAR GFSP..KDVL   VRWLQGSQELPRE.KYLTWASRQEPSQG...TTTFAVTSILRVAAEDW..KKGDTFSC MVGHEA.LPLAFT QKTIDRLA...GKPTHVNVSVVMAEVDGTCY
IGHD    (A)AQAPVKLSLNLLASSDPP..EAASWLLCEVS QFSP..PNIL   IMRLEIQREVKTS.GFAPARPPQRS.....TTFPAKSVLRVPAPPS..PQPATYTC VVSHEDSRTLLNA SRSLEVS    (Y)VTGHRMK
IGHE    (D)SNPRGVSAYLSRPSPFDLF..RKSFTITCLVS QLPFSKGTVN    LTWSRASGKPEN..NKSTRKEEQRN.....GTLTVTSTLPVGTRDW.IEGETYQC RVTHFH..LPRAL MRSTTKTS
IGHG1   (G)QPREPQVYTLPPSRDELT..KNQVSLTCLVK GFYP.SDIA   VEWESNGQPEN..NYKTTPPVLDSD.....GSFFLYSKLTVDKSRW.QQGNVFSC SVMHEA.LHNHYT QKSLSLSP...GK
IGHG2   (G)QPREPQVYTLPPSREEMT..KNQVSLTCLVK GFYP.SDIA   VEWESNGQPEN..NYKTTPPMLDSD.....GSFFLYSKLTVDKSRW.QQGNVFSC SVMHEA.LHNHYT QKSLSLSP...GK
IGHG3   (G)QPREPQVYTLPPSREEMT..KNQVSLTCLVK GFYP.SDIA   VEWESSGQPEN..NYNTTPPMLDSD.....GSFFLYSKLTVDKSRW.QQGNVFSC SVMHEA.LHNRFT QKSLSLSP...GK
IGHG4   (G)QPREPQVYTLPPSQEEMT..KNQVSLTCLVK GFYP.SDIA   VEWESNGQPEN..NYKTTPPVLDSD.....GSFFLYSRLTVDKSRW.QEGNVFSC SVMHEA.LHNHYT QKSLSLSL...GK
IGHM    (D)QDTAIRVFAIPPSFASIFL..TKSTKITCLVT DLTTY.DSVT   ISWTRQNGEAV...KTHTNISESHPN.....ATFSAVGERSICEDDW..NSGERFTC TVTHTD..LPSPL KQTISRPK
```

**Hinge**
```
IGHA1          (V)PSTPPTPSPSTPPTPSPS
IGHA2          (V)PPPP
IGHD    (H1)   (E)SPKAQASSVPTAQPQAESSLAKATTAPATTRNT       (H2)  (G)RGGEERKKEREKEEQEERETKTP
IGHG1          (E)PKSCDKTHTCPPCP
IGHG2          (E)RKCCVECPPCP
IGHG3   (H1)(E)LKTPLGDTTHTCPRCP   (H2)  (E)PKSCDTPPPCPRCP   (H3)  (E)PKSCDTPPPCPRCP   (H4)  (E)PKSCDTPPPCPRCP
IGHG4          (S)SKYGPPCPSCP
```

**CK**
```
IGKC    (G)TVAAPSVFIFPPSDEQLK...SGTASVVCLLN NFYP..REAK   VQWKVDNALQSG..NSQESVTEQDSKD.....STYSLSSTLTLSKADY..EKHKVYAC EVTHQG..LSSPV TKSFNRGEC
```

**CL**
```
IGLC1   (G)QPKANPTVTLFPPSSEELQ...ANKATLVCLIS DFYP..GAVT   VAWKADGSPVKA..GVETTKPSKQSN.....NKYAASSYLSLTPEQW..KSHRSYSC QVTHE....GSTV EKTVAPTECS
IGLC2   (G)QPKANPTVTLFPPSSEELQ...ANKATLVCLIS DFYP..GAVT   VAWKADSSPVKA..GVETTTPSKQSN.....NKYAASSYLSLTPEQW..KSHKSYSC QVTHE....GSTV EKTVAPTECS
IGLC3   (G)QPKAAPSVTLFPPSSEELQ...ANKATLVCLIS DFYP..GAVT   VAWKADSSPVKA..GVETTTPSKQSN.....NKYAASSYLSLTPEQW..KSHKSYSC QVTHE....GSTV EKTVAPTECS
IGLC6   (G)QPKAAPSVTLFPPSSEELQ...ANKATLVCLIS DFYP..GAVK   VAWKADSSPVNT..GVETTTPSKQSN.....NKYAASSYLSLTPDQW..KSHRSYSC QVTHE....GSTV EKTVSPAECS
IGLC7   (G)QPKAAPSVTLFPPSSEELQ...ANKATLVCLVS DFYP..GAVT   VAWKADGSPVKV..GVETTKPSKQSN.....NKYAASSYLSLTPEQW..KSHRSYSC RVTHE....GSTV EKTVSPAECS
```

# Conserved motifs in camel Ig constant domains

**CH1**

IGHG2 (1)    (A)STKAESV PLTARSGDTP...GSTVAFGCLVW GYIP..EPVT  VTWNSGAVSS....GIKTFPSVLMSL......GLYSLSSLVTLPTSTS...TGKTFIS NVAHPA..SSTKV DKSV

**CH2**

IGHG2     (A)PELLGGPSVF FPPKPKDVLSI.SGRPEVTCVVV DVGQEDPEVS  FNWYIDGVEVR...TANTKPKEEQFN......STYRVVSVLTIQHQDW..LTGKELKC KVNNKA..LPAPI ERTISKAK

IGHG3 c   (A)PELPGGPSVF FPPKPKDVLSI.SGRPEVTCVVV DVGKEDPEVN  FNWYIDGVEVR...TANTKPKEEQFN......STYRVVSVLTIQHQDW..LTGKEFKC KVNNKA..LPAPI ERTISKPK

**CH3**

IGHG2      (G)QTREPQVYTLAPHREELA...KDTVSITCLVI GFYP..ADIN  VEWQRNGRPESEG.AYAFTLPQLDND......GTYFLYSKLSVGKNTW..QQGETFTC VVMHEA.LHNHST QKSITQSS...GK

IGHG3 c    (G)QTREPQVYTLAPHRESLA...KDTVSVTCLVK GFYP..PDIR  VEWQRNRQPESEG.AYATTLPQLDND......GTYFLYSKLSVGKNTW..QRGETFTC VVMHEA.LHNHYT QKSITQSS...GK

**Hinge**

IGHG2      (E)PKIPQPQPKPQPQPQPQPKPQPKPEPECTCPKCP

IGHG3 c    (G)TNEVCKCPKCP

# Conserved motifs in human TCR constant domains

TRAC (1)    (N)IQNPDPAVYQLRESK......SSDKSVCLFT DFDS...QTN  VSQSKDS.......RVYITDKTVLDMRS.MDFKSNSAVAWSNKS.........DFKC ANAFNN............SIIPE DTFFPSP

TRBC1      (E)DLNKVFPPEVAVFEPSEAEISH..TQKATLVCLAT GFFP..DHVE  LSWWVNGKEVHS..GVSTDPQPLKEQPAL.NDSRYCLSSRLRVSATFWQ.NPRNHFRC QVQFYGLSENDEWTQDRAKPVTQIV SAEAWGRA

TRBC2      (E)DLNKVFPPEVAVFEPSEAEISH..TQKATLVCLAT GFYP..DHVE  LSWWVNGKEVHS..GVSTDPQPLKEQPAL.NDSRYCLSSRLRVSATFWQ.NPRNHFRC QVQFYGLSENDEWTQDRAKPVTQIV SAEAWGRA

TRDC       (R)SQPHT KPSVF VMKNG......TNVACLVK GFYP..KDIR  IKLVSSKKITEF.DPAIVISPSG.........KYNAVKLGKYED......SNSVTC SVQHDN............KTVHST DFEVKTDST

TRGC1      (D)KQLDADVSPKPTISLPSIAETKL..QKAGTYLCLLE KFFP..DVIK  IHWQEKKSNTIL..GSQEGNTMKTND.......PYKFSWLTVPEK..SLDKEHRC IVRHENN............KNGVDQ EIIFPPIKT

TRGC2(2x)  (D)KQLDADVSPKPTIFLPSIAETKL..QKAGTYLCLLE KFFP..DIIK  IHWQEKKSNTIL..GSQEGNTMKTND.......TYNKFSWLTVPEE....SLDKEHRC IVRHENN............KNGVDQ EIIFPPIKT

TRGC2(3x)  (D)KQLDADVSPKPTIFLPSIAETKL..QKAGTYLCLLE KFFP..DIIK  IHWQEKKSNTIL..GSQEGNTMKTND.......TYNKFSWLTVPEE....SLDKEHRC IVRHENN............KNGVDQ EIIFPPIKT

## Conserved GXGT motif in nurse shark Ig J-segments

### nurse shark IGH J-segments

```
IGHJ1          ..SYEYGGGTVVTVNP
IGHJ2          .SFDEYGGGTVVT
IGHJ3          ..SYEYGGGTVVT
IGHJ4          .NYAACGDGTAVT
IGHJ5           GYWGQGTMVTVTT
IGHJ6          .ALDYWGQGTRVTVT
IGHJ7            ACGSGTAVTVTP
IGHJ8            YGGGTGVTVNP
IGHJ9            YGGGTVVTVNP
IGHJ10          ACGDGTFVTVNP
IGHJ11          YGADTVVTVNP
IGHJ12         YAACGAGTAVTVNP
IGHJ13          YGSGTVLTVNP
IGHJ14         ACGDGTAVTVNP
IGHJ15          YGGGTVVTVNP
IGHJ16        IYDECGDGTAVTVNP
IGHJ17          CGDGTVVTVNP
IGHJ18          CGGGTVVTVNP
IGHJ19          YGGGTVVTVNP
IGHJ20         YAAVGDGTAVTVNP
IGHJ21          CGEGTAVTVNP
IGHJ22         AACGHGTAVTVTS
IGHJ23          YGGGTVVTVNP
IGHJ24          CGDGTTVTVHP
IGHJ25         YAACGEGTTVTVNP
IGHJ26         YAACGHGTAVTVNA
IGHJ27         ACGHGTAVTVNP
IGHJ28          CGDGTAVTVNP
IGHJ29         YAACGDATAVTVNP
IGHJ30         ACGDGTVVTVSP
IGHJ31          CGDGTAVTVNP
IGHJ32         YAACGDGTAVTVNP
IGHJ33          CGDNTAVTVNP
IGHJ34         ACGHGTTVTVAP
IGHJ35          CGLGTVLTVNP
IGHJ36         AACGDGTAVTVNP
IGHJ37         ACGLGTAVTVNP
IGHJ38         YAACGDGTAVTVNP
IGHJ39         AACGDGTAVTVNP
IGHJ40          YGGGTVVTVNP
IGHJ41         ACGDGTAVTVNP
IGHJ42         AACGDGTAVTVNP
IGHJ43          CGGGTDVTVNT
IGHJ44         AACGDGTAVTVNP
IGHJ45         YAACGDGTAVTVNP
IGHJ46         YAACGDGTSVTVNP
IGHJ47         YAACGDGTAVTVNP
IGHJ48         AACGEGTAVTVNP
IGHJ49         YAACGDGTTVTVKP
IGHJ50         LDYWGDGTFLEVTS
IGHJ51          CGDGTAVTVNP
IGHJ52         ACGDGTAVTVNP
```

### nurse shark IGl J-segments

```
IGlJlSl*01     FAFGKGTKLRLSR
IGlJlS2*01     FAFGKGTKLRLSR
IGlJlS3*01     FVFGKGTKLRLSR
IGlJlS4*01     FAFGKGTKLRLSR
IGlJlS5*01     RAFGKGTKLRLSR
IGlJlS6*01     VVFGKGTKLRLSR
```

## Conserved GXGT motif in mouse TCR J-segments

### mouse TRA J-segments

```
TRAJ59          .LLKREDKATFATGGYEAEED..
TRAJ58          .QQGTGSKLSFGKGAKLTVSP..
TRAJ57          .NQGGSAKLIFGEGTKLTVSS..
TRAJ56          .ATGGNNKLTFGQGTVLSVIP..
TRAJ53          NSGGSNYKLTFGKGTLLTVTP..
TRAJ52          NTGANTGKLTFGHGTILRVHP..
TRAJ50          .ASSSFSKLVFGQGTSLSVVP..
TRAJ49          ..NTGYQNFYFGKGTSLTVIP..
TRAJ48          .ANYGNEKITFGAGTKLTIKP..
TRAJ47          ...HYANKMICGLGTILRVRP..
TRAJ46          .RRQQCRHAGFGDGDELGVST..
TRAJ45          .NTEGADRLTFGKGTQLIIQP..
TRAJ44          .VTGSGGKLTLGAGTRLQVNL..
TRAJ43          ...NNNNAPRFGAGTKLSVKP..
TRAJ42          NSGGSNAKLTFGKGTKLSVKS..
TRAJ41          ...VSNTSSMLAEAPEYWSHP..
TRAJ40          .VNTGNYKYVFGAGTRLKVIA..
TRAJ39          .NNNAGAKLTFGGGTRLTVRP..
TRAJ38          .NVGDNSKLIWGLGTSLVVNP..
TRAJ37          ..TGNTGKLIFGLGTTLQVQP..
TRAJ35          .QTGFASALTFGSGTKVIPCLP.
TRAJ34          ..SSNTDKVVFGTGTRLQVSP..
TRAJ33          ...DSNYQLIWGSGTKLIIKP..
TRAJ32          NYGSSGNKLIFGIGTLLSVKP..
TRAJ31          ...NSNNRIFFGDGTQLVVKP..
TRAJ30          ..DTNAYKVIFGKGTHLHVLP..
TRAJ29          ..NSGSRELVLGREARLSMIE...
TRAJ28          LPGTGSNRLTFGKGTKFSLIP..
TRAJ27          ..NTNTGKLTFGDGTVLTVKP..
TRAJ25          ...RTKVSSVFGTWRLLLVKP..
TRAJ24          .ELASLGKLQFGTGTQVVVTP..
TRAJ23          ..NYNQGKLIFGQGTKLSIKP..
TRAJ22          ..SSGSWQLIFGSGTQLTVMP..
TRAJ21          ...SNYNVLYFGSGTKLTVEP..
TRAJ20          ...SGNYKLGVRSVTKMSVRA..
TRAJ19          .IYRGFHKFSSGIESKHNVSP..
TRAJ18          DRGSALGRLHFGAGTQLIVIP..
TRAJ17          .TNSAGNKLTFGIGTRVLVRP..
TRAJ16          .ATSSGQKLVFGQGTILKVYL..
TRAJ15          ..YQGGRALIFGTGTTVSVSP..
TRAJ13          ...NSGTYQRFGTGTKLQVVP..
TRAJ12          ..GTGGYKVVFGSGTRLLVSP..
TRAJ11          ..DSGYNKLTFGKGTVLLVSP..
TRAJ9           ..RNMGYKLTFGTGTSLLVDP..
TRAJ7           ..DYSNNRLTLGKGTQVVVLP..
TRAJ6           .TSGGNYKPTFGKGTSLVVHP..
TRAJ5           .GTQVVGQLTFGRGTRLQVYA..
TRAJ4           .LSGSFNKLTFGAGTRLLCAH..
TRAJ3           .EFSYSSKLIFGAETKLRNPPY.
TRAJ2           NTGGLSGKLTFGEGTQVTVIS..
```

## mouse TRB J-segments

```
TRBJ1-1        ..NTEVFFGKGTRLTVV
TRBJ1-2        ..NSDYTFGSGTRLLVI
TRBJ1-3        .SGNTLYFGEGSRLIVV
TRBJ1-4        .SNERLFFGHGTKLSVL
TRBJ1-5         .NNQAPLFGEGTRLSVL
TRBJ1-6        SYNSPLYFAAGTRLTVT
TRBJ1-7        ..PVLDDHGLGKELRYK
TRBJ2-1        .NYAEQFFGPGTRLTVL
TRBJ2-2        .NTGQLYFGEGSKLTVL
TRBJ2-3        .SAETLYFGSGTRLTVL
TRBJ2-4        .SQNTLYFGAGTRLSVL
TRBJ2-5        .NQDTQYFGPGTRLLVL
TRBV2-7        ..SYEQYFGPGTRLTVL
```

## mouse TRD J-segments

```
TRDJ1          ...TDKLVFGQGTQVTVEP.
TRDJ2          SWDTRQMFFGTGIELFVEP.
```

```
                  FR1                        CDR1           FR2
VHH#3G    QVQLQDSGGGLVQAGGSLRLSCAVSGR   TFSAHS--VYTMG   WFRQAPGKEREFVA
VHH#3E    QVQLQESGGGLVQPGGSLRLSCAASGR   TFSDHSGYTYTIG   WFRQAPGKEREFVA
VHH#1A    QVQLQESGGGLVQPGGSLRLSCATSGF   DFS--VSWMY---   WVRQAPGKGLEWVS
VHH#2B    QVQLQESGGGLVQPGGSLRLSCATSGF   TFS--DYWMY---   WVRQAPGKGLEWVS
VHH#12B   QVQLQESGGGLVQPGGSLRLSCAASGF   EFE--NHWMY---   WVRQAPGKGLEWVS
VHH#7B    QVQLQESGGGLVQPGGSLRLSCAASGS   IFRVNA-----MG   WYRQVPGNQREFVA
          ***** ******* ********* **       *           * ** **    * *


                CDR2                          FR2
VHH#3G    RIYWSSANTYYADSVKG   RFTISRDNAKNTVDLLMNSLKPEDTAVYYCAA
VHH#3E    RIYWSSGNTYYADSVKG   RFAISRDIAKNTVDLTMNNLEPEDTAVYYCAA
VHH#1A    EINTNGLITKYVDSVKG   RFTISRDNAKNTLYLQMDSLIPEDTALYYCAR
VHH#2B    TVNTNGLITRYADSVKG   RFTISRDNAKYTLYLQMNSLKSEDTAVYYCTK
VHH#12B   TVNTNGLITRYADSVKG   RFTISRDNAKYTLYLQMNSLKSEDTAVYYCTK
VHH#7B    -IITSGDNLNYADAVKG   RFTISTDNVKKTVYLQMNVLKPEDTAVYYCNA
          * * *** ** ** *    * *   * *   *   **** ***


                CDR3              FR4       HINGE
VHH#3G    RDGIPTSRTVGSYNY   WGQGTQVTVSS   EPKTPKPQP
VHH#3E    RDGIPTSRSVESYNY   WGQGTQVTVSS   EPKTPKPQP
VHH#1A    ----------SPSGSF   RGQGTQVTVSS   EPKTPKPQP
VHH#2B    -VVPPYSDDSRTNAD   WGQGTQVTVSS   EPKTPKPQP
VHH#12B   -VLPPYSDDSRTNAD   WGQGTQVTVSS   EPKTPKPQP
VHH#7B    --ILQTSRWSIPSNY   WGQGTQVTVSS   EPKTPKPQP
                            *********** *********
```

Fig. 10A

```
                  FR1                          CDR1     FR2
VHH#m3F   QVQLQDSGGGLVQAGGSLRLSCAASGG   TFSSIIMA   WFRQAPGKEREFVGA
VHH#m4B   QVQLQDSGGGLVQAGGSLRLSCGVSGL   SFSGYTMG   WFRQAPGKEREFAAA
VHH#m9A   EVQLVESGGGLVQAGGSLRLSCAASGG   TLSSYITG   WFRQAPGKEREFVGA
VHH#m9E   QVQLVESGGGLVQAGGSLRLSCAASEG   TLSGYILG   WFRQAPGKEREFVGA
          ***   ***************** *       *       ***********  *


                CDR2                          FR2
VHH#m3F   VSWSGGTTVYADSVLG   RFEISRDSARKSVYLQMNSLKPEDTAVYYCAA
VHH#m4B   IGWNSGTTEYRNSVKG   RFTISRDNAKNTVYLQMNSLKPEDTAVYYCAA
VHH#m9A   VSWSSSTIVYADSVEG   RFTISRDNHQNTVYLQMDSLKPEDTAVYYCAA
VHH#m9E   VSWSGGTIVYADSVKG   RFEISRDNARNTVYLQMDSLKSEDTAVYYCAA
          *    *   ** * **  ****   **** ***** *** *********


                CDR3              FR4       HINGE
VHH#m3F   RPYQKYNWA-SASYNV   WGQGTQVTVSS   EPKTPKPQP
VHH#m4B   SP--KYMTAYERSYDF   WGQGTQVTVSS   EPKTPKPQP
VHH#m9A   RPYQKYNWA-SASYNV   WGQGTQVTVSS   ---------
VHH#m9E   RPYQRFNWA-SASYNV   WGRGTQVTVSS   ---------
            *     * *    **   ** *******
```

* 1 AA change in FR4 on position 108
as represented in the following sequence alignment:

DP47        EVQLLESGGGLVQPGGSLRLSCAASGFTFS  SYAMS  WVRQAPGKGLEWVS  AISGSGGSTYY

VHH#12B     QVQLQESGGGLVQPGGSLRLSCAASGFEFE  NHWMY  WVRQAPGKGLEWVS  TVNTNGLITRY

DP47        ADSVKG  RFTISRDNSKNTLYLQMNSLRAEDTAVYYCAK  ---------------  --------------
VHH#12B     ADSVKG  RFTISRDNAKYTLYLQMNSLKSEDTAVYYCTK  VLPPYSDDSRTNAD  WGQGTQVTVSS

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 2006004559 W
- US 76170806 P
- WO 9426087 O, O'Connor

### Non-patent literature cited in the description

- **KABAT, E.A. et al.** Sequences of Proteins of Immunological Interest. U.S. Department of Health and Human Services, 1991
- **TATUSOVA, T. A. et al.** *FEMS Microbiol Lett,* 1999, vol. 174, 187-188
- **KARLI ; ALTSCHUL.** *Proc. Natl. Acad. Sci. USA,* 1990, vol. 87 (6), 2264-8
- **LE GALL et al.** *Protein Engineering, Design & Selection,* 2004, vol. 17, 357-366
- **KIPRIYANOV et al.** *Int. J. Cancer,* 1998, vol. 77, 763-772
- **LE GALL et al.** *J. Immunol. Methods,* 2004, vol. 285, 111-127
- **LE GALL et al.** *FEBS Letters,* 1995, vol. 453, 164-168
- **KIPRIYANOV et al.** *Protein Engineering,* 1997, vol. 10, 445-453
- **THOMPSON et al.** *Nucleic Acids Research,* 1997, vol. 25, 4876-4882
- **CHENNA R et al.** *Nucleic Acids Res,* 2003, vol. 31, 3497-3500
- **ALTSCHUL et al.** *J. Mol. Biol.,* 1990, vol. 215, 403-410
- **GISH, W. ; STATES, D.J.** *Nature Genet.,* 1993, vol. 3, 266-272
- **MADDEN et al.** *Meth. Enzymol.,* 1996, vol. 266, 131-141
- **ALTSCHUL et al.** *Nucleic Acids Res.,* 1997, vol. 25, 3389-3402
- **ZHANG et al.** *J Comput Biol,* 2000, vol. 7 (1-2), 203-14
- **ZHANG, J. ; MADDEN, T.L.** *Genome Res.,* 1997, vol. 7, 649-656
- Current Protocols in Molecular Biology. 1991, vol. 2, 16.4.1-16.7.8
- **DAILEY et al.** *J. Virol.,* 1985, vol. 54, 739-749
- **PEAR et al.** *Proc. Natl. Acad. Sci. U.S.A.,* 1993, vol. 90, 8392-8396
- Current Protocols in Molecular Biology. Greene Publishing Associates and John Wiley & Sons Inc, 1993
- **MACK.** Remington's Pharmaceutical Sciences. 1982
- **KNIGHT et al.** *J. Exp. Med.,* 1978, vol. 147, 1653
- **REINERSTEN et al.** *New Eng. J. Med.,* 1978, vol. 299, 515
- **LINDSTROM et al.** *Adv. Immunol.,* 1988, vol. 42, 233
- **STUART et al.** *Ann. Rev. Immunol.,* 1984, vol. 42, 233
- **VAN EDEN et al.** *Nature,* 1988, vol. 331, 171
- **BLOM, A.B. et al.** *Osteoarthritis Cartilage,* 2004, vol. 12, 627-635
- **MARON et al.** *J. Exp. Med.,* 1980, vol. 152, 1115
- **KANASAWA et al.** *Diabetologia,* 1984, vol. 27, 113
- **PATERSON et al.** Textbook of Immunopathology. Grune and Stratton, 1986, 179-213
- **MCFARLIN et al.** *Science,* 1973, vol. 179, 478
- **SAT et al.** *J. Immunol.,* 1987, vol. 138, 179

SEQUENCE LISTING

<110> Domantis Limited
      Beckmann, Roland

<120> Fusion Proteins that Contain Natural
  Junctions

<130> 3440.1017002

<140> PCT/GB2007/000227
<141> 2007-01-24

<150> PCT/GB2006/004559
<151> 2006-12-05

<150> US 60/761,708
<151> 2006-01-24

<160> 542

<170> FastSEQ for Windows Version 4.0

<210> 1
<211> 17
<212> PRT
<213> Homo sapiens

<400> 1
Ala Glu Tyr Phe Gln His Trp Gly Gln Gly Thr Leu Val Thr Val Ser
 1               5                   10                  15
Ser

<210> 2
<211> 17
<212> PRT
<213> Homo sapiens

<400> 2
Tyr Trp Tyr Phe Asp Leu Trp Gly Arg Gly Thr Leu Val Thr Val Ser
 1               5                   10                  15
Ser

<210> 3
<211> 15
<212> PRT
<213> Homo sapiens

<400> 3
Ala Phe Asp Val Trp Gly Gln Gly Thr Met Val Thr Val Ser Ser
 1               5                   10                  15

<210> 4
<211> 15
<212> PRT
<213> Homo sapiens

<400> 4
Tyr Phe Asp Tyr Trp Gly Gln Gly Thr Leu Val Thr Val Ser Ser
1               5                   10                  15

<210> 5
<211> 16
<212> PRT
<213> Homo sapiens

<400> 5
Asn Trp Phe Asp Ser Trp Gly Gln Gly Thr Leu Val Thr Val Ser Ser
1               5                   10                  15

<210> 6
<211> 20
<212> PRT
<213> Homo sapiens

<400> 6
Tyr Tyr Tyr Tyr Tyr Gly Met Asp Val Trp Gly Gln Gly Thr Thr Val
1               5                   10                  15
Thr Val Ser Ser
          20

<210> 7
<211> 12
<212> PRT
<213> Homo sapiens

<400> 7
Trp Thr Phe Gly Gln Gly Thr Lys Val Glu Ile Lys
1               5                   10

<210> 8
<211> 12
<212> PRT
<213> Homo sapiens

<400> 8
Tyr Thr Phe Gly Gln Gly Thr Lys Leu Glu Ile Lys
1               5                   10

<210> 9
<211> 12
<212> PRT
<213> Homo sapiens

<400> 9

```
Phe Thr Phe Gly Pro Gly Thr Lys Val Asp Ile Lys
 1               5                   10
```

```
<210> 10
<211> 12
<212> PRT
<213> Homo sapiens

<400> 10
Leu Thr Phe Gly Gly Gly Thr Lys Val Glu Ile Lys
 1               5                   10
```

```
<210> 11
<211> 12
<212> PRT
<213> Homo sapiens

<400> 11
Ile Thr Phe Gly Gln Gly Thr Arg Leu Glu Ile Lys
 1               5                   10
```

```
<210> 12
<211> 12
<212> PRT
<213> Homo sapiens

<400> 12
Tyr Val Phe Gly Thr Gly Thr Lys Val Thr Val Leu
 1               5                   10
```

```
<210> 13
<211> 12
<212> PRT
<213> Homo sapiens

<400> 13
Val Val Phe Gly Gly Gly Thr Lys Leu Thr Val Leu
 1               5                   10
```

```
<210> 14
<211> 12
<212> PRT
<213> Homo sapiens

<400> 14
Val Val Phe Gly Gly Gly Thr Lys Leu Thr Val Leu
 1               5                   10
```

```
<210> 15
<211> 12
<212> PRT
<213> Homo sapiens
```

<400> 15
Phe Val Phe Gly Gly Gly Thr Gln Leu Ile Ile Leu
1               5                   10


<210> 16
<211> 12
<212> PRT
<213> Homo sapiens

<400> 16
Trp Val Phe Gly Glu Gly Thr Glu Leu Thr Val Leu
1               5                   10


<210> 17
<211> 12
<212> PRT
<213> Homo sapiens

<400> 17
Asn Val Phe Gly Ser Gly Thr Lys Val Thr Val Leu
1               5                   10


<210> 18
<211> 12
<212> PRT
<213> Homo sapiens

<400> 18
Ala Val Phe Gly Gly Gly Thr Gln Leu Thr Val Leu
1               5                   10


<210> 19
<211> 15
<212> PRT
<213> Homo sapiens

<400> 19
Asn Thr Glu Ala Phe Phe Gly Gln Gly Thr Arg Leu Thr Val Val
1               5                   10                  15


<210> 20
<211> 15
<212> PRT
<213> Homo sapiens

<400> 20
Asn Tyr Gly Tyr Thr Phe Gly Ser Gly Thr Arg Leu Thr Val Val
1               5                   10                  15


<210> 21
<211> 16

```
<212> PRT
<213> Homo sapiens

<400> 21
Ser Gly Asn Thr Ile Tyr Phe Gly Glu Gly Ser Trp Leu Thr Val Val
1               5                   10                  15


<210> 22
<211> 16
<212> PRT
<213> Homo sapiens

<400> 22
Thr Asn Glu Lys Leu Phe Phe Gly Ser Gly Thr Gln Leu Ser Val Leu
1               5                   10                  15


<210> 23
<211> 16
<212> PRT
<213> Homo sapiens

<400> 23
Ser Asn Gln Pro Gln His Phe Gly Asp Gly Thr Arg Leu Ser Ile Leu
1               5                   10                  15


<210> 24
<211> 17
<212> PRT
<213> Homo sapiens

<400> 24
Ser Tyr Asn Ser Pro Leu His Phe Gly Asn Gly Thr Arg Leu Thr Val
1               5                   10                  15
Thr


<210> 25
<211> 16
<212> PRT
<213> Homo sapiens

<400> 25
Ser Tyr Asn Glu Gln Phe Phe Gly Pro Gly Thr Arg Leu Thr Val Leu
1               5                   10                  15


<210> 26
<211> 16
<212> PRT
<213> Homo sapiens

<400> 26
Asn Thr Gly Glu Leu Phe Phe Gly Glu Gly Ser Arg Leu Thr Val Leu
1               5                   10                  15
```

```
<210> 27
<211> 15
<212> PRT
<213> Homo sapiens

<400> 27
Leu Arg Gly Ala Ala Gly Arg Leu Gly Gly Gly Leu Leu Val Leu
1               5                   10                  15


<210> 28
<211> 16
<212> PRT
<213> Homo sapiens

<400> 28
Ser Thr Asp Thr Gln Tyr Phe Gly Pro Gly Thr Arg Leu Thr Val Leu
1               5                   10                  15


<210> 29
<211> 16
<212> PRT
<213> Homo sapiens

<400> 29
Ala Lys Asn Ile Gln Tyr Phe Gly Ala Gly Thr Arg Leu Ser Val Leu
1               5                   10                  15


<210> 30
<211> 15
<212> PRT
<213> Homo sapiens

<400> 30
Gln Glu Thr Gln Tyr Phe Gly Pro Gly Thr Arg Leu Leu Val Leu
1               5                   10                  15


<210> 31
<211> 17
<212> PRT
<213> Homo sapiens

<400> 31
Ser Gly Ala Asn Val Leu Thr Phe Gly Ala Gly Ser Arg Leu Thr Val
1               5                   10                  15
Leu


<210> 32
<211> 15
<212> PRT
<213> Homo sapiens
```

<400> 32
Ser Tyr Glu Gln Tyr Phe Gly Pro Gly Thr Arg Leu Thr Val Thr
1               5                   10                  15


<210> 33
<211> 16
<212> PRT
<213> Homo sapiens

<400> 33
Asn Tyr Tyr Lys Lys Leu Phe Gly Ser Gly Thr Thr Leu Val Val Thr
1               5                   10                  15


<210> 34
<211> 16
<212> PRT
<213> Homo sapiens

<400> 34
Asn Tyr Tyr Lys Lys Leu Phe Gly Ser Gly Thr Thr Leu Val Val Thr
1               5                   10                  15


<210> 35
<211> 20
<212> PRT
<213> Homo sapiens

<400> 35
Gly Gln Glu Leu Gly Lys Lys Ile Lys Val Phe Gly Pro Gly Thr Lys
1               5                   10                  15
Leu Ile Ile Thr
            20


<210> 36
<211> 19
<212> PRT
<213> Homo sapiens

<400> 36
Thr Thr Gly Trp Phe Lys Ile Phe Ala Glu Gly Thr Lys Leu Ile Val
1               5                   10                  15
Thr Ser Pro


<210> 37
<211> 19
<212> PRT
<213> Homo sapiens

<400> 37
Ser Ser Asp Trp Ile Lys Thr Phe Ala Lys Gly Thr Arg Leu Ile Val
1               5                   10                  15

Thr Ser Pro

```
<210> 38
<211> 16
<212> PRT
<213> Homo sapiens

<400> 38
Thr Asp Lys Leu Ile Phe Gly Lys Gly Thr Arg Val Thr Val Glu Pro
 1               5                   10                  15


<210> 39
<211> 17
<212> PRT
<213> Homo sapiens

<400> 39
Leu Thr Ala Gln Leu Phe Phe Gly Lys Gly Thr Gln Leu Ile Val Glu
 1               5                   10                  15
Pro


<210> 40
<211> 19
<212> PRT
<213> Homo sapiens

<400> 40
Ser Trp Asp Thr Arg Gln Met Phe Phe Gly Thr Gly Ile Lys Leu Phe
 1               5                   10                  15
Val Glu Pro


<210> 41
<211> 15
<212> PRT
<213> Homo sapiens

<400> 41
Arg Pro Leu Ile Phe Gly Lys Gly Thr Tyr Leu Glu Val Gln Gln
 1               5                   10                  15


<210> 42
<211> 19
<212> PRT
<213> Homo sapiens

<400> 42
Tyr Arg Val Asn Arg Lys Leu Thr Phe Gly Ala Asn Thr Arg Gly Ile
 1               5                   10                  15
Met Lys Leu
```

```
<210> 43
<211> 17
<212> PRT
<213> Homo sapiens

<400> 43
Lys Glu Gly Asn Arg Lys Phe Thr Phe Gly Met Gly Thr Gln Val Arg
 1               5                   10                  15
Val


<210> 44
<211> 19
<212> PRT
<213> Homo sapiens

<400> 44
Glu Thr Ser Gly Ser Arg Leu Thr Phe Gly Glu Gly Thr Gln Leu Thr
 1               5                   10                  15
Val Asn Pro


<210> 45
<211> 20
<212> PRT
<213> Homo sapiens

<400> 45
Thr Gln Gly Gly Ser Glu Lys Leu Val Phe Gly Lys Gly Thr Lys Leu
 1               5                   10                  15
Thr Val Asn Pro
                20


<210> 46
<211> 20
<212> PRT
<213> Homo sapiens

<400> 46
Tyr Thr Gly Ala Asn Ser Lys Leu Thr Phe Gly Lys Gly Ile Thr Leu
 1               5                   10                  15
Ser Val Arg Pro
                20


<210> 47
<211> 19
<212> PRT
<213> Homo sapiens

<400> 47
Ile Gln Gly Ala Gln Lys Leu Val Phe Gly Gln Gly Thr Arg Leu Thr
 1               5                   10                  15
```

```
Ile Asn Pro


<210> 48
<211> 21
<212> PRT
<213> Homo sapiens

<400> 48
Asn Ser Gly Gly Ser Asn Tyr Lys Leu Thr Phe Gly Lys Gly Thr Leu
1               5                   10                  15
Leu Thr Val Asn Pro
                20


<210> 49
<211> 22
<212> PRT
<213> Homo sapiens

<400> 49
Asn Ala Gly Gly Thr Ser Tyr Gly Lys Leu Thr Phe Gly Gln Gly Thr
1               5                   10                  15
Ile Leu Thr Val His Pro
                20


<210> 50
<211> 19
<212> PRT
<213> Homo sapiens

<400> 50
Lys Thr Ser Tyr Asp Lys Val Ile Phe Gly Pro Gly Thr Ser Leu Ser
1               5                   10                  15
Val Ile Pro


<210> 51
<211> 18
<212> PRT
<213> Homo sapiens

<400> 51
Asn Thr Gly Asn Gln Phe Tyr Phe Gly Thr Gly Thr Ser Leu Thr Val
1               5                   10                  15
Ile Pro


<210> 52
<211> 20
<212> PRT
<213> Homo sapiens

<400> 52
```

```
Ser Asn Phe Gly Asn Glu Lys Leu Thr Phe Gly Thr Gly Thr Arg Leu
1               5                   10                  15
Thr Ile Ile Pro
                20


<210> 53
<211> 18
<212> PRT
<213> Homo sapiens

<400> 53
Glu Tyr Gly Asn Lys Leu Val Phe Gly Ala Gly Thr Ile Leu Arg Val
1               5                   10                  15
Lys Ser


<210> 54
<211> 20
<212> PRT
<213> Homo sapiens

<400> 54
Lys Lys Ser Ser Gly Asp Lys Leu Thr Phe Gly Thr Gly Thr Arg Leu
1               5                   10                  15
Ala Val Arg Pro
                20


<210> 55
<211> 21
<212> PRT
<213> Homo sapiens

<400> 55
Tyr Ser Gly Gly Gly Ala Asp Gly Leu Thr Phe Gly Lys Gly Thr His
1               5                   10                  15
Leu Ile Ile Gln Pro
                20


<210> 56
<211> 20
<212> PRT
<213> Homo sapiens

<400> 56
Asn Thr Gly Thr Ala Ser Lys Leu Thr Phe Gly Thr Gly Thr Arg Leu
1               5                   10                  15
Gln Val Thr Leu
                20


<210> 57
<211> 17
<212> PRT
<213> Homo sapiens
```

<400> 57
Asn Asn Asn Asp Met Arg Phe Gly Ala Gly Thr Arg Leu Thr Val Lys
1               5                   10                  15
Pro


<210> 58
<211> 20
<212> PRT
<213> Homo sapiens

<400> 58
Asn Ser Asn Ser Gly Tyr Ala Leu Asn Phe Gly Lys Gly Thr Ser Leu
1               5                   10                  15
Leu Val Thr Pro
            20


<210> 59
<211> 20
<212> PRT
<213> Homo sapiens

<400> 59
Thr Thr Ser Gly Thr Tyr Lys Tyr Ile Phe Gly Thr Gly Thr Arg Leu
1               5                   10                  15
Lys Val Leu Ala
            20


<210> 60
<211> 20
<212> PRT
<213> Homo sapiens

<400> 60
Asn Asn Asn Ala Gly Asn Met Leu Thr Phe Gly Gly Gly Thr Arg Leu
1               5                   10                  15
Met Val Lys Pro
            20


<210> 61
<211> 20
<212> PRT
<213> Homo sapiens

<400> 61
Asn Ala Gly Asn Asn Arg Lys Leu Ile Trp Gly Leu Gly Thr Ser Leu
1               5                   10                  15
Ala Val Asn Pro
            20


<210> 62
<211> 20

```
<212> PRT
<213> Homo sapiens

<400> 62
Gly Ser Gly Asn Thr Gly Lys Leu Ile Phe Gly Gln Gly Thr Thr Leu
1               5                   10                  15
Gln Val Lys Pro
            20


<210> 63
<211> 19
<212> PRT
<213> Homo sapiens

<400> 63
Gln Thr Gly Ala Asn Asn Leu Phe Phe Gly Thr Gly Thr Arg Leu Thr
1               5                   10                  15
Val Ile Pro



<210> 64
<211> 19
<212> PRT
<213> Homo sapiens

<400> 64
Ile Gly Phe Gly Asn Val Leu His Cys Gly Ser Gly Thr Gln Val Ile
1               5                   10                  15
Val Leu Pro



<210> 65
<211> 19
<212> PRT
<213> Homo sapiens

<400> 65
Ser Tyr Asn Thr Asp Lys Leu Ile Phe Gly Thr Gly Thr Arg Leu Gln
1               5                   10                  15
Val Phe Pro



<210> 66
<211> 18
<212> PRT
<213> Homo sapiens

<400> 66
Asp Ser Asn Tyr Gln Leu Ile Trp Gly Ala Gly Thr Lys Leu Ile Ile
1               5                   10                  15
Lys Pro
```

```
<210> 67
<211> 21
<212> PRT
<213> Homo sapiens

<400> 67
Asn Tyr Gly Gly Ala Thr Asn Lys Leu Ile Phe Gly Thr Gly Thr Leu
1               5                   10                  15
Leu Ala Val Gln Pro
            20


<210> 68
<211> 18
<212> PRT
<213> Homo sapiens

<400> 68
Asn Asn Asn Ala Arg Leu Met Phe Gly Asp Gly Thr Gln Leu Val Val
1               5                   10                  15
Lys Pro


<210> 69
<211> 18
<212> PRT
<213> Homo sapiens

<400> 69
Asn Arg Asp Asp Lys Ile Ile Phe Gly Lys Gly Thr Arg Leu His Ile
1               5                   10                  15
Leu Pro


<210> 70
<211> 19
<212> PRT
<213> Homo sapiens

<400> 70
Asn Ser Gly Asn Thr Pro Leu Val Phe Gly Lys Gly Thr Arg Leu Ser
1               5                   10                  15
Val Ile Ala


<210> 71
<211> 21
<212> PRT
<213> Homo sapiens

<400> 71
Tyr Ser Gly Ala Gly Ser Tyr Gln Leu Thr Phe Gly Lys Gly Thr Lys
1               5                   10                  15
Leu Ser Val Ile Pro
            20
```

77

```
<210> 72
<211> 19
<212> PRT
<213> Homo sapiens

<400> 72
Asn Thr Asn Ala Gly Lys Ser Thr Phe Gly Asp Gly Thr Thr Leu Thr
1               5                   10                  15
Val Lys Pro


<210> 73
<211> 19
<212> PRT
<213> Homo sapiens

<400> 73
Asp Asn Tyr Gly Gln Asn Phe Val Phe Gly Pro Gly Thr Arg Leu Ser
1               5                   10                  15
Val Leu Pro


<210> 74
<211> 19
<212> PRT
<213> Homo sapiens

<400> 74
Glu Gly Gln Gly Phe Ser Phe Ile Phe Gly Lys Gly Thr Arg Leu Leu
1               5                   10                  15
Val Lys Pro


<210> 75
<211> 20
<212> PRT
<213> Homo sapiens

<400> 75
Thr Thr Asp Ser Trp Gly Lys Phe Glu Phe Gly Ala Gly Thr Gln Val
1               5                   10                  15
Val Val Thr Pro
            20


<210> 76
<211> 20
<212> PRT
<213> Homo sapiens

<400> 76
Ile Tyr Asn Gln Gly Gly Lys Leu Ile Phe Gly Gln Gly Thr Glu Leu
1               5                   10                  15
```

Ser Val Lys Pro
                20


<210> 77
<211> 20
<212> PRT
<213> Homo sapiens

<400> 77
Ser Ser Gly Ser Ala Arg Gln Leu Thr Phe Gly Ser Gly Thr Gln Leu
1               5                   10                  15
Thr Val Leu Pro
                20


<210> 78
<211> 18
<212> PRT
<213> Homo sapiens

<400> 78
Tyr Asn Phe Asn Lys Phe Tyr Phe Gly Ser Gly Thr Lys Leu Asn Val
1               5                   10                  15
Lys Pro


<210> 79
<211> 18
<212> PRT
<213> Homo sapiens

<400> 79
Ser Asn Asp Tyr Lys Leu Ser Phe Gly Ala Gly Thr Thr Val Thr Val
1               5                   10                  15
Arg Ala


<210> 80
<211> 19
<212> PRT
<213> Homo sapiens

<400> 80
Tyr Gln Arg Phe Tyr Asn Phe Thr Phe Gly Lys Gly Ser Lys His Asn
1               5                   10                  15
Val Thr Pro


<210> 81
<211> 21
<212> PRT
<213> Homo sapiens

<400> 81

```
Asp Arg Gly Ser Thr Leu Gly Arg Leu Tyr Phe Gly Arg Gly Thr Gln
1               5                   10                  15
Leu Thr Val Trp Pro
                20
```

```
<210> 82
<211> 20
<212> PRT
<213> Homo sapiens
```

```
<400> 82
Ile Lys Ala Ala Gly Asn Lys Leu Thr Phe Gly Gly Gly Thr Arg Val
1               5                   10                  15
Leu Val Lys Pro
                20
```

```
<210> 83
<211> 19
<212> PRT
<213> Homo sapiens
```

```
<400> 83
Phe Ser Asp Gly Gln Lys Leu Leu Phe Ala Arg Gly Thr Met Leu Lys
1               5                   10                  15
Val Asp Leu
```

```
<210> 84
<211> 19
<212> PRT
<213> Homo sapiens
```

```
<400> 84
Asn Gln Ala Gly Thr Ala Leu Ile Phe Gly Lys Gly Thr Thr Leu Ser
1               5                   10                  15
Val Ser Ser
```

```
<210> 85
<211> 17
<212> PRT
<213> Homo sapiens
```

```
<400> 85
Ile Tyr Ser Thr Phe Ile Phe Gly Ser Gly Thr Arg Leu Ser Val Lys
1               5                   10                  15
Pro
```

```
<210> 86
<211> 20
<212> PRT
<213> Homo sapiens
```

```
<400> 86
Asn Ser Gly Gly Tyr Gln Lys Val Thr Phe Gly Ile Gly Thr Lys Leu
1               5                   10                  15
Gln Val Ile Pro
            20


<210> 87
<211> 19
<212> PRT
<213> Homo sapiens

<400> 87
Met Asp Ser Ser Tyr Lys Leu Ile Phe Gly Ser Gly Thr Arg Leu Leu
1               5                   10                  15
Val Arg Pro


<210> 88
<211> 19
<212> PRT
<213> Homo sapiens

<400> 88
Asn Ser Gly Tyr Ser Thr Leu Thr Phe Gly Lys Gly Thr Met Leu Leu
1               5                   10                  15
Val Ser Pro


<210> 89
<211> 21
<212> PRT
<213> Homo sapiens

<400> 89
Ile Leu Thr Gly Gly Gly Asn Lys Leu Thr Phe Gly Thr Gly Thr Gln
1               5                   10                  15
Leu Lys Val Glu Leu
            20


<210> 90
<211> 20
<212> PRT
<213> Homo sapiens

<400> 90
Gly Asn Thr Gly Gly Phe Lys Thr Ile Phe Gly Ala Gly Thr Arg Leu
1               5                   10                  15
Phe Val Lys Ala
            20


<210> 91
<211> 19
```

```
<212> PRT
<213> Homo sapiens

<400> 91
Asn Thr Gly Phe Gln Lys Leu Val Phe Gly Thr Gly Thr Arg Leu Leu
1               5                   10                  15
Val Ser Pro


<210> 92
<211> 19
<212> PRT
<213> Homo sapiens

<400> 92
Asp Tyr Gly Asn Asn Arg Leu Ala Phe Gly Lys Gly Asn Gln Val Val
1               5                   10                  15
Val Ile Pro


<210> 93
<211> 20
<212> PRT
<213> Homo sapiens

<400> 93
Ala Ser Gly Gly Ser Tyr Ile Pro Thr Phe Gly Arg Gly Thr Ser Leu
1               5                   10                  15
Ile Val His Pro
                20


<210> 94
<211> 19
<212> PRT
<213> Homo sapiens

<400> 94
Asp Thr Gly Arg Arg Ala Leu Thr Phe Gly Ser Gly Thr Arg Leu Gln
1               5                   10                  15
Val Gln Pro


<210> 95
<211> 20
<212> PRT
<213> Homo sapiens

<400> 95
Phe Ser Gly Gly Tyr Asn Lys Leu Ile Phe Gly Ala Gly Thr Arg Leu
1               5                   10                  15
Ala Val His Pro
                20
```

```
<210> 96
<211> 20
<212> PRT
<213> Homo sapiens

<400> 96
Gly Tyr Ser Ser Ala Ser Lys Ile Ile Phe Gly Ser Gly Thr Arg Leu
1               5                   10                  15
Ser Ile Arg Pro
            20


<210> 97
<211> 21
<212> PRT
<213> Homo sapiens

<400> 97
Asn Thr Gly Gly Thr Ile Asp Lys Leu Thr Phe Gly Lys Gly Thr His
1               5                   10                  15
Val Phe Ile Ile Ser
            20


<210> 98
<211> 20
<212> PRT
<213> Homo sapiens

<400> 98
Tyr Glu Ser Ile Thr Ser Gln Leu Gln Phe Gly Lys Gly Thr Arg Val
1               5                   10                  15
Ser Thr Ser Pro
            20


<210> 99
<211> 17
<212> PRT
<213> Unknown

<220>
<223> Mouse

<400> 99
Tyr Trp Tyr Phe Asp Val Trp Gly Ala Gly Thr Thr Val Thr Val Ser
1               5                   10                  15
Ser


<210> 100
<211> 15
<212> PRT
<213> Unknown

<220>
<223> Mouse
```

```
<400> 100
Tyr Phe Asp Tyr Trp Gly Gln Gly Thr Thr Leu Thr Val Ser Ser
1               5                   10                  15


<210> 101
<211> 15
<212> PRT
<213> Unknown

<220>
<223> Mouse

<400> 101
Trp Phe Ala Tyr Trp Gly Gln Gly Thr Leu Val Thr Val Ser Ala
1               5                   10                  15


<210> 102
<211> 17
<212> PRT
<213> Unknown

<220>
<223> Mouse

<400> 102
Tyr Tyr Ala Met Asp Tyr Trp Gly Gln Gly Thr Ser Val Thr Val Ser
1               5                   10                  15
Ser


<210> 103
<211> 18
<212> PRT
<213> Unknown

<220>
<223> Llama

<400> 103
Gly Tyr Arg Tyr Leu Glu Val Trp Gly Gln Gly Thr Leu Val Thr Val
1               5                   10                  15
Ser Ser


<210> 104
<211> 16
<212> PRT
<213> Unknown

<220>
<223> Llama

<400> 104
```

```
Asn Ala Leu Asp Ala Trp Gly Gln Gly Thr Leu Val Thr Val Ser Ser
1                   5                   10                  15


<210> 105
<211> 15
<212> PRT
<213> Unknown

<220>
<223> Llama

<400> 105
Glu Tyr Asp Tyr Trp Gly Gln Gly Thr Gln Val Thr Val Ser Ser
1                   5                   10                  15


<210> 106
<211> 15
<212> PRT
<213> Unknown

<220>
<223> Llama

<400> 106
Pro Gln Phe Glu Tyr Trp Gly Gln Gly Thr Leu Val Thr Val Ser
1                   5                   10                  15


<210> 107
<211> 14
<212> PRT
<213> Unknown

<220>
<223> Llama

<400> 107
Asp Phe Gly Ser Trp Gly Gln Gly Thr Leu Val Thr Val Ser
1                   5                   10


<210> 108
<211> 17
<212> PRT
<213> Unknown

<220>
<223> Sheep

<400> 108
Tyr Ala Asp Phe His Leu Trp Asp Gln Gly Ala Leu Val Thr Val Ser
1                   5                   10                  15
Ser
```

```
<210> 109
<211> 17
<212> PRT
<213> Unknown

<220>
<223> Sheep

<400> 109
Cys Trp Asp Ser Gly Leu Trp Gly Gln Arg Thr Pro Val Thr Val Ser
 1               5                  10                  15
Leu


<210> 110
<211> 15
<212> PRT
<213> Unknown

<220>
<223> Sheep

<400> 110
Ala Phe Asp Ser Trp Gly Gln Arg Ala Pro Val Thr Val Ser Ser
 1               5                  10                  15


<210> 111
<211> 15
<212> PRT
<213> Unknown

<220>
<223> Sheep

<400> 111
Tyr Ile Asp Tyr Trp Gly Pro Gly Leu Leu Val Thr Val Ser Ser
 1               5                  10                  15


<210> 112
<211> 15
<212> PRT
<213> Unknown

<220>
<223> Sheep

<400> 112
Asp Trp Leu Lys His Trp Gly Gln Gly Pro Arg Arg Cys Leu Leu
 1               5                  10                  15


<210> 113
<211> 17
<212> PRT
<213> Unknown
```

```
<220>
<223> Sheep

<400> 113
Tyr Tyr Gly Val Asp Val Trp Gly Arg Gly Leu Leu Val Thr Val Ser
 1               5                   10                  15
Ser


<210> 114
<211> 14
<212> PRT
<213> Unknown

<220>
<223> Pig

<400> 114
Leu Leu Cys Trp Gly Pro Gly Val Glu Val Val Val Ser Ser
 1               5                   10


<210> 115
<211> 12
<212> PRT
<213> Unknown

<220>
<223> Mouse

<400> 115
Trp Thr Phe Gly Gly Gly Thr Lys Leu Glu Ile Lys
 1               5                   10


<210> 116
<211> 12
<212> PRT
<213> Unknown

<220>
<223> Mouse

<400> 116
Tyr Thr Phe Gly Gly Gly Thr Lys Leu Glu Ile Lys
 1               5                   10


<210> 117
<211> 12
<212> PRT
<213> Unknown

<220>
<223> Mouse
```

```
<400> 117
Ile Thr Phe Ser Asp Gly Thr Arg Leu Glu Ile Lys
1               5                   10


<210> 118
<211> 12
<212> PRT
<213> Unknown

<220>
<223> Mouse

<400> 118
Phe Thr Phe Gly Ser Gly Thr Lys Leu Glu Ile Lys
1               5                   10


<210> 119
<211> 12
<212> PRT
<213> Unknown

<220>
<223> Mouse

<400> 119
Leu Thr Phe Gly Ala Gly Thr Lys Leu Glu Leu Lys
1               5                   10


<210> 120
<211> 12
<212> PRT
<213> Unknown

<220>
<223> Possum

<400> 120
Leu Thr Phe Gly Gln Gly Thr Lys Leu Glu Ile Lys
1               5                   10


<210> 121
<211> 12
<212> PRT
<213> Unknown

<220>
<223> Possum

<400> 121
Phe Thr Phe Gly Gly Gly Thr Lys Val Glu Ile Lys
1               5                   10


<210> 122
```

```
<211> 11
<212> PRT
<213> Unknown

<220>
<223> Sheep

<400> 122
Ser Phe Gly Gln Gly Thr Lys Leu Glu Ile Lys
 1               5                   10


<210> 123
<211> 12
<212> PRT
<213> Unknown

<220>
<223> Sheep

<400> 123
Phe Thr Phe Gly Pro Gly Thr Arg Val Glu Ile Lys
 1               5                   10


<210> 124
<211> 11
<212> PRT
<213> Unknown

<220>
<223> Sheep

<400> 124
Tyr Ala Phe Gly Gly Gly Thr Lys Val Glu Ile
 1               5                   10


<210> 125
<211> 12
<212> PRT
<213> Unknown

<220>
<223> Sheep

<400> 125
Leu Ala Phe Gly Gly Gly Thr Asn Val Glu Ile Lys
 1               5                   10


<210> 126
<211> 12
<212> PRT
<213> Unknown

<220>
<223> Mouse
```

```
<400> 126
Trp Val Phe Gly Gly Gly Thr Lys Leu Thr Val Leu
1               5                   10


<210> 127
<211> 12
<212> PRT
<213> Unknown

<220>
<223> Mouse

<400> 127
Tyr Val Phe Gly Gly Gly Thr Lys Val Thr Val Leu
1               5                   10


<210> 128
<211> 12
<212> PRT
<213> Unknown

<220>
<223> Mouse

<400> 128
Phe Ile Phe Gly Ser Gly Thr Lys Val Thr Val Leu
1               5                   10


<210> 129
<211> 12
<212> PRT
<213> Unknown

<220>
<223> Mouse

<400> 129
Gly Ser Phe Ser Ser Asn Gly Leu Leu Tyr Ala Gly
1               5                   10


<210> 130
<211> 12
<212> PRT
<213> Unknown

<220>
<223> Mouse

<400> 130
Trp Val Phe Gly Gly Gly Thr Arg Leu Thr Val Leu
1               5                   10
```

EP 2 441 838 A2

```
<210> 131
<211> 12
<212> PRT
<213> Unknown

<220>
<223> Possum

<400> 131
Tyr Ile Phe Gly Gly Gly Thr Gln Leu Thr Val Ile
 1               5                   10


<210> 132
<211> 12
<212> PRT
<213> Unknown

<220>
<223> Possum

<400> 132
Trp Val Phe Gly Glu Gly Thr His Val Thr Val Leu
 1               5                   10


<210> 133
<211> 12
<212> PRT
<213> Unknown

<220>
<223> Possum

<400> 133
Trp Val Phe Gly Gly Gly Thr His Leu Thr Val Leu
 1               5                   10


<210> 134
<211> 12
<212> PRT
<213> Unknown

<220>
<223> Sheep

<400> 134
Gly Val Phe Gly Ser Gly Thr Arg Leu Thr Val Leu
 1               5                   10


<210> 135
<211> 101
<212> PRT
<213> Unknown

<220>
```

91

<223> Mouse

<400> 135
Glu Ser Ala Arg Asn Pro Thr Ile Tyr Pro Leu Thr Leu Pro Pro Val
1                   5                   10                  15
Leu Cys Ser Asp Pro Val Ile Ile Gly Cys Leu Ile His Asp Tyr Phe
                20                  25                  30
Pro Phe Gly Thr Met Asn Val Thr Trp Gly Lys Ser Gly Lys Asp Ile
            35                  40                  45
Thr Thr Val Asn Phe Pro Pro Ala Leu Ala Ser Gly Gly Arg Tyr Thr
        50                  55                  60
Met Ser Ser Gln Leu Thr Leu Pro Ala Val Glu Cys Pro Glu Gly Glu
65                  70                  75                  80
Ser Val Lys Cys Ser Val Gln His Asp Ser Asn Pro Val Gln Glu Leu
                85                  90                  95
Asp Val Asn Cys Ser
                100

<210> 136
<211> 94
<212> PRT
<213> Unknown

<220>
<223> Mouse

<400> 136
Gly Asp Lys Lys Glu Pro Asp Met Phe Leu Leu Ser Glu Cys Lys Ala
1                   5                   10                  15
Pro Glu Glu Asn Glu Lys Ile Asn Leu Gly Cys Leu Val Ile Gly Ser
                20                  25                  30
Gln Pro Leu Lys Ile Ser Trp Glu Pro Lys Lys Ser Ser Ile Val Glu
            35                  40                  45
His Val Phe Pro Ser Glu Met Arg Asn Gly Asn Tyr Thr Met Val Leu
            50                  55                  60
Gln Val Thr Val Leu Ala Ser Glu Leu Asn Leu Asn His Thr Cys Thr
65                  70                  75                  80
Ile Asn Lys Pro Lys Arg Lys Glu Lys Pro Phe Lys Phe Pro
                85                  90

<210> 137
<211> 91
<212> PRT
<213> Unknown

<220>
<223> Mouse

<400> 137
Ala Ser Ile Arg Asn Pro Gln Leu Tyr Pro Leu Lys Pro Cys Lys Gly
1                   5                   10                  15
Thr Ala Ser Met Thr Leu Gly Cys Leu Val Lys Asp Tyr Phe Pro Asn
                20                  25                  30
Pro Val Thr Val Thr Trp Tyr Ser Asp Ser Leu Asn Met Ser Thr Val
            35                  40                  45
Asn Phe Pro Ala Leu Gly Ser Glu Leu Lys Val Thr Thr Ser Gln Val

```
          50                    55                    60
Thr Ser Trp Gly Lys Ser Ala Lys Asn Phe Thr Cys His Val Thr His
65                    70                    75                    80
Pro Pro Ser Phe Asn Glu Ser Arg Thr Ile Leu
                    85                    90


<210> 138
<211> 97
<212> PRT
<213> Unknown

<220>
<223> Mouse

<400> 138
Ala Lys Thr Thr Pro Pro Ser Val Tyr Pro Leu Ala Pro Gly Ser Ala
 1               5                   10                  15
Ala Gln Thr Asn Ser Met Val Thr Leu Gly Cys Leu Val Lys Gly Tyr
                20                  25                  30
Phe Pro Glu Pro Val Thr Val Thr Trp Asn Ser Gly Ser Leu Ser Ser
            35                  40                  45
Gly Val His Thr Phe Pro Ala Val Leu Glu Ser Asp Leu Tyr Thr Leu
        50                  55                  60
Ser Ser Ser Val Thr Val Pro Ser Ser Pro Arg Pro Ser Glu Thr Val
65                  70                  75                  80
Thr Cys Asn Val Ala His Pro Ala Ser Ser Thr Lys Val Asp Lys Lys
                85                  90                  95
Ile


<210> 139
<211> 97
<212> PRT
<213> Unknown

<220>
<223> Mouse

<400> 139
Ala Lys Thr Thr Ala Pro Ser Val Tyr Pro Leu Ala Pro Val Cys Gly
 1               5                   10                  15
Asp Thr Thr Gly Ser Ser Val Thr Leu Gly Cys Leu Val Lys Gly Tyr
                20                  25                  30
Phe Pro Glu Pro Val Thr Leu Thr Trp Asn Ser Gly Ser Leu Ser Ser
            35                  40                  45
Gly Val His Thr Phe Pro Ala Val Leu Gln Ser Asp Leu Tyr Thr Leu
        50                  55                  60
Ser Ser Ser Val Thr Val Thr Ser Ser Thr Trp Pro Ser Gln Ser Ile
65                  70                  75                  80
Thr Cys Asn Val Ala His Pro Ala Ser Ser Thr Lys Val Asp Lys Lys
                85                  90                  95
Ile


<210> 140
```

```
<211> 97
<212> PRT
<213> Unknown

<220>
<223> Mouse

<400> 140
Ala Lys Thr Thr Pro Pro Ser Val Tyr Pro Leu Ala Pro Gly Cys Gly
1               5                   10                  15
Asp Thr Thr Gly Ser Ser Val Thr Ser Gly Cys Leu Val Lys Gly Tyr
            20                  25                  30
Phe Pro Glu Pro Val Thr Val Thr Trp Asn Ser Gly Ser Leu Ser Ser
        35                  40                  45
Ser Val His Thr Phe Pro Ala Leu Leu Gln Ser Gly Leu Tyr Thr Met
        50                  55                  60
Ser Ser Ser Val Thr Val Pro Ser Ser Thr Trp Pro Ser Gln Thr Val
65                  70                  75                  80
Thr Cys Ser Val Ala His Pro Ala Ser Ser Thr Thr Val Asp Lys Lys
                85                  90                  95
Leu
```

```
<210> 141
<211> 97
<212> PRT
<213> Unknown

<220>
<223> Mouse

<400> 141
Ala Lys Thr Thr Ala Pro Ser Val Tyr Pro Leu Ala Pro Val Cys Gly
1               5                   10                  15
Gly Thr Thr Gly Ser Ser Val Thr Leu Gly Cys Leu Val Lys Gly Tyr
            20                  25                  30
Phe Pro Glu Pro Val Thr Leu Thr Trp Asn Ser Gly Ser Leu Ser Ser
        35                  40                  45
Gly Val His Thr Phe Pro Ala Leu Leu Gln Ser Gly Leu Tyr Thr Leu
        50                  55                  60
Ser Ser Ser Val Thr Val Thr Ser Asn Thr Trp Pro Ser Gln Thr Ile
65                  70                  75                  80
Thr Cys Asn Val Ala His Pro Ala Ser Ser Thr Lys Val Asp Lys Lys
                85                  90                  95
Ile
```

```
<210> 142
<211> 97
<212> PRT
<213> Unknown

<220>
<223> Mouse

<400> 142
```

```
Ala Thr Thr Thr Ala Pro Ser Val Tyr Pro Leu Val Pro Gly Cys Ser
1               5                   10                  15
Asp Thr Ser Gly Ser Ser Val Thr Leu Gly Cys Leu Val Lys Gly Tyr
            20                  25                  30
Phe Pro Glu Pro Val Thr Val Lys Trp Asn Tyr Gly Ala Leu Ser Ser
        35                  40                  45
Gly Val Arg Thr Val Ser Ser Val Leu Gln Ser Gly Phe Tyr Ser Leu
    50                  55                  60
Ser Ser Leu Val Thr Val Pro Ser Ser Thr Trp Pro Ser Gln Thr Val
65                  70                  75                  80
Ile Cys Asn Val Ala His Pro Ala Ser Lys Thr Glu Leu Ile Lys Arg
                85                  90                  95
Ile
```

<210> 143
<211> 105
<212> PRT
<213> Unknown

<220>
<223> Mouse

<400> 143
```
Glu Ser Gln Ser Phe Pro Asn Val Phe Pro Leu Val Ser Cys Glu Ser
1               5                   10                  15
Pro Leu Ser Asp Lys Asn Leu Val Ala Met Gly Cys Leu Ala Arg Asp
            20                  25                  30
Phe Leu Pro Ser Thr Ile Ser Phe Thr Trp Asn Tyr Gln Asn Asn Thr
        35                  40                  45
Glu Val Ile Gln Gly Ile Arg Thr Phe Pro Thr Leu Arg Thr Gly Gly
    50                  55                  60
Lys Tyr Leu Ala Thr Ser Gln Val Leu Leu Ser Pro Lys Ser Ile Leu
65                  70                  75                  80
Glu Gly Ser Asp Glu Tyr Leu Val Cys Lys Ile His Tyr Gly Gly Lys
                85                  90                  95
Asn Arg Asp Leu His Val Pro Ile Pro
            100                 105
```

<210> 144
<211> 101
<212> PRT
<213> Unknown

<220>
<223> Mouse

<400> 144
```
Ser Cys Gln Pro Ser Leu Ser Leu Gln Arg Pro Ala Leu Glu Asp Leu
1               5                   10                  15
Leu Leu Gly Ser Asp Ala Ser Ile Thr Cys Thr Leu Asn Gly Leu Arg
            20                  25                  30
Asn Pro Glu Gly Ala Ala Phe Thr Trp Glu Pro Ser Thr Gly Lys Asp
        35                  40                  45
Ala Val Gln Lys Lys Ala Ala Gln Asn Ser Cys Gly Cys Tyr Ser Val
    50                  55                  60
```

```
Ser Ser Val Leu Pro Gly Cys Ala Glu Arg Trp Asn Ser Gly Ala Ser
65              70              75              80
Phe Lys Cys Thr Val Thr His Pro Glu Ser Gly Thr Leu Thr Gly Thr
                85              90              95
Ile Ala Lys Val Thr
                100
```

```
<210> 145
<211> 107
<212> PRT
<213> Unknown

<220>
<223> Mouse

<400> 145
Val Arg Pro Val Asn Ile Thr Glu Pro Thr Leu Glu Leu Leu His Ser
1               5               10              15
Ser Cys Asp Pro Asn Ala Phe His Ser Thr Ile Gln Leu Tyr Cys Phe
                20              25              30
Ile Tyr Gly His Ile Leu Asn Asp Val Ser Val Ser Trp Leu Met Asp
            35              40              45
Asp Arg Glu Ile Thr Asp Thr Leu Ala Gln Thr Val Leu Ile Lys Glu
            50              55              60
Glu Gly Lys Leu Ala Ser Thr Cys Ser Lys Leu Asn Ile Thr Glu Gln
65              70              75              80
Gln Trp Met Ser Glu Ser Thr Phe Thr Cys Lys Val Thr Ser Gln Gly
                85              90              95
Val Asp Tyr Leu Ala His Thr Arg Arg Cys Pro
            100             105
```

```
<210> 146
<211> 107
<212> PRT
<213> Unknown

<220>
<223> Mouse

<400> 146
Val Pro Glu Val Ser Ser Val Phe Ile Phe Pro Pro Lys Pro Lys Asp
1               5               10              15
Val Leu Thr Ile Thr Leu Thr Pro Lys Val Thr Cys Val Val Val Asp
                20              25              30
Ile Ser Lys Asp Asp Pro Glu Val Gln Phe Ser Trp Phe Val Asp Asp
            35              40              45
Val Glu Val His Thr Ala Gln Thr Gln Pro Arg Glu Glu Gln Phe Asn
            50              55              60
Ser Thr Phe Arg Ser Val Ser Glu Leu Pro Ile Met His Gln Asp Trp
65              70              75              80
Leu Asn Gly Lys Glu Phe Lys Cys Arg Val Asn Ser Ala Ala Phe Pro
                85              90              95
Ala Pro Ile Glu Lys Thr Ile Ser Lys Thr Lys
            100             105
```

```
<210> 147
<211> 110
<212> PRT
<213> Unknown

<220>
<223> Mouse

<400> 147
Ala Pro Asn Leu Leu Gly Gly Pro Ser Val Phe Ile Phe Pro Pro Lys
1               5                   10                  15
Ile Lys Asp Val Leu Met Ile Ser Leu Ser Pro Ile Val Thr Cys Val
            20                  25                  30
Val Val Asp Val Ser Glu Asp Asp Pro Asp Val Gln Ile Ser Trp Phe
        35                  40                  45
Val Asn Asn Val Glu Val His Thr Ala Gln Thr Gln Thr His Arg Glu
        50                  55                  60
Asp Tyr Asn Ser Thr Leu Arg Val Val Ser Ala Leu Pro Ile Gln His
65                  70                  75                  80
Gln Asp Trp Met Ser Gly Lys Glu Phe Lys Cys Lys Val Asn Asn Lys
                85                  90                  95
Asp Leu Pro Ala Pro Ile Glu Arg Thr Ile Ser Lys Pro Lys
            100                 105                 110


<210> 148
<211> 110
<212> PRT
<213> Unknown

<220>
<223> Mouse

<400> 148
Ala Pro Asn Leu Glu Gly Gly Pro Ser Val Phe Ile Phe Pro Pro Asn
1               5                   10                  15
Ile Lys Asp Val Leu Met Ile Ser Leu Thr Pro Lys Val Thr Cys Val
            20                  25                  30
Val Val Asp Val Ser Glu Asp Asp Pro Asp Val Gln Ile Ser Trp Phe
        35                  40                  45
Val Asn Asn Val Glu Val His Thr Ala Gln Thr Gln Thr His Arg Glu
        50                  55                  60
Asp Tyr Asn Ser Thr Ile Arg Val Val Ser Thr Leu Pro Ile Gln His
65                  70                  75                  80
Gln Asp Trp Met Ser Gly Lys Glu Phe Lys Cys Lys Val Asn Asn Lys
                85                  90                  95
Asp Leu Pro Ser Pro Ile Glu Arg Thr Ile Ser Lys Ile Lys
            100                 105                 110


<210> 149
<211> 110
<212> PRT
<213> Unknown

<220>
<223> Mouse
```

```
<400> 149
Ala Pro Asp Leu Leu Gly Gly Pro Ser Val Phe Ile Phe Pro Pro Lys
1               5                   10                  15
Ile Lys Asp Val Leu Met Ile Ser Leu Ser Pro Met Val Thr Cys Val
            20                  25                  30
Val Val Asp Val Ser Glu Asp Asp Pro Asp Val Gln Ile Ser Trp Phe
        35                  40                  45
Val Asn Asn Val Glu Val His Thr Ala Gln Thr Gln Thr His Arg Glu
        50                  55                  60
Asp Tyr Asn Ser Thr Leu Arg Val Val Ser Ala Leu Pro Ile Gln His
65                  70                  75                  80
Gln Asp Trp Met Ser Gly Lys Glu Phe Lys Cys Lys Val Asn Asn Arg
                85                  90                  95
Ala Leu Pro Ser Pro Ile Glu Lys Thr Ile Ser Lys Pro Arg
            100                 105                 110


<210> 150
<211> 110
<212> PRT
<213> Unknown

<220>
<223> Mouse

<400> 150
Pro Gly Asn Ile Leu Gly Gly Pro Ser Val Phe Ile Phe Pro Pro Lys
1               5                   10                  15
Pro Lys Asp Ala Leu Met Ile Ser Leu Thr Pro Lys Val Thr Cys Val
            20                  25                  30
Val Val Asp Val Ser Glu Asp Asp Pro Asp Val His Val Ser Trp Phe
        35                  40                  45
Val Asp Asn Lys Glu Val His Thr Ala Trp Thr Gln Pro Arg Glu Ala
        50                  55                  60
Gln Tyr Asn Ser Thr Phe Arg Val Val Ser Ala Leu Pro Ile Gln His
65                  70                  75                  80
Gln Asp Trp Met Arg Gly Lys Glu Phe Lys Cys Lys Val Asn Asn Lys
                85                  90                  95
Ala Leu Pro Ala Pro Ile Glu Arg Thr Ile Ser Lys Pro Lys
            100                 105                 110


<210> 151
<211> 113
<212> PRT
<213> Unknown

<220>
<223> Mouse

<400> 151
Ala Val Ala Glu Met Asn Pro Asn Val Asn Val Phe Val Pro Pro Arg
1               5                   10                  15
Asp Gly Phe Ser Gly Pro Ala Pro Arg Lys Ser Lys Leu Ile Cys Glu
            20                  25                  30
Ala Thr Asn Phe Thr Pro Lys Pro Ile Thr Val Ser Trp Leu Lys Asp
            35                  40                  45
Gly Lys Leu Val Glu Ser Gly Phe Thr Thr Asp Pro Val Thr Ile Glu
```

```
         50                    55                    60
Asn Lys Gly Ser Thr Pro Gln Thr Tyr Lys Val Ile Ser Thr Leu Thr
65                    70                    75                    80
Ile Ser Glu Ile Asp Trp Leu Asn Leu Asn Val Tyr Thr Cys Arg Val
                 85                    90                    95
Asp His Arg Gly Leu Thr Phe Leu Lys Asn Val Ser Ser Thr Cys Ala
                100                   105                   110
Ala
```

```
<210> 152
<211> 131
<212> PRT
<213> Unknown

<220>
<223> Mouse

<400> 152
Val Asn Thr Phe Pro Pro Gln Val His Leu Leu Pro Pro Pro Ser Glu
 1               5                    10                    15
Glu Leu Ala Leu Asn Glu Leu Leu Ser Leu Thr Cys Leu Val Arg Ala
                20                    25                    30
Phe Asn Pro Lys Glu Val Leu Val Arg Trp Leu His Gly Asn Glu Glu
             35                    40                    45
Leu Ser Pro Glu Ser Tyr Leu Val Phe Glu Pro Leu Lys Glu Pro Gly
         50                    55                    60
Glu Gly Ala Thr Thr Tyr Leu Val Thr Ser Val Leu Arg Val Ser Ala
65                    70                    75                    80
Glu Thr Trp Lys Gln Gly Asp Gln Tyr Ser Cys Met Val Gly His Glu
                 85                    90                    95
Ala Leu Pro Met Asn Phe Thr Gln Lys Thr Ile Asp Arg Leu Ser Gly
                100                   105                   110
Lys Pro Thr Asn Val Ser Val Ser Val Ile Met Ser Glu Gly Asp Gly
             115                   120                   125
Ile Cys Tyr
         130
```

```
<210> 153
<211> 129
<212> PRT
<213> Unknown

<220>
<223> Mouse

<400> 153
Gly Ala Met Ala Pro Ser Asn Leu Thr Val Asn Ile Leu Thr Thr Ser
 1               5                    10                    15
Thr His Pro Glu Met Ser Ser Trp Leu Leu Cys Glu Val Ser Gly Phe
                20                    25                    30
Phe Pro Glu Asn Ile His Leu Met Trp Leu Gly Val His Ser Lys Met
             35                    40                    45
Lys Ser Thr Asn Phe Val Thr Ala Asn Pro Thr Ala Gln Pro Gly Gly
         50                    55                    60
Thr Phe Gln Thr Trp Ser Val Leu Arg Leu Pro Val Ala Leu Ser Ser
```

```
                65                      70                      75                      80
                Ser Leu Asp Thr Tyr Thr Cys Val Val Glu His Glu Ala Ser Lys Thr
                            85                      90                      95
                Lys Leu Asn Ala Ser Lys Ser Leu Ala Ile Ser Gly Cys Tyr His Leu
                        100                     105                     110
                Leu Pro Glu Ser Asp Gly Pro Ser Arg Arg Pro Asp Gly Pro Ala Leu
                        115                     120                     125
                Ala


                <210> 154
                <211> 107
                <212> PRT
                <213> Unknown

                <220>
                <223> Mouse

                <400> 154
                Asp His Glu Pro Arg Gly Val Ile Thr Tyr Leu Ile Pro Pro Ser Pro
                 1               5                      10                      15
                Leu Asp Leu Tyr Gln Asn Gly Ala Pro Lys Leu Thr Cys Leu Val Val
                            20                      25                      30
                Asp Leu Glu Ser Glu Lys Asn Val Asn Val Thr Trp Asn Gln Glu Lys
                        35                      40                      45
                Lys Thr Ser Val Ser Ala Ser Gln Trp Tyr Thr Lys His His Asn Asn
                    50                      55                      60
                Ala Thr Thr Ser Ile Thr Ser Ile Leu Pro Val Val Ala Lys Asp Trp
                65                      70                      75                      80
                Ile Glu Gly Tyr Gly Tyr Gln Cys Ile Val Asp His Pro Asp Phe Pro
                            85                      90                      95
                Lys Pro Ile Val Arg Ser Ile Thr Lys Thr Pro
                        100                     105


                <210> 155
                <211> 107
                <212> PRT
                <213> Unknown

                <220>
                <223> Mouse

                <400> 155
                Gly Arg Pro Lys Ala Pro Gln Val Tyr Thr Ile Pro Pro Pro Lys Glu
                 1               5                      10                      15
                Gln Met Ala Lys Asp Lys Val Ser Leu Thr Cys Met Ile Thr Asp Phe
                            20                      25                      30
                Phe Pro Glu Asp Ile Thr Val Glu Trp Gln Trp Asn Gly Gln Pro Ala
                        35                      40                      45
                Glu Asn Tyr Lys Asn Thr Gln Pro Ile Met Asn Thr Asn Gly Ser Tyr
                    50                      55                      60
                Phe Val Tyr Ser Lys Leu Asn Val Gln Lys Ser Asn Trp Glu Ala Gly
                65                      70                      75                      80
                Asn Thr Phe Thr Cys Ser Val Leu His Glu Gly Leu His Asn His His
                            85                      90                      95
                Thr Glu Lys Ser Leu Ser His Ser Pro Gly Lys
```

100      105

```
<210> 156
<211> 107
<212> PRT
<213> Unknown

<220>
<223> Mouse

<400> 156
Gly Ser Val Arg Ala Pro Gln Val Tyr Val Leu Pro Pro Pro Glu Glu
1               5                   10                  15
Glu Met Thr Lys Lys Gln Val Thr Leu Thr Cys Met Val Thr Asp Phe
            20                  25                  30
Met Pro Glu Asp Ile Tyr Val Glu Trp Thr Asn Asn Gly Lys Thr Glu
        35                  40                  45
Leu Asn Tyr Lys Asn Thr Glu Pro Val Leu Asp Ser Asp Gly Ser Tyr
        50                  55                  60
Phe Met Tyr Ser Lys Leu Arg Val Glu Lys Lys Asn Trp Val Glu Arg
65                  70                  75                  80
Asn Ser Tyr Ser Cys Ser Val Val His Glu Gly Leu His Asn His His
                85                  90                  95
Thr Thr Lys Ser Phe Ser Arg Thr Pro Gly Lys
            100                 105


<210> 157
<211> 107
<212> PRT
<213> Unknown

<220>
<223> Mouse

<400> 157
Gly Leu Val Arg Ala Pro Gln Val Tyr Thr Leu Pro Pro Pro Ala Glu
1               5                   10                  15
Gln Leu Ser Arg Lys Asp Val Ser Leu Thr Cys Leu Val Val Gly Phe
            20                  25                  30
Asn Pro Gly Asp Ile Ser Val Glu Trp Thr Ser Asn Gly His Thr Glu
        35                  40                  45
Glu Asn Tyr Lys Asp Thr Ala Pro Val Leu Asp Ser Asp Gly Ser Tyr
        50                  55                  60
Phe Ile Tyr Ser Lys Leu Asn Met Lys Thr Ser Lys Trp Glu Lys Thr
65                  70                  75                  80
Asp Ser Phe Ser Cys Asn Val Arg His Glu Gly Leu Lys Asn Tyr Tyr
                85                  90                  95
Leu Lys Lys Thr Ile Ser Arg Ser Pro Gly Lys
            100                 105


<210> 158
<211> 107
<212> PRT
<213> Unknown
```

```
<220>
<223> Mouse

<400> 158
Gly Pro Val Arg Ala Pro Gln Val Tyr Val Leu Pro Pro Pro Ala Glu
1               5                   10                  15
Glu Met Thr Lys Lys Glu Phe Ser Leu Thr Cys Met Ile Thr Gly Phe
            20                  25                  30
Leu Pro Ala Glu Ile Ala Val Asp Trp Thr Ser Asn Gly Arg Thr Glu
        35                  40                  45
Gln Asn Tyr Lys Asn Thr Ala Thr Val Leu Asp Ser Asp Gly Ser Tyr
    50                  55                  60
Phe Met Tyr Ser Lys Leu Arg Val Gln Lys Ser Thr Trp Glu Arg Gly
65                  70                  75                  80
Ser Leu Phe Ala Cys Ser Val Val His Glu Val Leu His Asn His Leu
                85                  90                  95
Thr Thr Lys Thr Ile Ser Arg Ser Leu Gly Lys
            100                 105


<210> 159
<211> 107
<212> PRT
<213> Unknown

<220>
<223> Mouse

<400> 159
Gly Arg Ala Gln Thr Pro Gln Val Tyr Thr Ile Pro Pro Pro Arg Glu
1               5                   10                  15
Gln Met Ser Lys Lys Lys Val Ser Leu Thr Cys Leu Val Thr Asn Phe
            20                  25                  30
Phe Ser Glu Ala Ile Ser Val Glu Trp Glu Arg Asn Gly Glu Leu Glu
        35                  40                  45
Gln Asp Tyr Lys Asn Thr Pro Pro Ile Leu Asp Ser Asp Gly Thr Tyr
    50                  55                  60
Phe Leu Tyr Ser Lys Leu Thr Val Asp Thr Asp Ser Trp Leu Gln Gly
65                  70                  75                  80
Glu Ile Phe Thr Cys Ser Val Val His Glu Ala Leu His Asn His His
                85                  90                  95
Thr Gln Lys Asn Leu Ser Arg Ser Pro Gly Lys
            100                 105


<210> 160
<211> 106
<212> PRT
<213> Unknown

<220>
<223> Mouse

<400> 160
Ser Pro Ser Thr Asp Ile Leu Thr Phe Thr Ile Pro Pro Ser Phe Ala
1               5                   10                  15
Asp Ile Phe Leu Ser Lys Ser Ala Asn Leu Thr Cys Leu Val Ser Asn
            20                  25                  30
```

Leu Ala Thr Tyr Glu Thr Leu Asn Ile Ser Trp Ala Ser Gln Ser Gly
            35                  40                  45
Glu Pro Leu Glu Thr Lys Ile Lys Ile Met Glu Ser His Pro Asn Gly
    50                  55                  60
Thr Phe Ser Ala Lys Gly Val Ala Ser Val Cys Val Glu Asp Trp Asn
65                  70                  75                  80
Asn Arg Lys Glu Phe Val Cys Thr Val Thr His Arg Asp Leu Pro Ser
                85                  90                  95
Pro Gln Lys Lys Phe Ile Ser Lys Pro Asn
                100                 105


<210> 161
<211> 11
<212> PRT
<213> Unknown

<220>
<223> Mouse

<400> 161
Gly Pro Thr Pro Pro Pro Pro Ile Thr Ile Pro
 1               5                   10


<210> 162
<211> 12
<212> PRT
<213> Unknown

<220>
<223> Mouse

<400> 162
Gly Ile Cys Ser Pro Pro Thr Thr Pro Pro Pro Pro
 1               5                   10


<210> 163
<211> 11
<212> PRT
<213> Unknown

<220>
<223> Mouse

<400> 163
Gly Pro Pro Pro Pro Cys Pro Pro Cys Pro Pro
 1               5                   10


<210> 164
<211> 8
<212> PRT
<213> Unknown

<220>
<223> Mouse

<400> 164
Gly Pro Pro Pro Pro Cys Pro Pro
1               5


<210> 165
<211> 10
<212> PRT
<213> Unknown

<220>
<223> Mouse

<400> 165
Gly Pro Pro Pro Pro Cys Pro Pro Leu Pro
1               5               10


<210> 166
<211> 35
<212> PRT
<213> Unknown

<220>
<223> Mouse

<400> 166
Glu Ser Trp Asp Ser Gln Ser Ser Lys Arg Val Thr Pro Thr Leu Gln
1               5               10                  15
Ala Lys Asn His Ser Thr Glu Ala Thr Lys Ala Ile Thr Thr Lys Lys
            20                  25                  30
Asp Ile Glu
        35


<210> 167
<211> 13
<212> PRT
<213> Unknown

<220>
<223> Mouse

<400> 167
Val Pro Arg Asp Cys Gly Cys Lys Pro Cys Ile Cys Thr
1               5               10


<210> 168
<211> 16
<212> PRT
<213> Unknown

<220>
<223> Mouse

<400> 168

Glu Pro Arg Gly Pro Thr Ile Lys Pro Cys Pro Pro Cys Lys Cys Pro
1               5                   10                  15

<210> 169
<211> 22
<212> PRT
<213> Unknown

<220>
<223> Mouse

<400> 169
Glu Pro Ser Gly Pro Ile Ser Thr Ile Asn Pro Cys Pro Pro Cys Lys
1               5                   10                  15
Glu Cys His Lys Cys Pro
            20

<210> 170
<211> 21
<212> PRT
<213> Unknown

<220>
<223> Mouse

<400> 170
Glu Pro Arg Val Pro Ile Thr Gln Asn Pro Cys Pro Pro Leu Lys Glu
1               5                   10                  15
Cys Pro Pro Cys Ala
            20

<210> 171
<211> 16
<212> PRT
<213> Unknown

<220>
<223> Mouse

<400> 171
Glu Pro Arg Ile Pro Lys Pro Ser Thr Pro Pro Gly Ser Ser Cys Pro
1               5                   10                  15

<210> 172
<211> 107
<212> PRT
<213> Unknown

<220>
<223> Mouse

<400> 172
Arg Ala Asp Ala Ala Pro Thr Val Ser Ile Phe Pro Pro Ser Ser Glu
1               5                   10                  15

```
Gln Leu Thr Ser Gly Gly Ala Ser Val Val Cys Phe Leu Asn Asn Phe
        20                  25                  30
Tyr Pro Lys Asp Ile Asn Val Lys Trp Lys Ile Asp Gly Ser Glu Arg
        35                  40                  45
Gln Asn Gly Val Leu Asn Ser Trp Thr Asp Gln Asp Ser Lys Asp Ser
        50                  55                  60
Thr Tyr Ser Met Ser Ser Thr Leu Thr Leu Thr Lys Asp Glu Tyr Glu
65                  70                  75                  80
Arg His Asn Ser Tyr Thr Cys Glu Ala Thr His Lys Thr Ser Thr Ser
                85                  90                  95
Pro Ile Val Lys Ser Phe Asn Arg Asn Glu Cys
                100                 105
```

<210> 173
<211> 107
<212> PRT
<213> Unknown

<220>
<223> Mouse

<400> 173
```
Arg Ala Asp Ala Ala Pro Thr Val Ser Ile Phe Pro Pro Ser Ser Glu
1               5                   10                  15
Gln Leu Thr Ser Gly Gly Ala Ser Val Val Cys Phe Leu Asn Asn Phe
        20                  25                  30
Tyr Pro Arg Asp Ile Asn Val Lys Trp Lys Ile Asp Gly Ser Glu Arg
        35                  40                  45
Gln Asn Gly Val Leu Asn Ser Trp Thr Asp Gln Asp Ser Lys Asp Ser
        50                  55                  60
Thr Tyr Ser Met Ser Ser Thr Leu Thr Leu Thr Lys Asp Glu Tyr Glu
65                  70                  75                  80
Arg His Asn Ser Tyr Thr Cys Glu Ala Thr His Lys Thr Ser Thr Ser
                85                  90                  95
Pro Ile Val Lys Ser Phe Asn Arg Asn Glu Cys
                100                 105
```

<210> 174
<211> 106
<212> PRT
<213> Unknown

<220>
<223> Mouse

<400> 174
```
Gly Gln Pro Lys Ser Ser Pro Ser Val Thr Leu Phe Pro Pro Ser Ser
1               5                   10                  15
Glu Glu Leu Glu Thr Asn Lys Ala Thr Leu Val Cys Thr Ile Thr Asp
        20                  25                  30
Phe Tyr Pro Gly Val Val Thr Val Asp Trp Lys Val Asp Gly Thr Pro
        35                  40                  45
Val Thr Gln Gly Met Glu Thr Thr Gln Pro Ser Lys Gln Ser Asn Asn
        50                  55                  60
Lys Tyr Met Ala Ser Ser Tyr Leu Thr Leu Thr Ala Arg Ala Trp Glu
65                  70                  75                  80
```

```
Arg His Ser Ser Tyr Ser Cys Gln Val Thr His Glu Gly His Thr Val
            85                  90                  95
Glu Lys Ser Leu Ser Arg Ala Asp Cys Ser
            100                 105
```

```
<210> 175
<211> 105
<212> PRT
<213> Unknown

<220>
<223> Mouse

<400> 175
Gly Gln Pro Lys Ser Thr Pro Thr Leu Thr Val Phe Pro Pro Ser Ser
 1           5                   10                  15
Glu Glu Leu Lys Glu Asn Lys Ala Thr Leu Val Cys Leu Ile Ser Asn
            20                  25                  30
Phe Ser Pro Ser Gly Val Thr Val Ala Trp Lys Ala Asn Gly Thr Pro
            35                  40                  45
Ile Thr Gln Gly Val Asp Thr Ser Asn Pro Thr Lys Glu Gly Asn Lys
        50                  55                  60
Phe Met Ala Ser Ser Phe Leu His Leu Thr Ser Asp Gln Trp Arg Ser
65                  70                  75                  80
His Asn Ser Phe Thr Cys Gln Val Thr His Glu Gly Asp Thr Val Glu
            85                  90                  95
Lys Ser Leu Ser Pro Ala Glu Cys Leu
            100                 105
```

```
<210> 176
<211> 105
<212> PRT
<213> Unknown

<220>
<223> Mouse

<400> 176
Gly Gln Pro Lys Ser Thr Pro Thr Leu Thr Met Phe Pro Pro Ser Pro
 1           5                   10                  15
Glu Glu Leu Gln Glu Asn Lys Ala Thr Leu Val Cys Leu Ile Ser Asn
            20                  25                  30
Phe Ser Pro Ser Gly Val Thr Val Ala Trp Lys Ala Asn Gly Thr Pro
            35                  40                  45
Ile Thr Gln Gly Val Asp Thr Ser Asn Pro Thr Lys Glu Asp Asn Lys
        50                  55                  60
Tyr Met Ala Ser Ser Phe Leu His Leu Thr Ser Asp Gln Trp Arg Ser
65                  70                  75                  80
His Asn Ser Phe Thr Cys Gln Val Thr His Glu Gly Asp Thr Val Glu
            85                  90                  95
Lys Ser Leu Ser Pro Ala Glu Cys Leu
            100                 105
```

```
<210> 177
<211> 102
```

```
<212> PRT
<213> Homo sapiens

<400> 177
Ala Ser Pro Thr Ser Pro Lys Val Phe Pro Leu Ser Leu Cys Ser Thr
1               5                   10                  15
Gln Pro Asp Gly Asn Val Val Ile Ala Cys Leu Val Gln Gly Phe Phe
            20                  25                  30
Pro Gln Glu Pro Leu Ser Val Thr Trp Ser Glu Ser Gly Gln Gly Val
        35                  40                  45
Thr Ala Arg Asn Phe Pro Pro Ser Gln Asp Ala Ser Gly Asp Leu Tyr
    50                  55                  60
Thr Thr Ser Ser Gln Leu Thr Leu Pro Ala Thr Gln Cys Leu Ala Gly
65                  70                  75                  80
Lys Ser Val Thr Cys His Val Lys His Tyr Thr Asn Pro Ser Gln Asp
                85                  90                  95
Val Thr Val Pro Cys Pro
            100
```

```
<210> 178
<211> 102
<212> PRT
<213> Homo sapiens

<400> 178
Ala Ser Pro Thr Ser Pro Lys Val Phe Pro Leu Ser Leu Asp Ser Thr
1               5                   10                  15
Pro Gln Asp Gly Asn Val Val Val Ala Cys Leu Val Gln Gly Phe Phe
            20                  25                  30
Pro Gln Glu Pro Leu Ser Val Thr Trp Ser Glu Ser Gly Gln Asn Val
        35                  40                  45
Thr Ala Arg Asn Phe Pro Pro Ser Gln Asp Ala Ser Gly Asp Leu Tyr
    50                  55                  60
Thr Thr Ser Ser Gln Leu Thr Leu Pro Ala Thr Gln Cys Pro Asp Gly
65                  70                  75                  80
Lys Ser Val Thr Cys His Val Lys His Tyr Thr Asn Pro Ser Gln Asp
                85                  90                  95
Val Thr Val Pro Cys Pro
            100
```

```
<210> 179
<211> 101
<212> PRT
<213> Homo sapiens

<400> 179
Ala Pro Thr Lys Ala Pro Asp Val Phe Pro Ile Ile Ser Gly Cys Arg
1               5                   10                  15
His Pro Lys Asp Asn Ser Pro Val Val Leu Ala Cys Leu Ile Thr Gly
            20                  25                  30
Tyr His Pro Thr Ser Val Thr Val Thr Trp Tyr Met Gly Thr Gln Ser
        35                  40                  45
Gln Pro Gln Arg Thr Phe Pro Glu Ile Gln Arg Arg Asp Ser Tyr Tyr
    50                  55                  60
Met Thr Ser Ser Gln Leu Ser Thr Pro Leu Gln Gln Trp Arg Gln Gly
65                  70                  75                  80
```

```
Glu Tyr Lys Cys Val Val Gln His Thr Ala Ser Lys Ser Lys Lys Glu
                85                  90              95
Ile Phe Arg Trp Pro
                100


<210> 180
<211> 103
<212> PRT
<213> Homo sapiens

<400> 180
Ala Ser Thr Gln Ser Pro Ser Val Phe Pro Leu Thr Arg Cys Cys Lys
1               5                   10              15
Asn Ile Pro Ser Asn Ala Thr Ser Val Thr Leu Gly Cys Leu Ala Thr
                20                  25              30
Gly Tyr Phe Pro Glu Pro Val Met Val Thr Cys Asp Thr Gly Ser Leu
        35                  40                  45
Asn Gly Thr Thr Met Thr Leu Pro Ala Thr Thr Leu Thr Leu Ser Gly
        50                  55                  60
His Tyr Ala Thr Ile Ser Leu Leu Thr Val Ser Gly Ala Trp Ala Lys
65                  70                  75                  80
Gln Met Phe Thr Cys Arg Val Ala His Thr Pro Ser Ser Thr Asp Trp
                85                  90                  95
Val Asp Asn Lys Thr Phe Ser
                100


<210> 181
<211> 98
<212> PRT
<213> Homo sapiens

<400> 181
Ala Ser Thr Lys Gly Pro Ser Val Phe Pro Leu Ala Pro Ser Ser Lys
1               5                   10              15
Ser Thr Ser Gly Gly Thr Ala Ala Leu Gly Cys Leu Val Lys Asp Tyr
                20                  25              30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
        50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Arg Val


<210> 182
<211> 98
<212> PRT
<213> Homo sapiens

<400> 182
Ala Ser Thr Lys Gly Pro Ser Val Phe Pro Leu Ala Pro Cys Ser Arg
1               5                   10              15
```

Ser Thr Ser Glu Ser Thr Ala Ala Leu Gly Cys Leu Val Lys Asp Tyr
          20                      25                      30
Phe Pro Glu Pro Val Thr Val Ser Trp Asn Ser Gly Ala Leu Thr Ser
          35                      40                      45
Gly Val His Thr Phe Pro Ala Val Leu Gln Ser Ser Gly Leu Tyr Ser
          50                      55                      60
Leu Ser Ser Val Val Thr Val Pro Ser Ser Asn Phe Gly Thr Gln Thr
65                      70                      75                      80
Tyr Thr Cys Asn Val Asp His Lys Pro Ser Asn Thr Lys Val Asp Lys
                    85                      90                      95
Thr Val


<210> 183
<211> 98
<212> PRT
<213> Homo sapiens


<400> 183
Ala Ser Thr Lys Gly Pro Ser Val Phe Pro Leu Ala Pro Cys Ser Arg
  1                   5                      10                      15
Ser Thr Ser Gly Gly Thr Ala Ala Leu Gly Cys Leu Val Lys Asp Tyr
          20                      25                      30
Phe Pro Glu Pro Val Thr Val Ser Trp Asn Ser Gly Ala Leu Thr Ser
          35                      40                      45
Gly Val His Thr Phe Pro Ala Val Leu Gln Ser Ser Gly Leu Tyr Ser
          50                      55                      60
Leu Ser Ser Val Val Thr Val Pro Ser Ser Ser Leu Gly Thr Gln Thr
65                      70                      75                      80
Tyr Thr Cys Asn Val Asn His Lys Pro Ser Asn Thr Lys Val Asp Lys
                    85                      90                      95
Arg Val


<210> 184
<211> 98
<212> PRT
<213> Homo sapiens


<400> 184
Ala Ser Thr Lys Gly Pro Ser Val Phe Pro Leu Ala Pro Cys Ser Arg
  1                   5                      10                      15
Ser Thr Ser Glu Ser Thr Ala Ala Leu Gly Cys Leu Val Lys Asp Tyr
          20                      25                      30
Phe Pro Glu Pro Val Thr Val Ser Trp Asn Ser Gly Ala Leu Thr Ser
          35                      40                      45
Gly Val His Thr Phe Pro Ala Val Leu Gln Ser Ser Gly Leu Tyr Ser
          50                      55                      60
Leu Ser Ser Val Val Thr Val Pro Ser Ser Ser Leu Gly Thr Lys Thr
65                      70                      75                      80
Tyr Thr Cys Asn Val Asp His Lys Pro Ser Asn Thr Lys Val Asp Lys
                    85                      90                      95
Arg Val

```
<210> 185
<211> 104
<212> PRT
<213> Homo sapiens

<400> 185
Gly Ser Ala Ser Ala Pro Thr Leu Phe Pro Leu Val Ser Cys Glu Asn
1               5                   10                  15
Ser Pro Ser Asp Thr Ser Ser Val Ala Val Gly Cys Leu Ala Gln Asp
            20                  25                  30
Phe Leu Pro Asp Ser Ile Thr Leu Ser Trp Lys Tyr Lys Asn Asn Ser
        35                  40                  45
Asp Ile Ser Ser Thr Arg Gly Phe Pro Ser Val Leu Arg Gly Gly Lys
    50                  55                  60
Tyr Ala Ala Thr Ser Gln Val Leu Leu Pro Ser Lys Asp Val Met Gln
65                  70                  75                  80
Gly Thr Asp Glu His Val Val Cys Lys Val Gln His Pro Asn Gly Asn
                85                  90                  95
Lys Glu Lys Asn Val Pro Leu Pro
                100
```

```
<210> 186
<211> 101
<212> PRT
<213> Homo sapiens

<400> 186
Cys Cys His Pro Arg Leu Ser Leu His Arg Pro Ala Leu Glu Asp Leu
1               5                   10                  15
Leu Leu Gly Ser Glu Ala Asn Leu Thr Cys Thr Leu Thr Gly Leu Arg
            20                  25                  30
Asp Ala Ser Gly Val Thr Phe Thr Trp Thr Pro Ser Ser Gly Lys Ser
        35                  40                  45
Ala Val Gln Gly Pro Pro Glu Arg Asp Leu Cys Gly Cys Tyr Ser Val
        50                  55                  60
Ser Ser Val Leu Pro Gly Cys Ala Glu Pro Trp Asn His Gly Lys Thr
65                  70                  75                  80
Phe Thr Cys Thr Ala Ala Tyr Pro Glu Ser Lys Thr Pro Leu Thr Ala
                85                  90                  95
Thr Leu Ser Lys Ser
                100
```

```
<210> 187
<211> 101
<212> PRT
<213> Homo sapiens

<400> 187
Cys Cys His Pro Arg Leu Ser Leu His Arg Pro Ala Leu Glu Asp Leu
1               5                   10                  15
Leu Leu Gly Ser Glu Ala Asn Leu Thr Cys Thr Leu Thr Gly Leu Arg
            20                  25                  30
Asp Ala Ser Gly Ala Thr Phe Thr Trp Thr Pro Ser Ser Gly Lys Ser
        35                  40                  45
Ala Val Gln Gly Pro Pro Glu Arg Asp Leu Cys Gly Cys Tyr Ser Val
        50                  55                  60
```

Ser Ser Val Leu Pro Gly Cys Ala Gln Pro Trp Asn His Gly Glu Thr
65                70                75                80
Phe Thr Cys Thr Ala Ala His Pro Glu Leu Lys Thr Pro Leu Thr Ala
                85                90                95
Asn Ile Thr Lys Ser
                100


<210> 188
<211> 108
<212> PRT
<213> Homo sapiens


<400> 188
Glu Cys Pro Ser His Thr Gln Pro Leu Gly Val Tyr Leu Leu Thr Pro
  1                5                10                15
Ala Val Gln Asp Leu Trp Leu Arg Asp Lys Ala Thr Phe Thr Cys Phe
                20                25                30
Val Val Gly Ser Asp Leu Lys Asp Ala His Leu Thr Trp Glu Val Ala
                35                40                45
Gly Lys Val Pro Thr Gly Gly Val Glu Glu Gly Leu Leu Glu Arg His
                50                55                60
Ser Asn Gly Ser Gln Ser Gln His Ser Arg Leu Thr Leu Pro Arg Ser
65                70                75                80
Leu Trp Asn Ala Gly Thr Ser Val Thr Cys Thr Leu Asn His Pro Ser
                85                90                95
Leu Pro Pro Gln Arg Leu Met Ala Leu Arg Glu Pro
                100                105


<210> 189
<211> 107
<212> PRT
<213> Homo sapiens


<400> 189
Val Cys Ser Arg Asp Phe Thr Pro Pro Thr Val Lys Ile Leu Gln Ser
  1                5                10                15
Ser Cys Asp Gly Gly Gly His Phe Pro Pro Thr Ile Gln Leu Leu Cys
                20                25                30
Leu Val Ser Gly Tyr Thr Pro Gly Thr Ile Asn Ile Thr Trp Leu Glu
                35                40                45
Asp Gly Gln Val Met Asp Val Asp Leu Ser Thr Ala Ser Thr Thr Gln
                50                55                60
Glu Gly Glu Leu Ala Ser Thr Gln Ser Glu Leu Thr Leu Ser Gln Lys
65                70                75                80
His Trp Leu Ser Asp Arg Thr Tyr Thr Cys Gln Val Thr Tyr Gln Gly
                85                90                95
His Thr Phe Glu Asp Ser Thr Lys Lys Cys Ala
                100                105


<210> 190
<211> 110
<212> PRT
<213> Homo sapiens


<400> 190

```
Ala Pro Glu Leu Leu Gly Gly Pro Ser Val Phe Leu Phe Pro Pro Lys
1               5                   10                  15
Pro Lys Asp Thr Leu Met Ile Ser Arg Thr Pro Glu Val Thr Cys Val
            20                  25                  30
Val Val Asp Val Ser His Glu Asp Pro Glu Val Lys Phe Asn Trp Tyr
        35                  40                  45
Val Asp Gly Val Glu Val His Asn Ala Lys Thr Lys Pro Arg Glu Glu
    50                  55                  60
Gln Tyr Asn Ser Thr Tyr Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Ala Leu Pro Ala Pro Ile Glu Lys Thr Ile Ser Lys Ala Lys
            100                 105                 110
```

<210> 191
<211> 109
<212> PRT
<213> Homo sapiens

<400> 191
```
Ala Pro Pro Val Ala Gly Pro Ser Val Phe Leu Phe Pro Pro Lys Pro
1               5                   10                  15
Lys Asp Thr Leu Met Ile Ser Arg Thr Pro Glu Val Thr Cys Val Val
        20                  25                  30
Val Asp Val Ser His Glu Asp Pro Glu Val Gln Phe Asn Trp Tyr Val
    35                  40                  45
Asp Gly Val Glu Val His Asn Ala Lys Thr Lys Pro Arg Glu Glu Gln
    50                  55                  60
Phe Asn Ser Thr Phe Arg Val Val Ser Val Leu Thr Val Val His Gln
65                  70                  75                  80
Asp Trp Leu Asn Gly Lys Glu Tyr Lys Cys Lys Val Ser Asn Lys Gly
                85                  90                  95
Leu Pro Ala Pro Ile Glu Lys Thr Ile Ser Lys Thr Lys
            100                 105
```

<210> 192
<211> 110
<212> PRT
<213> Homo sapiens

<400> 192
```
Ala Pro Glu Leu Leu Gly Gly Pro Ser Val Phe Leu Phe Pro Pro Lys
1               5                   10                  15
Pro Lys Asp Thr Leu Met Ile Ser Arg Thr Pro Glu Val Thr Cys Val
            20                  25                  30
Val Val Asp Val Ser His Glu Asp Pro Glu Val Gln Phe Lys Trp Tyr
        35                  40                  45
Val Asp Gly Val Glu Val His Asn Ala Lys Thr Lys Pro Arg Glu Glu
    50                  55                  60
Gln Tyr Asn Ser Thr Phe Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Ala Leu Pro Ala Pro Ile Glu Lys Thr Ile Ser Lys Thr Lys
            100                 105                 110
```

```
<210> 193
<211> 110
<212> PRT
<213> Homo sapiens

<400> 193
Ala Pro Glu Phe Leu Gly Gly Pro Ser Val Phe Leu Phe Pro Pro Lys
1               5                   10                  15
Pro Lys Asp Thr Leu Met Ile Ser Arg Thr Pro Glu Val Thr Cys Val
            20                  25                  30
Val Val Asp Val Ser Gln Glu Asp Pro Glu Val Gln Phe Asn Trp Tyr
            35                  40                  45
Val Asp Gly Val Glu Val His Asn Ala Lys Thr Lys Pro Arg Glu Glu
        50                  55                  60
Gln Phe Asn Ser Thr Tyr Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Gly Leu Pro Ser Ser Ile Glu Lys Thr Ile Ser Lys Ala Lys
                100                 105                 110


<210> 194
<211> 112
<212> PRT
<213> Homo sapiens

<400> 194
Val Ile Ala Glu Leu Pro Pro Lys Val Ser Val Phe Val Pro Pro Arg
1               5                   10                  15
Asp Gly Phe Phe Gly Asn Pro Arg Lys Ser Lys Leu Ile Cys Gln Ala
            20                  25                  30
Thr Gly Phe Ser Pro Arg Gln Ile Gln Val Ser Trp Leu Arg Glu Gly
            35                  40                  45
Lys Gln Val Gly Ser Gly Val Thr Thr Asp Gln Val Gln Ala Glu Ala
        50                  55                  60
Lys Glu Ser Gly Pro Thr Thr Tyr Lys Val Thr Ser Thr Leu Thr Ile
65                  70                  75                  80
Lys Glu Ser Asp Trp Leu Gly Gln Ser Met Phe Thr Cys Arg Val Asp
                85                  90                  95
His Arg Gly Leu Thr Phe Gln Gln Asn Ala Ser Ser Met Cys Val Pro
                100                 105                 110


<210> 195
<211> 131
<212> PRT
<213> Homo sapiens

<400> 195
Gly Asn Thr Phe Arg Pro Glu Val His Leu Leu Pro Pro Pro Ser Glu
1               5                   10                  15
Glu Leu Ala Leu Asn Glu Leu Val Thr Leu Thr Cys Leu Ala Arg Gly
            20                  25                  30
Phe Ser Pro Lys Asp Val Leu Val Arg Trp Leu Gln Gly Ser Gln Glu
            35                  40                  45
```

```
Leu Pro Arg Glu Lys Tyr Leu Thr Trp Ala Ser Arg Gln Glu Pro Ser
    50                      55                  60
Gln Gly Thr Thr Thr Phe Ala Val Thr Ser Ile Leu Arg Val Ala Ala
65                      70                  75                  80
Glu Asp Trp Lys Lys Gly Asp Thr Phe Ser Cys Met Val Gly His Glu
                85                  90                  95
Ala Leu Pro Leu Ala Phe Thr Gln Lys Thr Ile Asp Arg Leu Ala Gly
                100                 105                 110
Lys Pro Thr His Val Asn Val Ser Val Val Met Ala Glu Val Asp Gly
            115                 120                 125
Thr Cys Tyr
    130


<210> 196
<211> 131
<212> PRT
<213> Homo sapiens


<400> 196
Gly Asn Thr Phe Arg Pro Glu Val His Leu Leu Pro Pro Pro Ser Glu
    1           5                   10                  15
Glu Leu Ala Leu Asn Glu Leu Val Thr Leu Thr Cys Leu Ala Arg Gly
                20                  25                  30
Phe Ser Pro Lys Asp Val Leu Val Arg Trp Leu Gln Gly Ser Gln Glu
            35                  40                  45
Leu Pro Arg Glu Lys Tyr Leu Thr Trp Ala Ser Arg Gln Glu Pro Ser
    50                      55                  60
Gln Gly Thr Thr Thr Phe Ala Val Thr Ser Ile Leu Arg Val Ala Ala
65                      70                  75                  80
Glu Asp Trp Lys Lys Gly Asp Thr Phe Ser Cys Met Val Gly His Glu
                85                  90                  95
Ala Leu Pro Leu Ala Phe Thr Gln Lys Thr Ile Asp Arg Leu Ala Gly
                100                 105                 110
Lys Pro Thr His Val Asn Val Ser Val Val Met Ala Glu Val Asp Gly
            115                 120                 125
Thr Cys Tyr
    130


<210> 197
<211> 117
<212> PRT
<213> Homo sapiens


<400> 197
Ala Ala Gln Ala Pro Val Lys Leu Ser Leu Asn Leu Leu Ala Ser Ser
    1           5                   10                  15
Asp Pro Pro Glu Ala Ala Ser Trp Leu Leu Cys Glu Val Ser Gly Phe
            20                  25                  30
Ser Pro Pro Asn Ile Leu Leu Met Trp Leu Glu Asp Gln Arg Glu Val
            35                  40                  45
Asn Thr Ser Gly Phe Ala Pro Ala Arg Pro Pro Pro Gln Pro Arg Ser
        50                  55                  60
Thr Thr Phe Trp Ala Trp Ser Val Leu Arg Val Pro Ala Pro Pro Ser
65                      70                  75                  80
Pro Gln Pro Ala Thr Tyr Thr Cys Val Val Ser His Glu Asp Ser Arg
                85                  90                  95
```

```
Thr Leu Leu Asn Ala Ser Arg Ser Leu Glu Val Ser Tyr Val Thr Asp
            100                 105                 110
His Gly Pro Met Lys
            115


<210> 198
<211> 108
<212> PRT
<213> Homo sapiens

<400> 198
Asp Ser Asn Pro Arg Gly Val Ser Ala Tyr Leu Ser Arg Pro Ser Pro
1               5                   10                  15
Phe Asp Leu Phe Ile Arg Lys Ser Pro Thr Ile Thr Cys Leu Val Val
            20                  25                  30
Asp Leu Ala Pro Ser Lys Gly Thr Val Asn Leu Thr Trp Ser Arg Ala
        35                  40                  45
Ser Gly Lys Pro Val Asn His Ser Thr Arg Lys Glu Glu Lys Gln Arg
    50                  55                  60
Asn Gly Thr Leu Thr Val Thr Ser Thr Leu Pro Val Gly Thr Arg Asp
65                  70                  75                  80
Trp Ile Glu Gly Glu Thr Tyr Gln Cys Arg Val Thr His Pro His Leu
            85                  90                  95
Pro Arg Ala Leu Met Arg Ser Thr Thr Lys Thr Ser
            100                 105


<210> 199
<211> 107
<212> PRT
<213> Homo sapiens

<400> 199
Gly Gln Pro Arg Glu Pro Gln Val Tyr Thr Leu Pro Pro Ser Arg Asp
1               5                   10                  15
Glu Leu Thr Lys Asn Gln Val Ser Leu Thr Cys Leu Val Lys Gly Phe
            20                  25                  30
Tyr Pro Ser Asp Ile Ala Val Glu Trp Glu Ser Asn Gly Gln Pro Glu
        35                  40                  45
Asn Asn Tyr Lys Thr Thr Pro Pro Val Leu Asp Ser Asp Gly Ser Phe
    50                  55                  60
Phe Leu Tyr Ser Lys Leu Thr Val Asp Lys Ser Arg Trp Gln Gln Gly
65                  70                  75                  80
Asn Val Phe Ser Cys Ser Val Met His Glu Ala Leu His Asn His Tyr
            85                  90                  95
Thr Gln Lys Ser Leu Ser Leu Ser Pro Gly Lys
            100                 105


<210> 200
<211> 107
<212> PRT
<213> Homo sapiens

<400> 200
Gly Gln Pro Arg Glu Pro Gln Val Tyr Thr Leu Pro Pro Ser Arg Glu
1               5                   10                  15
```

116

```
Glu Met Thr Lys Asn Gln Val Ser Leu Thr Cys Leu Val Lys Gly Phe
          20                    25                    30
Tyr Pro Ser Asp Ile Ala Val Glu Trp Glu Ser Asn Gly Gln Pro Glu
          35                    40                    45
Asn Asn Tyr Lys Thr Thr Pro Pro Met Leu Asp Ser Asp Gly Ser Phe
          50                    55                    60
Phe Leu Tyr Ser Lys Leu Thr Val Asp Lys Ser Arg Trp Gln Gln Gly
65                    70                    75                    80
Asn Val Phe Ser Cys Ser Val Met His Glu Ala Leu His Asn His Tyr
                 85                    90                    95
Thr Gln Lys Ser Leu Ser Leu Ser Pro Gly Lys
              100                   105


<210> 201
<211> 107
<212> PRT
<213> Homo sapiens


<400> 201
Gly Gln Pro Arg Glu Pro Gln Val Tyr Thr Leu Pro Pro Ser Arg Glu
  1               5                   10                  15
Glu Met Thr Lys Asn Gln Val Ser Leu Thr Cys Leu Val Lys Gly Phe
          20                    25                    30
Tyr Pro Ser Asp Ile Ala Val Glu Trp Glu Ser Ser Gly Gln Pro Glu
          35                    40                    45
Asn Asn Tyr Asn Thr Thr Pro Pro Met Leu Asp Ser Asp Gly Ser Phe
          50                    55                    60
Phe Leu Tyr Ser Lys Leu Thr Val Asp Lys Ser Arg Trp Gln Gln Gly
65                    70                    75                    80
Asn Ile Phe Ser Cys Ser Val Met His Glu Ala Leu His Asn Arg Phe
                 85                    90                    95
Thr Gln Lys Ser Leu Ser Leu Ser Pro Gly Lys
              100                   105


<210> 202
<211> 107
<212> PRT
<213> Homo sapiens


<400> 202
Gly Gln Pro Arg Glu Pro Gln Val Tyr Thr Leu Pro Pro Ser Gln Glu
  1               5                   10                  15
Glu Met Thr Lys Asn Gln Val Ser Leu Thr Cys Leu Val Lys Gly Phe
          20                    25                    30
Tyr Pro Ser Asp Ile Ala Val Glu Trp Glu Ser Asn Gly Gln Pro Glu
          35                    40                    45
Asn Asn Tyr Lys Thr Thr Pro Pro Val Leu Asp Ser Asp Gly Ser Phe
          50                    55                    60
Phe Leu Tyr Ser Arg Leu Thr Val Asp Lys Ser Arg Trp Gln Glu Gly
65                    70                    75                    80
Asn Val Phe Ser Cys Ser Val Met His Glu Ala Leu His Asn His Tyr
                 85                    90                    95
Thr Gln Lys Ser Leu Ser Leu Ser Leu Gly Lys
              100                   105
```

```
<210> 203
<211> 106
<212> PRT
<213> Homo sapiens

<400> 203
Asp Gln Asp Thr Ala Ile Arg Val Phe Ala Ile Pro Pro Ser Phe Ala
1               5                   10                  15
Ser Ile Phe Leu Thr Lys Ser Thr Lys Leu Thr Cys Leu Val Thr Asp
                20                  25                  30
Leu Thr Thr Tyr Asp Ser Val Thr Ile Ser Trp Thr Arg Gln Asn Gly
            35                  40                  45
Glu Ala Val Lys Thr His Thr Asn Ile Ser Glu Ser His Pro Asn Ala
        50                  55                  60
Thr Phe Ser Ala Val Gly Glu Ala Ser Ile Cys Glu Asp Asp Trp Asn
65                  70                  75                  80
Ser Gly Glu Arg Phe Thr Cys Thr Val Thr His Thr Asp Leu Pro Ser
                85                  90                  95
Pro Leu Lys Gln Thr Ile Ser Arg Pro Lys
                100                 105


<210> 204
<211> 19
<212> PRT
<213> Homo sapiens

<400> 204
Val Pro Ser Thr Pro Pro Thr Pro Ser Pro Ser Thr Pro Pro Thr Pro
1               5                   10                  15
Ser Pro Ser


<210> 205
<211> 6
<212> PRT
<213> Homo sapiens

<400> 205
Val Pro Pro Pro Pro Pro
1               5


<210> 206
<211> 58
<212> PRT
<213> Homo sapiens

<400> 206
Glu Ser Pro Lys Ala Gln Ala Ser Ser Val Pro Thr Ala Gln Pro Gln
1               5                   10                  15
Ala Glu Gly Ser Leu Ala Lys Ala Thr Thr Ala Pro Ala Thr Thr Arg
                20                  25                  30
Asn Thr Gly Arg Gly Gly Glu Glu Lys Lys Lys Glu Lys Glu Lys Glu
            35                  40                  45
Glu Gln Glu Glu Arg Glu Thr Lys Thr Pro
        50                  55
```

```
<210> 207
<211> 15
<212> PRT
<213> Homo sapiens

<400> 207
Glu Pro Lys Ser Cys Asp Lys Thr His Thr Cys Pro Pro Cys Pro
1               5                   10                  15


<210> 208
<211> 12
<212> PRT
<213> Homo sapiens

<400> 208
Glu Arg Lys Cys Cys Val Glu Cys Pro Pro Cys Pro
1               5                   10


<210> 209
<211> 62
<212> PRT
<213> Homo sapiens

<400> 209
Glu Leu Lys Thr Pro Leu Gly Asp Thr Thr His Thr Cys Pro Arg Cys
1               5                   10                  15
Pro Glu Pro Lys Ser Cys Asp Thr Pro Pro Pro Cys Pro Arg Cys Pro
            20                  25                  30
Glu Pro Lys Ser Cys Asp Thr Pro Pro Pro Cys Pro Arg Cys Pro Glu
        35                  40                  45
Pro Lys Ser Cys Asp Thr Pro Pro Pro Cys Pro Arg Cys Pro
    50                  55                  60


<210> 210
<211> 12
<212> PRT
<213> Homo sapiens

<400> 210
Glu Ser Lys Tyr Gly Pro Pro Cys Pro Ser Cys Pro
1               5                   10


<210> 211
<211> 107
<212> PRT
<213> Homo sapiens

<400> 211
Gly Thr Val Ala Ala Pro Ser Val Phe Ile Phe Pro Pro Ser Asp Glu
1               5                   10                  15
Gln Leu Lys Ser Gly Thr Ala Ser Val Val Cys Leu Leu Asn Asn Phe
            20                  25                  30
```

```
Tyr Pro Arg Glu Ala Lys Val Gln Trp Lys Val Asp Asn Ala Leu Gln
        35                  40                  45
Ser Gly Asn Ser Gln Glu Ser Val Thr Glu Gln Asp Ser Lys Asp Ser
        50                  55                  60
Thr Tyr Ser Leu Ser Ser Thr Leu Thr Leu Ser Lys Ala Asp Tyr Glu
65                  70                  75                  80
Lys His Lys Val Tyr Ala Cys Glu Val Thr His Gln Gly Leu Ser Ser
                85                  90                  95
Pro Val Thr Lys Ser Phe Asn Arg Gly Glu Cys
            100                 105
```

```
<210> 212
<211> 106
<212> PRT
<213> Homo sapiens
```

```
<400> 212
Gly Gln Pro Lys Ala Asn Pro Thr Val Thr Leu Phe Pro Pro Ser Ser
 1               5                   10                  15
Glu Glu Leu Gln Ala Asn Lys Ala Thr Leu Val Cys Leu Ile Ser Asp
            20                  25                  30
Phe Tyr Pro Gly Ala Val Thr Val Ala Trp Lys Ala Asp Gly Ser Pro
        35                  40                  45
Val Lys Ala Gly Val Glu Thr Thr Lys Pro Ser Lys Gln Ser Asn Asn
        50                  55                  60
Lys Tyr Ala Ala Ser Ser Tyr Leu Ser Leu Thr Pro Glu Gln Trp Lys
65                  70                  75                  80
Ser His Arg Ser Tyr Ser Cys Gln Val Thr His Glu Gly Ser Thr Val
                85                  90                  95
Glu Lys Thr Val Ala Pro Thr Glu Cys Ser
            100                 105
```

```
<210> 213
<211> 106
<212> PRT
<213> Homo sapiens
```

```
<400> 213
Gly Gln Pro Lys Ala Ala Pro Ser Val Thr Leu Phe Pro Pro Ser Ser
 1               5                   10                  15
Glu Glu Leu Gln Ala Asn Lys Ala Thr Leu Val Cys Leu Ile Ser Asp
            20                  25                  30
Phe Tyr Pro Gly Ala Val Thr Val Ala Trp Lys Ala Asp Ser Ser Pro
        35                  40                  45
Val Lys Ala Gly Val Glu Thr Thr Thr Pro Ser Lys Gln Ser Asn Asn
        50                  55                  60
Lys Tyr Ala Ala Ser Ser Tyr Leu Ser Leu Thr Pro Glu Gln Trp Lys
65                  70                  75                  80
Ser His Arg Ser Tyr Ser Cys Gln Val Thr His Glu Gly Ser Thr Val
                85                  90                  95
Glu Lys Thr Val Ala Pro Thr Glu Cys Ser
            100                 105
```

```
<210> 214
<211> 106
```

```
<212> PRT
<213> Homo sapiens

<400> 214
Gly Gln Pro Lys Ala Ala Pro Ser Val Thr Leu Phe Pro Pro Ser Ser
1               5                   10                  15
Glu Glu Leu Gln Ala Asn Lys Ala Thr Leu Val Cys Leu Ile Ser Asp
            20                  25                  30
Phe Tyr Pro Gly Ala Val Thr Val Ala Trp Lys Ala Asp Ser Ser Pro
        35                  40                  45
Val Lys Ala Gly Val Glu Thr Thr Thr Pro Ser Lys Gln Ser Asn Asn
    50                  55                  60
Lys Tyr Ala Ala Ser Ser Tyr Leu Ser Leu Thr Pro Glu Gln Trp Lys
65                  70                  75                  80
Ser His Lys Ser Tyr Ser Cys Gln Val Thr His Glu Gly Ser Thr Val
                85                  90                  95
Glu Lys Thr Val Ala Pro Thr Glu Cys Ser
            100                 105


<210> 215
<211> 106
<212> PRT
<213> Homo sapiens

<400> 215
Gly Gln Pro Lys Ala Ala Pro Ser Val Thr Leu Phe Pro Pro Ser Ser
1               5                   10                  15
Glu Glu Leu Gln Ala Asn Lys Ala Thr Leu Val Cys Leu Ile Ser Asp
            20                  25                  30
Phe Tyr Pro Gly Ala Val Lys Val Ala Trp Lys Ala Asp Gly Ser Pro
        35                  40                  45
Val Asn Thr Gly Val Glu Thr Thr Thr Pro Ser Lys Gln Ser Asn Asn
    50                  55                  60
Lys Tyr Ala Ala Ser Ser Tyr Leu Ser Leu Thr Pro Glu Gln Trp Lys
65                  70                  75                  80
Ser His Arg Ser Tyr Ser Cys Gln Val Thr His Glu Gly Ser Thr Val
                85                  90                  95
Glu Lys Thr Val Ala Pro Ala Glu Cys Ser
            100                 105


<210> 216
<211> 106
<212> PRT
<213> Homo sapiens

<400> 216
Gly Gln Pro Lys Ala Ala Pro Ser Val Thr Leu Phe Pro Pro Ser Ser
1               5                   10                  15
Glu Glu Leu Gln Ala Asn Lys Ala Thr Leu Val Cys Leu Val Ser Asp
            20                  25                  30
Phe Tyr Pro Gly Ala Val Thr Val Ala Trp Lys Ala Asp Gly Ser Pro
        35                  40                  45
Val Lys Val Gly Val Glu Thr Thr Lys Pro Ser Lys Gln Ser Asn Asn
    50                  55                  60
Lys Tyr Ala Ala Ser Ser Tyr Leu Ser Leu Thr Pro Glu Gln Trp Lys
65                  70                  75                  80
```

Ser His Arg Ser Tyr Ser Cys Arg Val Thr His Glu Gly Ser Thr Val
                85                      90                      95
Glu Lys Thr Val Ala Pro Ala Glu Cys Ser
                100                     105


<210> 217
<211> 98
<212> PRT
<213> Unknown

<220>
<223> Camel

<400> 217
Ala Ser Thr Lys Ala Pro Ser Val Tyr Pro Leu Thr Ala Arg Ser Gly
 1               5                   10                  15
Asp Thr Pro Gly Ser Thr Val Ala Phe Gly Cys Leu Val Trp Gly Tyr
                20                  25                  30
Ile Pro Glu Pro Val Thr Val Thr Trp Asn Ser Gly Ala Val Ser Ser
            35                  40                  45
Gly Ile His Thr Phe Pro Ser Val Leu Met Ser Leu Gly Leu Tyr Ser
        50                  55                  60
Leu Ser Ser Leu Val Thr Leu Pro Thr Ser Thr Ser Thr Gly Lys Thr
65                  70                  75                      80
Phe Ile Ser Asn Val Ala His Pro Ala Ser Ser Thr Lys Val Asp Lys
                85                  90                      95
Ser Val


<210> 218
<211> 110
<212> PRT
<213> Unknown

<220>
<223> Camel

<400> 218
Ala Pro Glu Leu Leu Gly Gly Pro Ser Val Phe Ile Phe Pro Pro Lys
 1               5                   10                  15
Pro Lys Asp Val Leu Ser Ile Ser Gly Arg Pro Glu Val Thr Cys Val
                20                  25                  30
Val Val Asp Val Gly Gln Glu Asp Pro Glu Val Ser Phe Asn Trp Tyr
                35                  40                  45
Ile Asp Gly Val Glu Val Arg Thr Ala Asn Thr Lys Pro Lys Glu Glu
        50                  55                  60
Gln Phe Asn Ser Thr Tyr Arg Val Val Ser Val Leu Thr Ile Gln His
65                  70                  75                      80
Gln Asp Trp Leu Thr Gly Lys Glu Leu Lys Cys Lys Val Asn Asn Lys
                85                  90                      95
Ala Leu Pro Ala Pro Ile Glu Arg Thr Ile Ser Lys Ala Lys
            100                 105                 110


<210> 219
<211> 110

122

```
<212> PRT
<213> Unknown

<220>
<223> Camel

<400> 219
Ala Pro Glu Leu Pro Gly Gly Pro Ser Val Phe Val Phe Pro Pro Lys
1               5                   10                  15
Pro Lys Asp Val Leu Ser Ile Ser Gly Arg Pro Glu Val Thr Cys Val
            20                  25                  30
Val Val Asp Val Gly Lys Glu Asp Pro Glu Val Asn Phe Asn Trp Tyr
        35                  40                  45
Ile Asp Gly Val Glu Val Arg Thr Ala Asn Thr Lys Pro Lys Glu Glu
    50                  55                  60
Gln Phe Asn Ser Thr Tyr Arg Val Val Ser Val Leu Thr Ile Gln His
65                  70                  75                  80
Gln Asp Trp Leu Thr Gly Lys Glu Phe Lys Cys Lys Val Asn Asn Lys
            85                  90                  95
Ala Leu Pro Ala Pro Ile Glu Arg Thr Ile Ser Lys Pro Lys
            100                 105                 110


<210> 220
<211> 109
<212> PRT
<213> Unknown

<220>
<223> Camel

<400> 220
Gly Gln Thr Arg Glu Pro Gln Val Tyr Thr Leu Ala Pro His Arg Glu
1               5                   10                  15
Glu Leu Ala Lys Asp Thr Val Ser Ile Thr Cys Leu Val Ile Gly Phe
            20                  25                  30
Tyr Pro Ala Asp Ile Asn Val Glu Trp Gln Arg Asn Gly Arg Pro Glu
        35                  40                  45
Ser Glu Gly Ala Tyr Ala Thr Thr Leu Pro Gln Leu Asp Asn Asp Gly
        50                  55                  60
Thr Tyr Phe Leu Tyr Ser Lys Leu Ser Val Gly Lys Asn Thr Trp Gln
65                  70                  75                  80
Gln Gly Glu Thr Phe Thr Cys Val Val Met His Glu Ala Leu His Asn
            85                  90                  95
His Ser Thr Gln Lys Ser Ile Thr Gln Ser Ser Gly Lys
            100                 105


<210> 221
<211> 109
<212> PRT
<213> Unknown

<220>
<223> Camel

<400> 221
Gly Gln Thr Arg Glu Pro Gln Val Tyr Thr Leu Ala Pro His Arg Glu
```

```
      1                 5                    10                    15
      Glu Leu Ala Lys Asp Thr Val Ser Val Thr Cys Leu Val Lys Gly Phe
                  20                    25                    30
      Tyr Pro Pro Asp Ile Asn Val Glu Trp Gln Arg Asn Arg Gln Pro Glu
              35                    40                    45
      Ser Glu Gly Ala Tyr Ala Thr Thr Leu Pro Gln Leu Asp Asn Asp Gly
          50                    55                    60
      Thr Tyr Phe Leu Tyr Ser Lys Leu Ser Val Gly Lys Asn Thr Trp Gln
      65                    70                    75                    80
      Arg Gly Glu Thr Phe Thr Cys Val Val Met His Glu Ala Leu His Asn
                  85                    90                    95
      His Tyr Thr Gln Lys Ser Ile Thr Gln Ser Ser Gly Lys
                  100                   105
```

```
<210> 222
<211> 35
<212> PRT
<213> Unknown

<220>
<223> Camel

<400> 222
Glu Pro Lys Ile Pro Gln Pro Gln Pro Lys Pro Gln Pro Gln Pro Gln
1                 5                    10                    15
Pro Gln Pro Lys Pro Gln Pro Lys Pro Glu Pro Glu Cys Thr Cys Pro
            20                    25                    30
Lys Cys Pro
        35
```

```
<210> 223
<211> 12
<212> PRT
<213> Unknown

<220>
<223> Camel

<400> 223
Gly Thr Asn Glu Val Cys Lys Cys Pro Lys Cys Pro
1                 5                    10
```

```
<210> 224
<211> 91
<212> PRT
<213> Homo sapiens

<400> 224
Asn Ile Gln Asn Pro Asp Pro Ala Val Tyr Gln Leu Arg Asp Ser Lys
1                 5                    10                    15
Ser Ser Asp Lys Ser Val Cys Leu Phe Thr Asp Phe Asp Ser Gln Thr
            20                    25                    30
Asn Val Ser Gln Ser Lys Asp Ser Asp Val Tyr Ile Thr Asp Lys Thr
            35                    40                    45
Val Leu Asp Met Arg Ser Met Asp Phe Lys Ser Asn Ser Ala Val Ala
```

```
              50                    55                    60
Trp Ser Asn Lys Ser Asp Phe Ala Cys Ala Asn Ala Phe Asn Asn Ser
65                    70                    75                    80
Ile Ile Pro Glu Asp Thr Phe Phe Pro Ser Pro
                    85                    90


<210> 225
<211> 129
<212> PRT
<213> Homo sapiens

<400> 225
Glu Asp Leu Asn Lys Val Phe Pro Pro Glu Val Ala Val Phe Glu Pro
1                   5                   10                  15
Ser Glu Ala Glu Ile Ser His Thr Gln Lys Ala Thr Leu Val Cys Leu
                  20                  25                  30
Ala Thr Gly Phe Phe Pro Asp His Val Glu Leu Ser Trp Trp Val Asn
                  35                  40                  45
Gly Lys Glu Val His Ser Gly Val Ser Thr Asp Pro Gln Pro Leu Lys
                  50                  55                  60
Glu Gln Pro Ala Leu Asn Asp Ser Arg Tyr Cys Leu Ser Ser Arg Leu
65                  70                  75                  80
Arg Val Ser Ala Thr Phe Trp Gln Asn Pro Arg Asn His Phe Arg Cys
                  85                  90                  95
Gln Val Gln Phe Tyr Gly Leu Ser Glu Asn Asp Glu Trp Thr Gln Asp
                  100                 105                 110
Arg Ala Lys Pro Val Thr Gln Ile Val Ser Ala Glu Ala Trp Gly Arg
                  115                 120                 125
Ala


<210> 226
<211> 129
<212> PRT
<213> Homo sapiens

<400> 226
Glu Asp Leu Lys Asn Val Phe Pro Pro Glu Val Ala Val Phe Glu Pro
1                   5                   10                  15
Ser Glu Ala Glu Ile Ser His Thr Gln Lys Ala Thr Leu Val Cys Leu
                  20                  25                  30
Ala Thr Gly Phe Tyr Pro Asp His Val Glu Leu Ser Trp Trp Val Asn
                  35                  40                  45
Gly Lys Glu Val His Ser Gly Val Ser Thr Asp Pro Gln Pro Leu Lys
                  50                  55                  60
Glu Gln Pro Ala Leu Asn Asp Ser Arg Tyr Cys Leu Ser Ser Arg Leu
65                  70                  75                  80
Arg Val Ser Ala Thr Phe Trp Gln Asn Pro Arg Asn His Phe Arg Cys
                  85                  90                  95
Gln Val Gln Phe Tyr Gly Leu Ser Glu Asn Asp Glu Trp Thr Gln Asp
                  100                 105                 110
Arg Ala Lys Pro Val Thr Gln Ile Val Ser Ala Glu Ala Trp Gly Arg
                  115                 120                 125
Ala
```

```
<210> 227
<211> 93
<212> PRT
<213> Homo sapiens

<400> 227
Arg Ser Gln Pro His Thr Lys Pro Ser Val Phe Val Met Lys Asn Gly
1               5                   10                  15
Thr Asn Val Ala Cys Leu Val Lys Glu Phe Tyr Pro Lys Asp Ile Arg
            20                  25                  30
Ile Asn Leu Val Ser Ser Lys Lys Ile Thr Glu Phe Asp Pro Ala Ile
        35                  40                  45
Val Ile Ser Pro Ser Gly Lys Tyr Asn Ala Val Lys Leu Gly Lys Tyr
        50                  55                  60
Glu Asp Ser Asn Ser Val Thr Cys Ser Val Gln His Asp Asn Lys Thr
65                  70                  75                  80
Val His Ser Thr Asp Phe Glu Val Lys Thr Asp Ser Thr
                85                  90


<210> 228
<211> 110
<212> PRT
<213> Homo sapiens

<400> 228
Asp Lys Gln Leu Asp Ala Asp Val Ser Pro Lys Pro Thr Ile Phe Leu
1               5                   10                  15
Pro Ser Ile Ala Glu Thr Lys Leu Gln Lys Ala Gly Thr Tyr Leu Cys
            20                  25                  30
Leu Leu Glu Lys Phe Phe Pro Asp Val Ile Lys Ile His Trp Gln Glu
        35                  40                  45
Lys Lys Ser Asn Thr Ile Leu Gly Ser Gln Glu Gly Asn Thr Met Lys
        50                  55                  60
Thr Asn Asp Thr Tyr Met Lys Phe Ser Trp Leu Thr Val Pro Glu Lys
65                  70                  75                  80
Ser Leu Asp Lys Glu His Arg Cys Ile Val Arg His Glu Asn Asn Lys
                85                  90                  95
Asn Gly Val Asp Gln Glu Ile Ile Phe Pro Pro Ile Lys Thr
            100                 105                 110


<210> 229
<211> 110
<212> PRT
<213> Homo sapiens

<400> 229
Asp Lys Gln Leu Asp Ala Asp Val Ser Pro Lys Pro Thr Ile Phe Leu
1               5                   10                  15
Pro Ser Ile Ala Glu Thr Lys Leu Gln Lys Ala Gly Thr Tyr Leu Cys
            20                  25                  30
Leu Leu Glu Lys Phe Phe Pro Asp Ile Ile Lys Ile His Trp Gln Glu
        35                  40                  45
Lys Lys Ser Asn Thr Ile Leu Gly Ser Gln Glu Gly Asn Thr Met Lys
        50                  55                  60
Thr Asn Asp Thr Tyr Met Lys Phe Ser Trp Leu Thr Val Pro Glu Glu
```

```
      65                      70                      75                      80
      Ser Leu Asp Lys Glu His Arg Cys Ile Val Arg His Glu Asn Asn Lys
                          85                      90                      95
      Asn Gly Ile Asp Gln Glu Ile Ile Phe Pro Pro Ile Lys Thr
                          100                     105                     110



<210> 230
<211> 110
<212> PRT
<213> Homo sapiens

<400> 230
Asp Lys Gln Leu Asp Ala Asp Val Ser Pro Lys Pro Thr Ile Phe Leu
1               5                   10                  15
Pro Ser Ile Ala Glu Thr Lys Leu Gln Lys Ala Gly Thr Tyr Leu Cys
            20                  25                  30
Leu Leu Glu Lys Phe Phe Pro Asp Ile Ile Lys Ile His Trp Gln Glu
        35                  40                  45
Lys Lys Ser Asn Thr Ile Leu Gly Ser Gln Glu Gly Asn Thr Met Lys
        50                  55                  60
Thr Asn Asp Thr Tyr Met Lys Phe Ser Trp Leu Thr Val Pro Glu Glu
65                  70                  75                  80
Ser Leu Asp Lys Glu His Arg Cys Ile Val Arg His Glu Asn Asn Lys
                    85                  90                  95
Asn Gly Ile Asp Gln Glu Ile Ile Phe Pro Pro Ile Lys Thr
            100                 105                 110



<210> 231
<211> 14
<212> PRT
<213> Unknown

<220>
<223> Nurse Shark

<400> 231
Ser Tyr Glu Tyr Gly Gly Gly Thr Val Val Thr Val Asn Pro
1               5                   10


<210> 232
<211> 12
<212> PRT
<213> Unknown

<220>
<223> Nurse Shark

<400> 232
Ser Phe Asp Glu Tyr Gly Gly Gly Thr Val Val Thr
1               5                   10


<210> 233
<211> 11
<212> PRT
```

<213> Unknown

<220>
<223> Nurse Shark

<400> 233
Ser Tyr Glu Tyr Gly Gly Gly Thr Val Val Thr
1               5                   10

<210> 234
<211> 12
<212> PRT
<213> Unknown

<220>
<223> Nurse Shark

<400> 234
Asn Tyr Ala Ala Cys Gly Asp Gly Thr Ala Val Thr
1               5                   10

<210> 235
<211> 13
<212> PRT
<213> Unknown

<220>
<223> Nurse Shark

<400> 235
Gly Tyr Trp Gly Gln Gly Thr Met Val Thr Val Thr Thr
1               5                   10

<210> 236
<211> 14
<212> PRT
<213> Unknown

<220>
<223> Nurse Shark

<400> 236
Ala Leu Asp Tyr Trp Gly Gln Gly Thr Arg Val Thr Val Thr
1               5                   10

<210> 237
<211> 12
<212> PRT
<213> Unknown

<220>
<223> Nurse Shark

<400> 237

```
Ala Cys Gly Ser Gly Thr Ala Val Thr Val Thr Pro
 1               5                   10
```

```
<210> 238
<211> 11
<212> PRT
<213> Unknown

<220>
<223> Nurse Shark

<400> 238
```
```
Tyr Gly Gly Gly Thr Gly Val Thr Val Asn Pro
 1               5                   10
```

```
<210> 239
<211> 11
<212> PRT
<213> Unknown

<220>
<223> Nurse Shark

<400> 239
```
```
Tyr Gly Gly Gly Thr Val Val Thr Val Asn Pro
 1               5                   10
```

```
<210> 240
<211> 12
<212> PRT
<213> Unknown

<220>
<223> Nurse Shark

<400> 240
```
```
Ala Cys Gly Asp Gly Thr Phe Val Thr Val Asn Pro
 1               5                   10
```

```
<210> 241
<211> 11
<212> PRT
<213> Unknown

<220>
<223> Nurse Shark

<400> 241
```
```
Tyr Gly Ala Asp Thr Val Val Thr Val Asn Pro
 1               5                   10
```

```
<210> 242
<211> 14
```

```
<212> PRT
<213> Unknown

<220>
<223> Nurse Shark

<400> 242
Tyr Ala Ala Cys Gly Ala Gly Thr Ala Val Thr Val Asn Pro
 1               5                   10


<210> 243
<211> 11
<212> PRT
<213> Unknown

<220>
<223> Nurse Shark

<400> 243
Tyr Gly Ser Gly Thr Val Leu Thr Val Asn Pro
 1               5                   10


<210> 244
<211> 12
<212> PRT
<213> Unknown

<220>
<223> Nurse Shark

<400> 244
Ala Cys Gly Asp Gly Thr Ala Val Thr Val Asn Pro
 1               5                   10


<210> 245
<211> 11
<212> PRT
<213> Unknown

<220>
<223> Nurse Shark

<400> 245
Tyr Gly Gly Gly Thr Val Val Thr Val Asn Pro
 1               5                   10


<210> 246
<211> 15
<212> PRT
<213> Unknown

<220>
<223> Nurse Shark
```

```
<400> 246
Ile Tyr Asp Glu Cys Gly Asp Gly Thr Ala Val Thr Val Asn Pro
 1               5                   10                  15


<210> 247
<211> 11
<212> PRT
<213> Unknown

<220>
<223> Nurse Shark

<400> 247
Cys Gly Asp Gly Thr Val Val Thr Val Asn Pro
 1               5                   10


<210> 248
<211> 11
<212> PRT
<213> Unknown

<220>
<223> Nurse Shark

<400> 248
Cys Gly Gly Gly Thr Val Val Thr Val Asn Pro
 1               5                   10


<210> 249
<211> 11
<212> PRT
<213> Unknown

<220>
<223> Nurse Shark

<400> 249
Tyr Gly Gly Gly Thr Val Val Thr Val Asn Pro
 1               5                   10


<210> 250
<211> 14
<212> PRT
<213> Unknown

<220>
<223> Nurse Shark

<400> 250
Tyr Ala Ala Val Gly Asp Gly Thr Ala Val Thr Val Asn Pro
 1               5                   10


<210> 251
```

```
<211> 11
<212> PRT
<213> Unknown

<220>
<223> Nurse Shark

<400> 251
Cys Gly Glu Gly Thr Ala Val Thr Val Asn Pro
 1               5                   10
```

```
<210> 252
<211> 13
<212> PRT
<213> Unknown

<220>
<223> Nurse Shark

<400> 252
Ala Ala Cys Gly His Gly Thr Ala Val Thr Val Thr Ser
 1               5                   10
```

```
<210> 253
<211> 11
<212> PRT
<213> Unknown

<220>
<223> Nurse Shark

<400> 253
Tyr Gly Gly Gly Thr Val Val Thr Val Asn Pro
 1               5                   10
```

```
<210> 254
<211> 11
<212> PRT
<213> Unknown

<220>
<223> Nurse Shark

<400> 254
Cys Gly Asp Gly Thr Thr Val Thr Val His Pro
 1               5                   10
```

```
<210> 255
<211> 14
<212> PRT
<213> Unknown

<220>
<223> Nurse Shark
```

```
<400> 255
Tyr Ala Ala Cys Gly Pro Gly Thr Thr Val Thr Val Asn Pro
1               5                   10


<210> 256
<211> 14
<212> PRT
<213> Unknown

<220>
<223> Nurse Shark

<400> 256
Tyr Ala Ala Cys Gly His Gly Thr Ala Val Thr Val Asn Ala
1               5                   10


<210> 257
<211> 12
<212> PRT
<213> Unknown

<220>
<223> Nurse Shark

<400> 257
Ala Cys Gly His Gly Thr Ala Val Thr Val Asn Pro
1               5                   10


<210> 258
<211> 11
<212> PRT
<213> Unknown

<220>
<223> Nurse Shark

<400> 258
Cys Gly Asp Gly Thr Ala Val Thr Val Asn Pro
1               5                   10


<210> 259
<211> 14
<212> PRT
<213> Unknown

<220>
<223> Nurse Shark

<400> 259
Tyr Ala Ala Cys Gly Asp Ala Thr Ala Val Thr Val Asn Pro
1               5                   10
```

```
<210> 260
<211> 12
<212> PRT
<213> Unknown

<220>
<223> Nurse Shark

<400> 260
Ala Cys Gly Asp Gly Thr Val Val Thr Val Ser Pro
 1               5                   10


<210> 261
<211> 11
<212> PRT
<213> Unknown

<220>
<223> Nurse Shark

<400> 261
Cys Gly Asp Gly Thr Ala Val Thr Val Asn Pro
 1               5                   10


<210> 262
<211> 14
<212> PRT
<213> Unknown

<220>
<223> Nurse Shark

<400> 262
Tyr Ala Ala Cys Gly Asp Gly Thr Ala Val Thr Val Asn Pro
 1               5                   10


<210> 263
<211> 11
<212> PRT
<213> Unknown

<220>
<223> Nurse Shark

<400> 263
Cys Gly Asp Asn Thr Ala Val Thr Val Asn Pro
 1               5                   10


<210> 264
<211> 12
<212> PRT
<213> Unknown

<220>
```

<223> Nurse Shark

<400> 264
Ala Cys Gly His Gly Thr Thr Val Thr Val Ala Pro
1               5                   10


<210> 265
<211> 11
<212> PRT
<213> Unknown

<220>
<223> Nurse Shark

<400> 265
Cys Gly Asp Gly Thr Val Leu Thr Val Asn Pro
1               5                   10


<210> 266
<211> 13
<212> PRT
<213> Unknown

<220>
<223> Nurse Shark

<400> 266
Ala Ala Cys Gly Asp Gly Thr Ala Val Thr Val Asn Pro
1               5                   10


<210> 267
<211> 12
<212> PRT
<213> Unknown

<220>
<223> Nurse Shark

<400> 267
Ala Cys Gly Leu Gly Thr Ala Val Thr Val Asn Pro
1               5                   10


<210> 268
<211> 14
<212> PRT
<213> Unknown

<220>
<223> Nurse Shark

<400> 268
Tyr Ala Ala Cys Gly Asp Gly Thr Ala Val Thr Val Asn Pro
1               5                   10

```
<210> 269
<211> 13
<212> PRT
<213> Unknown

<220>
<223> Nurse Shark

<400> 269
Ala Ala Cys Gly Asp Gly Thr Ala Val Thr Val Asn Pro
 1               5                   10


<210> 270
<211> 11
<212> PRT
<213> Unknown

<220>
<223> Nurse Shark

<400> 270
Tyr Gly Gly Gly Thr Val Val Thr Val Asn Pro
 1               5                   10


<210> 271
<211> 12
<212> PRT
<213> Unknown

<220>
<223> Nurse Shark

<400> 271
Ala Cys Gly Asp Gly Thr Ala Val Thr Val Asn Pro
 1               5                   10


<210> 272
<211> 13
<212> PRT
<213> Unknown

<220>
<223> Nurse Shark

<400> 272
Ala Ala Cys Gly Asp Gly Thr Ala Val Thr Val Asn Pro
 1               5                   10


<210> 273
<211> 11
<212> PRT
<213> Unknown
```

```
<220>
<223> Nurse Shark

<400> 273
Cys Gly Gly Gly Thr Asp Val Thr Val Asn Thr
1               5                   10


<210> 274
<211> 13
<212> PRT
<213> Unknown

<220>
<223> Nurse Shark

<400> 274
Ala Ala Cys Gly Asp Gly Thr Ala Val Thr Val Asn Pro
1               5                   10


<210> 275
<211> 14
<212> PRT
<213> Unknown

<220>
<223> Nurse Shark

<400> 275
Tyr Ala Ala Cys Gly Asp Gly Thr Ala Val Thr Val Asn Pro
1               5                   10


<210> 276
<211> 14
<212> PRT
<213> Unknown

<220>
<223> Nurse Shark

<400> 276
Tyr Ala Ala Cys Gly Asp Gly Thr Ser Val Thr Val Asn Pro
1               5                   10


<210> 277
<211> 14
<212> PRT
<213> Unknown

<220>
<223> Nurse Shark

<400> 277
Tyr Ala Ala Cys Gly Asp Gly Thr Ala Val Thr Val Asn Pro
1               5                   10
```

```
<210> 278
<211> 13
<212> PRT
<213> Unknown

<220>
<223> Nurse Shark

<400> 278
Ala Ala Cys Gly Glu Gly Thr Ala Val Thr Val Asn Pro
 1               5                   10


<210> 279
<211> 14
<212> PRT
<213> Unknown

<220>
<223> Nurse Shark

<400> 279
Tyr Ala Ala Cys Gly Asp Gly Thr Thr Val Thr Val Lys Pro
 1               5                   10


<210> 280
<211> 14
<212> PRT
<213> Unknown

<220>
<223> Nurse Shark

<400> 280
Leu Asp Tyr Trp Gly Asp Gly Thr Phe Leu Glu Val Thr Ser
 1               5                   10


<210> 281
<211> 11
<212> PRT
<213> Unknown

<220>
<223> Nurse Shark

<400> 281
Cys Gly Asp Gly Thr Ala Val Thr Val Asn Pro
 1               5                   10


<210> 282
<211> 12
<212> PRT
<213> Unknown
```

```
<220>
<223> Nurse Shark

<400> 282
Ala Cys Gly Asp Gly Thr Ala Val Thr Val Asn Pro
1               5                   10


<210> 283
<211> 13
<212> PRT
<213> Unknown

<220>
<223> Nurse Shark

<400> 283
Phe Ala Phe Gly Lys Gly Thr Lys Leu Arg Leu Ser Arg
1               5                   10


<210> 284
<211> 13
<212> PRT
<213> Unknown

<220>
<223> Nurse Shark

<400> 284
Phe Ala Phe Gly Lys Gly Thr Lys Leu Arg Leu Ser Arg
1               5                   10


<210> 285
<211> 13
<212> PRT
<213> Unknown

<220>
<223> Nurse Shark

<400> 285
Phe Val Phe Gly Lys Gly Thr Lys Leu Arg Leu Ser Arg
1               5                   10


<210> 286
<211> 13
<212> PRT
<213> Unknown

<220>
<223> Nurse Shark

<400> 286
Phe Ala Phe Gly Lys Gly Thr Lys Leu Arg Leu Ser Arg
```

```
        1               5                   10


<210> 287
<211> 13
<212> PRT
<213> Unknown

<220>
<223> Nurse Shark

<400> 287
Arg Ala Phe Gly Lys Gly Thr Lys Leu Arg Leu Ser Arg
 1               5                   10


<210> 288
<211> 13
<212> PRT
<213> Unknown

<220>
<223> Nurse Shark

<400> 288
Val Val Phe Gly Glu Gly Thr Lys Leu Arg Leu Ser Arg
 1               5                   10


<210> 289
<211> 20
<212> PRT
<213> Unknown

<220>
<223> Mouse

<400> 289
Leu Leu Lys Arg Glu Asp Lys Ala Thr Phe Ala Thr Gly Gly Tyr Glu
 1               5                   10                  15
Ala Glu Glu Asp
                20


<210> 290
<211> 20
<212> PRT
<213> Unknown

<220>
<223> Mouse

<400> 290
Gln Gln Gly Thr Gly Ser Lys Leu Ser Phe Gly Lys Gly Ala Lys Leu
 1               5                   10                  15
Thr Val Ser Pro
                20
```

```
<210> 291
<211> 20
<212> PRT
<213> Unknown

<220>
<223> Mouse

<400> 291
Asn Gln Gly Gly Ser Ala Lys Leu Ile Phe Gly Glu Gly Thr Lys Leu
 1               5                   10                  15
Thr Val Ser Ser
            20


<210> 292
<211> 20
<212> PRT
<213> Unknown

<220>
<223> Mouse

<400> 292
Ala Thr Gly Gly Asn Asn Lys Leu Thr Phe Gly Gln Gly Thr Val Leu
 1               5                   10                  15
Ser Val Ile Pro
            20


<210> 293
<211> 21
<212> PRT
<213> Unknown

<220>
<223> Mouse

<400> 293
Asn Ser Gly Gly Ser Asn Tyr Lys Leu Thr Phe Gly Lys Gly Thr Leu
 1               5                   10                  15
Leu Thr Val Thr Pro
            20


<210> 294
<211> 21
<212> PRT
<213> Unknown

<220>
<223> Mouse

<400> 294
Asn Thr Gly Ala Asn Thr Gly Lys Leu Thr Phe Gly His Gly Thr Ile
 1               5                   10                  15
Leu Arg Val His Pro
```

20

```
<210> 295
<211> 20
<212> PRT
<213> Unknown

<220>
<223> Mouse

<400> 295
Ala Ser Ser Ser Phe Ser Lys Leu Val Phe Gly Gln Gly Thr Ser Leu
1               5                   10                  15
Ser Val Val Pro
            20


<210> 296
<211> 19
<212> PRT
<213> Unknown

<220>
<223> Mouse

<400> 296
Asn Thr Gly Tyr Gln Asn Phe Tyr Phe Gly Lys Gly Thr Ser Leu Thr
1               5                   10                  15
Val Ile Pro


<210> 297
<211> 20
<212> PRT
<213> Unknown

<220>
<223> Mouse

<400> 297
Ala Asn Tyr Gly Asn Glu Lys Ile Thr Phe Gly Ala Gly Thr Lys Leu
1               5                   10                  15
Thr Ile Lys Pro
            20


<210> 298
<211> 18
<212> PRT
<213> Unknown

<220>
<223> Mouse

<400> 298
His Tyr Ala Asn Lys Met Ile Cys Gly Leu Gly Thr Ile Leu Arg Val
```

```
 1              5                    10                   15
Arg Pro



<210> 299
<211> 20
<212> PRT
<213> Unknown

<220>
<223> Mouse

<400> 299
Arg Arg Gln Gln Cys Arg His Ala Gly Phe Gly Asp Gly Asp Glu Leu
 1              5                    10                   15
Gly Val Ser Thr
            20



<210> 300
<211> 20
<212> PRT
<213> Unknown

<220>
<223> Mouse

<400> 300
Asn Thr Glu Gly Ala Asp Arg Leu Thr Phe Gly Lys Gly Thr Gln Leu
 1              5                    10                   15
Ile Ile Gln Pro
            20



<210> 301
<211> 20
<212> PRT
<213> Unknown

<220>
<223> Mouse

<400> 301
Val Thr Gly Ser Gly Gly Lys Leu Thr Leu Gly Ala Gly Thr Arg Leu
 1              5                    10                   15
Gln Val Asn Leu
            20



<210> 302
<211> 18
<212> PRT
<213> Unknown

<220>
<223> Mouse
```

<400> 302
Asn Asn Asn Asn Ala Pro Arg Phe Gly Ala Gly Thr Lys Leu Ser Val
1               5                   10                  15
Lys Pro


<210> 303
<211> 21
<212> PRT
<213> Unknown

<220>
<223> Mouse

<400> 303
Asn Ser Gly Gly Ser Asn Ala Lys Leu Thr Phe Gly Lys Gly Thr Lys
1               5                   10                  15
Leu Ser Val Lys Ser
                20


<210> 304
<211> 18
<212> PRT
<213> Unknown

<220>
<223> Mouse

<400> 304
Val Ser Asn Thr Ser Ser Met Leu Ala Glu Ala Pro His Tyr Trp Ser
1               5                   10                  15
His Pro


<210> 305
<211> 20
<212> PRT
<213> Unknown

<220>
<223> Mouse

<400> 305
Val Asn Thr Gly Asn Tyr Lys Tyr Val Phe Gly Ala Gly Thr Arg Leu
1               5                   10                  15
Lys Val Ile Ala
                20


<210> 306
<211> 20
<212> PRT
<213> Unknown

<220>

<223> Mouse

<400> 306
Asn Asn Asn Ala Gly Ala Lys Leu Thr Phe Gly Gly Gly Thr Arg Leu
1               5                   10                  15
Thr Val Arg Pro
            20

<210> 307
<211> 20
<212> PRT
<213> Unknown

<220>
<223> Mouse

<400> 307
Asn Val Gly Asp Asn Ser Lys Leu Ile Trp Gly Leu Gly Thr Ser Leu
1               5                   10                  15
Val Val Asn Pro
            20

<210> 308
<211> 19
<212> PRT
<213> Unknown

<220>
<223> Mouse

<400> 308
Thr Gly Asn Thr Gly Lys Leu Ile Phe Gly Leu Gly Thr Thr Leu Gln
1               5                   10                  15
Val Gln Pro

<210> 309
<211> 21
<212> PRT
<213> Unknown

<220>
<223> Mouse

<400> 309
Gln Thr Gly Phe Ala Ser Ala Leu Thr Phe Gly Ser Gly Thr Lys Val
1               5                   10                  15
Ile Pro Cys Leu Pro
            20

<210> 310
<211> 19
<212> PRT
<213> Unknown

<220>
<223> Mouse

<400> 310
Ser Ser Asn Thr Asp Lys Val Val Phe Gly Thr Gly Thr Arg Leu Gln
1               5               10              15
Val Ser Pro


<210> 311
<211> 18
<212> PRT
<213> Unknown

<220>
<223> Mouse

<400> 311
Asp Ser Asn Tyr Gln Leu Ile Trp Gly Ser Gly Thr Lys Leu Ile Ile
1               5               10              15
Lys Pro


<210> 312
<211> 21
<212> PRT
<213> Unknown

<220>
<223> Mouse

<400> 312
Asn Tyr Gly Ser Ser Gly Asn Lys Leu Ile Phe Gly Ile Gly Thr Leu
1               5               10              15
Leu Ser Val Lys Pro
              20


<210> 313
<211> 18
<212> PRT
<213> Unknown

<220>
<223> Mouse

<400> 313
Asn Ser Asn Asn Arg Ile Phe Phe Gly Asp Gly Thr Gln Leu Val Val
1               5               10              15
Lys Pro


<210> 314
<211> 19

146

<212> PRT
<213> Unknown

<220>
<223> Mouse

<400> 314
Asp Thr Asn Ala Tyr Lys Val Ile Phe Gly Lys Gly Thr His Leu His
1               5                   10                  15
Val Leu Pro


<210> 315
<211> 19
<212> PRT
<213> Unknown

<220>
<223> Mouse

<400> 315
Asn Ser Gly Ser Arg Glu Leu Val Leu Gly Arg Glu Ala Arg Leu Ser
1               5                   10                  15
Met Ile Glu


<210> 316
<211> 21
<212> PRT
<213> Unknown

<220>
<223> Mouse

<400> 316
Leu Pro Gly Thr Gly Ser Asn Arg Leu Thr Phe Gly Lys Gly Thr Lys
1               5                   10                  15
Phe Ser Leu Ile Pro
                20


<210> 317
<211> 19
<212> PRT
<213> Unknown

<220>
<223> Mouse

<400> 317
Asn Thr Asn Thr Gly Lys Leu Thr Phe Gly Asp Gly Thr Val Leu Thr
1               5                   10                  15
Val Lys Pro

```
<210> 318
<211> 18
<212> PRT
<213> Unknown

<220>
<223> Mouse

<400> 318
Arg Thr Lys Val Ser Ser Val Phe Gly Thr Trp Arg Arg Leu Leu Val
 1               5                   10                  15
Lys Pro


<210> 319
<211> 20
<212> PRT
<213> Unknown

<220>
<223> Mouse

<400> 319
Glu Leu Ala Ser Leu Gly Lys Leu Gln Phe Gly Thr Gly Thr Gln Val
 1               5                   10                  15
Val Val Thr Pro
             20


<210> 320
<211> 19
<212> PRT
<213> Unknown

<220>
<223> Mouse

<400> 320
Asn Tyr Asn Gln Gly Lys Leu Ile Phe Gly Gln Gly Thr Lys Leu Ser
 1               5                   10                  15
Ile Lys Pro


<210> 321
<211> 19
<212> PRT
<213> Unknown

<220>
<223> Mouse

<400> 321
Ser Ser Gly Ser Trp Gln Leu Ile Phe Gly Ser Gly Thr Gln Leu Thr
 1               5                   10                  15
Val Met Pro
```

```
<210> 322
<211> 18
<212> PRT
<213> Unknown

<220>
<223> Mouse

<400> 322
Ser Asn Tyr Asn Val Leu Tyr Phe Gly Ser Gly Thr Lys Leu Thr Val
 1               5                  10                  15
Glu Pro


<210> 323
<211> 18
<212> PRT
<213> Unknown

<220>
<223> Mouse

<400> 323
Ser Gly Asn Tyr Lys Leu Gly Val Glu Ser Val Thr Met Met Ser Val
 1               5                  10                  15
Arg Ala


<210> 324
<211> 20
<212> PRT
<213> Unknown

<220>
<223> Mouse

<400> 324
Ile Tyr Arg Gly Phe His Lys Phe Ser Ser Gly Ile Glu Ser Lys His
 1               5                  10                  15
Asn Val Ser Pro
             20


<210> 325
<211> 21
<212> PRT
<213> Unknown

<220>
<223> Mouse

<400> 325
Asp Arg Gly Ser Ala Leu Gly Arg Leu His Phe Gly Ala Gly Thr Gln
 1               5                  10                  15
```

149

```
Leu Ile Val Ile Pro
            20


<210> 326
<211> 20
<212> PRT
<213> Unknown

<220>
<223> Mouse

<400> 326
Thr Asn Ser Ala Gly Asn Lys Leu Thr Phe Gly Ile Gly Thr Arg Val
 1               5                   10                  15
Leu Val Arg Pro
            20


<210> 327
<211> 20
<212> PRT
<213> Unknown

<220>
<223> Mouse

<400> 327
Ala Thr Ser Ser Gly Gln Lys Leu Val Phe Gly Gln Gly Thr Ile Leu
 1               5                   10                  15
Lys Val Tyr Leu
            20


<210> 328
<211> 19
<212> PRT
<213> Unknown

<220>
<223> Mouse

<400> 328
Tyr Gln Gly Gly Arg Ala Leu Ile Phe Gly Thr Gly Thr Thr Val Ser
 1               5                   10                  15
Val Ser Pro


<210> 329
<211> 18
<212> PRT
<213> Unknown

<220>
<223> Mouse

<400> 329
```

```
Asn Ser Gly Thr Tyr Gln Arg Phe Gly Thr Gly Thr Lys Leu Gln Val
1               5                   10                  15
Val Pro
```

```
<210> 330
<211> 19
<212> PRT
<213> Unknown

<220>
<223> Mouse

<400> 330
Gly Thr Gly Gly Tyr Lys Val Val Phe Gly Ser Gly Thr Arg Leu Leu
1               5                   10                  15
Val Ser Pro
```

```
<210> 331
<211> 19
<212> PRT
<213> Unknown

<220>
<223> Mouse

<400> 331
Asp Ser Gly Tyr Asn Lys Leu Thr Phe Gly Lys Gly Thr Val Leu Leu
1               5                   10                  15
Val Ser Pro
```

```
<210> 332
<211> 19
<212> PRT
<213> Unknown

<220>
<223> Mouse

<400> 332
Arg Asn Met Gly Tyr Lys Leu Thr Phe Gly Thr Gly Thr Ser Leu Leu
1               5                   10                  15
Val Asp Pro
```

```
<210> 333
<211> 19
<212> PRT
<213> Unknown

<220>
<223> Mouse
```

<400> 333
Asp Tyr Ser Asn Asn Arg Leu Thr Leu Gly Lys Gly Thr Gln Val Val
1               5                   10                  15
Val Leu Pro


<210> 334
<211> 20
<212> PRT
<213> Unknown

<220>
<223> Mouse

<400> 334
Thr Ser Gly Gly Asn Tyr Lys Pro Thr Phe Gly Lys Gly Thr Ser Leu
1               5                   10                  15
Val Val His Pro
                20


<210> 335
<211> 20
<212> PRT
<213> Unknown

<220>
<223> Mouse

<400> 335
Gly Thr Gln Val Val Gly Gln Leu Thr Phe Gly Arg Gly Thr Arg Leu
1               5                   10                  15
Gln Val Tyr Ala
                20


<210> 336
<211> 20
<212> PRT
<213> Unknown

<220>
<223> Mouse

<400> 336
Leu Ser Gly Ser Phe Asn Lys Leu Thr Phe Gly Ala Gly Thr Arg Leu
1               5                   10                  15
Leu Cys Ala His
                20


<210> 337
<211> 21
<212> PRT
<213> Unknown

```
<220>
<223> Mouse

<400> 337
Glu Phe Ser Tyr Ser Ser Lys Leu Ile Phe Gly Ala Glu Thr Lys Leu
1               5                   10                  15
Arg Asn Pro Pro Tyr
                20


<210> 338
<211> 21
<212> PRT
<213> Unknown

<220>
<223> Mouse

<400> 338
Asn Thr Gly Gly Leu Ser Gly Lys Leu Thr Phe Gly Glu Gly Thr Gln
1               5                   10                  15
Val Thr Val Ile Ser
                20


<210> 339
<211> 15
<212> PRT
<213> Unknown

<220>
<223> Mouse

<400> 339
Asn Thr Glu Val Phe Phe Gly Lys Gly Thr Arg Leu Thr Val Val
1               5                   10                  15


<210> 340
<211> 15
<212> PRT
<213> Unknown

<220>
<223> Mouse

<400> 340
Asn Ser Asp Tyr Thr Phe Gly Ser Gly Thr Arg Leu Leu Val Ile
1               5                   10                  15


<210> 341
<211> 16
<212> PRT
<213> Unknown

<220>
<223> Mouse
```

<400> 341
Ser Gly Asn Thr Leu Tyr Phe Gly Glu Gly Ser Arg Leu Ile Val Val
1               5                   10                  15

<210> 342
<211> 16
<212> PRT
<213> Unknown

<220>
<223> Mouse

<400> 342
Ser Asn Glu Arg Leu Phe Phe Gly His Gly Thr Lys Leu Ser Val Leu
1               5                   10                  15

<210> 343
<211> 16
<212> PRT
<213> Unknown

<220>
<223> Mouse

<400> 343
Asn Asn Gln Ala Pro Leu Phe Gly Glu Gly Thr Arg Leu Ser Val Leu
1               5                   10                  15

<210> 344
<211> 17
<212> PRT
<213> Unknown

<220>
<223> Mouse

<400> 344
Ser Tyr Asn Ser Pro Leu Tyr Phe Ala Ala Gly Thr Arg Leu Thr Val
1               5                   10                  15
Thr

<210> 345
<211> 15
<212> PRT
<213> Unknown

<220>
<223> Mouse

<400> 345
Pro Val Leu Asp Asp His Gly Leu Gly Lys Glu Leu Arg Tyr Lys
1               5                   10                  15

```
<210> 346
<211> 16
<212> PRT
<213> Unknown

<220>
<223> Mouse

<400> 346
Asn Tyr Ala Glu Gln Phe Phe Gly Pro Gly Thr Arg Leu Thr Val Leu
 1               5                   10                  15


<210> 347
<211> 16
<212> PRT
<213> Unknown

<220>
<223> Mouse

<400> 347
Asn Thr Gly Gln Leu Tyr Phe Gly Glu Gly Ser Lys Leu Thr Val Leu
 1               5                   10                  15


<210> 348
<211> 16
<212> PRT
<213> Unknown

<220>
<223> Mouse

<400> 348
Ser Ala Glu Thr Leu Tyr Phe Gly Ser Gly Thr Arg Leu Thr Val Leu
 1               5                   10                  15


<210> 349
<211> 16
<212> PRT
<213> Unknown

<220>
<223> Mouse

<400> 349
Ser Gln Asn Thr Leu Tyr Phe Gly Ala Gly Thr Arg Leu Ser Val Leu
 1               5                   10                  15


<210> 350
<211> 16
<212> PRT
<213> Unknown
```

EP 2 441 838 A2

```
<220>
<223> Mouse

<400> 350
Asn Gln Asp Thr Gln Tyr Phe Gly Pro Gly Thr Arg Leu Leu Val Leu
1               5                   10                  15


<210> 351
<211> 15
<212> PRT
<213> Unknown

<220>
<223> Mouse

<400> 351
Ser Tyr Glu Gln Tyr Phe Gly Pro Gly Thr Arg Leu Thr Val Leu
1               5                   10                  15


<210> 352
<211> 16
<212> PRT
<213> Unknown

<220>
<223> Mouse

<400> 352
Thr Asp Lys Leu Val Phe Gly Gln Gly Thr Gln Val Thr Val Glu Pro
1               5                   10                  15


<210> 353
<211> 19
<212> PRT
<213> Unknown

<220>
<223> Mouse

<400> 353
Ser Trp Asp Thr Arg Gln Met Phe Phe Gly Thr Gly Ile Glu Leu Phe
1               5                   10                  15
Val Glu Pro


<210> 354
<211> 52
<212> PRT
<213> Unknown

<220>
<223> Camelid
```

<400> 354
Gln Val Gln Leu Gln Asp Ser Gly Gly Gly Leu Val Gln Ala Gly Gly
1               5                   10                  15
Ser Leu Arg Leu Ser Cys Ala Val Ser Gly Arg Thr Phe Ser Ala His
            20                  25                  30
Ser Val Tyr Thr Met Gly Trp Phe Arg Gln Ala Pro Gly Lys Glu Arg
            35                  40                  45
Glu Phe Val Ala
            50


<210> 355
<211> 54
<212> PRT
<213> Unknown

<220>
<223> Camelid

<400> 355
Gln Val Gln Leu Gln Glu Ser Gly Gly Gly Leu Val Gln Pro Gly Gly
1               5                   10                  15
Ser Leu Arg Leu Ser Cys Ala Ala Ser Gly Arg Thr Phe Ser Asp His
            20                  25                  30
Ser Gly Tyr Thr Tyr Thr Ile Gly Trp Phe Arg Gln Ala Pro Gly Lys
            35                  40                  45
Glu Arg Glu Phe Val Ala
            50


<210> 356
<211> 49
<212> PRT
<213> Unknown

<220>
<223> Camelid

<400> 356
Gln Val Gln Leu Gln Glu Ser Gly Gly Gly Leu Val Gln Pro Gly Gly
1               5                   10                  15
Ser Leu Arg Leu Ser Cys Ala Thr Ser Gly Phe Asp Phe Ser Val Ser
            20                  25                  30
Trp Met Tyr Trp Val Arg Gln Ala Pro Gly Lys Gly Leu Glu Trp Val
            35                  40                  45
Ser


<210> 357
<211> 49
<212> PRT
<213> Unknown

<220>
<223> Camelid

<400> 357

```
Gln Val Gln Leu Gln Glu Ser Gly Gly Gly Leu Val Gln Pro Gly Gly
1               5                   10                  15
Ser Leu Arg Leu Ser Cys Ala Thr Ser Gly Phe Thr Phe Ser Asp Tyr
            20                  25                  30
Trp Met Tyr Trp Val Arg Gln Ala Pro Gly Lys Gly Leu Glu Trp Val
        35                  40                  45
Ser
```

```
<210> 358
<211> 49
<212> PRT
<213> Unknown

<220>
<223> Camelid

<400> 358
Gln Val Gln Leu Gln Glu Ser Gly Gly Gly Leu Val Gln Pro Gly Gly
1               5                   10                  15
Ser Leu Arg Leu Ser Cys Ala Ala Ser Gly Phe Glu Phe Glu Asn His
            20                  25                  30
Trp Met Tyr Trp Val Arg Gln Ala Pro Gly Lys Gly Leu Glu Trp Val
        35                  40                  45
Ser
```

```
<210> 359
<211> 49
<212> PRT
<213> Unknown

<220>
<223> Camelid

<400> 359
Gln Val Gln Leu Gln Glu Ser Gly Gly Gly Leu Val Gln Pro Gly Gly
1               5                   10                  15
Ser Leu Arg Leu Ser Cys Ala Ala Ser Gly Ser Ile Phe Arg Val Asn
            20                  25                  30
Ala Met Gly Trp Tyr Arg Gln Val Pro Gly Asn Gln Arg Glu Phe Val
        35                  40                  45
Ala
```

```
<210> 360
<211> 49
<212> PRT
<213> Unknown

<220>
<223> Camelid

<400> 360
Arg Ile Tyr Trp Ser Ser Ala Asn Thr Tyr Tyr Ala Asp Ser Val Lys
```

```
                1               5                       10                      15
                Gly Arg Phe Thr Ile Ser Arg Asp Asn Ala Lys Asn Thr Val Asp Leu
                            20                      25                      30
                Leu Met Asn Ser Leu Lys Pro Glu Asp Thr Ala Val Tyr Tyr Cys Ala
                            35                      40                      45
                Ala
```

```
                <210> 361
                <211> 49
                <212> PRT
                <213> Unknown

                <220>
                <223> Camelid

                <400> 361
                Arg Ile Tyr Trp Ser Ser Gly Asn Thr Tyr Tyr Ala Asp Ser Val Lys
                1               5                       10                      15
                Gly Arg Phe Ala Ile Ser Arg Asp Ile Ala Lys Asn Thr Val Asp Leu
                            20                      25                      30
                Thr Met Asn Asn Leu Glu Pro Glu Asp Thr Ala Val Tyr Tyr Cys Ala
                            35                      40                      45
                Ala
```

```
                <210> 362
                <211> 49
                <212> PRT
                <213> Unknown

                <220>
                <223> Camelid

                <400> 362
                Glu Ile Asn Thr Asn Gly Leu Ile Thr Lys Tyr Val Asp Ser Val Lys
                1               5                       10                      15
                Gly Arg Phe Thr Ile Ser Arg Asp Asn Ala Lys Asn Thr Leu Tyr Leu
                            20                      25                      30
                Gln Met Asp Ser Leu Ile Pro Glu Asp Thr Ala Leu Tyr Tyr Cys Ala
                            35                      40                      45
                Arg
```

```
                <210> 363
                <211> 49
                <212> PRT
                <213> Unknown

                <220>
                <223> Camelid

                <400> 363
                Thr Val Asn Thr Asn Gly Leu Ile Thr Arg Tyr Ala Asp Ser Val Lys
                1               5                       10                      15
```

```
Gly Arg Phe Thr Ile Ser Arg Asp Asn Ala Lys Tyr Thr Leu Tyr Leu
            20                  25                  30
Gln Met Asn Ser Leu Lys Ser Glu Asp Thr Ala Val Tyr Tyr Cys Thr
            35                  40                  45
Lys
```

```
<210> 364
<211> 49
<212> PRT
<213> Unknown

<220>
<223> Camelid

<400> 364
Thr Val Asn Thr Asn Gly Leu Ile Thr Arg Tyr Ala Asp Ser Val Lys
1               5                   10                  15
Gly Arg Phe Thr Ile Ser Arg Asp Asn Ala Lys Tyr Thr Leu Tyr Leu
            20                  25                  30
Gln Met Asn Ser Leu Lys Ser Glu Asp Thr Ala Val Tyr Tyr Cys Thr
            35                  40                  45
Lys
```

```
<210> 365
<211> 48
<212> PRT
<213> Unknown

<220>
<223> Camelid

<400> 365
Ile Ile Thr Ser Gly Asp Asn Leu Asn Tyr Ala Asp Ala Val Lys Gly
1               5                   10                  15
Arg Phe Thr Ile Ser Thr Asp Asn Val Lys Lys Thr Val Tyr Leu Gln
            20                  25                  30
Met Asn Val Leu Lys Pro Glu Asp Thr Ala Val Tyr Tyr Cys Asn Ala
            35                  40                  45
```

```
<210> 366
<211> 35
<212> PRT
<213> Unknown

<220>
<223> Camelid

<400> 366
Arg Asp Gly Ile Pro Thr Ser Arg Thr Val Gly Ser Tyr Asn Tyr Trp
1               5                   10                  15
Gly Gln Gly Thr Gln Val Thr Val Ser Ser Glu Pro Lys Thr Pro Lys
            20                  25                  30
Pro Gln Pro
```

35

<210> 367
<211> 35
<212> PRT
<213> Unknown

<220>
<223> Camelid

<400> 367
Arg Asp Gly Ile Pro Thr Ser Arg Ser Val Glu Ser Tyr Asn Tyr Trp
1               5                   10                  15
Gly Gln Gly Thr Gln Val Thr Val Ser Ser Glu Pro Lys Thr Pro Lys
                20                  25                  30
Pro Gln Pro
            35

<210> 368
<211> 26
<212> PRT
<213> Unknown

<220>
<223> Camelid

<400> 368
Ser Pro Ser Gly Ser Phe Arg Gly Gln Gly Thr Gln Val Thr Val Ser
1               5                   10                  15
Ser Glu Pro Lys Thr Pro Lys Pro Gln Pro
                20                  25

<210> 369
<211> 34
<212> PRT
<213> Unknown

<220>
<223> Camelid

<400> 369
Val Val Pro Pro Tyr Ser Asp Asp Ser Arg Thr Asn Ala Asp Trp Gly
1               5                   10                  15
Gln Gly Thr Gln Val Thr Val Ser Ser Glu Pro Lys Thr Pro Lys Pro
                20                  25                  30
Gln Pro

<210> 370
<211> 34
<212> PRT
<213> Unknown

<220>

<223> Camelid

<400> 370
Val Leu Pro Pro Tyr Ser Asp Asp Ser Arg Thr Asn Ala Asp Trp Gly
1               5                   10                  15
Gln Gly Thr Gln Val Thr Val Ser Ser Glu Pro Lys Thr Pro Lys Pro
            20                  25                  30
Gln Pro


<210> 371
<211> 33
<212> PRT
<213> Unknown

<220>
<223> Camelid

<400> 371
Ile Leu Gln Thr Ser Arg Trp Ser Ile Pro Ser Asn Tyr Trp Gly Gln
1               5                   10                  15
Gly Thr Gln Val Thr Val Ser Ser Glu Pro Lys Thr Pro Lys Pro Gln
            20                  25                  30
Pro


<210> 372
<211> 50
<212> PRT
<213> Unknown

<220>
<223> Camelid

<400> 372
Gln Val Gln Leu Gln Asp Ser Gly Gly Gly Leu Val Gln Ala Gly Gly
1               5                   10                  15
Ser Leu Arg Leu Ser Cys Ala Ala Ser Gly Gly Thr Phe Ser Ser Ile
            20                  25                  30
Ile Met Ala Trp Phe Arg Gln Ala Pro Gly Lys Glu Arg Glu Phe Val
            35                  40                  45
Gly Ala
    50


<210> 373
<211> 50
<212> PRT
<213> Unknown

<220>
<223> Camelid

<400> 373
Gln Val Gln Leu Gln Asp Ser Gly Gly Gly Leu Val Gln Ala Gly Gly
1               5                   10                  15

```
Ser Leu Arg Leu Ser Cys Gly Val Ser Gly Leu Ser Phe Ser Gly Tyr
            20                  25                  30
Thr Met Gly Trp Phe Arg Gln Ala Pro Gly Lys Glu Arg Glu Phe Ala
            35                  40                  45
Ala Ala
    50


<210> 374
<211> 50
<212> PRT
<213> Unknown

<220>
<223> Camelid

<400> 374
Glu Val Gln Leu Val Glu Ser Gly Gly Gly Leu Val Gln Ala Gly Gly
 1               5                  10                  15
Ser Leu Arg Leu Ser Cys Ala Ala Ser Gly Gly Thr Leu Ser Ser Tyr
            20                  25                  30
Ile Thr Gly Trp Phe Arg Gln Ala Pro Gly Lys Glu Arg Glu Phe Val
            35                  40                  45
Gly Ala
    50


<210> 375
<211> 50
<212> PRT
<213> Unknown

<220>
<223> Camelid

<400> 375
Gln Val Gln Leu Val Glu Ser Gly Gly Gly Leu Val Gln Ala Gly Gly
 1               5                  10                  15
Ser Leu Arg Leu Ser Cys Ala Ala Ser Glu Gly Thr Leu Ser Gly Tyr
            20                  25                  30
Ile Leu Gly Trp Phe Arg Gln Ala Pro Gly Lys Glu Arg Glu Phe Val
            35                  40                  45
Gly Ala
    50


<210> 376
<211> 48
<212> PRT
<213> Unknown

<220>
<223> Camelid

<400> 376
Val Ser Trp Ser Gly Gly Thr Thr Val Tyr Ala Asp Ser Val Leu Gly
 1               5                  10                  15
Arg Phe Glu Ile Ser Arg Asp Ser Ala Arg Lys Ser Val Tyr Leu Gln
```

163

```
              20                  25                  30
Met Asn Ser Leu Lys Pro Glu Asp Thr Ala Val Tyr Tyr Cys Ala Ala
        35                  40                  45
```

<210> 377
<211> 48
<212> PRT
<213> Unknown

<220>
<223> Camelid

<400> 377
```
Ile Gly Trp Asn Ser Gly Thr Thr Glu Tyr Arg Asn Ser Val Lys Gly
 1               5                  10                  15
Arg Phe Thr Ile Ser Arg Asp Asn Ala Lys Asn Thr Val Tyr Leu Gln
            20                  25                  30
Met Asn Ser Leu Lys Pro Glu Asp Thr Ala Val Tyr Tyr Cys Ala Ala
        35                  40                  45
```

<210> 378
<211> 48
<212> PRT
<213> Unknown

<220>
<223> Camelid

<400> 378
```
Val Ser Trp Ser Ser Ser Thr Ile Val Tyr Ala Asp Ser Val Glu Gly
 1               5                  10                  15
Arg Phe Thr Ile Ser Arg Asp Asn His Gln Asn Thr Val Tyr Leu Gln
            20                  25                  30
Met Asp Ser Leu Lys Pro Glu Asp Thr Ala Val Tyr Tyr Cys Ala Ala
        35                  40                  45
```

<210> 379
<211> 48
<212> PRT
<213> Unknown

<220>
<223> Camelid

<400> 379
```
Val Ser Trp Ser Gly Gly Thr Ile Val Tyr Ala Asp Ser Val Lys Gly
 1               5                  10                  15
Arg Phe Glu Ile Ser Arg Asp Asn Ala Arg Asn Thr Val Tyr Leu Gln
            20                  25                  30
Met Asp Ser Leu Lys Ser Glu Asp Thr Ala Val Tyr Tyr Cys Ala Ala
        35                  40                  45
```

<210> 380
<211> 35

```
<212> PRT
<213> Unknown

<220>
<223> Camelid

<400> 380
Arg Pro Tyr Gln Lys Tyr Asn Trp Ala Ser Ala Ser Tyr Asn Val Trp
1               5                   10                  15
Gly Gln Gly Thr Gln Val Thr Val Ser Ser Glu Pro Lys Thr Pro Lys
            20                  25                  30
Pro Gln Pro
        35


<210> 381
<211> 34
<212> PRT
<213> Unknown

<220>
<223> Camelid

<400> 381
Ser Pro Lys Tyr Met Thr Ala Tyr Glu Arg Ser Tyr Asp Phe Trp Gly
1               5                   10                  15
Gln Gly Thr Gln Val Thr Val Ser Ser Glu Pro Lys Thr Pro Lys Pro
            20                  25                  30
Gln Pro


<210> 382
<211> 26
<212> PRT
<213> Unknown

<220>
<223> Camelid

<400> 382
Arg Pro Tyr Gln Lys Tyr Asn Trp Ala Ser Ala Ser Tyr Asn Val Trp
1               5                   10                  15
Gly Gln Gly Thr Gln Val Thr Val Ser Ser
            20                  25


<210> 383
<211> 26
<212> PRT
<213> Unknown

<220>
<223> Camelid

<400> 383
Arg Pro Tyr Gln Arg Phe Asn Trp Ala Ser Ala Ser Tyr Asn Val Trp
1               5                   10                  15
```

```
Gly Arg Gly Thr Gln Val Thr Val Ser Ser
            20                  25


<210> 384
<211> 98
<212> PRT
<213> Homo sapiens

<400> 384
Glu Val Gln Leu Leu Glu Ser Gly Gly Gly Leu Val Gln Pro Gly Gly
 1               5                  10                  15
Ser Leu Arg Leu Ser Cys Ala Ala Ser Gly Phe Thr Phe Ser Ser Tyr
            20                  25                  30
Ala Met Ser Trp Val Arg Gln Ala Pro Gly Lys Gly Leu Glu Trp Val
        35                  40                  45
Ser Ala Ile Ser Gly Ser Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys


<210> 385
<211> 123
<212> PRT
<213> Unknown

<220>
<223> Camelid

<400> 385
Gln Val Gln Leu Gln Glu Ser Gly Gly Gly Leu Val Gln Pro Gly Gly
 1               5                  10                  15
Ser Leu Arg Leu Ser Cys Ala Ala Ser Gly Phe Glu Phe Glu Asn His
            20                  25                  30
Trp Met Tyr Trp Val Arg Gln Ala Pro Gly Lys Gly Leu Glu Trp Val
        35                  40                  45
Ser Thr Val Asn Thr Asn Gly Leu Ile Thr Arg Tyr Ala Asp Ser Val
    50                  55                  60
Lys Gly Arg Phe Thr Ile Ser Arg Asp Asn Ala Lys Tyr Thr Leu Tyr
65                  70                  75                  80
Leu Gln Met Asn Ser Leu Lys Ser Glu Asp Thr Ala Val Tyr Tyr Cys
                85                  90                  95
Thr Lys Val Leu Pro Pro Tyr Ser Asp Asp Ser Arg Thr Asn Ala Asp
            100                 105                 110
Trp Gly Gln Gly Thr Gln Val Thr Val Ser Ser
            115                 120


<210> 386
<211> 4
<212> PRT
<213> Unknown
```

```
<220>
<223> Conserved motif in antibody FR4

<220>
<221> VARIANT
<222> 2
<223> Xaa = Any Amino Acid

<400> 386
Gly Xaa Gly Thr
 1


<210> 387
<211> 6
<212> PRT
<213> Unknown

<220>
<223> Conserved motif in antibody FR4

<220>
<221> VARIANT
<222> 2, 5
<223> Xaa = Any Amino Acid

<220>
<221> VARIANT
<222> 6
<223> Xaa = Val or Leu

<400> 387
Gly Xaa Gly Thr Xaa Xaa
 1               5


<210> 388
<211> 4
<212> PRT
<213> Unknown

<220>
<223> Conserved motif in antibody FR3

<400> 388
Glu Asp Thr Ala
 1


<210> 389
<211> 4
<212> PRT
<213> Unknown

<220>
<223> Conserved motif in antibody FR3

<400> 389
```

```
Val Tyr Tyr Cys
  1


<210> 390
<211> 8
<212> PRT
<213> Unknown

<220>
<223> Conserved motif in antibody FR3

<400> 390
Glu Asp Thr Ala Val Tyr Tyr Cys
  1               5


<210> 391
<211> 4
<212> PRT
<213> Unknown

<220>
<223> Conserved motif in antibody constant region

<220>
<221> VARIANT
<222> 1
<223> Xaa = Ser, Ala or Gly

<220>
<221> VARIANT
<222> 3
<223> Xaa = Lys, Asp or Ser

<400> 391
Xaa Pro Xaa Val
  1


<210> 392
<211> 5
<212> PRT
<213> Unknown

<220>
<223> Conserved motif in antibody constant region

<220>
<221> VARIANT
<222> 1
<223> Xaa = Ser, Ala or Gly

<220>
<221> VARIANT
<222> 3
<223> Xaa = Lys, Asp or Ser
```

```
<400> 392
Xaa Pro Xaa Val Phe
 1               5


<210> 393
<211> 5
<212> PRT
<213> Unknown

<220>
<223> Conserved motif in antibody constant region

<220>
<221> VARIANT
<222> 5
<223> Xaa = Ser, Arg or Thr

<400> 393
Lys Val Asp Lys Xaa
 1               5


<210> 394
<211> 3
<212> PRT
<213> Unknown

<220>
<223> Conserved motif in antibody constant region

<400> 394
Val Thr Val
 1


<210> 395
<211> 6
<212> PRT
<213> Unknown

<220>
<223> Conserved motif

<220>
<221> VARIANT
<222> 2, 5
<223> Xaa = Any Amino Acid

<400> 395
Gly Xaa Gly Thr Xaa Val
 1               5


<210> 396
<211> 6
<212> PRT
<213> Unknown
```

```
<220>
<223> Conserved motif

<220>
<221> VARIANT
<222> 2, 5
<223> Xaa = Any Amino Acid

<400> 396
Gly Xaa Gly Thr Xaa Leu
 1               5


<210> 397
<211> 4
<212> PRT
<213> Unknown

<220>
<223> Conserved motif

<400> 397
Pro Ser Val Phe
 1


<210> 398
<211> 4
<212> PRT
<213> Unknown

<220>
<223> Conserved motif

<400> 398
Ser Pro Lys Val
 1


<210> 399
<211> 4
<212> PRT
<213> Unknown

<220>
<223> Conserved motif

<400> 399
Ser Pro Asp Val
 1


<210> 400
<211> 4
<212> PRT
<213> Unknown
```

```
<220>
<223> Conserved motif

<400> 400
Ser Pro Ser Val
 1


<210> 401
<211> 4
<212> PRT
<213> Unknown

<220>
<223> Conserved motif

<400> 401
Ala Pro Lys Val
 1


<210> 402
<211> 4
<212> PRT
<213> Unknown

<220>
<223> Conserved motif

<400> 402
Ala Pro Asp Val
 1


<210> 403
<211> 4
<212> PRT
<213> Unknown

<220>
<223> Conserved motif

<400> 403
Ala Pro Ser Val
 1


<210> 404
<211> 4
<212> PRT
<213> Unknown

<220>
<223> Conserved motif

<400> 404
Gly Pro Lys Val
 1
```

```
<210> 405
<211> 4
<212> PRT
<213> Unknown

<220>
<223> Conserved motif

<400> 405
Gly Pro Asp Val
 1


<210> 406
<211> 4
<212> PRT
<213> Unknown

<220>
<223> Conserved motif

<400> 406
Gly Pro Ser Val
 1


<210> 407
<211> 5
<212> PRT
<213> Unknown

<220>
<223> Conserved motif

<400> 407
Ser Pro Lys Val Phe
 1                5


<210> 408
<211> 5
<212> PRT
<213> Unknown

<220>
<223> Conserved motif

<400> 408
Ser Pro Asp Val Phe
 1                5


<210> 409
<211> 5
<212> PRT
<213> Unknown
```

```
<220>
<223> Conserved motif

<400> 409
Ser Pro Ser Val Phe
 1                   5


<210> 410
<211> 5
<212> PRT
<213> Unknown

<220>
<223> Conserved motif

<400> 410
Ala Pro Lys Val Phe
 1                   5


<210> 411
<211> 5
<212> PRT
<213> Unknown

<220>
<223> Conserved motif

<400> 411
Ala Pro Asp Val Phe
 1                   5


<210> 412
<211> 5
<212> PRT
<213> Unknown

<220>
<223> Conserved motif

<400> 412
Ala Pro Ser Val Phe
 1                   5


<210> 413
<211> 5
<212> PRT
<213> Unknown

<220>
<223> Conserved motif

<400> 413
Gly Pro Lys Val Phe
```

```
            1                 5
```

```
<210> 414
<211> 5
<212> PRT
<213> Unknown

<220>
<223> Conserved motif

<400> 414
Gly Pro Asp Val Phe
 1                 5


<210> 415
<211> 5
<212> PRT
<213> Unknown

<220>
<223> Conserved motif

<400> 415
Gly Pro Ser Val Phe
 1                 5


<210> 416
<211> 5
<212> PRT
<213> Unknown

<220>
<223> Conserved motif

<400> 416
Lys Val Asp Lys Ser
 1                 5


<210> 417
<211> 5
<212> PRT
<213> Unknown

<220>
<223> Conserved motif

<400> 417
Lys Val Asp Lys Arg
 1                 5


<210> 418
<211> 5
<212> PRT
```

```
<213> Unknown

<220>
<223> Conserved motif

<400> 418
Lys Val Asp Lys Thr
 1               5


<210> 419
<211> 4
<212> PRT
<213> Unknown

<220>
<223> antibody FR

<400> 419
Thr Val Ser Ser
 1


<210> 420
<211> 6
<212> PRT
<213> Unknown

<220>
<223> antibody FR

<220>
<221> VARIANT
<222> 1
<223> Xaa = Leu, Met or Thr

<400> 420
Xaa Val Thr Val Ser Ser
 1               5


<210> 421
<211> 6
<212> PRT
<213> Unknown

<220>
<223> antibody FR

<400> 421
Leu Val Thr Val Ser Ser
 1               5


<210> 422
<211> 6
<212> PRT
<213> Unknown
```

```
<220>
<223> antibody FR

<400> 422
Met Val Thr Val Ser Ser
 1               5


<210> 423
<211> 6
<212> PRT
<213> Unknown

<220>
<223> antibody FR

<400> 423
Thr Val Thr Val Ser Ser
 1               5


<210> 424
<211> 5
<212> PRT
<213> Unknown

<220>
<223> antibody FR

<220>
<221> VARIANT
<222> 1
<223> Xaa = Lys or Arg

<220>
<221> VARIANT
<222> 2
<223> Xaa = Val or Leu

<220>
<221> VARIANT
<222> 3
<223> Xaa = Glu or Asp

<400> 424
Xaa Xaa Xaa Ile Lys
 1               5


<210> 425
<211> 5
<212> PRT
<213> Unknown

<220>
<223> antibody FR
```

```
<220>
<221> VARIANT
<222> 1
<223> Xaa = Lys, Gln or Glu

<220>
<221> VARIANT
<222> 2
<223> Xaa = Val or Leu

<220>
<221> VARIANT
<222> 3
<223> Xaa = Thr or Ile

<220>
<221> VARIANT
<222> 4
<223> Xaa = Val or Ile

<400> 425
Xaa Xaa Xaa Xaa Leu
 1               5


<210> 426
<211> 5
<212> PRT
<213> Unknown

<220>
<223> antibody FR

<400> 426
Lys Val Glu Ile Lys
 1               5


<210> 427
<211> 5
<212> PRT
<213> Unknown

<220>
<223> antibody FR

<400> 427
Lys Val Asp Ile Lys
 1               5


<210> 428
<211> 5
<212> PRT
<213> Unknown

<220>
<223> antibody FR
```

177

```
<400> 428
Lys Leu Glu Ile Lys
 1               5


<210> 429
<211> 5
<212> PRT
<213> Unknown

<220>
<223> antibody FR

<400> 429
Lys Leu Asp Ile Lys
 1               5


<210> 430
<211> 5
<212> PRT
<213> Unknown

<220>
<223> antibody FR

<400> 430
Arg Val Glu Ile Lys
 1               5


<210> 431
<211> 5
<212> PRT
<213> Unknown

<220>
<223> antibody FR

<400> 431
Arg Val Asp Ile Lys
 1               5


<210> 432
<211> 5
<212> PRT
<213> Unknown

<220>
<223> antibody FR

<400> 432
Arg Leu Glu Ile Lys
 1               5
```

```
<210> 433
<211> 5
<212> PRT
<213> Unknown

<220>
<223> antibody FR

<400> 433
Arg Leu Asp Ile Lys
 1               5


<210> 434
<211> 5
<212> PRT
<213> Unknown

<220>
<223> antibody FR

<400> 434
Lys Val Thr Val Leu
 1               5


<210> 435
<211> 5
<212> PRT
<213> Unknown

<220>
<223> antibody FR

<400> 435
Lys Val Thr Ile Leu
 1               5


<210> 436
<211> 5
<212> PRT
<213> Unknown

<220>
<223> antibody FR

<400> 436
Lys Val Ile Val Leu
 1               5


<210> 437
<211> 5
<212> PRT
<213> Unknown

<220>
```

```
<223> antibody FR

<400> 437
Lys Val Ile Ile Leu
 1               5


<210> 438
<211> 5
<212> PRT
<213> Unknown

<220>
<223> antibody FR

<400> 438
Lys Leu Thr Val Leu
 1               5


<210> 439
<211> 5
<212> PRT
<213> Unknown

<220>
<223> antibody FR

<400> 439
Lys Leu Thr Ile Leu
 1               5


<210> 440
<211> 5
<212> PRT
<213> Unknown

<220>
<223> antibody FR

<400> 440
Lys Leu Ile Val Leu
 1               5


<210> 441
<211> 5
<212> PRT
<213> Unknown

<220>
<223> antibody FR

<400> 441
Lys Leu Ile Ile Leu
 1               5
```

```
<210> 442
<211> 5
<212> PRT
<213> Unknown

<220>
<223> antibody FR

<400> 442
Gln Val Thr Val Leu
 1               5


<210> 443
<211> 5
<212> PRT
<213> Unknown

<220>
<223> antibody FR

<400> 443
Gln Val Thr Ile Leu
 1               5


<210> 444
<211> 5
<212> PRT
<213> Unknown

<220>
<223> antibody FR

<400> 444
Gln Val Ile Val Leu
 1               5


<210> 445
<211> 5
<212> PRT
<213> Unknown

<220>
<223> antibody FR

<400> 445
Gln Val Ile Ile Leu
 1               5


<210> 446
<211> 5
<212> PRT
<213> Unknown
```

EP 2 441 838 A2

```
<220>
<223> antibody FR

<400> 446
Gln Leu Thr Val Leu
 1               5


<210> 447
<211> 5
<212> PRT
<213> Unknown

<220>
<223> antibody FR

<400> 447
Gln Leu Thr Ile Leu
 1               5


<210> 448
<211> 5
<212> PRT
<213> Unknown

<220>
<223> antibody FR

<400> 448
Gln Leu Ile Val Leu
 1               5


<210> 449
<211> 5
<212> PRT
<213> Unknown

<220>
<223> antibody FR

<400> 449
Gln Leu Ile Ile Leu
 1               5


<210> 450
<211> 5
<212> PRT
<213> Unknown

<220>
<223> antibody FR

<400> 450
Glu Val Thr Val Leu
 1               5
```

182

```
<210> 451
<211> 5
<212> PRT
<213> Unknown

<220>
<223> antibody FR

<400> 451
Glu Val Thr Ile Leu
 1               5


<210> 452
<211> 5
<212> PRT
<213> Unknown

<220>
<223> antibody FR

<400> 452
Glu Val Ile Val Leu
 1               5


<210> 453
<211> 5
<212> PRT
<213> Unknown

<220>
<223> antibody FR

<400> 453
Glu Val Ile Ile Leu
 1               5


<210> 454
<211> 5
<212> PRT
<213> Unknown

<220>
<223> antibody FR

<400> 454
Glu Leu Thr Val Leu
 1               5


<210> 455
<211> 5
<212> PRT
<213> Unknown
```

```
<220>
<223> antibody FR

<400> 455
Glu Leu Thr Ile Leu
 1               5


<210> 456
<211> 5
<212> PRT
<213> Unknown

<220>
<223> antibody FR

<400> 456
Glu Leu Ile Val Leu
 1               5


<210> 457
<211> 5
<212> PRT
<213> Unknown

<220>
<223> antibody FR

<400> 457
Glu Leu Ile Ile Leu
 1               5


<210> 458
<211> 3
<212> PRT
<213> Unknown

<220>
<223> antibody FR

<220>
<221> VARIANT
<222> 1
<223> Xaa = Glu or Asp

<400> 458
Xaa Ile Lys
 1


<210> 459
<211> 3
<212> PRT
<213> Unknown
```

```
<220>
<223> antibody FR

<220>
<221> VARIANT
<222> 1
<223> Xaa = Thr or Ile

<220>
<221> VARIANT
<222> 2
<223> Xaa = Val or Ile

<400> 459
Xaa Xaa Leu
 1


<210> 460
<211> 3
<212> PRT
<213> Unknown

<220>
<223> antibody FR

<400> 460
Glu Ile Lys
 1


<210> 461
<211> 3
<212> PRT
<213> Unknown

<220>
<223> antibody FR

<400> 461
Asp Ile Lys
 1


<210> 462
<211> 3
<212> PRT
<213> Unknown

<220>
<223> antibody FR

<400> 462
Thr Val Leu
 1


<210> 463
```

```
<211> 3
<212> PRT
<213> Unknown

<220>
<223> antibody FR

<400> 463
Thr Ile Leu
 1


<210> 464
<211> 3
<212> PRT
<213> Unknown

<220>
<223> antibody FR

<400> 464
Ile Val Leu
 1


<210> 465
<211> 3
<212> PRT
<213> Unknown

<220>
<223> antibody FR

<400> 465
Ile Ile Leu
 1


<210> 466
<211> 4
<212> PRT
<213> Unknown

<220>
<223> Lamda constant domain

<400> 466
Gln Pro Lys Ala
 1


<210> 467
<211> 3
<212> PRT
<213> Unknown

<220>
<223> Kappa constant domain
```

```
<400> 467
Thr Val Ala
 1


<210> 468
<211> 4
<212> PRT
<213> Unknown

<220>
<223> Conserved motif

<220>
<221> VARIANT
<222> 1
<223> Xaa = Ala or Gly

<400> 468
Xaa Pro Ser Val
 1


<210> 469
<211> 3
<212> PRT
<213> Unknown

<220>
<223> CH1

<400> 469
Ser Thr Lys
 1


<210> 470
<211> 5
<212> PRT
<213> Unknown

<220>
<223> Kappa constant domain

<220>
<221> VARIANT
<222> 1
<223> Xaa = Ala or Gly

<400> 470
Xaa Pro Ser Val Phe
 1               5


<210> 471
<211> 6
<212> PRT
```

```
<213> Artificial Sequence

<220>
<223> Natural junction

<400> 471
Lys Val Glu Ile Lys Arg
 1               5


<210> 472
<211> 10
<212> PRT
<213> Unknown

<220>
<223> antibody FR4

<400> 472
Gly Gln Gly Thr Lys Val Thr Val Ser Ser
 1               5                   10


<210> 473
<211> 3
<212> PRT
<213> Unknown

<220>
<223> CH1

<400> 473
Ala Ser Thr
 1


<210> 474
<211> 7
<212> PRT
<213> Unknown

<220>
<223> CH1

<400> 474
Ala Ser Thr Lys Gly Pro Ser
 1               5


<210> 475
<211> 8
<212> PRT
<213> Unknown

<220>
<223> CH1

<400> 475
```

```
Ala Ser Thr Lys Gly Pro Ser Gly
 1               5


<210> 476
<211> 9
<212> PRT
<213> Unknown

<220>
<223> antibody FR4

<220>
<221> VARIANT
<222> 2
<223> Xaa = Any Amino Acid

<220>
<221> VARIANT
<222> 5
<223> Xaa = Lys, Gln or Glu

<220>
<221> VARIANT
<222> 6
<223> Xaa = Val or Leu

<220>
<221> VARIANT
<222> 7
<223> Xaa = Thr or Ile

<400> 476
Gly Xaa Gly Thr Xaa Xaa Xaa Val Leu
 1               5


<210> 477
<211> 10
<212> PRT
<213> Unknown

<220>
<223> antibody FR4

<400> 477
Gly Gln Gly Thr Lys Val Glu Ile Lys Arg
 1               5                   10


<210> 478
<211> 10
<212> PRT
<213> Unknown

<220>
<223> antibody FR4
```

```
<220>
<221> VARIANT
<222> 2
<223> Xaa = Any Amino Acid

<220>
<221> VARIANT
<222> 5
<223> Xaa = Leu, Met or Thr

<400> 478
Gly Xaa Gly Thr Xaa Val Thr Val Ser Ser
 1               5                   10


<210> 479
<211> 10
<212> PRT
<213> Unknown

<220>
<223> antibody FR4

<220>
<221> VARIANT
<222> 2
<223> Xaa = Any Amino Acid

<400> 479
Gly Xaa Gly Thr Leu Val Thr Val Ser Ser
 1               5                   10


<210> 480
<211> 10
<212> PRT
<213> Unknown

<220>
<223> antibody FR4

<220>
<221> VARIANT
<222> 2
<223> Xaa = Any Amino Acid

<400> 480
Gly Xaa Gly Thr Met Val Thr Val Ser Ser
 1               5                   10


<210> 481
<211> 10
<212> PRT
<213> Unknown

<220>
<223> antibody FR4
```

```
<220>
<221> VARIANT
<222> 2
<223> Xaa = Any Amino Acid

<400> 481
Gly Xaa Gly Thr Thr Val Thr Val Ser Ser
 1               5                   10


<210> 482
<211> 5
<212> PRT
<213> Unknown

<220>
<223> natural junction

<400> 482
Lys Val Thr Val Leu
 1               5


<210> 483
<211> 5
<212> PRT
<213> Unknown

<220>
<223> natural junction

<400> 483
Leu Val Thr Val Leu
 1               5


<210> 484
<211> 6
<212> PRT
<213> Unknown

<220>
<223> natural junction

<400> 484
Leu Val Thr Val Ser Ser
 1               5


<210> 485
<211> 10
<212> PRT
<213> Unknown

<220>
<223> antibody FR4
```

```
<220>
<221> VARIANT
<222> 2
<223> Xaa = any Amino Acid

<220>
<221> VARIANT
<222> 5
<223> Xaa = Lys or Arg

<220>
<221> VARIANT
<222> 6
<223> Xaa = Val or Leu

<220>
<221> VARIANT
<222> 7
<223> Xaa = Glu or Asp

<400> 485
Gly Xaa Gly Thr Xaa Xaa Xaa Ile Lys Arg
 1               5                   10


<210> 486
<211> 10
<212> PRT
<213> Unknown

<220>
<223> antibody FR4

<220>
<221> VARIANT
<222> 2
<223> Xaa = Any Amino Acid

<400> 486
Gly Xaa Gly Thr Lys Val Glu Ile Lys Arg
 1               5                   10


<210> 487
<211> 10
<212> PRT
<213> Unknown

<220>
<223> antibody FR4

<220>
<221> VARIANT
<222> 2
<223> Xaa = Any Amino Acid

<400> 487
Gly Xaa Gly Thr Lys Leu Glu Ile Lys Arg
```

```
         1                 5                      10
```

```
<210> 488
<211> 10
<212> PRT
<213> Unknown

<220>
<223> antibody FR4

<220>
<221> VARIANT
<222> 2
<223> Xaa = Any Amino Acid

<400> 488
Gly Xaa Gly Thr Lys Val Asp Ile Lys Arg
 1                 5                      10
```

```
<210> 489
<211> 10
<212> PRT
<213> Unknown

<220>
<223> antibody FR4

<220>
<221> VARIANT
<222> 2
<223> Xaa = Any Amino Acid

<400> 489
Gly Xaa Gly Thr Arg Lys Glu Ile Lys Arg
 1                 5                      10
```

```
<210> 490
<211> 6
<212> PRT
<213> Unknown

<220>
<223> Kappa constant domain

<400> 490
Thr Val Ala Ala Pro Ser
 1                 5
```

```
<210> 491
<211> 9
<212> PRT
<213> Unknown

<220>
```

```
<223> Kappa constant domain

<400> 491
Thr Val Ala Leu Ala Ala Pro Ser Gly
 1               5


<210> 492
<211> 9
<212> PRT
<213> Unknown

<220>
<223> antibody FR4

<220>
<221> VARIANT
<222> 2
<223> Xaa = Any Amino Acid

<220>
<221> VARIANT
<222> 5
<223> Xaa = Lys, Gln or Glu

<220>
<221> VARIANT
<222> 6
<223> Xaa = Val or Leu

<220>
<221> VARIANT
<222> 7
<223> Xaa = Thr or Ile


<220>
<221> VARIANT
<222> 8
<223> Xaa = Val or Ile

<400> 492
Gly Xaa Gly Thr Xaa Xaa Xaa Xaa Leu
 1               5


<210> 493
<211> 9
<212> PRT
<213> Unknown

<220>
<223> antibody FR4

<220>
<221> VARIANT
<222> 2
<223> Xaa = Any Amino Acid
```

```
<400> 493
Gly Xaa Gly Thr Lys Val Thr Val Leu
 1               5


<210> 494
<211> 9
<212> PRT
<213> Unknown

<220>
<223> antibody FR4

<220>
<221> VARIANT
<222> 2
<223> Xaa = Any Amino Acid

<400> 494
Gly Xaa Gly Thr Lys Val Thr Ile Leu
 1               5


<210> 495
<211> 9
<212> PRT
<213> Unknown

<220>
<223> antibody FR4

<220>
<221> VARIANT
<222> 2
<223> Xaa = Any Amino Acid

<400> 495
Gly Xaa Gly Thr Lys Val Ile Val Leu
 1               5


<210> 496
<211> 9
<212> PRT
<213> Unknown

<220>
<223> antibody FR4

<220>
<221> VARIANT
<222> 2
<223> Xaa = Any Amino Acid

<400> 496
Gly Xaa Gly Thr Lys Val Ile Ile Leu
 1               5
```

```
<210> 497
<211> 9
<212> PRT
<213> Unknown

<220>
<223> antibody FR4

<220>
<221> VARIANT
<222> 2
<223> Xaa = Any Amino Acid

<400> 497
Gly Xaa Gly Thr Lys Leu Thr Val Leu
 1               5


<210> 498
<211> 9
<212> PRT
<213> Unknown

<220>
<223> antibody FR4

<220>
<221> VARIANT
<222> 2
<223> Xaa = Any Amino Acid

<400> 498
Gly Xaa Gly Thr Lys Leu Thr Ile Leu
 1               5


<210> 499
<211> 9
<212> PRT
<213> Unknown

<220>
<223> antibody FR4

<220>
<221> VARIANT
<222> 2
<223> Xaa = Any Amino Acid

<400> 499
Gly Xaa Gly Thr Lys Leu Ile Val Leu
 1               5


<210> 500
<211> 9
```

```
<212> PRT
<213> Unknown

<220>
<223> antibody FR4

<220>
<221> VARIANT
<222> 2
<223> Xaa = Any Amino Acid

<400> 500
Gly Xaa Gly Thr Lys Leu Ile Ile Leu
 1               5


<210> 501
<211> 9
<212> PRT
<213> Unknown

<220>
<223> antibody FR4

<220>
<221> VARIANT
<222> 2
<223> Xaa = Any Amino Acid

<400> 501
Gly Xaa Gly Thr Gln Val Thr Val Leu
 1               5


<210> 502
<211> 9
<212> PRT
<213> Unknown

<220>
<223> antibody FR4

<220>
<221> VARIANT
<222> 2
<223> Xaa = Any Amino Acid

<400> 502
Gly Xaa Gly Thr Gln Val Thr Ile Leu
 1               5


<210> 503
<211> 9
<212> PRT
<213> Unknown

<220>
```

```
<223> antibody FR4

<220>
<221> VARIANT
<222> 2
<223> Xaa = Any Amino Acid

<400> 503
Gly Xaa Gly Thr Gln Val Ile Val Leu
 1               5


<210> 504
<211> 9
<212> PRT
<213> Unknown

<220>
<223> antibody FR4

<220>
<221> VARIANT
<222> 2
<223> Xaa = Any Amino Acid

<400> 504
Gly Xaa Gly Thr Gln Val Ile Ile Leu
 1               5


<210> 505
<211> 9
<212> PRT
<213> Unknown

<220>
<223> antibody FR4

<220>
<221> VARIANT
<222> 2
<223> Xaa = Any Amino Acid

<400> 505
Gly Xaa Gly Thr Gln Leu Thr Val Leu
 1               5


<210> 506
<211> 9
<212> PRT
<213> Unknown

<220>
<223> antibody FR4

<220>
<221> VARIANT
```

```
<222> 2
<223> Xaa = Any Amino Acid

<400> 506
Gly Xaa Gly Thr Gln Leu Thr Ile Leu
 1               5


<210> 507
<211> 9
<212> PRT
<213> Unknown

<220>
<223> antibody FR4

<220>
<221> VARIANT
<222> 2
<223> Xaa = Any Amino Acid

<400> 507
Gly Xaa Gly Thr Gln Leu Ile Val Leu
 1               5


<210> 508
<211> 9
<212> PRT
<213> Unknown

<220>
<223> antibody FR4

<220>
<221> VARIANT
<222> 2
<223> Xaa = Any Amino Acid

<400> 508
Gly Xaa Gly Thr Gln Leu Ile Ile Leu
 1               5


<210> 509
<211> 9
<212> PRT
<213> Unknown

<220>
<223> antibody FR4

<220>
<221> VARIANT
<222> 2
<223> Xaa = Any Amino Acid

<400> 509
```

```
Gly Xaa Gly Thr Glu Val Thr Val Leu
 1               5
```

```
<210> 510
<211> 9
<212> PRT
<213> Unknown

<220>
<223> antibody FR4

<220>
<221> VARIANT
<222> 2
<223> Xaa = Any Amino Acid

<400> 510
Gly Xaa Gly Thr Glu Val Thr Ile Leu
 1               5
```

```
<210> 511
<211> 9
<212> PRT
<213> Unknown

<220>
<223> antibody FR4

<220>
<221> VARIANT
<222> 2
<223> Xaa = Any Amino Acid

<400> 511
Gly Xaa Gly Thr Glu Val Ile Val Leu
 1               5
```

```
<210> 512
<211> 9
<212> PRT
<213> Unknown

<220>
<223> antibody FR4

<220>
<221> VARIANT
<222> 2
<223> Xaa = Any Amino Acid

<400> 512
Gly Xaa Gly Thr Glu Val Ile Ile Leu
 1               5
```

```
<210> 513
<211> 9
<212> PRT
<213> Unknown

<220>
<223> antibody FR4

<220>
<221> VARIANT
<222> 2
<223> Xaa = Any Amino Acid

<400> 513
Gly Xaa Gly Thr Glu Leu Thr Val Leu
 1               5


<210> 514
<211> 9
<212> PRT
<213> Unknown

<220>
<223> antibody FR4

<220>
<221> VARIANT
<222> 2
<223> Xaa = Any Amino Acid

<400> 514
Gly Xaa Gly Thr Glu Leu Thr Ile Leu
 1               5


<210> 515
<211> 9
<212> PRT
<213> Unkown

<220>
<223> antibody FR4

<220>
<221> VARIANT
<222> 2
<223> Xaa = Any Amino Acid

<400> 515
Gly Xaa Gly Thr Glu Leu Ile Val Leu
 1               5


<210> 516
<211> 9
<212> PRT
<213> Unknown
```

```
<220>
<223> antibody FR4

<220>
<221> VARIANT
<222> 2
<223> Xaa = Any Amino Acid

<400> 516
Gly Xaa Gly Thr Glu Leu Ile Ile Leu
 1               5


<210> 517
<211> 330
<212> PRT
<213> Unknown

<220>
<223> IgG1 CH

<400> 517
Ala Ser Thr Lys Gly Pro Ser Val Phe Pro Leu Ala Pro Ser Ser Lys
 1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
        50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
        130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
        210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
```

```
              275                    280                    285
       Leu Tyr Ser Lys Leu Thr Val Asp Lys Ser Arg Trp Gln Gln Gly Asn
              290                    295                    300
       Val Phe Ser Cys Ser Val Met His Glu Ala Leu His Asn His Tyr Thr
       305                    310                    315                    320
       Gln Lys Ser Leu Ser Leu Ser Pro Gly Lys
                            325                    330
```

```
       <210> 518
       <211> 106
       <212> PRT
       <213> Unknown

       <220>
       <223> CK

       <400> 518
       Thr Val Ala Ala Pro Ser Val Phe Ile Phe Pro Pro Ser Asp Glu Gln
        1                    5                   10                    15
       Leu Lys Ser Gly Thr Ala Ser Val Val Cys Leu Leu Asn Asn Phe Tyr
                     20                    25                    30
       Pro Arg Glu Ala Lys Val Gln Trp Lys Val Asp Asn Ala Leu Gln Ser
                     35                    40                    45
       Gly Asn Ser Gln Glu Ser Val Thr Glu Gln Asp Ser Lys Asp Ser Thr
                     50                    55                    60
       Tyr Ser Leu Ser Ser Thr Leu Thr Leu Ser Lys Ala Asp Tyr Glu Lys
       65                    70                    75                    80
       His Lys Val Tyr Ala Cys Glu Val Thr His Gln Gly Leu Ser Ser Pro
                            85                    90                    95
       Val Thr Lys Ser Phe Asn Arg Gly Glu Cys
                     100                   105
```

```
       <210> 519
       <211> 106
       <212> PRT
       <213> Unknown

       <220>
       <223> CL2

       <400> 519
       Gly Gln Pro Lys Ala Ala Pro Ser Val Thr Leu Phe Pro Pro Ser Ser
        1                    5                   10                    15
       Glu Glu Leu Gln Ala Asn Lys Ala Thr Leu Val Cys Leu Ile Ser Asp
                     20                    25                    30
       Phe Tyr Pro Gly Ala Val Thr Val Ala Trp Lys Ala Asp Ser Ser Pro
                     35                    40                    45
       Val Lys Ala Gly Val Glu Thr Thr Thr Pro Ser Lys Gln Ser Asn Asn
                     50                    55                    60
       Lys Tyr Ala Ala Ser Ser Tyr Leu Ser Leu Thr Pro Glu Gln Trp Lys
       65                    70                    75                    80
       Ser His Arg Ser Tyr Ser Cys Gln Val Thr His Glu Gly Ser Thr Val
                            85                    90                    95
       Glu Lys Thr Val Ala Pro Thr Glu Cys Ser
                     100                   105
```

```
<210> 520
<211> 8
<212> PRT
<213> Artificial Sequence

<220>
<223> Antibody hinge

<400> 520
Thr His Thr Cys Pro Pro Cys Pro
 1               5


<210> 521
<211> 9
<212> PRT
<213> Artificial Sequence

<220>
<223> Antibody hinge

<400> 521
Gly Thr His Thr Cys Pro Pro Cys Pro
 1               5


<210> 522
<211> 18
<212> PRT
<213> Artificial Sequence

<220>
<223> Unnatural junction

<400> 522
Thr Phe Gly Gln Gly Thr Lys Val Glu Ile Lys Ala Ser Thr Lys Gly
 1               5                   10                  15
Pro Ser


<210> 523
<211> 19
<212> PRT
<213> Artificial Sequence

<220>
<223> Unnatural junction

<400> 523
Thr Phe Gly Gln Gly Thr Lys Val Glu Ile Lys Arg Ala Ser Thr Lys
 1               5                   10                  15
Gly Pro Ser


<210> 524
```

<211> 19
<212> PRT
<213> Artificial Sequence

<220>
<223> Natural junction

<400> 524
Thr Phe Gly Gln Gly Thr Leu Val Thr Val Ser Ser Ala Ser Thr Lys
1               5                   10                  15
Gly Pro Ser


<210> 525
<211> 20
<212> PRT
<213> Artificial Sequence

<220>
<223> Unnatural junction

<400> 525
Phe Asp Tyr Trp Gly Gln Gly Thr Leu Val Thr Val Ser Ser Thr Val
1               5                   10                  15
Ala Ala Pro Ser
            20


<210> 526
<211> 21
<212> PRT
<213> Artificial Sequence

<220>
<223> Unnatural junction

<400> 526
Phe Asp Tyr Trp Gly Gln Gly Thr Leu Val Thr Val Ser Ser Arg Thr
1               5                   10                  15
Val Ala Ala Pro Ser
                20


<210> 527
<211> 20
<212> PRT
<213> Artificial Sequence

<220>
<223> Natural junction

<400> 527
Phe Asp Tyr Trp Gly Gln Gly Thr Lys Val Glu Ile Lys Arg Thr Val
1               5                   10                  15
Ala Ala Pro Ser
                20

```
<210> 528
<211> 12
<212> PRT
<213> Artificial Sequence

<220>
<223> Linker

<400> 528
Lys Val Glu Ile Lys Arg Thr Val Ala Ala Pro Ser
 1               5                   10


<210> 529
<211> 9
<212> PRT
<213> Artificial Sequence

<220>
<223> Linker


<400> 529
Leu Val Thr Val Ser Ser Ala Ser Thr
 1               5


<210> 530
<211> 15
<212> PRT
<213> Artificial Sequence

<220>
<223> Linker

<400> 530
Leu Val Thr Val Ser Ser Gly Gly Gly Gly Ser Gly Gly Gly Ser
 1               5                   10                  15


<210> 531
<211> 16
<212> PRT
<213> Artificial Sequence

<220>
<223> Linker

<400> 531
Gly Gln Gly Thr Lys Val Glu Ile Lys Arg Thr Val Ala Ala Pro Ser
 1               5                   10                  15


<210> 532
<211> 16
<212> PRT
<213> Artificial Sequence
```

```
<220>
<223> Linker

<400> 532
Gly Gln Gly Thr Asn Val Glu Ile Asn Arg Thr Val Ala Ala Pro Ser
 1               5                  10                  15


<210> 533
<211> 16
<212> PRT
<213> Artificial Sequence

<220>
<223> Linker

<400> 533
Gly Gln Gly Thr Asn Val Glu Ile Asn Gln Thr Val Ala Ala Pro Ser
 1               5                  10                  15


<210> 534
<211> 16
<212> PRT
<213> Artificial Sequence

<220>
<223> Linker

<400> 534
Gly Gln Gly Thr Asn Val Glu Ile Gln Arg Thr Val Ala Ala Pro Ser
 1               5                  10                  15


<210> 535
<211> 16
<212> PRT
<213> Artificial Sequence

<220>
<223> Linker

<400> 535
Gly Gln Gly Thr Leu Val Thr Val Ser Ser Thr Val Ala Ala Pro Ser
 1               5                  10                  15


<210> 536
<211> 6
<212> PRT
<213> Artificial Sequence

<220>
<223> Linker

<400> 536
Thr Val Ala Ala Pro Ser
```

1                    5

<210> 537
<211> 6
<212> PRT
<213> Artificial Sequence

<220>
<223> Linker

<400> 537
Ser Ala Lys Thr Thr Pro
 1               5

<210> 538
<211> 10
<212> PRT
<213> Artificial Sequence

<220>
<223> Linker

<400> 538
Ser Ala Lys Thr Thr Pro Lys Leu Gly Gly
 1               5                   10

<210> 539
<211> 17
<212> PRT
<213> Artificial Sequence

<220>
<223> Linker

<400> 539
Ala Lys Thr Thr Pro Lys Leu Glu Glu Gly Glu Phe Ser Glu Ala Arg
 1               5                   10                  15
Val

<210> 540
<211> 9
<212> PRT
<213> Artificial Sequence

<220>
<223> Linker

<400> 540
Ala Lys Thr Thr Pro Lys Leu Glu Glu
 1               5

<210> 541

```
<211> 6
<212> PRT
<213> Homo sapiens

<400> 541
Lys Val Glu Ile Lys Arg
 1               5


<210> 542
<211> 12
<212> PRT
<213> Artificial Sequence

<220>
<223> Linker

<400> 542
Leu Val Thr Val Ser Ser Ala Ser Thr Gly Pro Ser
 1               5                   10
```